# EUROPEAN PATENT APPLICATION

(11) **EP 1 741 781 A2**
(43) Date of publication of application: **10.01.2007**
(21) Application number: 06075998.2
(22) Date of filing: 18.02.2003
(51) Int. Cl.: C12N 15/11, C12P 19/34, C07H 21/02, C07H 21/04, A01N 43/04, A61K 31/713

(54) **RNA interference mediated treatment of Alzheimer's disease using short interfering nucleic acid (siNA)**

(30) Priority: 20.02.2002 US 358580 P; 11.03.2002 US 363124 P; 06.06.2002 US 386782 P; 25.07.2002 US 205309; 29.08.2002 US 406784 P; 05.09.2002 US 408378 P; 09.09.2002 US 409293 P; 15.01.2003 US 440129 P
(62) Divisional of application: 03709133.7
(71) Applicant: Sirna Therapeutics, Inc., Boulder, CO 80301 (US)
(72) Inventor: McSwiggen, James, Boulder, CO 80301 (US); Beigelman, Leonid, Brisbane, CA 94005 (US)
(74) Representative: Maschio, Antonio

(57) **Abstract**

The present invention concerns methods and reagents useful in modulating BACE gene expression in a variety of applications, including use in therapeutic, diagnostic, target validation, and genomic discovery applications. Specifically, the invention relates to small nucleic acid molecules, such as short interfering nucleic acid (siNA), short interfering RNA (siRNA), double-stranded RNA (dsRNA), micro-RNA (miRNA), and short hairpin RNA (shRNA) molecules capable of mediating RNA interference (RNAi) against beta-secretase (BACE), amyloid precurson protein (APP), pin-1, presenillin 1 (PS-1) and/or presenillin 2 (PS-2) gene expression and/or activity. The small nucleic acid molecules are useful in the treatment of Alzheimer's disease and any other condition that responds to modulation of BACE, APP, pin-1, PS-1 and/or PS-2 expression or activity.

## Description

This invention claims the benefit of McSwiggen USSN 10/205,309, filed July 25, 2002, of Beigelman USSN 60/358,580, filed February 20, 2002, of Beigelman USSN 60/363,124, filed March 11, 2002, of Beigelman USSN 60/386,782, filed June 6, 2002, of Beigelman USSN 60/406,784, filed August 29,2002, of Beigelman USSN 60/408,378, filed September 5, 2002, of Beigelman USSN 60/409,293, filed September 9, 2002, and of Beigelman USSN 60/440,129, filed January 15, 2003. These applications are hereby incorporated by reference herein in their entireties, including the drawings.

### Field Of The Invention

The present invention concerns methods and reagents useful in modulating gene expression associated with Alzheimer's disease in a variety of applications, including use in therapeutic, diagnostic, target validation, and genomic discovery applications. The present invention concerns compounds, compositions, and methods for the study, diagnosis, and treatment of conditions and diseases that respond to the modulation of beta-secretase (BACE), amyloid precurson protein (APP), pin-1, presenillin 1 (PS-1) and/or presenillin 2 (PS-2) gene expression and/or activity. The present invention also concerns compounds, compositions, and methods relating to conditions and diseases that respond to the modulation of expression and/or activity of genes involved in beta-secretase (BACE), amyloid precurson protein (APP), pin-1, presenillin 1 (PS-1) and/or presenillin 2 (PS-2) pathways. Specifically, the invention relates to small nucleic acid molecules, such as short interfering nucleic acid (siNA), short interfering RNA (siRNA), double-stranded RNA (dsRNA), micro-RNA (miRNA), and short hairpin RNA (shRNA) molecules capable of mediating RNA interference (RNAi) against beta-secretase (BACE), amyloid precurson protein (APP), pin-1, presenillin 1 (PS-1) and/or presenillin 2 (PS-2) gene expression.

### Background Of The Invention

The following is a discussion of relevant art pertaining to RNAi. The discussion is provided only for understanding of the invention that follows. The summary is not an admission that any of the work described below is prior art to the claimed invention.

RNA interference refers to the process of sequence-specific post-transcriptional gene silencing in animals mediated by short interfering RNAs (siRNAs) (Fire et al., 1998, Nature, 391, 806). The corresponding process in plants is commonly referred to as post-transcriptional gene silencing or RNA silencing and is also referred to as quelling in fungi. The process of post-transcriptional gene silencing is thought to be an evolutionarily-conserved cellular defense mechanism used to prevent the expression of foreign genes and is commonly shared by diverse flora and phyla (Fire et al., 1999, Trends Genet., 15, 358). Such protection from foreign gene expression may have evolved in response to the production of double-stranded RNAs (dsRNAs) derived from viral infection or from the random integration of transposon elements into a host genome via a cellular response that specifically destroys homologous single-stranded RNA or viral genomic RNA. The presence of dsRNA in cells triggers the RNAi response though a mechanism that has yet to be fully characterized. This mechanism appears to be different from the interferon response that results from dsRNA-mediated activation of protein kinase PKR and 2',5'-oligoadenylate synthetase resulting in non-specific cleavage of mRNA by ribonuclease L.

The presence of long dsRNAs in cells stimulates the activity of a ribonuclease III enzyme referred to as dicer. Dicer is involved in the processing of the dsRNA into short pieces of dsRNA known as short interfering RNAs (siRNAs) (Berstein et al., 2001, Nature, 409, 363). Short interfering RNAs derived from dicer activity are typically about 21 to about 23 nucleotides in length and comprise about 19 base pair duplexes (Elbashir et al., 2001, Genes Dev., 15, 188). Dicer has also been implicated in the excision of 21- and 22-nucleotide small temporal RNAs (stRNAs) from precursor RNA of conserved structure that are implicated in translational control (Hutvagner et al., 2001, Science, 293, 834). The RNAi response also features an endonuclease complex, commonly referred to as an RNA-induced silencing complex (RISC), which mediates cleavage of single-stranded RNA having sequence complementary to the antisense strand of the siRNA duplex. Cleavage of the target RNA takes place in the middle of the region complementary to the antisense strand of the siRNA duplex (Elbashir et al., 2001, Genes Dev., 15, 188).

RNAi has been studied in a variety of systems. Fire et al., 1998, Nature, 391, 806, were the first to observe RNAi in *C*. *elegans.* Wianny and Goetz, 1999, Nature Cell Biol., 2, 70, describe RNAi mediated by dsRNA in mouse embryos. Hammond et al., 2000, Nature, 404, 293, describe RNAi in *Drosophila* cells transfected with dsRNA. Elbashir et al., 2001, Nature, 411, 494, describe RNAi induced by introduction of duplexes of synthetic 21-nucleotide RNAs in cultured mammalian cells including human embryonic kidney and HeLa cells. Recent work in Drosophila embryonic lysates (Elbashir et al., 2001, EMBO J., 20, 6877) has revealed certain requirements for siRNA length, structure, chemical composition, and sequence that are essential to mediate efficient RNAi activity. These studies have shown that 21-nucleotide siRNA duplexes are most active when containing 3'-terminal dinucleotide overhangs. Furthemore, complete substitution of one or both siRNA strands with 2'-deoxy (2'-H) or 2'-O-methyl nucleotides abolishes RNAi activity, whereas substitution of the 3'-terminal siRNA overhang nucleotides with 2'-deoxy nucleotides (2'-H) was shown to be tolerated. Single mismatch sequences in the center of the siRNA duplex were also shown to abolish RNAi activity. In addition, these studies also indicate that the position of the cleavage site in the target RNA is defined by the 5'-end of the siRNA guide sequence rather than the 3'-end of the guide sequence (Elbashir et al., 2001, EMBO J., 20, 6877). Other studies have indicated that a 5'-phosphate on the target-complementary strand of a siRNA duplex is required for siRNA activity and that ATP is utilized to maintain the 5'-phosphate moiety on the siRNA (Nykanen et al., 2001, Cell, 107, 309).

Studies have shown that replacing the 3'-terminal nucleotide overhanging segments of a 21-mer siRNA duplex having two-nucleotide 3'-overhangs with deoxyribonucleotides does not have an adverse effect on RNAi activity. Replacing up to four nucleotides on each end of the siRNA with deoxyribonucleotides has been reported to be well-tolerated, whereas complete substitution with deoxyribonucleotides results in no RNAi activity (Elbashir et al., 2001, EMBO J., 20, 6877). In addition, Elbashir *et al., supra,* also report that substitution of siRNA with 2'-O-methyl nucleotides completely abolishes RNAi activity. Li et al., International PCT Publication No. WO 00/44914, and Beach *et al.,* International PCT Publication No. WO 01/68836 preliminarily suggest that siRNA may include modifications to either the phosphate-sugar backbone or the nucleoside to include at least one of a nitrogen or sulfur heteroatom, however, neither application postulates to what extent such modifications would be tolerated in siRNA molecules, nor provides any further guidance or examples of such modified siRNA. Kreutzer et al., Canadian Patent Application No. 2,359,180, also describe certain chemical modifications for use in dsRNA constructs in order to counteract activation of double-stranded RNA-dependent protein kinase PKR, specifically 2'-amino or 2'-O-methyl nucleotides, and nucleotides containing a 2'-O or 4'-C methylene bridge. However, Kreutzer *et al.* similarly fails to provide examples or guidance as to what extent these modifications would be tolerated in siRNA molecules.

Parrish et al., 2000, Molecular Cell, 6, 1977-1087, tested certain chemical modifications targeting the unc-22 gene in *C*. *elegans* using long (>25 nt) siRNA transcripts. The authors describe the introduction of thiophosphate residues into these siRNA transcripts by incorporating thiophosphate nucleotide analogs with T7 and T3 RNA polymerase and observed that RNAs with two phosphorothioate modified bases also had substantial decreases in effectiveness as RNAi. Further, Parrish *et al.* reported that phosphorothioate modification of more than two residues greatly destabilized the RNAs *in vitro* such that interference activities could not be assayed. *Id*. at 1081. The authors also tested certain modifications at the 2'-position of the nucleotide sugar in the long siRNA transcripts and found that substituting deoxynucleotides for ribonucleotides produced a substantial decrease in interference activity, especially in the case of Uridine to Thymidine and/or Cytidine to deoxy-Cytidine substitutions. *Id.* In addition, the authors tested certain base modifications, including substituting, in sense and antisense strands of the siRNA, 4-thiouracil, 5-bromouracil, 5-iodouracil, and 3-(aminoallyl)uracil for uracil, and inosine for guanosine. Whereas 4-thiouracil and 5-bromouracil substitution appeared to be tolerated, Parrish reported that inosine produced a substantial decrease in interference activity when incorporated in either strand. Parrish also reported that incorporation of 5-iodouracil and 3-(aminoallyl)uracil in the antisense strand resulted in a substantial decrease in RNAi activity as well.

The use of longer dsRNA has been described. For example, Beach *et al.,* International PCT Publication No. WO 01/68836, describes specific methods for attenuating gene expression using endogenously-derived dsRNA. Tuschl *et al.,* International PCT Publication No. WO 01/75164, describe a *Drosophila in vitro* RNAi system and the use of specific siRNA molecules for certain functional genomic and certain therapeutic applications; although Tuschl, 2001, Chem. Biochem., 2, 239-245, doubts that RNAi can be used to cure genetic diseases or viral infection due to the danger of activating interferon response. Li et al., International PCT Publication No. WO 00/44914, describe the use of specific dsRNAs for attenuating the expression of certain target genes. Zemicka-Goetz et al., International PCT Publication No. WO 01/36646, describe certain methods for inhibiting the expression of particular genes in mammalian cells using certain dsRNA molecules. Fire et al., International PCT Publication No. WO 99/32619, describe particular methods for introducing certain dsRNA molecules into cells for use in inhibiting gene expression. Plaetinck et al., International PCT Publication No. WO 00/01846, describe certain methods for identifying specific genes responsible for conferring a particular phenotype in a cell using specific dsRNA molecules. Mello et al., International PCT Publication No. WO 01/29058, describe the identification of specific genes involved in dsRNA-mediated RNAi. Deschamps Depaillette et al., International PCT Publication No. WO 99/07409, describe specific compositions consisting of particular dsRNA molecules combined with certain anti-viral agents, Waterhouse et al., International PCT Publication No. 99/53050, describe certain methods for decreasing the phenotypic expression of a nucleic acid in plant cells using certain dsRNAs. Driscoll et al., International PCT Publication No. WO 01/49844, describe specific DNA constructs for use in facilitating gene silencing in targeted organisms.

Others have reported on various RNAi and gene-silencing systems. For example, Parrish et al., 2000, Molecular Cell., 6, 1977-1087, describe specific chemically-modified siRNA constructs targeting the une-22 gene of C. *elegans.* Grossniklaus, International PCT Publication No. WO 01/38551, describes certain methods for regulating polycomb gene expression in plants using certain dsRNAs. Churikov et al., International PCT Publication No. WO 01/42443, describe certain methods for modifying genetic characteristics of an organism using certain dsRNAs. Cogoni et al., International PCT Publication No. WO 01/53475, describe certain methods for isolating a Neurospora silencing gene and uses thereof. Reed et al., International PCT Publication No. WO O1/68536, describe certain methods for gene silencing in plants. Honer et al., International PCT Publication No. WO 01/70944, describe certain methods of drug screening using transgenic nematodes as Parkinson's Disease models using certain dsRNAs. Deak et al., International PCT Publication No. WO 01/72774, describe certain Drosophila-derived gene products that may be related to RNAi in *Drosophila.* Arndt et al., International PCT Publication No. WO 01/92513 describe certain methods for mediating gene suppression by using factors that enhance RNAi. Tuschl et al., International PCT Publication No. WO 02/44321, describe certain synthetic siRNA constructs. Pachuk et al., International PCT Publication No. WO 00/63364, and Satishchandran et al., International PCT Publication No. WO 01/04313, describe certain methods and compositions for inhibiting the function of certain polynucleotide sequences using certain dsRNAs. Echeverri et al., International PCT Publication No. WO 02/38805, describe certain C. *elegans* genes identified via RNAi. Kreutzer et al., International PCT Publications Nos. WO 02/055692, WO 02/055693, and EP 1144623 B1 describes certain methods for inhibiting gene expression using RNAi. Graham et al., International PCT Publications Nos. WO 99/49029 and WO 01/70949, and AU 4037501 describe certain vector expressed siRNA molecules. Fire et al., US 6,506,559, describe certain methods for inhibiting gene expression in vitro using certain siRNA constructs that mediate RNAi.

McSwiggen et al., International PCT Publication No. WO 01/16312, describes nucleic acid mediated inhibition of BACE, PS-1, and PS-2 expression.

### SUMMARY OF THE INVENTION

This invention relates to compounds, compositions, and methods useful for modulating BACE expression by RNA interference (RNAi) using small nucleic acid molecules, such as short interfering nucleic acid (siNA), short interfering RNA (siRNA), double-stranded RNA (dsRNA), micro-RNA (miRNA), and short hairpin RNA (shRNA) molecules. This invention also relates to compounds, compositions, and methods useful for modulating the expression and activity of BACE genes, or genes involved in BACE pathways of gene expression and/or BACE activity by RNA interference (RNAi) using small nucleic acid molecules, such as short interfering nucleic acid (siNA), short interfering RNA (siRNA), double-stranded RNA (dsRNA), micro-RNA (miRNA), and short hairpin RNA (shRNA) molecules. In particular, the instant invention features small nucleic acid molecules, such as short interfering nucleic acid (siNA), short interfering RNA (siRNA), double-stranded RNA (dsRNA), micro-RNA (miRNA), and short hairpin RNA (shRNA) molecules and methods used to modulate the expression of BACE genes. A siNA of the invention can be unmodified or chemically-modified. A siNA of the instant invention can be chemically synthesized, expressed from a vector or enzymatically synthesized. The instant invention also features various chemically-modified synthetic short interfering nucleic acid (siNA) molecules capable of modulating BACE gene expression or activity in cells by RNA interference (RNAi). The use of chemically-modified siNA improves various properties of native siNA molecules through increased resistance to nuclease degradation *in vivo* and/or through improved cellular uptake. Further, contrary to earlier published studies, siNA having multiple chemical modifications retains its RNAi activity. The siNA molecules of the instant invention provide useful reagents and methods for a variety of therapeutic, diagnostic, target validation, genomic discovery, genetic engineering, and pharmacogenomic applications.

In one embodiment, the invention features one or more siNA molecules and methods that independently or in combination modulate the expression of gene(s) encoding proteins, such as BACE proteins, associated with the maintenance and/or development of Alzheimer's disease and other neurodegenerative disorders or conditions such as dementia, and stroke/cardiovascular accident (CVA), such as genes encoding sequences comprising those sequences referred to by GenBank Accession Nos. shown in Table I, referred to herein generally as BACE. The description below of the various aspects and embodiments is provided with reference to the exemplary BACE gene and BACE protein, including components or subunits thereof. However, the various aspects and embodiments are also directed to other genes which express other BACE related proteins or other proteins associated with Alheimer's disease, such as APP, PIN-1, PS-1 and PS-2, including mutant genes and splice variant genes thereof. The various aspects and embodiments are also directed to other genes that are involved in BACE, APP, PIN-1, PS-1 and PS-2 mediated pathways of signal transduction or gene expression that are involved in the progression, development, or maintenance of disease (e.g., Alzheimer's disease). Those additional genes can be analyzed for target sites using the methods described for BACEs herein. Thus, the inhibition and the effects of such inhibition of the other genes can be performed as described herein.

In one embodiment, the invention features a siNA molecule that down-regulates expression of a BACE gene, for example, wherein the BACE gene comprises BACE encoding sequence.

In one embodiment, the invention features a siNA molecule having RNAi activity against BACE RNA, wherein the siNA molecule comprises a sequence complementary to any RNA having BACE or other BACE encoding sequence, such as those sequences having GenBank Accession Nos. shown in **Table I**. Chemical modifications as shown in **Tables III and IV** or otherwise described herein can be applied to any siNA construct of the invention. Furthermore, the chemically modified constructs described in **Table IV** can be applied to any siNA sequence of the invention.

In another embodiment, the invention features a siNA molecule having RNAi activity against a BACE gene, wherein the siNA molecule comprises nucleotide sequence complementary to nucleotide sequence of a BACE gene, such as those BACE sequences having GenBank Accession Nos. shown in **Table I**. In another embodiment, a siNA molecule of the invention includes nucleotide sequence that can interact with nucleotide sequence of a BACE gene and thereby mediate silencing of BACE gene expression, for example, wherein the siNA mediates regulation of BACE gene expression by cellular processes that modulate the chromatin structure of the BACE gene and prevent transcription of the BACE gene.

In another embodiment, the invention features a siNA molecule comprising nucleotide sequence, for example, nucleotide sequence in the antisense region of the siNA molecule that is complementary to a nucleotide sequence or portion of sequence of a BACE gene. In another embodiment, the invention features a siNA molecule comprising a region, for example, the antisense region of the siNA construct, complementary to a sequence or portion of sequence comprising a BACE gene sequence.

In one embodiment, the antisense region of BACE siNA constructs can comprise a sequence complementary to sequence having any of SEQ ID NOs. 1-325 or 651-654. The antisense region can also comprise sequence having any of SEQ ID NOs. 326-650, 659-662, 667-670, 675-678, 695, 697, 699, 701, 703, or 704. In another embodiment, the sense region of BACE constructs can comprise sequence having any of SEQ ID NOs. 1-325, 651-658, 663-666, 671-674, 694, 696, 698, 700, or 702. The sense region can comprise a sequence of SEQ ID NO. 683 and the antisense region can comprise a sequence of SEQ ID NO. 684. The sense region can comprise a sequence of SEQ ID NO. 685 and the antisense region can comprise a sequence of SEQ ID NO. 686. The sense region can comprise a sequence of SEQ ID NO. 687 and the antisense region can comprise a sequence of SEQ ID NO. 688. The sense region can comprise a sequence of SEQ ID NO. 689 and the antisense region can comprise a sequence of SEQ ID NO. 690. The sense region can comprise a sequence of SEQ ID NO. 691 and the antisense region can comprise a sequence of SEQ ID NO. 692. The sense region can comprise a sequence of SEQ ID NO. 689 and the antisense region can comprise a sequence of SEQ ID NO. 693.

In one embodiment, a siNA molecule of the invention comprises any of SEQ ID NOs. 1-704. The sequences shown in SEQ ID NOs: 1-704 are not limiting. A siNA molecule of the invention can comprise any contiguous BACE sequence (e.g., about 19 to about 25, or about 19, 20, 21, 22, 23, 24 or 25 contiguous BACE nucleotides).

In yet another embodiment, the invention features a siNA molecule comprising a sequence, for example, the antisense sequence of the siNA construct, complementary to a sequence or portion of sequence comprising sequence represented by GenBank Accession Nos. shown in **Table I**. Chemical modifications in **Tables III and IV** and descrbed herein can be applied to any siRNA costruct of the invention. Furthermore, the chemically modified constructs described in **Table IV** can be applied to any siNA sequence of the invention.

In one embodiment of the invention a siNA molecule comprises an antisense strand having about 19 to about 29 nucleotides, wherein the antisense strand is complementary to a RNA sequence encoding a BACE protein, and wherein said siNA further comprises a sense strand having about 19 to about 29 (e.g., about 19, 20, 21, 22, 23, 24, 25, 26, 27, 28 or 29) nucleotides, and wherein said sense strand and said antisense strand are distinct nucleotide sequences with at least about 19 complementary nucleotides.

In another embodiment of the invention a siNA molecule of the invention comprises an antisense region having about 19 to about 29 (e.g., about 19, 20, 21, 22, 23, 24, 25, 26, 27, 28 or 29) nucleotides, wherein the antisense region is complementary to a RNA sequence encoding a BACE protein, and wherein said siNA further comprises a sense region having about 19 to about 29 nucleotides, wherein said sense region and said antisense region comprise a linear molecule with at least about 19 complementary nucleotides.

In one embodiment of the invention a siNA molecule comprises an antisense strand comprising a nucleotide sequence that is complementary to a nucleotide sequence or a portion thereof encoding a BACE protein. The siNA further comprises a sense strand, wherein said sense strand comprises a nucleotide sequence of a BACE gene or a portion thereof.

In another embodiment, a siNA molecule comprises an antisense region comprising a nucleotide sequence that is complementary to a nucleotide sequence or a portion thereof encoding a BACE protein. The siNA molecule further comprises a sense region, wherein said sense region comprises a nucleotide sequence of a BACE gene or a portion thereof.

In one embodiment, a siNA molecule of the invention has RNAi activity that modulates expression of RNA encoded by a BACE gene. Because BACE genes can share some degree of sequence homology with each other, siNA molecules can be designed to target a class of BACE genes (and associated receptor or ligand genes) or alternately specific BACE genes by selecting sequences that are either shared amongst different BACE targets or alternatively that are unique for a specific BACE target. Therefore, in one embodiment, the siNA molecule can be designed to target conserved regions of BACE RNA sequence having homology between several BACE genes so as to target several BACE genes (e.g., different BACE isoforms, splice variants, mutant genes etc.) with one siNA molecule. In another embodiment, the siNA molecule can be designed to target a sequence that is unique to a specific BACE RNA sequence due to the high degree of specificity that the siNA molecule requires to mediate RNAi activity.

In one embodiment, nucleic acid molecules of the invention that act as mediators of the RNA interference gene silencing response are double-stranded nucleic acid molecules. In another embodiment, the siNA molecules of the invention consist of duplexes containing about 19 base pairs between oligonucleotides comprising about 19 to about 25 (*e.g*., about 19, 20, 21, 22, 23, 24 or 25) nucleotides. In yet another embodiment, siNA molecules of the invention comprise duplexes with overhanging ends of about about 1 to about 3 (*e.g*., about 1, 2, or 3) nucleotides, for example, about 21-nucleotide duplexes with about 19 base pairs and 3'-temiinal mononucleotide, dinucleotide, or trinucleotide overhangs.

In one embodiment, the invention features one or more chemically-modified siNA constructs having specificity for BACE expressing nucleic acid molecules, such as RNA encoding a BACE protein. Non-limiting examples of such chemical modifications include without limitation phosphorothioate internucleotide linkages, 2'-deoxyribonucleotides, 2'-O-methyl ribonucleotides, 2'-deoxy-2'-fluoro ribonucleotides, "universal base" nucleotides, "acyclic" nucleotides, 5-C-methyl nucleotides, and terminal glyceryl and/or inverted deoxy abasic residue incorporation. These chemical modifications, when used in various siNA constructs, are shown to preserve RNAi activity in cells while at the same time, dramatically increasing the serum stability of these compounds. Furthermore, contrary to the data published by Parrish *et al., supra,* applicant demonstrates that multiple (greater than one) phosphorothioate substitutions are well-tolerated and confer substantial increases in serum stability for modified siNA constructs.

In one embodiment, a siNA molecule of the invention comprises modified nucleotides while maintaining the ability to mediate RNAi. The modified nucleotides can be used to improve in *vitro* or in *vivo* characteristics such as stability, activity, and/or bioavailability. For example, a siNA molecule of the invention can comprise modified nucleotides as a percentage of the total number of nucleotides present in the siNA molecule. As such, a siNA molecule of the invention can generally comprise about 5% to about 100% modified nucleotides (e.g., about 5%, 10%, 15%, 20%, 25%, 30%, 35%, 40%, 45%, 50%, 55%, 60%, 65%, 70%, 75%, 80%, 85%, 90%, 95% or 100% modified nucleotides). The actual percentage of modified nucleotides present in a given siNA molecule will depend on the total number of nucleotides present in the siNA. If the siNA molecule is single stranded, the percent modification can be based upon the total number of nucleotides present in the single stranded siNA molecules. Likewise, if the siNA molecule is double stranded, the percent modification can be based upon the total number of nucleotides present in the sense strand, antisense strand, or both the sense and antisense strands.

In a non-limiting example, the introduction of chemically-modified nucleotides into nucleic acid molecules provides a powerful tool in overcoming potential limitations of *in vivo* stability and bioavailability inherent to native RNA molecules that are delivered exogenously. For example, the use of chemically-modified nucleic acid molecules can enable a lower dose of a particular nucleic acid molecule for a given therapeutic effect since chemically-modified nucleic acid molecules tend to have a longer half-life in serum. Furthermore, certain chemical modifications can improve the bioavailability of nucleic acid molecules by targeting particular cells or tissues and/or improving cellular uptake of the nucleic acid molecule. Therefore, even if the activity of a chemically-modified nucleic acid molecule is reduced as compared to a native nucleic acid molecule, for example, when compared to an all-RNA nucleic acid molecule, the overall activity of the modified nucleic acid molecule can be greater than that of the native molecule due to improved stability and/or delivery of the molecule. Unlike native unmodified siNA, chemically-modified siNA can also minimize the possibility of activating interferon activity in humans.

The antisense region of a siNA molecule of the invention can comprise a phosphorothioate internucleotide linkage at the 3'-end of said antisense region. The antisense region can comprise about one to about five phosphorothioate internucleotide linkages at the 5'-end of said antisense region. The 3'-terminal nucleotide overhangs of a siNA molecule of the invention can comprise ribonucleotides or deoxyribonucleotides that are chemically-modified at a nucleic acid sugar, base, or backbone. The 3'-terminal nucleotide overhangs can comprise one or more universal base ribonucleotides. The 3'-terminal nucleotide overhangs can comprise one or more acyclic nucleotides.

One embodiment of the invention provides an expression vector comprising a nucleic acid sequence encoding at least one siNA molecule of the invention in a manner that allows expression of the nucleic acid molecule. Another embodiment of the invention provides a mammalian cell comprising such an expression vector. The mammalian cell can be a human cell. The siNA molecule of the expression vector can comprise a sense region and an antisense region. The antisense region can comprise sequence complementary to a RNA or DNA sequence encoding BACE and the sense region can comprise sequence complementary to the antisense region. The siNA molecule can comprise two distinct strands having complementary sense and antisense regions. The siNA molecule can comprise a single strand having complementary sense and antisense regions.

In one embodiment, the invention features a chemically-modified short interfering nucleic acid (siNA) molecule capable of mediating RNA interference (RNAi) against a BACE inside a cell or reconstituted *in vitro* system, wherein the chemical modification comprises one or more (*e.g*., about 1, 2, 3, 4, 5, 6, 7, 8, 9, 10, or more) nucleotides comprising a backbone modified internucleotide linkage having Formula I: 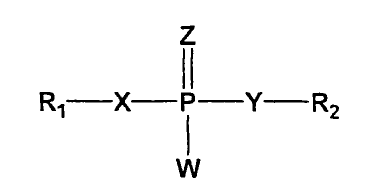 wherein each R1 and R2 is independently any nucleotide, non-nucleotide, or polynucleotide which can be naturally-occurring or chemically-modified, each X and Y is independently O, S, N, alkyl, or substituted alkyl, each Z and W is independently O, S, N, alkyl, substituted alkyl, O-alkyl, S-alkyl, alkaryl, or aralkyl, and wherein W, X, Y, and Z are optionally not all O.

The chemically-modified internucleotide linkages having Formula I, for example, wherein any Z, W, X, and/or Y independently comprises a sulfur atom, can be present in one or both oligonucleotide strands of the siNA duplex, for example, in the sense strand, the antisense strand, or both strands. The siNA molecules of the invention can comprise one or more (*e.g*., about 1, 2, 3, 4, 5, 6, 7, 8, 9, 10, or more) chemically-modified internucleotide linkages having Formula I at the 3'-end, the 5'-end, or both of the 3' and 5'-ends of the sense strand, the antisense strand, or both strands. For example, an exemplary siNA molecule of the invention can comprise about 1 to about 5 or more (*e.g*., about 1, 2, 3, 4, 5, or more) chemically-modified internucleotide linkages having Formula I at the 5'-end of the sense strand, the antisense strand, or both strands. In another non-limiting example, an exemplary siNA molecule of the invention can comprise one or more (*e.g*., about 1, 2, 3, 4, 5, 6, 7, 8, 9, 10, or more) pyrimidine nucleotides with chemically-modified internucleotide linkages having Formula I in the sense strand, the antisense strand, or both strands. In yet another non-limiting example, an exemplary siNA molecule of the invention can comprise one or more (*e.g*., about 1, 2, 3, 4, 5, 6, 7, 8, 9, 10, or more) purine nucleotides with chemically-modified internucleotide linkages having Formula I in the sense strand, the antisense strand, or both strands. In another embodiment, a siNA molecule of the invention having internucleotide linkage(s) of Formula I also comprises a chemically-modified nucleotide or non-nucleotide having any of Formulae I-VII.

In one embodiment, the invention features a chemically-modified short interfering nucleic acid (siNA) molecule capable of mediating RNA interference (RNAi) against a BACE inside a cell or reconstituted *in vitro* system, wherein the chemical modification comprises one or more (*e.g*., about 1, 2, 3, 4, 5, 6, 7, 8, 9, 10, or more) nucleotides or non-nucleotides having Formula II: 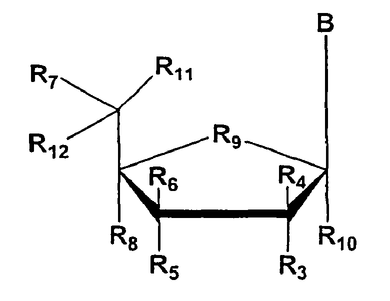 wherein each R3, R4, R5, R6, R7, R8, R10, R11 and R12 is independently H, OH, alkyl, substituted alkyl, alkaryl or aralkyl, F, Cl, Br, CN, CF3, OCF3, OCN, O-alkyl, S-alkyl, N-alkyl, O-alkenyl, S-alkenyl, N-alkenyl, SO-alkyl, alkyl-OSH, alkyl-OH, O-alkyl-OH, O-alkyl-SH, S-alkyl-OH, S-alkyl-SH, alkyl-S-alkyl, alkyl-O-alkyl, ONO2, NO2 N3, NH2, aminoalkyl, aminoacid, aminoacyl, ONH2, O-aminoalkyl, O-aminoacid, O-aminoacyl, heterocycloalkyl, heterocycloalkaryl, aminoalkylamino, polyalklylamino, substituted silyl, or group having Formula I; R9 is O, S, CH2, S=O, CHF, or CF2, and B is a nucleosidic base such as adenine, guanine, uracil, cytosine, thymine, 2-aminoadenosine, 5-methylcytosine, 2,6-diaminopurine, or any other non-naturally occurring base that can be complementary or non-complementary to target RNA or a non-nucleosidic base such as phenyl, naphthyl, 3-nitropyrrole, 5-nitroindole, nebularine, pyridone, pyridinone, or any other non-naturally occurring universal base that can be complementary or non-complementary to target RNA.

The chemically-modified nucleotide or non-nucleotide of Formula II can be present in one or both oligonucleotide strands of the siNA duplex, for example in the sense strand, the antisense strand, or both strands. The siNA molecules of the invention can comprise one or more chemically-modified nucleotide or non-nucleotide of Formula II at the 3'-end, the 5'-end, or both of the 3' and 5'-ends of the sense strand, the antisense strand, or both strands. For example, an exemplary siNA molecule of the invention can comprise about 1 to about 5 or more (*e.g*., about 1, 2, 3, 4, 5, or more) chemically-modified nucleotides or non-nucleotides of Formula II at the 5'-end of the sense strand, the antisense strand, or both strands. In anther non-limiting example, an exemplary siNA molecule of the invention can comprise about 1 to about 5 or more (*e.g*., about 1, 2, 3, 4, 5, or more) chemically-modified nucleotides or non-nucleotides of Formula II at the 3'-end of the sense strand, the antisense strand, or both strands.

In one embodiment, the invention features a chemically-modified short interfering nucleic acid (siNA) molecule capable of mediating RNA interference (RNAi) against a BACE inside a cell or reconstituted *in vitro* system, wherein the chemical modification comprises one or more (*e.g*., about 1, 2, 3, 4, 5, 6, 7, 8, 9, 10, or more) nucleotides or non-nucleotides having Formula III: 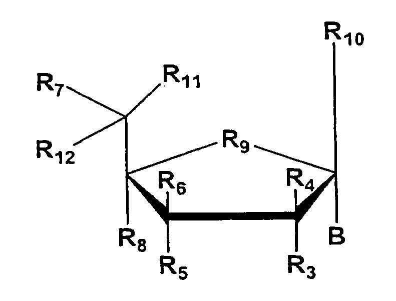 wherein each R3, R4, R5, R6, R7, R8, R10, R11 and R12 is independently H, OH, alkyl, substituted alkyl, alkaryl or aralkyl, F, Cl, Br, CN, CF3, OCF3, OCN, O-alkyl, S-alkyl, N-alkyl, O-alkenyl, S-alkenyl, N-alkenyl, SO-alkyl, alkyl-OSH, alkyl-OH, O-alkyl-OH, O-alkyl-SH, S-alkyl-OH, S-alkyl-SH, alkyl-S-alkyl, alkyl-O-alkyl, ONO2, NO2, N3, NH2, aminoalkyl, aminoacid, aminoacyl, ONH2, O-aminoalkyl, O-aminoacid, O-aminoacyl, heterocycloalkyl, heterocycloalkaryl, aminoalkylamino, polyalklylamino, substituted silyl, or group having Fonnula I; R9 is O, S, CH2, S=O, CHF, or CF2, and B is a nucleosidic base such as adenine, guanine, uracil, cytosine, thymine, 2-aminoadenosine, 5-methylcytosine, 2,6-diaminopurine, or any other non-naturally occurring base that can be employed to be complementary or non-complementary to target RNA or a non-nucleosidic base such as phenyl, naphthyl, 3-nitropyrrole, 5-nitroindole, nebularine, pyridone, pyridinone, or any other non-naturally occurring universal base that can be complementary or non-complementary to target RNA.

The chemically-modified nucleotide or non-nucleotide of Formula III can be present in one or both oligonucleotide strands of the siNA duplex, for example, in the sense strand, the antisense strand, or both strands. The siNA molecules of the invention can comprise one or more chemically-modified nucleotide or non-nucleotide of Formula III at the 3'-end, the 5'-end, or both of the 3' and 5'-ends of the sense strand, the antisense strand, or both strands. For example, an exemplary siNA molecule of the invention can comprise about 1 to about 5 or more (*e.g*., about 1, 2, 3, 4, 5, or more) chemically-modified nucleotide(s) or non-nucleotide(s) of Formula III at the 5'-end of the sense strand, the antisense strand, or both strands. In anther non-limiting example, an exemplary siNA molecule of the invention can comprise about 1 to about 5 or more (*e.g*., about . 1, 2, 3, 4, 5, or more) chemically-modified nucleotide or non-nucleotide of Fonnula III at the 3'-end of the sense strand, the antisense strand, or both strands.

hi another embodiment, a siNA molecule of the invention comprises a nucleotide having Formula II or III, wherein the nucleotide having Formula II or III is in an inverted configuration. For example, the nucleotide having Formula II or III is connected to the siNA construct in a 3'-3', 3'-2', 2'-3', or 5'-5' configuration, such as at the 3'-end, the 5'-end, or both of the 3' and 5'-ends of one or both siNA strands.

In one embodiment, the invention features a chemically-modified short interfering nucleic acid (siNA) molecule capable of mediating RNA interference (RNAi) against a BACE inside a cell or reconstituted *in vitro* system, wherein the chemical modification comprises a 5'-terminal phosphate group having Formula IV: 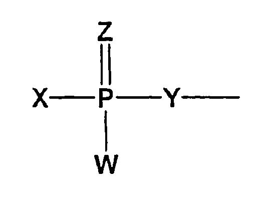 wherein each X and Y is independently O, S, N, alkyl, substituted alkyl, or alkylhalo; wherein each Z and W is independently O, S, N, alkyl, substituted alkyl, O-alkyl, S-alkyl, alkaryl, aralkyl, or alkylhalo; and wherein W, X, Y and Z are not all O.

In one embodiment, the invention features a siNA molecule having a 5'-terminal phosphate group having Formula IV on the target-complementary strand, for example, a strand complementary to a target RNA, wherein the siNA molecule comprises an all RNA siNA molecule. In another embodiment, the invention features a siNA molecule having a 5'-terminal phosphate group having Formula IV on the target-complementary strand wherein the siNA molecule also comprises about 1 to about 3 (*e.g*., about 1, 2, or 3) nucleotide 3'-terminal nucleotide overhangs having about 1 to about 4 (*e.g*., about 1, 2, 3, or 4) deoxyribonucleotides on the 3'-end of one or both strands. In another embodiment, a 5'-terminal phosphate group having Formula IV is present on the target-complementary strand of a siNA molecule of the invention, for example a siNA molecule having chemical modifications having any of Formulae I-VII.

In one embodiment, the invention features a chemically-modified short interfering nucleic acid (siNA) molecule capable of mediating RNA interference (RNAi) against a BACE inside a cell or reconstituted *in vitro* system, wherein the chemical modification comprises one or more phosphorothioate internucleotide linkages. For example, in a non-limiting example, the invention features a chemically-modified short interfering nucleic acid (siNA) having about 1, 2, 3, 4, 5, 6, 7, 8 or more phosphorothioate internucleotide linkages in one siNA strand. In yet another embodiment, the invention features a chemically-modified short interfering nucleic acid (siNA) individually having about 1, 2, 3, 4, 5, G, 7, 8 or more phosphorothioate internucleotide linkages in both siNA strands. The phosphorothioate internucleotide linkages can be present in one or both oligonucleotide strands of the siNA duplex, for example in the sense strand, the antisense strand, or both strands. The siNA molecules of the invention can comprise one or more phosphorothioate internucleotide linkages at the 3'-end, the 5'-end, or both of the 3'- and 5'-ends of the sense strand, the antisense strand, or both strands. For example, an exemplary siNA molecule of the invention can comprise about 1 to about 5 or more (*e.g.,* about 1, 2, 3, 4, 5, or more) consecutive phosphorothioate internucleotide linkages at the 5'-end of the sense strand, the antisense strand, or both strands. In another non-limiting example, an exemplary siNA molecule of the invention can comprise one or more (*e.g*., about 1, 2, 3, 4, 5, 6, 7, 8, 9, 10, or more) pyrimidine phosphorothioate internucleotide linkages in the sense strand, the antisense strand, or both strands. In yet another non-limiting example, an exemplary siNA molecule of the invention can comprise one or more (*e.g*., about 1, 2, 3, 4, 5, 6, 7, S, 9, 10, or more) purine phosphorothioate internucleotide linkages in the sense strand, the antisense strand, or both strands.

In one embodiment, the invention features a siNA molecule, wherein the sense strand comprises one or more, for example, about 1, 2, 3, 4, 5, 6, 7, 8, 9, 10, or more phosphorothioate internucleotide linkages, and/or one or more (e.g., about 1, 2, 3, 4, 5, 6, 7, 8, 9, 10 or more) 2'-deoxy, 2'-O-methyl, 2'-deoxy-2'-fluoro, and/or about one or more (*e.g*., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the sense strand; and wherein the antisense strand comprises about 1 to about 10 or more, specifically about 1, 2, 3, 4, 5, 6, 7, 8, 9, 10, or more phosphorothioate internucleotide linkages, and/or one or more (*e.g*., about 1, 2, 3, 4, 5, 6, 7, 8, 9, 10 or more) 2'-deoxy, 2'-O-methyl, 2'-deoxy-2'-fluoro, and/or one or more (*e.g*., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In another embodiment, one or more, for example about 1, 2, 3, 4, 5, 6, 7, 8, 9, 10, or more, pyrimidine nucleotides of the sense and/or antisense siNA strand are chemically-modified with 2'-deoxy, 2'-O-methyl and/or 2'-deoxy-2'-fluoro nucleotides, with or without one or more, for example about 1, 2, 3, 4, 5, 6, 7, 8, 9, 10, or more, phosphorothioate internucleotide linkages and/or a terminal cap molecule at the 3'-end, the 5'-end, or both of the 3'- and 5'-ends, being present in the same or different strand.

In another embodiment, the invention features a siNA molecule, wherein the sense strand comprises about 1 to about 5, specifically about 1, 2, 3, 4, or 5 phosphorothioate internucleotide linkages, and/or one or more (*e.g*., about 1, 2, 3, 4, 5, or more) 2'-deoxy, 2'-O-methyl, 2'-deoxy-2'-fluoro, and/or one or more (*e.g*., about 1, 2, 3, 4, 5, or more) universal base modified nucleotides, and optionally a terminal cap molecule at the 3-end, the 5'-end, or both of the 3'- and 5'-ends of the sense strand; and wherein the antisense strand comprises about 1 to about 5 or more, specifically about 1, 2, 3, 4, 5, or more phosphorothioate internucleotide linkages, and/or one or more (*e.g*., about 1, 2, 3, 4, 5, 6, 7, 8, 9, 10 or more) 2'-deoxy, 2'-O-methyl, 2'-deoxy-2'-fluoro, and/or one or more (*e.g*., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In another embodiment, one or more, for example about 1, 2, 3, 4, 5, 6, 7, 8, 9, 10, or more, pyrimidine nucleotides of the sense and/or antisense siNA strand are chemically-modified with 2'-deoxy, 2'-O-methyl and/or 2'-deoxy-2'-fluoro nucleotides, with or without about 1 to about 5 or more, for example about 1, 2, 3, 4, 5, or more phosphorothioate internucleotide linkages and/or a terminal cap molecule at the 3'-end, the 5'-end, or both of the 3'- and 5'-ends, being present in the same or different strand.

In one embodiment, the invention features a siNA molecule, wherein the antisense strand comprises one or more, for example, about 1, 2, 3, 4, 5, 6, 7, 8, 9, 10, or more phosphorothioate internucleotide linkages, and/or about one or more (*e.g*., about 1, 2, 3, 4, 5, 6, 7, 8, 9, 10 or more) 2'-deoxy, 2'-O-methyl, 2'-deoxy-2'-fluoro, and/or one or more (e.g., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the sense strand; and wherein the antisense strand comprises about 1 to about 10 or more, specifically about 1, 2, 3, 4, 5, 6, 7, 8, 9, 10 or more phosphorothioate internucleotide linkages, and/or one or more (*e.g*., about 1, 2, 3, 4, 5, 6, 7, 8, 9, 10 or more) 2'-deoxy, 2'-O-methyl, 2'-deoxy-2'-fluoro, and/or one or more (e.g., about 1, 3, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In another embodiment, one or more, for example about 1, 2, 3, 4, 5, 6, 7, 8, 9, 10 or more pyrimidine nucleotides of the sense and/or antisense siNA strand are chemically-modified with 2'-deoxy, 2'-O-methyl and/or 2'-deoxy-2'-fluoro nucleotides, with or without one or more, for example, about 1, 2, 3, 4, 5, 6, 7, 8, 9, 10 or more phosphorothioate internucleotide linkages and/or a terminal cap molecule at the 3'-end, the 5'-end, or both of the 3' and 5'-ends, being present in the same or different strand.

In another embodiment, the invention features a siNA molecule, wherein the antisense strand comprises about 1 to about 5 or more, specifically about 1,2, 3, 4, 5 or more phosphorothioate internucleotide linkages, and/or one or more (*e.g*., about 1, 2, 3, 4, 5, 6, 7, 8, 9, 10 or more) 2'-deoxy, 2'-O-methyl, 2'-deoxy-2'-fluoro, and/or one or more (*e.g*., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the sense strand; and wherein the antisense strand comprises about 1 to about 5 or more, specifically about 1, 2, 3, 4, 5 or more phosphorothioate internucleotide linkages, and/or one or more (*e.g*., about 1, 2, 3, 4, 5, 6, 7, 8, 9, 10 or more) 2'-deoxy, 2'-0-methyl, 2'-deoxy-2'-fluoro, and/or one or more (*e.g*., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In another embodiment, one or more, for example about 1, 2, 3, 4, 5, 6, 7, 8, 9, 10 or more pyrimidine nucleotides of the sense and/or antisense siNA strand are chemically-modified with 2'-deoxy, 2'-O-methyl and/or 2'-deoxy-2'-fluoro nucleotides, with or without about 1 to about 5 or more, for example about 1, 2, 3, 4, 5 or more phosphorothioate internucleotide linkages and/or a terminal cap molecule at the 3'-end, the 5'-end, or both of the 3'- and 5'-ends, being present in the same or different strand.

In one embodiment, the invention features a chemically-modified short interfering nucleic acid (siNA) molecule having about 1 to about 5 or more, specifically about 1, 2, 3, 4, 5 or more phosphorothioate internucleotide linkages in each strand of the siNA molecule.

In another embodiment, the invention features a siNA molecule comprising 2'-5' internucleotide linkages. The 2'-5' internucleotide linkage(s) can be at the 3'-end, the 5'-end, or both of the 3'- and 5'-ends of one or both siNA sequence strands. In addition, the 2'-5' internucleotide linkage(s) can be present at various other positions within one or both siNA sequence strands, for example, about 1, 2, 3, 4, 5, 6, 7, 8, 9, 10, or more including every internucleotide linkage of a pyrimidine nucleotide in one or both strands of the siNA molecule can comprise a 2'-5' internucleotide linkage, or about 1, 2, 3, 4, 5, 6, 7, 8, 9, 10, or more including every internucleotide linkage of a purine nucleotide in one or both strands of the siNA molecule can comprise a 2'-5' internucleotide linkage.

In another embodiment, a chemically-modified siNA molecule of the invention comprises a duplex having two strands, one or both of which can be chemically-modified, wherein each strand is about 18 to about 27 (*e.g*., about 18, 19, 20, 21, 22, 23, 24, 25, 26, or 27) nucleotides in length, wherein the duplex has about 18 to about 23 (*e.g*., about 18, 19, 20, 21, 22, or 23) base pairs, and wherein the chemical modification comprises a structure having any of Formulae I-VII. For example, an exemplary chemically-modified siNA molecule of the invention comprises a duplex having two strands, one or both of which can be chemically-modified with a chemical modification having any of Formulae I-VII or any combination thereof, wherein each strand consists of about 21 nucleotides, each having a 2-nucleotide 3'-terminal nucleotide overhang, and wherein the duplex has about 19 base pairs. In another embodiment, a siNA molecule of the invention comprises a single stranded hairpin structure, wherein the siNA is about 36 to about 70 (*e.g*., about 36, 40, 45, 50, 55, 60, 65, or 70) nucleotides in length having about 18 to about 23 (*e.g*., about 18, 19, 20, 21, 22, or 23) base pairs, and wherein the siNA can include a chemical modification comprising a structure having any of Formulae I-VII or any combination thereof. For example, an exemplary chemically-modified siNA molecule of the invention comprises a linear oligonucleotide having about 42 to about 50 (*e.g*., about 42, 43, 44, 45, 46, 47, 48, 49, or 50) nucleotides that is chemically-modified with a chemical modification having any of Formulae I-VII or any combination thereof, wherein the linear oligonucleotide forms a hairpin structure having about 19 base pairs and a 2-nucleotide 3'-terminal nucleotide overhang. In another embodiment, a linear hairpin siNA molecule of the invention contains a stem loop motif, wherein the loop portion of the siNA molecule is biodegradable. For example, a linear hairpin siNA molecule of the invention is designed such that degradation of the loop portion of the siNA molecule *in vivo* can generate a double-stranded siNA molecule with 3'-terminal overhangs, such as 3'-terminal nucleotide overhangs comprising about 2 nucleotides.

In another embodiment, a siNA molecule of the invention comprises a circular nucleic acid molecule, wherein the siNA is about 38 to about 70 (*e.g*., about 38, 40, 45, 50, 55, 60, 65, or 70) nucleotides in length having about 18 to about 23 (*e.g*., about 18, 19, 20, 21, 22, or 23) base pairs, and wherein the siNA can include a chemical modification, which comprises a structure having any of Formulae I-VII or any combination thereof. For example, an exemplary chemically-modified siNA molecule of the invention comprises a circular oligonucleotide having about 42 to about 50 (*e.g*., about 42, 43, 44, 45, 46, 47, 48, 49, or 50) nucleotides that is chemically-modified with a chemical modification having any of Formulae I-VII or any combination thereof, wherein the circular oligonucleotide forms a dumbbell shaped structure having about 19 base pairs and 2 loops.

In another embodiment, a circular siNA molecule of the invention contains two loop motifs, wherein one or both loop portions of the siNA molecule is biodegradable. For example, a circular siNA molecule of the invention is designed such that degradation of the loop portions of the siNA molecule *in vivo* can generate a double-stranded siNA molecule with 3'-terminal overhangs, such as 3'-terminal nucleotide overhangs comprising about 2 nucleotides.

In one embodiment, a siNA molecule of the invention comprises at least one (*e.g*., about 1, 2, 3, 4, 5, 6, 7, 8, 9, 10, or more) abasic moiety, for example a compound having Formula V: 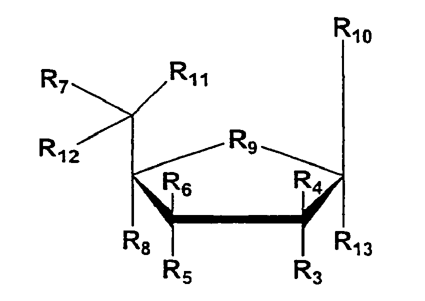 wherein each R3, R4, R5, R6, R7, R8, R10, R11, R12, and R13 is independently H, OH, alkyl, substituted allcyl, alkaryl or aralkyl, F, Cl, Br, CN, CF3, OCF3, OCN, O-alkyl, S-alkyl, N-allcyl, O-alkenyl, S-alkenyl, N-alkenyl, SO-alkyl, alkyl-OSH, alkyl-OH, O-alkyl-OH, 0-alkyl-SH, S-alkyl-OH, S-allcyl-SH, all:yl-S-alkyl, alkyl-0-alkyl, ON02, NO2, N3, NH2, aminoalkyl, aminoacid, aminoacyl, ONH2, O-aminoalkyl, O-aminoacid, O-aminoacyl, heterocycloalkyl, heterocycloalkaryl, aminoalkylamino, polyalklylamino, substituted silyl, or group having Formula 1; R9 is O, S, CH2, S=O, CHF, or CF2.

In one embodiment, a siNA molecule of the invention comprises at least one (*e.g.,* about 1, 2, 3, 4, 5, 6, 7, 8, 9, 10, or more) inverted abasic moiety, for example a compound having Formula VI: 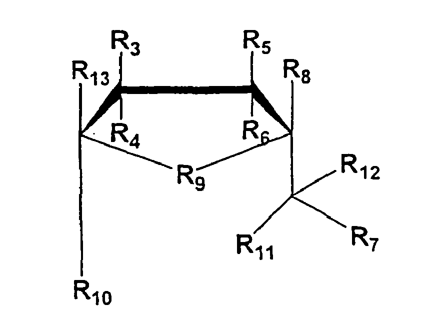 wherein each R3, R4, R5, R6, R7, R8, R10, R11, R12, and R13 is independently H, OH, alkyl, substituted alkyl, alkaryl or aralkyl, F, Cl, Br, CN, CF3, OCF3, OCN, O-alkyl, S-alkyl, N-alkyl, 0-alkenyl, S-alkenyl, N-alkenyl, SO-alkyl, all:yl-OSH, alkyl-OH, O-alkyl-OH, O-alkyl-SH, S-alkyl-OH, S-alkyl-SH, alk-yl-S-alk-yl, alkyl-0-alkyl, ON02, N02, N3, NH2, aminoalkyl, aminoacid, aminoacyl, ONH2, O-aminoalkyl, O-aminoacid, 0-aminoacyl, heterocycloalkyl, heterocycloalkaryl, aminoalkylamino, polyalk1ylamino, substituted silyl, or group having Fonnula I; R9 is O, S, CH2, S=O, CHF, or CF2, and either R2, R3, R8 or R13 serve as points of attachment to the siNA molecule of the invention.

In another embodiment, a siNA molecule of the invention comprises at least one (*e.g*., about 1, 2, 3, 4, 5, 6, 7, 8, 9, 10, or more) substituted polyalkyl moieties, for example a compound having Fonnula VII: 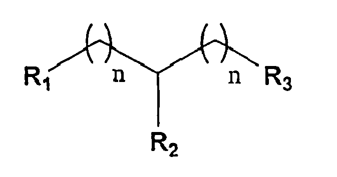 wherein each n is independently an integer from 1 to 12, each R1, R2 and R3 is independently H, OH, alkyl, substituted alkyl, alkaryl or aralkyl, F, Cl, Br, CN, CF3, OCF3, OCN, O-alkyl, S-alkyl, N-alkyl, O-alkenyl, S-alkenyl, N-alkenyl, SO-alkyl, alkyl-OSH, alkyl-OH, O-alklyl-OH, O-alkyl-SH, S-alkyl-OH, S-alkyl-SH, alkyl-S-alkyl, alkyl-O-alkyl, ONO2, NO2, N3, NH2, aminoalkyl, aminoacid, aminoacyl, ONH2, O-aminoalkyl, O-aminoacid, O-aminoacyl, heterocycloalkyl, heterocycloalkaryl, aminoalkylamino, polyalklylamino, substituted silyl, or a group having Formula I, and R1, R2 or R3 serves as points of attachment to the siNA molecule of the invention.

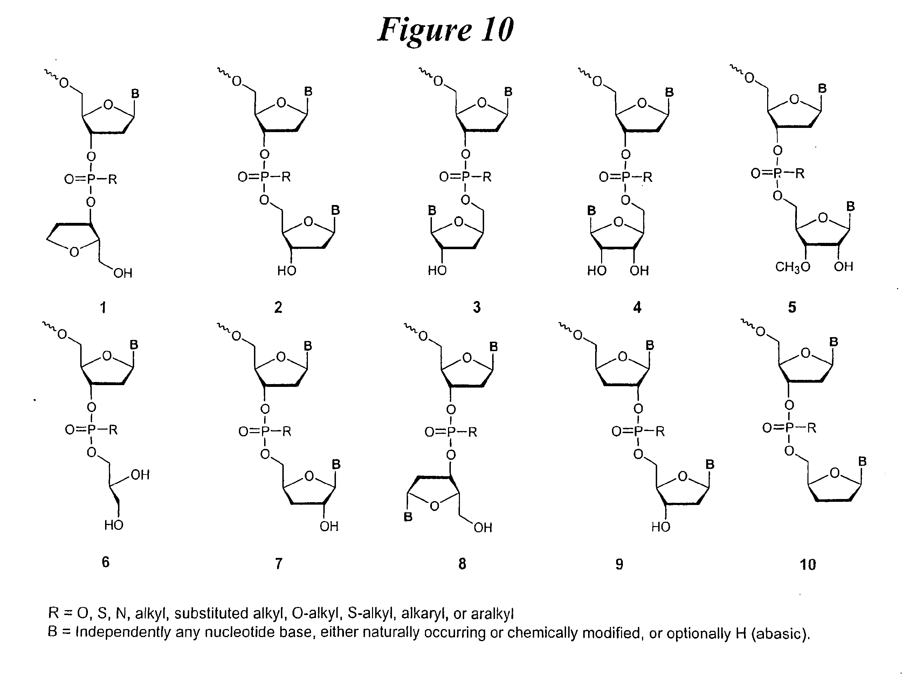

In another embodiment, the invention features a compound having Formula VII, wherein R1 and R2 are hydroxyl (OH) groups, n = 1, and R3 comprises O and is the point of attachment to the 3'-end, the 5'-end, or both of the 3' and 5'-ends of one or both strands of a double-stranded siNA molecule of the invention or to a single-stranded siNA molecule of the invention. This modification is referred to herein as "glyceryl" (for example modification 6 in **Figure 10**).

In another embodiment, a moiety having any of Formula V, VI or VII of the invention is at the 3'-end, the 5'-end, or both of the 3' and 5'-ends of a siNA molecule of the invention. For example, a moiety having Formula V, VI or VII can be present at the 3'-end, the 5'-end, or both of the 3' and 5'-ends of the antisense strand, the sense strand, or both antisense and sense strands of the siNA molecule. In addition, a moiety having Formula VII can be present at the 3'-end or the 5'-end of a hairpin siNA molecule as described herein.

In another embodiment, a siNA molecule of the invention comprises an abasic residue having Formula V or VI, wherein the abasic residue having Formula VI or VI is connected to the siNA construct in a 3'-3', 3'-2', 2'-3', or 5'-5' configuration, such as at the 3'-end, the 5'-end, or both of the 3' and 5'-ends of one or both siNA strands.

In one embodiment, a siNA molecule of the invention comprises one or more (e.g., about 1, 2, 3, 4, 5, 6, 7, 8, 9, 10, or more) locked nucleic acid (LNA) nucleotides, for example at the 5'-end, the 3'-end, both of the 5' and 3'-ends, or any combination thereof, of the siNA molecule.

In another embodiment, a siNA molecule of the invention comprises one or more (*e.g*., about 1, 2, 3, 4, 5, 6, 7, 8, 9, 10, or more) acyclic nucleotides, for example at the 5'-end, the 3'-end, both of the 5' and 3'-ends, or any combination thereof, of the siNA molecule.

In one embodiment, the invention features a chemically-modified short interfering nucleic acid (siNA) molecule of the invention, wherein the chemically-modified siNA comprises a sense region, where any (*e.g*., one or more or all) pyrimidine nucleotides present in the sense region are 2'-deoxy-2'-fluoro pyrimidine nucleotides (*e.g*., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and where any (*e.g*., one or more or all) purine nucleotides present in the sense region are 2'-deoxy purine nucleotides (*e.g*., wherein all purine nucleotides are 2'-deoxy purine nucleotides or alternately a plurality of purine nucleotides are 2'-deoxy purine nucleotides).

In one embodiment, the invention features a chemically-modified short interfering nucleic acid (siNA) molecule of the invention, wherein the chemically-modified siNA comprises a sense region, where any (*e.g.,* one or more or all) pyrimidine nucleotides present in the sense region are 2'-deoxy-2'-fluoro pyrimidine nucleotides (*e.g*., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and where any (*e.g*., one or more or all) purine nucleotides present in the sense region are 2'-deoxy purine nucleotides (*e.g*., wherein all purine nucleotides are 2'-deoxy purine nucleotides or alternately a plurality of purine nucleotides are 2'-deoxy purine nucleotides), wherein any nucleotides comprising a 3'-terminal nucleotide overhang that are present in said sense region are 2'-deoxy nucleotides.

In one embodiment, the invention features a chemically-modified short interfering nucleic acid (siNA) molecule of the invention, wherein the chemically-modified siNA comprises an antisense region, where any (*e.g*., one or more or all) pyrimidine nucleotides present in the antisense region are 2'-deoxy-2'-fluoro pyrimidine nucleotides (*e.g*., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any (*e.g*., one or more or all) purine nucleotides present in the antisense region are 2'-O-methyl purine nucleotides (*e.g*., wherein all purine nucleotides are 2'-O-methyl purine nucleotides or alternately a plurality of purine nucleotides are 2'-O-methyl purine nucleotides).

In one embodiment, the invention features a chemically-modified short interfering nucleic acid (siNA) molecule of the invention, wherein the chemically-modified siNA comprises an antisense region, where any (*e.g*., one or more or all) pyrimidine nucleotides present in the antisense region are 2'-deoxy-2'-fluoro pyrimidine nucleotides (*e.g.,* wherein all pyrimidine nucleotides are 3'-deoxy-2'-fluoro pyrimidine nucleotides or alternately a plurality of pyrimidine nucleotides are 2'-deoxy-2'-fluoro pyrimidine nucleotides), and wherein any (*e.g*., one or more or all) purine nucleotides present in the antisense region are 2'-O-methyl purine nucleotides (*e.g*., wherein all purine nucleotides are 2'-O-methyl purine nucleotides or alternately a plurality of purine nucleotides are 2'-O-methyl purine nucleotides), wherein any nucleotides comprising a 3'-terminal nucleotide overhang that are present in said antisense region are 2'-deoxy nucleotides.

In one embodiment, the invention features a chemically-modified short interfering nucleic acid (siNA) molecule of the invention, wherein the chemically-modified siNA comprises an antisense region, where any (*e.g*., one or more or all) pyrimidine nucleotides present in the antisense region are 2'-deoxy-2'-fluoro pyrimidine nucleotides (*e.g*., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and where any (*e.g*., one or more or all) purine nucleotides present in the antisense region are 2'-deoxy purine nucleotides (*e.g*., wherein all purine nucleotides are 2'-deoxy purine nucleotides or alternately a plurality of purine nucleotides are 2'-deoxy purine nucleotides).

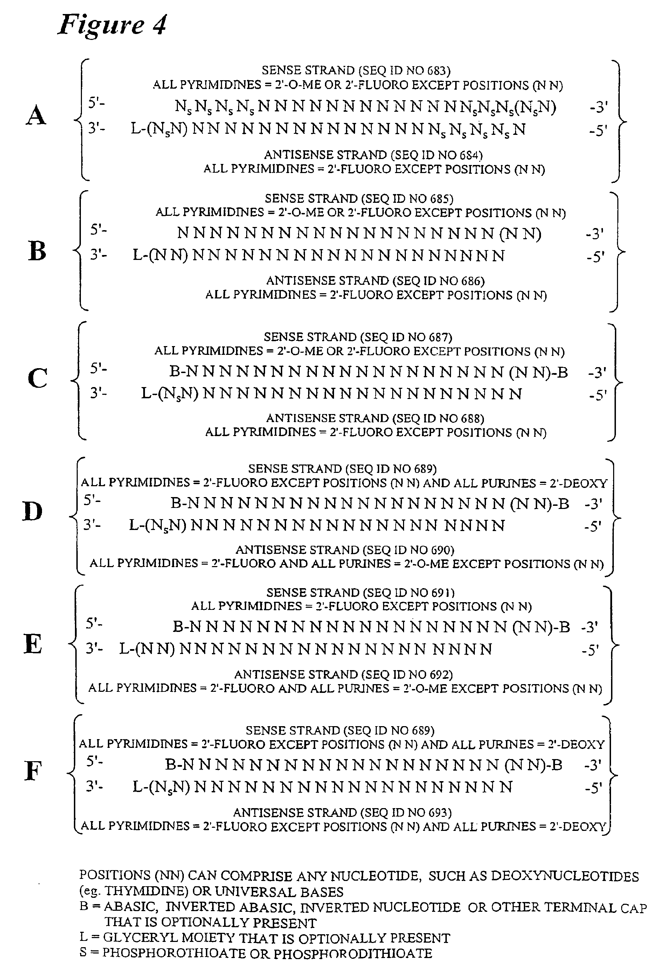

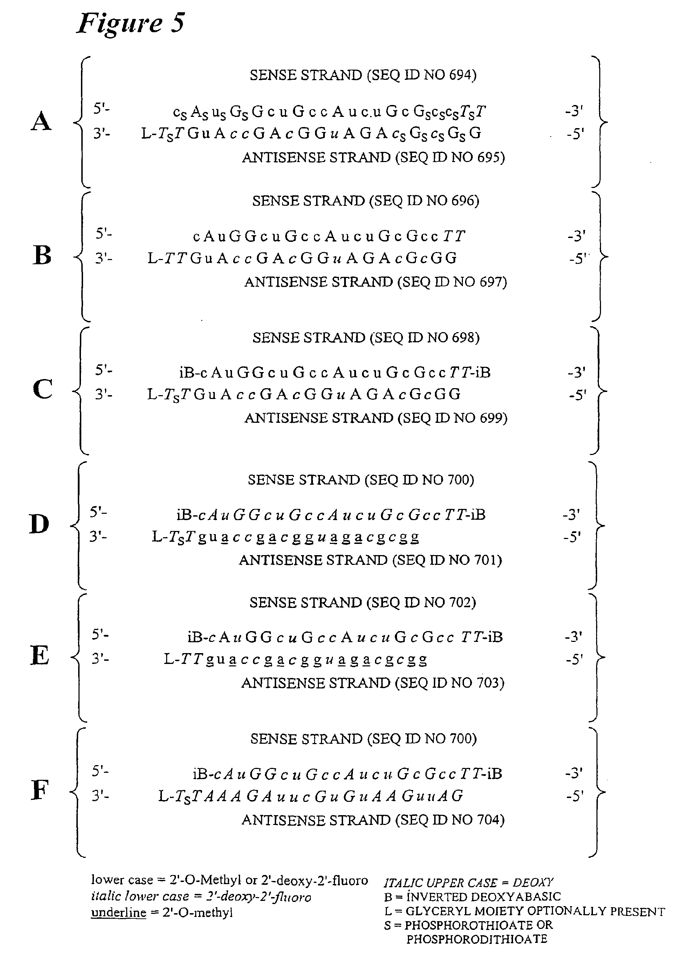

In one embodiment, the invention features a chemically-modified short interfering nucleic acid (siNA) molecule of the invention capable of mediating RNA interference (RNAi) against a BACE inside a cell or reconstituted *in vitro* system, wherein the chemically-modified siNA comprises a sense region, where one or more pyrimidine nucleotides present in the sense region are 2'-deoxy-2'-fluoro pyrimidine nucleotides (*e.g*., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and where one or more purine nucleotides present in the sense region are 2'-deoxy purine nucleotides (*e.g*., wherein all purine nucleotides are 2'-deoxy purine nucleotides or alternately a plurality of purine nucleotides are 2'-deoxy purine nucleotides), and inverted deoxy abasic modifications that are optionally present at the 3'-end, the 5'-end, or both of the 3' and 5'-ends of the sense region, the sense region optionally further comprising a 3'-terminal overhang having about 1 to about 4 (*e.g*., about 1, 2, 3, or 4) 2'-deoxyribonucleotides; and wherein the chemically-modified short interfering nucleic acid molecule comprises an antisense region, where one or more pyrimidine nucleotides present in the antisense region are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein one or more purine nucleotides present in the antisense region are 2'-O-methyl purine nucleotides (e.g., wherein all purine nucleotides are 2'-O-methyl purine nucleotides or alternately a plurality of purine nucleotides are 2'-O-methyl purine nucleotides), and a terminal cap modification, such as any modification described herein or shown in **Figure 10**, that is optionally present at the 3'-end, the 5'-end, or both of the 3' and 5'-ends of the antisense sequence, the antisense region optionally further comprising a 3'-terminal nucleotide overhang having about 1 to about 4 (e.g., about 1, 2, 3, or 4) 2'-deoxynucleotides, wherein the overhang nucleotides can further comprise one or more (e.g., 1, 2, 3, or 4) phosphorothioate internucleotide linkages. Non-limiting examples of these chemically-modified siNAs are shown in **Figures 4 and 5** and **Table III** herein.

In one embodiment, the invention features a chemically-modified short interfering nucleic acid (siNA) molecule of the invention capable of mediating RNA interference (RNAi) against a BACE inside a cell or reconstituted *in vitro* system, wherein the siNA comprises a sense region, where one or more pyrimidine nucleotides present in the sense region are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and where one or more purine nucleotides present in the sense region are purine ribonucleotides (e.g., wherein all purine nucleotides are purine ribonucleotides or alternately a plurality of purine nucleotides are purine ribonucleotides), and inverted deoxy abasic modifications that are optionally present at the 3'-end, the 5'-end, or both of the 3' and 5'-ends of the sense region, the sense region optionally further comprising a 3'-teminal overhang having about 1 to about 4 (e.g., about 1, 3. 3, or 4) 2'-deoxyribonucleotides; and wherein the siNA comprises an antisense region, where one or more pyrimidine nucleotides present in the antisense region are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any purine nucleotides present in the antisense region are 2'-O-methyl purine nucleotides (e.g., wherein all purine, nucleotides are 2'-O-methyl purine nucleotides or alternately a plurality of purine nucleotides are 2'-O-methyl purine nucleotides), and a terminal cap modification, such as any modification described herein or shown in **Figure 10**, that is optionally present at the 3'-end, the 5'-end, or both of the 3' and 5'-ends of the antisense sequence, the antisense region optionally further comprising a 3'-terminal nucleotide overhang having about 1 to about 4 (e.g., about 1, 2, 3, or 4) 2'-deoxynucleotides, wherein the overhang nucleotides can further comprise one or more (e.g., 1, 2, 3, or 4) phosphorothioate internucleotide linkages. Non-limiting examples of these chemically-modified siNAs are shown in **Figures 4 and 5** and **Table III** herein.

In one embodiment, the invention features a chemically-modified short interfering nucleic acid (siNA) molecule of the invention capable of mediating RNA interference (RNAi) against a BACE inside a cell or reconstituted *in vitro* system, wherein the chemically-modified siNA comprises a sense region, where one or more pyrimidine nucleotides present in the sense region are 2'-deoxy-2'-fluoro pyrimidine nucleotides (*e.g*., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and for example where one or more purine nucleotides present in the sense region are selected from the group consisting of 2'-deoxy nucleotides, locked nucleic acid (LNA) nucleotides, 2'-methoxyethyl nucleotides, 4'-thionucleotides, and 2'-O-methyl nucleotides (*e.g*., wherein all purine nucleotides are selected from the group consisting of 2'-deoxy nucleotides, locked nucleic acid (LNA) nucleotides, 2'-methoxyethyl nucleotides, 4'-thionucleotides, and 2'-O-methyl nucleotides or alternately a plurality of purine nucleotides are selected from the group consisting of 2'-deoxy nucleotides, locked nucleic acid (LNA) nucleotides, 2'-methoxyethyl nucleotides, 4'-thionucleotides, and 2'-O-methyl nucleotides), and wherein inverted deoxy abasic modifications are optionally present at the 3'-end, the 5'-end, or both of the 3' and 5'-ends of the sense region, the sense region optionally further comprising a 3'-terminal overhang having about 1 to about 4 (e.g., about 1, 2, 3, or 4) 2'-deoxyribonucleotides; and wherein the chemically-modified short interfering nucleic acid molecule comprises an antisense region, where one or more pyrimidine nucleotides present in the antisense region are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein one or more purine nucleotides present in the antisense region are selected from the group consisting of 2'-deoxy nucleotides, locked nucleic acid (LNA) nucleotides, 2'-methoxyethyl nucleotides, 4'-thionucleotides, and 2'-O-methyl nucleotides (e.g., wherein all purine nucleotides are selected from the group consisting of 2'-deoxy nucleotides, locked nucleic acid (LNA) nucleotides, 2'-methoxyethyl nucleotides, 4'-thionucleotides, and 2'-O-methyl nucleotides or alternately a plurality of purine nucleotides are selected from the group consisting of 2'-deoxy nucleotides, locked nucleic acid (LNA) nucleotides, 2'-methoxyethyl nucleotides, 4'-thionucleotides, and 2'-O-methyl nucleotides), and a terminal cap modification, such as any modification described herein or shown in Figure 10, that is optionally present at the 3'-end, the 5'-end, or both of the 3' and 5'-ends of the antisense sequence, the antisense region optionally further comprising a 3'-terminal nucleotide overhang having about 1 to about 4 (e.g., about 1, 2, 3, or 4) 2'-deoxynucleotides, wherein the overhang nucleotides can further comprise one or more (e.g., 1, 2, 3, or 4) phosphorothioate internucleotide linkages.

In another embodiment, any modified nucleotides present in the siNA molecules of the invention, preferably in the antisense strand of the siNA molecules of the invention, but also optionally in the sense and/or both antisense and sense strands, comprise modified nucleotides having properties or characteristics similar to naturally occurring ribonucleotides. For example, the invention features siNA molecules including modified nucleotides having a Northern conformation (e.g., Northern pseudorotation cycle, see for example Saenger, Principles of Nucleic Acid Structure, Springer-Verlag ed., 1984). As such, chemically modified nucleotides present in the siNA molecules of the invention, preferably in the antisense strand of the siNA molecules of the invention, but also optionally in the sense and/or both antisense and sense strands, are resistant to nuclease degradation while at the same time maintaining the capacity to mediate RNAi. Non-limiting examples of nucleotides having a northern configuration include locked nucleic acid (LNA) nucleotides (e.g., 2'-O,4'-C-methylene-(D-ribofuranosyl) nucleotides); 2'-methoxyethoxy (MOE) nucleotides; 2'-methyl-thio-ethyl, 2'-deoxy-2'-fluoro nucleotides, 2'-deoxy-2'-chloro nucleotides, 2'-azido nucleotides, and 2'-O-methyl nucleotides.

In one embodiment, the invention features a chemically-modified short interfering nucleic acid molecule (siNA) capable of mediating RNA interference (RNAi) against a BACE inside a cell or reconstituted *in vitro* system, wherein the chemical modification comprises a conjugate covalently attached to the chemically-modified siNA molecule. In another embodiment, the conjugate is covalently attached to the chemically-modified siNA molecule via a biodegradable linker. In one embodiment, the conjugate molecule is attached at the 3'-end of either the sense strand, the antisense strand, or both strands of the chemically-modified siNA molecule. In another embodiment, the conjugate molecule is attached at the 5'-end of either the sense strand, the antisense strand, or both strands of the chemically-modified siNA molecule. In yet another embodiment, the conjugate molecule is attached both the 3'-end and 5'-end of either the sense strand, the antisense strand, or both strands of the chemically-modified siNA molecule, or any combination thereof. In one embodiment, a conjugate molecule of the invention comprises a molecule that facilitates delivery of a chemically-modified siNA molecule into a biological system, such as a cell. In another embodiment, the conjugate molecule attached to the chemically-modified siNA molecule is a poly ethylene glycol, human serum albumin, or a ligand for a cellular receptor that can mediate cellular uptake. Examples of specific conjugate molecules contemplated by the instant invention that can be attached to chemically-modified siNA molecules are described in Vargeese et al., U.S. Serial No. 10/201,394, incorporated by reference herein. The type of conjugates used and the extent of conjugation of siNA molecules of the invention can be evaluated for improved pharmacokinetic profiles, bioavailability, and/or stability of siNA constructs while at the same time maintaining the ability of the siNA to mediate RNAi activity. As such, one skilled in the art can screen siNA constructs that are modified with various conjugates to determine whether the siNA conjugate complex possesses improved properties while maintaining the ability to mediate RNAi, for example in animal models as are generally known in the art.

In one embodiment, the invention features a short interfering nucleic acid (siNA) molecule of the invention, wherein the siNA further comprises a nucleotide, non-nucleotide, or mixed nucleotide/non-nucleotide linker that joins the sense region of the siNA to the antisense region of the siNA. In one embodiment, a nucleotide linker of the invention can be a linker of ≥ 2 nucleotides in length, for example 3, 4, 5, 6, 7, 8, 9, or 10 nucleotides in length. In another embodiment, the nucleotide linker can be a nucleic acid aptamer. By "aptamer" or "nucleic acid aptamer" as used herein is meant a nucleic acid molecule that binds specifically to a target molecule wherein the nucleic acid molecule has sequence that comprises a sequence recognized by the target molecule in its natural setting. Alternately, an aptamer can be a nucleic acid molecule that binds to a target molecule where the target molecule does not naturally bind to a nucleic acid. The target molecule can be any molecule of interest. For example, the aptamer can be used to bind to a ligand-binding domain of a protein, thereby preventing interaction of the naturally occurring ligand with the protein. This is a non-limiting example and those in the art will recognize that other embodiments can be readily generated using techniques generally known in the art. (See, for example, Gold et al., 1995, Annu. Rev. Biochem.. 64, 763; Brody and Gold, 2000, J. Biotechnol., 74, 5; Sun, 2000, Curr. Opin. Mol. Ther., 2, 100; Kusser, 2000, J Biotechnol., 74, 27; Hermann and Patel, 2000, Science, 287, 820; and Jayasena, 1999, Clinical Chemistry, 45, 1628.)

In yet another embodiment, a non-nucleotide linker of the invention comprises abasic nucleotide, polyether, polyamine, polyamide, peptide, carbohydrate, lipid, polyhydrocarbon, or other polymeric compounds (e.g. polyethylene glycols such as those having about 2 to about 100 ethylene glycol units). Specific examples include those described by Seela and Kaiser, Nucleic Acids Res. 1990, 18:6353 and Nucleic Acids Res. 1987, 15:3113; Cload and Schepartz, J. Am. Chem. Soc. 1991, 113:6324; Richardson and Schepartz, J. Am. Chem. Soc. 1991, 113:5109; Ma et al., Nucleic Acids Res. 1993, 21:2585 and Biochemistry 1993, 32:1751; Durand et al., Nucleic Acids Res. 1990, 18:6353; McCurdy et al., Nucleosides & Nucleotides 1991, 10:287; Jschke et al., Tetrahedron Lett. 1993, 34:301; Ono et al., Biochemistry 1991, 30:9914; Arnold *et al.,* International Publication No. WO 89/02439; Usman *et al.,* International Publication No. WO 95/06731; Dudycz *et al.,* International Publication No. WO 95/11910 and Ferentz and Verdine, *J. Am. Chem. Soc.* 1991, *113*:4000, all hereby incorporated by reference herein. A "non-nucleotide" further means any group or compound that can be incorporated into a nucleic acid chain in the place of one or more nucleotide units, including either sugar and/or phosphate substitutions, and allows the remaining bases to exhibit their enzymatic activity. The group or compound can be abasic in that it does not contain a commonly recognized nucleotide base, such as adenosine, guanine, cytosine, uracil or thymine, for example at the C1 position of the sugar.

In one embodiment, the invention features a short interfering nucleic acid (siNA) molecule capable of mediating RNA interference (RNAi) inside a cell or reconstituted in vitro system, wherein one or both strands of the siNA molecule that are assembled from two separate oligonucleotides do not comprise any ribonucleotides. All positions within the siNA can include chemically modified nucleotides and/or non-nucleotides such as nucleotides and or non-nucleotides having Formula I, II, III, IV, V, VI, or VII or any combination thereof to the extent that the ability of the siNA molecule to support RNAi activity in a cell is maintained.

In one embodiment, a siNA molecule of the invention is a single stranded siNA molecule that mediates RNAi activity in a cell or reconstituted in vitro system, wherein the siNA molecule comprises a single stranded polynucleotide having complementarity to a target nucleic acid sequence. In another embodiment, the single stranded siNA molecule of the invention comprises a 5'-terminal phosphate group. In another embodiment, the single stranded siNA molecule of the invention comprises a 5'-terminal phosphate group and a 3'-terminal phosphate group (e.g., a 2',3'-cyclic phosphate). In another embodiment, the single stranded siNA molecule of the invention comprises about 19 to about 29 nucleotides. In yet another embodiment, the single stranded siNA molecule of the invention comprises one or more chemically modified nucleotides or non-nucleotides described herein. For example, all the positions within the siNA molecule can include chemically-modified nucleotides such as nucleotides having any of Formulae I-VII, or any combination thereof to the extent that the ability of the siNA molecule to support RNAi activity in a cell is maintained.

In one embodiment, a siNA molecule of the invention is a single stranded siNA molecule that mediates RNAi activity in a cell or reconstituted in vitro system, wherein the siNA molecule comprises a single stranded polynucleotide having complementarity to a target nucleic acid sequence, and wherein one or more pyrimidine nucleotides present in the siNA are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any purine nucleotides present in the antisense region are 2'-O-methyl purine nucleotides (e.g., wherein all purine nucleotides are 2'-O-methyl purine nucleotides or alternately a plurality of purine nucleotides are 2'-O-methyl purine nucleotides), and a terminal cap modification, such as any modification described herein or shown in **Figure 10**, that is optionally present at the 3'-end, the 5'-end, or both of the 3' and 5'-ends of the antisense sequence, the siNA optionally further comprising about 1 to about 4 (e.g., about 1, 2, 3, or 4) terminal 2'-deoxynucleotides at the 3'-end of the siNA molecule, wherein the terminal nucleotides can further comprise one or more (e.g., 1, 2, 3, or 4) phosphorothioate internucleotide linkages, and wherein the siNA optionally further comprises a terminal phosphate group, such as a 5'-terminal phosphate group.

In one embodiment, a siNA molecule of the invention is a single stranded siNA molecule that mediates RNAi activity in a cell or reconstituted in vitro system, wherein the siNA molecule comprises a single stranded polynucleotide having complementarity to a target nucleic acid sequence, and wherein one or more pyrimidine nucleotides present in the siNA are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any purine nucleotides present in the antisense region are 2'-deoxy purine nucleotides (e.g., wherein all purine nucleotides are 2'-deoxy purine nucleotides or alternately a plurality of purine nucleotides are 2'-deoxy purine nucleotides), and a terminal cap modification, such as any modification described herein or shown in **Figure 10**, that is optionally present at the 3'-end, the 5'-end, or both of the 3' and 5'-ends of the antisense sequence, the siNA optionally further comprising about 1 to about 4 (e.g., about 1, 2, 3, or 4) terminal 2'-deoxynucleotides at the 3'-end of the siNA molecule, wherein the terminal nucleotides can further comprise one or more (e.g., 1, 2, 3, or 4) phosphorothioate internucleotide linkages, and wherein the siNA optionally further comprises a terminal phosphate group, such as a 5'-terminal phosphate group.

In one embodiment, a siNA molecule of the invention is a single stranded siNA molecule that mediates RNAi activity in a cell or reconstituted in vitro system, wherein the siNA molecule comprises a single stranded polynucleotide having complementarity to a target nucleic acid sequence, and wherein one or more pyrimidine nucleotides present in the siNA are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any purine nucleotides present in the antisense region are locked nucleic acid (LNA) nucleotides (e.g., wherein all purine nucleotides are LNA nucleotides or alternately a plurality of purine nucleotides are LNA nucleotides), and a terminal cap modification, such as any modification described herein or shown in **Figure 10**, that is optionally present at the 3'-end, the 5'-end, or both of the 3' and 5'-ends of the antisense sequence, the siNA optionally further comprising about 1 to about 4 (e.g., about 1, 2, 3, or 4) terminal 2'-deoxynucleotides at the 3'-end of the siNA molecule, wherein the terminal nucleotides can further comprise one or more (e.g., 1, 2, 3, or 4) phosphorothioate internucleotide linkages, and wherein the siNA optionally further comprises a terminal phosphate group, such as a 5'-terminal phosphate group.

In one embodiment, a siNA molecule of the invention is a single stranded siNA molecule that mediates RNAi activity in a cell or reconstituted in vitro system, wherein the siNA molecule comprises a single stranded polynucleotide having complementarity to a target nucleic acid sequence, and wherein one or more pyrimidine nucleotides present in the siNA are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any purine nucleotides present in the antisense region are 2'-methoxyethyl purine nucleotides (e.g., wherein all purine nucleotides are 2'-methoxyethyl purine nucleotides or alternately a plurality of purine nucleotides are 2'-methoxyethyl purine nucleotides), and a terminal cap modification, such as any modification described herein or shown in **Figure 10**, that is optionally present at the 3'-end, the 5'-end, or both of the 3' and 5'-ends of the antisense sequence, the siNA optionally further comprising about 1 to about 4 (e.g., about 1, 2, 3, or 4) terminal 2'-deoxynucleotides at the 3'-end of the siNA molecule, wherein the terminal nucleotides can further comprise one or more (e.g., 1, 2, 3, or 4) phosphorothioate internucleotide linkages, and wherein the siNA optionally further comprises a terminal phosphate group, such as a 5'-terminal phosphate group.

In another embodiment, any modified nucleotides present in the single stranded siNA molecules of the invention comprise modified nucleotides having properties or characteristics similar to naturally occurring ribonucleotides. For example, the invention features siNA molecules including modified nucleotides having a Northern conformation (e.g., Northern pseudorotation cycle, see for example Saenger, Principles of Nucleic Acid Structure, Springer-Verlag ed., 1984). As such, chemically modified nucleotides present in the single stranded siNA molecules of the invention are preferably resistant to nuclease degradation while at the same time maintaining the capacity to mediate RNAi.

In one embodiment, the invention features a method for modulating the expression of a BACE gene within a cell comprising: (a) synthesizing a siNA molecule of the invention, which can be chemically-modified, wherein one of the siNA strands comprises a sequence complementary to RNA of the BACE gene; and (b) introducing the siNA molecule into a cell under conditions suitable to modulate the expression of the BACE gene in the cell.

In one embodiment, the invention features a method for modulating the expression of a BACE gene within a cell comprising: (a) synthesizing a siNA molecule of the invention, which can be chemically-modified, wherein one of the siNA strands comprises a sequence complementary to RNA of the BACE gene and wherein the sense strand sequence of the siNA comprises a sequence identical to the sequence of the target RNA; and (b) introducing the siNA molecule into a cell under conditions suitable to modulate the expression of the BACE gene in the cell.

In another embodiment, the invention features a method for modulating the expression of more than one BACE gene within a cell comprising: (a) synthesizing siNA molecules of the invention, which can be chemically-modified, wherein one of the siNA strands comprises a sequence complementary to RNA of the BACE genes; and (b) introducing the siNA molecules into a cell under conditions suitable to modulate the expression of the BACE genes in the cell.

In another embodiment, the invention features a method for modulating the expression of more than one BACE gene within a cell comprising: (a) synthesizing a siNA molecule of the invention, which can be chemically-modified, wherein one of the siNA strands comprises a sequence complementary to RNA of the BACE gene and wherein the sense strand sequence of the siNA comprises a sequence identical to the sequence of the target RNA; and (b) introducing the siNA molecules into a cell under conditions suitable to modulate the expression of the BACE genes in the cell.

In one embodiment, the invention features a method of modulating the expression of a BACE gene in a tissue explant comprising: (a) synthesizing a siNA molecule of the invention, which can be chemically-modified, wherein one of the siNA strands comprises a sequence complementary to RNA of the BACE gene; and (b) introducing the siNA molecule into a cell of the tissue explant derived from a particular organism under conditions suitable to modulate the expression of the BACE gene in the tissue explant. In another embodiment, the method further comprises introducing the tissue explant back into the organism the tissue was derived from or into another organism under conditions suitable to modulate the expression of the BACE gene in that organism.

In one embodiment, the invention features a method of modulating the expression of a BACE gene in a tissue explant comprising: (a) synthesizing a siNA molecule of the invention, which can be chemically-modified, wherein one of the siNA strands comprises a sequence complementary to RNA of the BACE gene and wherein the sense strand sequence of the siNA comprises a sequence identical to the sequence of the target RNA; and (b) introducing the siNA molecule into a cell of the tissue explant derived from a particular organism under conditions suitable to modulate the expression of the BACE gene in the tissue explant. In another embodiment, the method further comprises introducing the tissue explant back into the organism the tissue was derived from or into another organism under conditions suitable to modulate the expression of the BACE gene in that organism.

In another embodiment, the invention features a method of modulating the expression of more than one BACE gene in a tissue explant comprising: (a) synthesizing siNA molecules of the invention, which can be chemically-modified, wherein one of the siNA strands comprises a sequence complementary to RNA of the BACE genes; and (b) introducing the siNA molecules into a cell of the tissue explant derived from a particular organism under conditions suitable to modulate the expression of the BACE genes in the tissue explant. In another embodiment, the method further comprises introducing the tissue explant back into the organism the tissue was derived from or into another organism under conditions suitable to modulate the expression of the BACE genes in that organism.

In one embodiment, the invention features a method of modulating the expression of a BACE gene in an organism comprising: (a) synthesizing a siNA molecule of the invention, which can be chemically-modified, wherein one of the siNA strands comprises a sequence complementary to RNA of the BACE gene; and (b) introducing the siNA molecule into the organism under conditions suitable to modulate the expression of the BACE gene in the organism.

In another embodiment, the invention features a method of modulating the expression of more than one BACE gene in an organism comprising: (a) synthesizing siNA molecules of the invention, which can be chemically-modified, wherein one of the siNA strands comprises a sequence complementary to RNA of the BACE genes; and (b) introducing the siNA molecules into the organism under conditions suitable to modulate the expression of the BACE genes in the organism.

In one embodiment, the invention features a method for modulating the expression of a BACE gene within a cell comprising: (a) synthesizing a siNA molecule of the invention, which can be chemically-modified, wherein the siNA comprises a single stranded sequence having complementarity to RNA of the BACE gene; and (b) introducing the siNA molecule into a cell under conditions suitable to modulate the expression of the BACE gene in the cell.

In another embodiment, the invention features a method for modulating the expression of more than one BACE gene within a cell comprising: (a) synthesizing siNA molecules of the invention, which can be chemically-modified, wherein the siNA comprises a single stranded sequence having complementarity to RNA of the BACE gene; and (b) contacting the siNA molecule with a cell in vitro or in vivo under conditions suitable to modulate the expression of the BACE genes in the cell.

In one embodiment, the invention features a method of modulating the expression of a BACE gene in a tissue explant comprising: (a) synthesizing a siNA molecule of the invention, which can be chemically-modified, wherein the siNA comprises a single stranded sequence having complementarity to RNA of the BACE gene; and (b) contacting the siNA molecule with a cell of the tissue explant derived from a particular organism under conditions suitable to modulate the expression of the BACE gene in the tissue explant. In another embodiment, the method further comprises introducing the tissue explant back into the organism the tissue was derived from or into another organism under conditions suitable to modulate the expression of the BACE gene in that organism.

In another embodiment, the invention features a method of modulating the expression of more than one BACE gene in a tissue explant comprising: (a) synthesizing siNA molecules of the invention, which can be chemically-modified, wherein the siNA comprises a single stranded sequence having complementarity to RNA of the BACE gene; and (b) introducing the siNA molecules into a cell of the tissue explant derived from a particular organism under conditions suitable to modulate the expression of the BACE genes in the tissue explant. In another embodiment, the method further comprises introducing the tissue explant back into the organism the tissue was derived from or into another organism under conditions suitable to modulate the expression of the BACE genes in that organism.

In one embodiment, the invention features a method of modulating the expression of a BACE gene in an organism comprising: (a) synthesizing a siNA molecule of the invention, which can be chemically-modified, wherein the siNA comprises a single stranded sequence having complementarity to RNA of the BACE gene; and (b) introducing the siNA molecule into the organism under conditions suitable to modulate the expression of the BACE gene in the organism.

In another embodiment, the invention features a method of modulating the expression of more than one BACE gene in an organism comprising: (a) synthesizing siNA molecules of the invention, which can be chemically-modified, wherein the siNA comprises a single stranded sequence having complementarity to RNA of the BACE gene; and (b) introducing the siNA molecules into the organism under conditions suitable to modulate the expression of the BACE genes in the organism.

In one embodiment, the invention features a method of modulating the expression of a BACE gene in an organism comprising contacting the organism with a siNA molecule of the invention under conditions suitable to modulate the expression of the BACE gene in the organism.

In another embodiment, the invention features a method of modulating the expression of more than one BACE gene in an organism comprising contacting the organism with one or more siNA molecules of the invention under conditions suitable to modulate the expression of the BACE genes in the organism.

The siNA molecules of the invention can be designed to inhibit target (BACE) gene expression through RNAi targeting of a variety of RNA molecules. In one embodiment, the siNA molecules of the invention are used to target various RNAs corresponding to a target gene. Non-limiting examples of such RNAs include messenger RNA (mRNA), alternate RNA splice variants of target gene(s), post-transcriptionally modified RNA of target gene(s), pre-mRNA of target gene(s), and/or RNA templates. If alternate splicing produces a family of transcripts that are distinguished by usage of appropriate exons, the instant invention can be used to inhibit gene expression through the appropriate exons to specifically inhibit or to distinguish among the functions of gene family members. For example, a protein that contains an alternatively spliced transmembrane domain can be expressed in both membrane bound and secreted forms. Use of the invention to target the exon containing the transmembrane domain can be used to determine the functional consequences of pharmaceutical targeting of membrane bound as opposed to the secreted form of the protein. Non-limiting examples of applications of the invention relating to targeting these RNA molecules include therapeutic pharmaceutical applications, pharmaceutical discovery applications, molecular diagnostic and gene function applications, and gene mapping, for example using single nucleotide polymorphism mapping with siNA molecules of the invention. Such applications can be implemented using known gene sequences or from partial sequences available from an expressed sequence tag (EST).

In another embodiment, the siNA molecules of the invention are used to target conserved sequences corresponding to a gene family or gene families such as BACE family genes. As such, siNA molecules targeting multiple BACE targets can provide increased therapeutic effect. In addition, siNA can be used to characterize pathways of gene function in a variety of applications. For example, the present invention can be used to inhibit the activity of target gene(s) in a pathway to determine the function of uncharacterized gene(s) in gene function analysis, mRNA function analysis, or translational analysis. The invention can be used to determine potential target gene pathways involved in various diseases and conditions toward pharmaceutical development. The invention can be used to understand pathways of gene expression involved in, for example, the progression and/or maintenance of Alzheimer's disease.

In one embodiment, siNA molecule(s) and/or methods of the invention are used to inhibit the expression of gene(s) that encode RNA referred to by Genbank Accession, for example BACE genes encoding RNA sequence(s) referred to herein by Genbank Accession number, for example Genbank Accession Nos. shown in Table I.

In one embodiment, the invention features a method comprising: (a) generating a library of siNA conshucts having a predetermined complexity; and (b) assaying the siNA constructs of (a) above, under conditions suitable to determine RNAi target sites within the target RNA sequence. In another embodiment, the siNA molecules of (a) have strands of a fixed length, for example, about 23 nucleotides in length. In yet another embodiment, the siNA molecules of (a) are of differing length, for example having strands of about 19 to about 25 (*e.g.,* about 19, 20, 21, 22, 23, 24, or 25) nucleotides in length. In one embodiment, the assay can comprise a reconstituted *in vitro* siNA assay as described herein. In another embodiment, the assay can comprise a cell culture system in which target RNA is expressed. In another embodiment, fragments of target RNA are analyzed for detectable levels of cleavage, for example by gel electrophoresis, northern blot analysis, or RNAse protection assays, to determine the most suitable target site(s) within the target RNA sequence. The target RNA sequence can be obtained as is known in the art, for example, by cloning and/or transcription for *in vitro* systems, and by cellular expression in *in vivo* systems.

In one embodiment, the invention features a method comprising: (a) generating a randomized library of siNA constructs having a predetermined complexity, such as of 4^{N}, where N represents the number of base paired nucleotides in each of the siNA construct strands (*eg*. for a siNA construct having 21 nucleotide sense and antisense strands with 19 base pairs, the complexity would be 4¹⁹); and (b) assaying the siNA constructs of (a) above, under conditions suitable to determine RNAi target sites within the target BACE RNA sequence. In another embodiment, the siNA molecules of (a) have strands of a fixed length, for example about 23 nucleotides in length. In yet another embodiment, the siNA molecules of (a) are of differing length, for example having strands of about 19 to about 25 (*e.g.,* about 19, 20, 21, 22, 23, 24, or 25) nucleotides in length. In one embodiment, the assay can comprise a reconstituted *in vitro* siNA assay as described in Example 7 herein. In another embodiment, the assay can comprise a cell culture system in which target RNA is expressed. In another embodiment, fragments of BACE RNA are analyzed for detectable levels of cleavage, for example by gel electrophoresis, northern blot analysis, or RNAse protection assays, to determine the most suitable target site(s) within the target BACE RNA sequence. The target BACE RNA sequence can be obtained as is known in the art, for example, by cloning and/or transcription for *in vitro* systems, and by cellular expression in in *vivo* systems.

In another embodiment, the invention features a method comprising: (a) analyzing the sequence of a RNA target encoded by a target gene; (b) synthesizing one or more sets of siNA molecules having sequence complementary to one or more regions of the RNA of (a); and (c) assaying the siNA molecules of (b) under conditions suitable to determine RNAi targets within the target RNA sequence. In one embodiment, the siNA molecules of (b) have strands of a fixed length, for example about 23 nucleotides in length. In another embodiment, the siNA molecules of (b) are of differing length, for example having strands of about 19 to about 25 (*e.g*., about 19, 20, 21, 22, 23, 24, or 25) nucleotides in length. In one embodiment, the assay can comprise a reconstituted *in vitro* siNA assay as described herein. In another embodiment, the assay can comprise a cell culture system in which target RNA is expressed. Fragments of target RN A are analyzed for detectable levels of cleavage, for example by gel electrophoresis, northern blot analysis, or RNAse protection assays, to determine the most suitable target site(s) within the target RNA sequence. The target RNA sequence can be obtained as is known in the art, for example, by cloning and/or transcription for *in vitro* systems, and by expression in *in vivo* systems.

By "target site" is meant a sequence within a target RNA that is "targeted" for cleavage mediated by a siNA construct which contains sequences within its antisense region that are complenientary to the target sequence.

By "detectable level of cleavage" is meant cleavage of target RNA (and formation of cleaved product RNAs) to an extent sufficient to discern cleavage products above the background of RNAs produced by random degradation of the target RNA. Production of cleavage products from 1-5% of the target RNA is sufficient to detect above the background for most methods of detection.

In one embodiment, the invention features a composition comprising a siNA molecule of the invention, which can be chemically-modified, in a pharmaceutically acceptable carrier or diluent. In another embodiment, the invention features a pharmaceutical composition comprising siNA molecules of the invention, which can be chemically-modified, targeting one or more genes in a pharmaceutically acceptable carrier or diluent. In another embodiment, the invention features a method for treating or preventing a disease or condition in a subject, comprising administering to the subject a composition of the invention under conditions suitable for the treatment or prevention of the disease or condition in the subject, alone or in conjunction with one or more other therapeutic compounds. In yet another embodiment, the invention features a method for reducing or preventing tissue rejection in a subject comprising administering to the subject a composition of the invention under conditions suitable for the reduction or prevention of tissue rejection in the subject.

In another embodiment, the invention features a method for validating a BACE gene target, comprising: (a) synthesizing a siNA molecule of the invention, which can be chemically-modified, wherein one of the siNA strands comprises a sequence complementary to RNA of a BACE target gene; (b) introducing the siNA molecule into a cell, tissue, or organism under conditions suitable for modulating expression of the BACE target gene in the cell, tissue, or organism; and (c) determining the function of the gene by assaying for any phenotypic change in the cell, tissue, or organism.

In another embodiment, the invention features a method for validating a BACE target comprising: (a) synthesizing a siNA molecule of the invention, which can be chemically-modified, wherein one of the siNA strands comprises a sequence complementary to RNA of a BACE target gene; (b) introducing the siNA molecule into a biological system under conditions suitable for modulating expression of the BACE target gene in the biological system; and (c) determining the function of the gene by assaying for any phenotypic change in the biological system.

By "biological system" is meant, material, in a purified or unpurified form, from biological sources, including but not limited to human, animal, plant, insect, bacterial, viral or other sources, wherein the system comprises the components required for RNAi acitivity. The term "biological system" includes, for example, a cell, tissue, or organism, or extract thereof. The term biological system also includes reconstituted RNAi systems that can be used in an *in vitro* setting.

By "phenotypic change" is meant any detectable change to a cell that occurs in response to contact or treatment with a nucleic acid molecule of the invention (e.g., siNA). Such detectable changes include, but are not limited to, changes in shape, size, proliferation, motility, protein expression or RNA expression or other physical or chemical changes as can be assayed by methods known in the art. The detectable change can also include expression of reporter genes/molecules such as Green Florescent Protein (GFP) or various tags that are used to identify an expressed protein or any other cellular component that can be assayed.

In one embodiment, the invention features a kit containing a siNA molecule of the invention, which can be chemically-modified, that can be used to modulate the expression of a BACE target gene in a cell, tissue, or organism. In another embodiment, the invention features a kit containing more than one siNA molecule of the invention, which can be chemically-modified, that can be used to modulate the expression of more than one BACE target gene in a cell, tissue, or organism.

In one embodiment, the invention features a cell containing one or more siNA molecules of the invention, which can be chemically-modified. In another embodiment, the cell containing a siNA molecule of the invention is a manunalian cell. In yet another embodiment, the cell containing a siNA molecule of the invention is a human cell.

In one embodiment, the synthesis of a siNA molecule of the invention, which can be chemically-modified, comprises: (a) synthesis of two complementary strands of the siNA molecule; (b) annealing the two complementary strands together under conditions suitable to obtain a double-stranded siNA molecule. In another embodiment, synthesis of the two complementary strands of the siNA molecule is by solid phase oligonucleotide synthesis. In yet another embodiment, synthesis of the two complementary strands of the siNA molecule is by solid phase tandem oligonucleotide synthesis.

In one embodiment, the invention features a method for synthesizing a siNA duplex molecule comprising: (a) synthesizing a first oligonucleotide sequence strand of the siNA molecule, wherein the first oligonucleotide sequence strand comprises a cleavable linker molecule that can be used as a scaffold for the synthesis of the second oligonucleotide sequence strand of the siNA; (b) synthesizing the second oligonucleotide sequence strand of siNA on the scaffold of the first oligonucleotide sequence strand, wherein the second oligonucleotide sequence strand further comprises a chemical moiety than can be used to purify the siNA duplex; (c) cleaving the linker molecule of (a) under conditions suitable for the two siNA oligonucleotide strands to hybridize and form a stable duplex; and (d) purifying the siNA duplex utilizing the chemical moiety of the second oligonucleotide sequence strand. In one embodiment, cleavage of the linker molecule in (c) above takes place during deprotection of the oligonucleotide, for example under hydrolysis conditions using an alkylamine base such as methylamine. In one embodiment, the method of synthesis comprises solid phase synthesis on a solid support such as controlled pore glass (CPG) or polystyrene, wherein the first sequence of (a) is synthesized on a cleavable linker, such as a succinyl linker, using the solid support as a scaffold. The cleavable linker in (a) used as a scaffold for synthesizing the second strand can comprise similar reactivity as the solid support derivatized linker, such that cleavage of the solid support derivatized linker and the cleavable linker of (a) takes place concomitantly. In another embodiment, the chemical moiety of (b) that can be used to isolate the attached oligonucleotide sequence comprises a trityl group, for example a dimethoxytrityl group, which can be employed in a trityl-on synthesis strategy as described herein. In yet another embodiment, the chemical moiety, such as a dimethoxytrityl group, is removed during purification, for example, using acidic conditions.

In a further embodiment, the method for siNA synthesis is a solution phase synthesis or hybrid phase synthesis wherein both strands of the siNA duplex are synthesized in tandem using a cleavable linker attached to the first sequence which acts a scaffold for synthesis of the second sequence. Cleavage of the linker under conditions suitable for hybridization of the separate siNA sequence strands results in formation of the double-stranded siNA molecule.

In another embodiment, the invention features a method for synthesizing a siNA duplex molecule comprising: (a) synthesizing one oligonucleotide sequence strand of the siNA molecule, wherein the sequence comprises a cleavable linker molecule that can be used as a scaffold for the synthesis of another oligonucleotide sequence; (b) synthesizing a second oligonucleotide sequence having complementarity to the first sequence strand on the scaffold of (a), wherein the second sequence comprises the other strand of the double-stranded siNA molecule and wherein the second sequence further comprises a chemical moiety than can be used to isolate the attached oligonucleotide sequence; (c) purifying the product of (b) utilizing the chemical moiety of the second oligonucleotide sequence strand under conditions suitable for isolating the full-length sequence comprising both siNA oligonucleotide strands connected by the cleavable linker and under conditions suitable for the two siNA oligonucleotide strands to hybridize and form a stable duplex. In one embodiment, cleavage of the linker molecule in (c) above takes place during deprotection of the oligonucleotide, for example under hydrolysis conditions. In another embodiment, cleavage of the linker molecule in (c) above takes place after deprotection of the oligonucleotide. In another embodiment, the method of synthesis comprises solid phase synthesis on a solid support such as controlled pore glass (CPG) or polystyrene, wherein the first sequence of (a) is synthesized on a cleavable linker, such as a succinyl linker, using the solid support as a scaffold. The cleavable linker in (a) used as a scaffold for synthesizing the second strand can comprise similar reactivity or differing reactivity as the solid support derivatized linker, such that cleavage of the solid support derivatized linker and the cleavable linker of (a) takes place either concomitantly or sequentially. In one embodiment, the chemical moiety of (b) that can be used to isolate the attached oligonucleotide sequence comprises a trityl group, for example a dimethoxytrityl group.

In another embodiment, the invention features a method for making a double-stranded siNA molecule in a single synthetic process comprising: (a) synthesizing an oligonucleotide having a first and a second sequence, wherein the first sequence is complementary to the second sequence, and the first oligonucleotide sequence is linked to the second sequence via a cleavable linker, and wherein a terminal 5'-protecting group, for example, a 5'-O-dimethoxytrityl group (5'-O-DMT) remains on the oligonucleotide having the second sequence; (b) deprotecting the oligonucleotide whereby the deprotection results in the cleavage of the linker joining the two oligonucleotide sequences; and (c) purifying the product of (b) under conditions suitable for isolating the double-stranded siNA molecule, for example using a trityl-on synthesis strategy as described herein.

In another embodiment, the method of synthesis of siNA molecules of the invention comprises the teachings of Scaringe *et al.,* US Patent Nos. 5,889,136; 6,008,400; and 6,111,086, incorporated by reference herein in their entirety.

In one embodiment, the invention features siNA constructs that mediate RNAi against a BACE, wherein the siNA construct comprises one or more chemical modifications, for example, one or more chemical modifications having any of Formulae I-VII or any combination thereof that increases the nuclease resistance of the siNA construct.

In another embodiment, the invention features a method for generating siNA molecules with increased nuclease resistance comprising (a) introducing nucleotides having any of Fonnulae I-VII or any combination thereof into a siNA molecule, and (b) assaying the siNA molecule of step (a) under conditions suitable for isolating siNA molecules having increased nuclease resistance.

In one embodiment, the invention features siNA constructs that mediate RNAi against a BACE, wherein the siNA construct comprises one or more chemical modifications described herein that modulates the binding affinity between the sense and antisense strands of the siNA construct.

In another embodiment, the invention features a method for generating siNA molecules with increased binding affinity between the sense and antisense strands of the siNA molecule comprising (a) introducing nucleotides having any of Formulae I-VII or any combination thereof into a siNA molecule, and (b) assaying the siNA molecule of step (a) under conditions suitable for isolating siNA molecules having increased binding affinity between the sense and antisense strands of the siNA molecule.

In one embodiment, the invention features siNA constructs that mediate RNAi against a BACE, wherein the siNA construct comprises one or more chemical modifications described herein that modulates the binding affinity between the antisense strand of the siNA construct and a complementary target RNA sequence within a cell.

In one embodiment, the invention features siNA constructs that mediate RNAi against a BACE, wherein the siNA construct comprises one or more chemical modifications described herein that modulates the binding affinity between the antisense strand of the siNA construct and a complementary target DNA sequence within a cell.

In another embodiment, the invention features a method for generating siNA molecules with increased binding affinity between the antisense strand of the siNA molecule and a complementary target RNA sequence comprising (a) introducing nucleotides having any of Formulae I-VII or any combination thereof into a siNA molecule, and (b) assaying the siNA molecule of step (a) under conditions suitable for isolating siNA molecules having increased binding affinity between the antisense strand of the siNA molecule and a complementary target RNA sequence.

In another embodiment, the invention features a method for generating siNA molecules with increased binding affinity between the antisense strand of the siNA molecule and a complementary target DNA sequence comprising (a) introducing nucleotides having any of Formulae I-VII or any combination thereof into a siNA molecule, and (b) assaying the siNA molecule of step (a) under conditions suitable for isolating siNA molecules having increased binding affinity between the antisense strand of the siNA molecule and a complementary target DNA sequence.

In one embodiment, the invention features siNA constructs that mediate RNAi against a BACE, wherein the siNA construct comprises one or more chemical modifications described herein that modulate the polymerase activity of a cellular polymerase capable of generating additional endogenous siNA molecules having sequence homology to the chemically-modified siNA construct.

In another embodiment, the invention features a method for generating siNA molecules capable of mediating increased polymerase activity of a cellular polymerase capable of generating additional endogenous siNA molecules having sequence homology to a chemically-modified siNA molecule comprising (a) introducing nucleotides having any of Fonnulae I-VII or any combination thereof into a siNA molecule, and (b) assaying the siNA molecule of step (a) under conditions suitable for isolating siNA molecules capable of mediating increased polymerase activity of a cellular polymerase capable of generating additional endogenous siNA molecules having sequence homology to the chemically-modified siNA molecule.

In one embodiment, the invention features chemically-modified siNA constructs that mediate RNAi against a BACE in a cell, wherein the chemical modifications do not significantly effect the interaction of siNA with a target RNA molecule, DNA molecule and/or proteins or other factors that are essential for RNAi in a manner that would decrease the efficacy of RNAi mediated by such siNA constructs.

In another embodiment, the invention features a method for generating siNA molecules with improved RNAi activity against BACE comprising (a) introducing nucleotides having any of Formulae I-VII or any combination thereof into a siNA molecule, and (b) assaying the siNA molecule of step (a) under conditions suitable for isolating siNA molecules having improved RNAi activity.

In yet another embodiment, the invention features a method for generating siNA molecules with improved RNAi activity against a BACE target RNA comprising (a) introducing nucleotides having any of Formulae I-VII or any combination thereof into a siNA molecule, and (b) assaying the siNA molecule of step (a) under conditions suitable for isolating siNA molecules having improved RNAi activity against the target RNA.

In yet another embodiment, the invention features a method for generating siNA molecules with improved RNAi activity against a BACE target DNA comprising (a) introducing nucleotides having any of Formulae I-VII or any combination thereof into a siNA molecule, and (b) assaying the siNA molecule of step (a) under conditions suitable for isolating siNA molecules having improved RNAi activity against the target DNA.

In one embodiment, the invention features siNA constructs that mediate RNAi against a BACE, wherein the siNA construct comprises one or more chemical modifications described herein that modulates the cellular uptake of the siNA construct.

In another embodiment, the invention features a method for generating siNA molecules against BACE with improved cellular uptake comprising (a) introducing nucleotides having any of Fommlae I-VII or any combination thereof into a siNA molecule, and (b) assaying the siNA molecule of step (a) under conditions suitable for isolating siNA molecules having improved cellular uptake.

In one embodiment, the invention features siNA constructs that mediate RNAi against a BACE, wherein the siNA construct comprises one or more chemical modifications described herein that increases the bioavailability of the siNA construct, for example, by attaching polymeric conjugates such as polyethyleneglycol or equivalent conjugates that improve the pharmacokinetics of the siNA construct, or by attaching conjugates that target specific tissue types or cell types *in vivo.* Non-limiting examples of such conjugates are described in Vargeese et al., U.S. Serial No. 10/201,394 incorporated by reference herein.

In one embodiment, the invention features a method for generating siNA molecules of the invention with improved bioavailability, comprising (a) introducing a conjugate into the structure of a siNA molecule, and (b) assaying the siNA molecule of step (a) under conditions suitable for isolating siNA molecules having improved bioavailability. Such conjugates can include ligands for cellular receptors, such as peptides derived from naturally occurring protein ligands; protein localization sequences, including cellular ZIP code sequences; antibodies; nucleic acid aptamers; vitamins and other co-factors, such as folate and N-acetylgalactosamine; polymers, such as polyethyleneglycol (PEG); phospholipids; polyamines, such as spermine or spermidine; and others.

In another embodiment, the invention features a method for generating siNA molecules of the invention with improved bioavailability comprising (a) introducing an excipient formulation to a siNA molecule, and (b) assaying the siNA molecule of step (a) under conditions suitable for isolating siNA molecules having improved bioavailability. Such excipients include polymers such as cyclodextrins, lipids, cationic lipids, polyamines, phospholipids, and others.

In another embodiment, the invention features a method for generating siNA molecules of the invention with improved bioavailability comprising (a) introducing nucleotides having any of Formulae I-VII or any combination thereof into a siNA molecule, and (b) assaying the siNA molecule of step (a) under conditions suitable for isolating siNA molecules having improved bioavailability.

In another embodiment, polyethylene glycol (PEG) can be covalently attached to siNA compounds of the present invention. The attached PEG can be any molecular weight, preferably from about 2,000 to about 50,000 daltons (Da).

The present invention can be used alone or as a component of a kit having at least one of the reagents necessary to carry out the *in vitro* or *in vivo* introduction of RNA to test samples and/or subjects. For example, preferred components of the kit include the siNA and a vehicle that promotes introduction of the siNA. Such a kit can also include instructions to allow a user of the kit to practice the invention.

The term "short interfering nucleic acid", "siNA", "short interfering RNA", "siRNA", "short interfering nucleic acid molecule", "short interfering oligonucleotide molecule", or "chemically-modified short interfering nucleic acid molecule" as used herein refers to any nucleic acid molecule capable of mediating RNA interference ("RNAi") or gene silencing in a sequence-specific manner; see for example Bass, 2001, Nature, 411, 428-429; Elbashir et al., 2001, Nature, 411, 494-498; and Kreutzer et al., International PCT Publication No. WO 00/44895; Zernicka-Goetz et al., International PCT Publication No. WO 01/36646; Fire, International PCT Publication No. WO 99/32619; Plaetinck et al., International PCT Publication No. WO 00/01846; Mello and Fire, International PCT Publication No. WO 01/29058; Deschamps-Depaillette, International PCT Publication No. WO 99/07409; and Li et al., International PCT Publication No. WO 00/44914; Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237; Hutvagner and Zamore, 2002, Science, 297, 2056-60; McManus et al., 2002, RNA, 8, 842-850; Reinhart et al., 2002, Gene & Dev., 16, 1616-1626; and Reinhart & Bartel, 2002, Science, 297, 1831). Non-limiting examples of siNA molecules of the invention are shown in **Figures 4-6,** and **Tables II and III** herein. For example the siNA can be a double-stranded polynucleotide molecule comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof. The siNA can be assembled from two separate oligonucleotides, where one strand is the sense strand and the other is the antisense strand, wherein the antisense and sense strands are self-complementary (i.e. each strand comprises nucleotide sequence that is complementary to nucleotide sequence in the other strand); the antisense strand comprises nucleotide sequence that is complementary to nucleotide sequence in a target nucleic acid molecule or a portion thereof and the sense strand comprises nucleotide sequence corresponding to the target nucleic acid sequence or a portion thereof. Alternatively, the siNA is assembled from a single oligonucleotide, where the self-complementary sense and antisense regions of the siNA are linked by means of a nucleic acid based or non-nucleic acid-based linker(s). The siNA can be a polynucleotide with a hairpin secondary structure, having self-complementary sense and antisense regions, wherein the antisense region comprises nucleotide sequence that is complementary to nucleotide sequence in a separate target nucleic acid molecule or a portion thereof and the sense region having nucleotide sequence corresponding to the target nucleic acid sequence or a portion thereof. The siNA can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either *in vivo* or *in vitro* to generate an active siNA molecule capable of mediating RNAi. The siNA can also comprise a single stranded polynucleotide having nucleotide sequence complementary to nucleotide sequence in a target nucleic acid molecule or a portion thereof (for example, where such siNA molecule does not require the presence within the siNA molecule of nucleotide sequence corresponding to the target nucleic acid sequence or a portion thereof), wherein the single stranded polynucleotide can further comprise a terminal phosphate group, such as a 5'-phosphate (see for example Martinez et al., 2002, Cell., 110, 563-574 and Schwarz et al., 2002, Molecular Cell, 10, 537-568), or 5',3'-diphosphate. In certain embodiments, the siNA molecules of the invention comprise nucleotide sequence that is complementary to nucleotide sequence of a target gene. In another embodiment, the siNA molecule of the invention interacts with nucleotide sequence of a target gene in a manner that causes inhibition of expression of the target gene. As used herein, siNA molecules need not be limited to those molecules containing only RNA, but further encompasses chemically-modified nucleotides and non-nucleotides. In certain embodiments, the short interfering nucleic acid molecules of the invention lack 2'-hydroxy (2'-OH) containing nucleotides. Applicant describes in certain embodiments short interfering nucleic acids that do not require the presence of nucleotides having a 2'-hydroxy group for mediating RNAi and as such, short interfering nucleic acid molecules of the invention optionally do not include any ribonucleotides (e.g., nucleotides having a 2'-OH group). Such siNA molecules that do not require the presence of ribonucleotides within the siNA molecule to support RNAi can however have an attached linker or linkers or other attached or associated groups, moieties, or chains containing one or more nucleotides with 2'-OH groups. Optionally, siNA molecules can comprise ribonucleotides at about 5, 10, 20, 30, 40, or 50% of the nucleotide positions. The modified short interfering nucleic acid molecules of the invention can also be referred to as short interfering modified oligonucleotides "siMON." As used herein, the term siNA is meant to be equivalent to other terms used to describe nucleic acid molecules that are capable of mediating sequence specific RNAi, for example short interfering RNA (siRNA), double-stranded RNA (dsRNA), micro-RNA (miRNA), short hairpin RNA (shRNA), short interfering oligonucleotide, short interfering nucleic acid, short interfering modified oligonucleotide, chemically-modified siRNA, post-transcriptional gene silencing RNA (ptgsRNA), and others. In addition, as used herein, the term RNAi is meant to be equivalent to other terms used to describe sequence specific RNA interference, such as post transcriptional gene silencing, or epigenetics. For example, siNA molecules of the invention can be used to epigenetically silence genes at both the post-transcriptional level or the pre-transcriptional level. In a non-limiting example, epigenetic regulation of gene expression by siNA molecules of the invention can result from siNA mediated modification of chromatin structure to alter gene expression (see, for example, Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237).

By "modulate" is meant that the expression of the gene, or level of RNA molecule or equivalent RNA molecules encoding one or more proteins or protein subunits, or activity of one or more proteins or protein subunits is up regulated or down regulated, such that expression, level, or activity is greater than or less than that observed in the absence of the modulator. For example, the term "modulate" can mean "inhibit," but the use of the word "modulate" is not limited to this definition.

By "inhibit" it is meant that the activity of a gene expression product or level of RNAs or equivalent RNAs encoding one or more gene products is reduced below that observed in the absence of the nucleic acid molecule of the invention. In one embodiment, inhibition with a siNA molecule preferably is below that level observed in the presence of an inactive or attenuated molecule that is unable to mediate an RNAi response. In another embodiment, inhibition of gene expression with the siNA molecule of the instant invention is greater in the presence of the siNA molecule than in its absence.

By "gene" or "target gene" is meant, a nucleic acid that encodes an RNA, for example, nucleic acid sequences including, but not limited to, structural genes encoding a polypeptide. The target gene can be a gene derived from a cell, an endogenous gene, a transgene, or exogenous genes such as genes of a pathogen, for example a virus, which is present in the cell after infection thereof. The cell containing the target gene can be derived from or contained in any organism, for example a plant, animal, protozoan, virus, bacterium, or fungus. Non-limiting examples of plants include monocots, dicots, or gymnosperms. Non-limiting examples of animals include vertebrates or invertebrates. Non-limiting examples of fungi include molds or yeasts.

By "BACE" or "beta secretase" as used herein is meant, any protein, peptide, or polypeptide, having beta-secretase activity, such as that involved in generating beta-amyloid. The term BACE also refers to nucleotide sequences that encode BACE protein.

By "APP" or "amyloid precurson protein" as used herein is meant, any protein, peptide, or polypeptide, that is processed to generate beta-amyloid. The term APP also refers to nucleotide sequences that encode amyloid precurson protein.

By "presenillin" or "PS", eg "PS-1" or "PS-2" as used herein is meant, any protein, peptide, or polypeptide having gamma-secretase activity, such as that involved in generating beta-amyloid. The term presenillin also refers to nucleotide sequences that encode presenillin protein, eg PS-1 or PS-2.

By "PIN-1" as used herein is meant, any protein, peptide, or polypeptide having peptidyl-prolyl cis/trans isomerase activity, such as that involved in the development of Neurofibrillary Tangles. The term PIN-1 also refers to nucleotide sequences that encode PIN-1 protein.

By "highly conserved sequence region" is meant, a nucleotide sequence of one or more regions in a target gene does not vary significantly from one generation to the other or from one biological system to the other.

By "sense region" is meant a nucleotide sequence of a siNA molecule having complementarity to an antisense region of the siNA molecule. In addition, the sense region of a siNA molecule can comprise a nucleic acid sequence having homology with a target nucleic acid sequence.

By "antisense region" is meant a nucleotide sequence of a siNA molecule having complementarity to a target nucleic acid sequence. In addition, the antisense region of a siNA molecule can optionally comprise a nucleic acid sequence having complementarity to a sense region of the siNA molecule.

By "target nucleic acid" is meant any nucleic acid sequence whose expression or activity is to be modulated. The target nucleic acid can be DNA or RNA.

By "complementarity" is meant that a nucleic acid can form hydrogen bond(s) with another nucleic acid sequence by either traditional Watson-Crick or other non-traditional types. In reference to the nucleic molecules of the present invention, the binding free energy for a nucleic acid molecule with its complementary sequence is sufficient to allow the relevant function of the nucleic acid to proceed, e.g., RNAi activity. Determination of binding free energies for nucleic acid molecules is well known in the art (see, e.g., Turner et al., 1987, CSH Symp. Qacant. Biol. LII pp.123-133; Frier et al., 1986, Proc. Mat. Acad. Sci. USA 83:9373-9377; Turner et al., 1987, J. Am. Chem. Soc. 109:3783-3785). A percent complementarity indicates the percentage of contiguous residues in a nucleic acid molecule that can form hydrogen bonds (e.g., Watson-Crick base pairing) with a second nucleic acid sequence (*e.g*., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence.

The siRNA molecules of the invention represent a novel therapeutic approach to treat a variety of pathologic neurodegenerative indications and conditions, including Alzheimer's disease, dementia, stroke (CVA), and any other diseases or conditions that are related to the levels of BACE in a cell or tissue, alone or in combination with other therapies. The reduction of BACE expression (specifically BACE RNA levels) and thus reduction in the level of the respective protein relieves, to some extent, the symptoms of the disease or condition.

In one embodiment of the present invention, each sequence of a siNA molecule of the invention is independently about 18 to about 24 nucleotides in length, in specific embodiments about 18, 19, 20, 21, 22, 23, or 24 nucleotides in length. In another embodiment, the siNA duplexes of the invention independently comprise about 17 to about 23 base pairs (e.g., about 17, 18, 19, 20, 21, 22 or 23). In yet another embodiment, siNA molecules of the invention comprising hairpin or circular structures are about 35 to about 55 (e.g., about 35, 40, 45, 50 or 55) nucleotides in length, or about 38 to about 44 (e.g., 38, 39, 40, 41, 42, 43 or 44) nucleotides in length and comprising about 16 to about 22 (e.g., about 16, 17, 18, 19, 20, 21 or 22) base pairs. Exemplary siNA molecules of the invention are shown in **Tables II and III and Figures 4 and 5**. Exemplary synthetic siNA molecules of the invention are shown in **Table III** and/or **Figures 4-5.**

As used herein "cell" is used in its usual biological sense, and does not refer to an entire multicellular organism, e.g., specifically does not refer to a human. The cell can be present in an organism, e.g., birds, plants and mammals such as humans, cows, sheep, apes, monkeys, swine, dogs, and cats. The cell can be prokaryotic (e.g., bacterial cell) or eukaryotic (e.g., mammalian or plant cell). The cell can be of somatic or germ line origin, totipotent or pluripotent, dividing or non-dividing. The cell can also be derived from or can comprise a gamete or embryo, a stem cell, or a fully differentiated cell.

The siNA molecules of the invention are added directly, or can be complexed with cationic lipids, packaged within liposomes, or otherwise delivered to target cells or tissues. The nucleic acid or nucleic acid complexes can be locally administered to relevant tissues *ex vivo,* or *in vivo* through injection, infusion pump or stent, with or without their incorporation in biopolymers. In particular embodiments, the nucleic acid molecules of the invention comprise sequences shown in **Tables II-III** and/or **Figures 4-5**. Examples of such nucleic acid molecules consist essentially of sequences defined in these tables and figures. Furthermore, the chemically modified constructs described in **Table IV** can be applied to any siNA sequence of the invention.

In another aspect, the invention provides mammalian cells containing one or more siNA molecules of this invention. The one or more siNA molecules can independently be targeted to the same or different sites.

By "RNA" is meant a molecule comprising at least one ribonucleotide residue. By "ribonucleotide" is meant a nucleotide with a hydroxyl group at the 2' position of a β-D-ribo-furanose moiety. The terms include double-stranded RNA, single-stranded RNA, isolated RNA such as partially purified RNA, essentially pure RNA, synthetic RNA, recombinantly produced RNA, as well as altered RNA that differs from naturally occurring RNA by the addition, deletion, substitution and/or alteration of one or more nucleotides. Such alterations can include addition of non-nucleotide material, such as to the end(s) of the siNA or internally, for example at one or more nucleotides of the RNA. Nucleotides in the RNA molecules of the instant invention can also comprise non-standard nucleotides, such as non-naturally occurring nucleotides or chemically synthesized nucleotides or deoxynucleotides. These altered RNAs can be referred to as analogs or analogs of naturally-occurring RNA.

By "subject" is meant an organism, which is a donor or recipient of explanted cells or the cells themselves. "Subject" also refers to an organism to which the nucleic acid molecules of the invention can be administered. In one embodiment, a subject is a mammal or mammalian cells. In another embodiment, a subject is a human or human cells.

The term "phosphorothioate" as used herein refers to an internucleotide linkage having Formula I, wherein Z and/or W comprise a sulfur atom. Hence, the term phosphorothioate refers to both phosphorothioate and phosphorodithioate internucleotide linkages.

The term "universal base" as used herein refers to nucleotide base analogs that form base pairs with each of the natural DNA/RIVA bases with little discrimination between them. Non-limiting examples of universal bases include C-phenyl, C-naphthyl and other aromatic derivatives, inosine, azole carboxamides, and nitroazole derivatives such as 3-nitropyrrole, 4-nitroindole, 5-nitroindole, and 6-nitroindole as known in the art (see for example Loakes, 2001, Nucleic Acids Research, 29, 3437-2447).

The term "acyclic nucleotide" as used herein refers to any nucleotide having an acyclic ribose sugar, for example where any of the ribose carbons (C1, C2, C3, C4, or C5), are independently or in combination absent from the nucleotide.

The nucleic acid molecules of the instant invention, individually, or in combination or in conjunction with other drugs, can be used to treat diseases or conditions discussed herein (e.g., Alzheimer's disease and other nuerodegenerative conditions). For example, to treat a particular disease or condition, the siNA molecules can be administered to a subject or can be administered to other appropriate cells evident to those skilled in the art, individually or in combination with one or more drugs under conditions suitable for the treatment.

In a further embodiment, the siNA molecules can be used in combination with other known treatments to treat conditions or diseases discussed above. For example, the described molecules could be used in combination with one or more known therapeutic agents to treat a disease or condition. Non-limiting examples of other therapeutic agents that can be readily combined with a siNA molecule of the invention are enzymatic nucleic acid molecules, allosteric nucleic acid molecules, antisense, decoy, or aptamer nucleic acid molecules, antibodies such as monoclonal antibodies, small molecules, and other organic and/or inorganic compounds including metals, salts and ions.

In one embodiment, the invention features an expression vector comprising a nucleic acid sequence encoding at least one siNA molecule of the invention, in a manner which allows expression of the siNA molecule. For example, the vector can contain sequence(s) encoding both strands of a siNA molecule comprising a duplex. The vector can also contain sequence(s) encoding a single nucleic acid molecule that is self-complementary and thus forms a siNA molecule. Non-limiting examples of such expression vectors are described in Paul et al., 2002, Nature Biotechnology, 19, 505; Miyagishi and Taira, 2002, Nature Biotechnology, 19, 497; Lee et al., 2002, Nature Biotechnology, 19, 500; and Novina et al., 2002, Nature Medicine, advance online publication doi:10.1035/nm725.

In another embodiment, the invention features a mammalian cell, for example, a human cell, including an expression vector of the invention.

In yet another embodiment, the expression vector of the invention comprises a sequence for a siNA molecule having complementarity to a RNA molecule referred to by a Genbank Accession numbers, for example Genbank Accession Nos. shown in Table I.

In one embodiment, an expression vector of the invention comprises a nucleic acid sequence encoding two or more siNA molecules, which can be the same or different.

In another aspect of the invention, siNA molecules that interact with target RNA molecules and down-regulate gene encoding target RNA molecules (for example target RNA molecules referred to by Genbank Accession numbers herein) are expressed from transcription units inserted into DNA or RNA vectors. The recombinant vectors can be DNA plasmids or viral vectors. siNA expressing viral vectors can be constructed based on, but not limited to, adeno-associated virus, retrovirus, adenovirus, or alphavirus. The recombinant vectors capable of expressing the siNA molecules can be delivered as described herein, and persist in target cells. Alternatively, viral vectors can be used that provide for transient expression of siNA molecules. Such vectors can be repeatedly administered as necessary. Once expressed, the siNA molecules bind and down-regulate gene function or expression via RNA interference (RNAi). Delivery of siNA expressing vectors can be systemic, such as by intravenous or intramuscular administration, by administration to target cells ex-planted from a subject followed by reintroduction into the subject, or by any other means that would allow for introduction into the desired target cell.

By "vectors" is meant any nucleic acid- and/or viral-based technique used to deliver a desired nucleic acid.

Other features and advantages of the invention will be apparent from the following description of the preferred embodiments thereof, and from the claims.

### BRIEF DESCRIPTION OF THE DRAWINGS

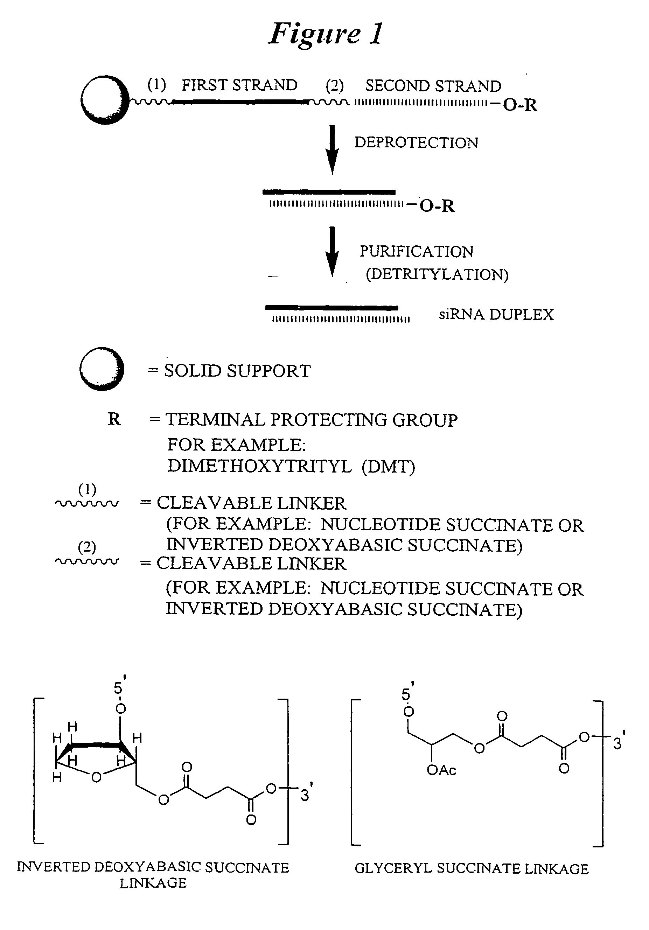

**Figure 1** shows a non-limiting example of a scheme for the synthesis of siNA molecules. The complementary siNA sequence strands, strand 1 and strand 2, are synthesized in tandem and are connected by a cleavable linkage, such as a nucleotide succinate or abasic succinate, which can be the same or different from the cleavable linker used for solid phase synthesis on a solid support. The synthesis can be either solid phase or solution phase, in the example shown, the synthesis is a solid phase synthesis. The synthesis is performed such that a protecting group, such as a dimethoxytrityl group, remains intact on the terminal nucleotide of the tandem oligonucleotide. Upon cleavage and deprotection of the oligonucleotide, the two siNA strands spontaneously hybridize to form a siNA duplex, which allows the purification of the duplex by utilizing the properties of the terminal protecting group, for example by applying a trityl on purification method wherein only duplexes/oligonucleotides with the terminal protecting group are isolated.

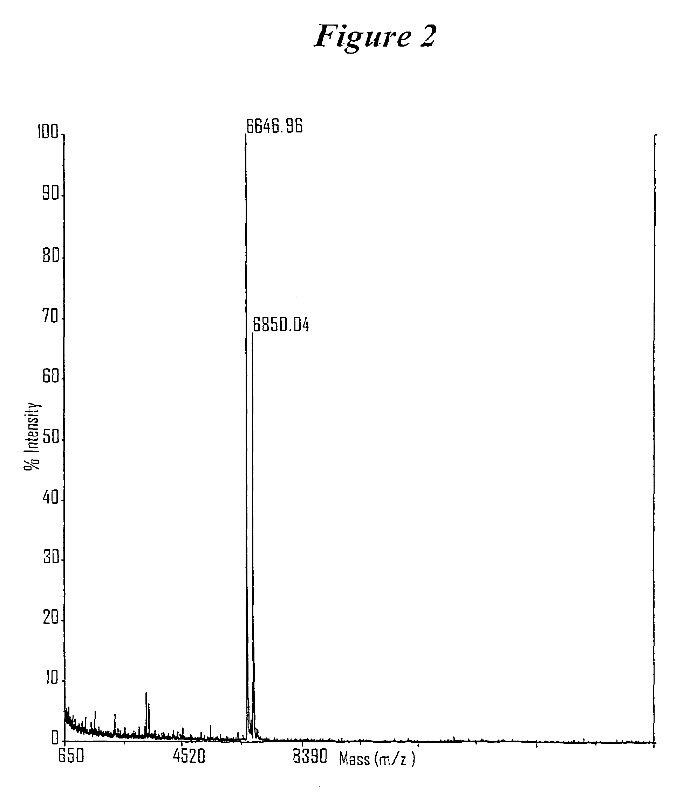

**Figure 2** shows a MALDI-TOV mass spectrum of a purified siNA duplex synthesized by a method of the invention. The two peaks shown correspond to the predicted mass of the separate siNA sequence strands. This result demonstrates that the siNA duplex generated from tandem synthesis can be purified as a single entity using a simple trityl-on purification methodology.

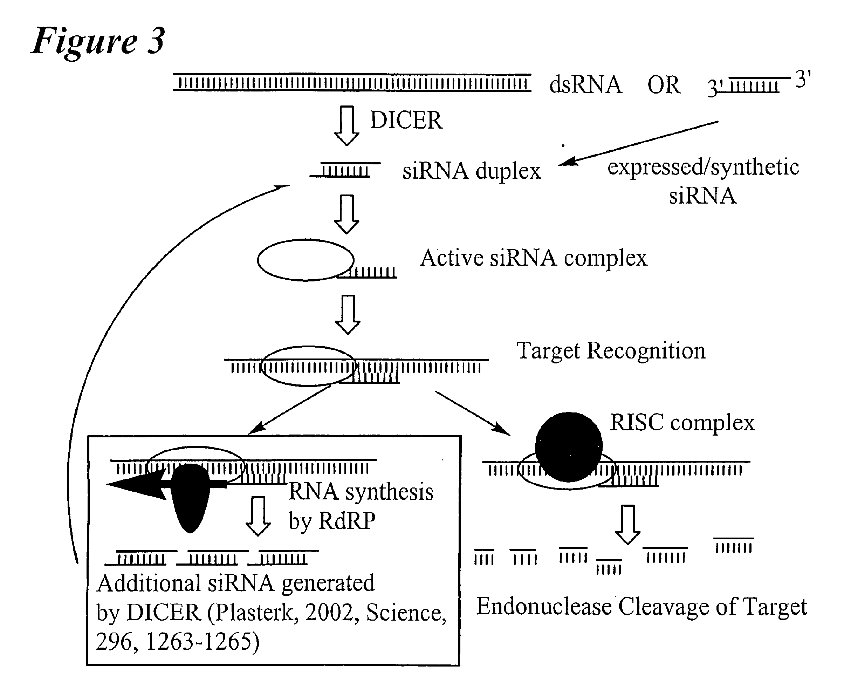

**Figure 3** shows a non-limiting proposed mechanistic representation of target RNA degradation involved in RNAi. Double-stranded RNA (dsRNA), which is generated by RNA-dependent RNA polymerase (RdRP) from foreign single-stranded RNA, for example viral, transposon, or other exogenous RNA, activates the DICER enzyme that in turn generates siNA duplexes. Alternately, synthetic or expressed siNA can be introduced directly into a cell by appropriate means. An active siNA complex fomis which recognizes a target RNA, resulting in degradation of the target RNA by the RISC endonuclease complex or in the synthesis of additional RNA by RNA-dependent RNA polymerase (RdRP), which can activate DICER and result in additional siNA molecules, thereby amplifying the RNAi response.
**Figure 4A-F** shows non-limiting examples of chemically-modified siNA constructs of the present invention. In the figure, N stands for any nucleotide (adenosine, guanosine, cytosine, uridine, or optionally thymidine, for example thymidine can be substituted in the overhanging regions designated by parenthesis (N N). Various modifications are shown for the sense and antisense strands of the siNA constructs.
**Figure 4A:** The sense strand comprises 21 nucleotides having four phosphorothioate 5'- and 3'-terminal internucleotide linkages, wherein the two terminal 3'-nucleotides are optionally base paired and wherein all pyrimidine nucleotides that may be present are 2'-O-methyl or 2'-deoxy-2'-fluoro modified nucleotides except for (N N) nucleotides, which can comprise ribonucleotides, deoxynucleotides, universal bases, or other chemical modifications described herein. The antisense strand comprises 21 nucleotides, optionally having a 3'-terminal glyceryl moiety and wherein the two terminal 3'-nucleotides are optionally complementary to the target RNA sequence, and having one 3'-terminal phosphorothioate internucleotide linkage and four 5'-terminal phosphorothioate internucleotide linkages and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein.
**Figure 4B:** The sense strand comprises 21 nucleotides wherein the two terminal 3'-nucleotides are optionally base paired and wherein all pyrimidine nucleotides that may be present are 2'-O-methyl or 2'-deoxy-2'-fluoro modified nucleotides except for (N N) nucleotides, which can comprise ribonucleotides, deoxynucleotides, universal bases, or other chemical modifications described herein. The antisense strand comprises 21 nucleotides, optionally having a 3'-terminal glyceryl moiety and wherein the two terminal 3'-nucleotides are optionally complementary to the target RNA sequence, and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein.
**Figure 4C:** The sense strand comprises 21 nucleotides having 5'- and 3'- terminal cap moieties wherein the two terminal 3'-nucleotides are optionally base paired and wherein all pyrimidine nucleotides that may be present are 2'-O-methyl or 2'-deoxy-2'-fluoro modified nucleotides except for (N N) nucleotides, which can comprise ribonucleotides, deoxynucleotides, universal bases, or other chemical modifications described herein. The antisense strand comprises 21 nucleotides, optionally having a 3'-terminal glyceryl moiety and wherein the two terminal 3'-nucleotides are optionally complementary to the target RNA sequence, and having one 3'-terminal phosphorothioate internucleotide linkage and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein.
**Figure 4D**: The sense strand comprises 21 nucleotides having 5'- and 3'- terminal cap moieties wherein the two terminal 3'-nucleotides are optionally base paired and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein and wherein and all purine nucleotides that may be present are 2'-deoxy nucleotides. The antisense strand comprises 21 nucleotides, optionally having a 3'-terminal glyceryl moiety and wherein the two terminal 3'-nucleotides are optionally complementary to the target RNA sequence, and having one 3'-terminal phosphorothioate internucleotide linkage and wherein all pyrimidine nucleotides that may be present are 2'-deoxy-2'-fluoro modified nucleotides and all purine nucleotides that may be present are 2'-O-methyl modified nucleotides except for (N N) nucleotides, which can comprise ribonucleotides, deoxynucleotides, universal bases, or other chemical modifications described herein.
**Figure 4E:** The sense strand comprises 21 nucleotides having 5'- and 3'- terminal cap moieties wherein the two terminal 3'-nucleotides are optionally base paired and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein. The antisense strand comprises 21 nucleotides, optionally having a 3'-terminal glyceryl moiety and wherein the two terminal 3'-nucleotides are optionally complementary to the target RNA sequence, and wherein all pyrimidine nucleotides that may be present are 2'-deoxy-2'-fluoro modified nucleotides and all purine nucleotides that may be present are 2'-O-methyl modified nucleotides except for (N N) nucleotides, which can comprise ribonucleotides, deoxynucleotides, universal bases, or other chemical modifications described herein.
**Figure 4F:** The sense strand comprises 21 nucleotides having 5'- and 3'- terminal cap moieties wherein the two temiinal 3'-nucleotides are optionally base paired and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein. The antisense strand comprises 21 nucleotides, optionally having a 3'-terminal glyceryl moiety and wherein the two terminal 3'-nucleotides are optionally complementary to the target RNA sequence, and having one 3'-terminal phosphorothioate internucleotide linkage and wherein all pyrimidine nucleotides that may be present are 2'-deoxy-2'-fluoro modified nucleotides and all purine nucleotides that may be present are 2'-deoxy nucleotides except for (N N) nucleotides, which can comprise ribonucleotides, deoxynucleotides, universal bases, or other chemical modifications described herein. The antisense strand of constructs A-F comprise sequence complementary to any target nucleic acid sequence of the invention.
**Figure 5A-F** shows non-limiting examples of specific chemically-modified siNA sequences of the invention. **A-F** applies the chemical modifications described in **Figure 4A-F** to a BACE siNA sequence.

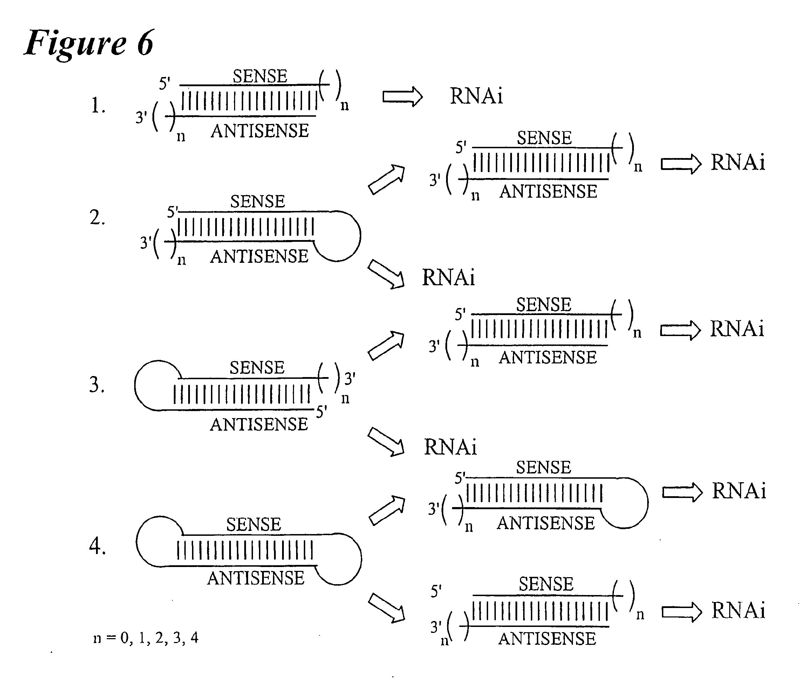

**Figure 6** shows non-limiting examples of different siNA constructs of the invention. The examples shown (constructs 1, 2, and 3) have about 19 representative base pairs; however, different embodiments of the invention include any number of base pairs described herein. Bracketed regions represent nucleotide overhangs, for example comprising about 1, 2, 3, or 4 nucleotides in length, preferably about 2 nucleotides. Constructs 1 and 2 can be used independently for RNAi activity. Construct 2 can comprise a polynucleotide or non-nucleotide linker, which can optionally be designed as a biodegradable linker. In one embodiment, the loop structure shown in construct 2 can comprise a biodegradable linker that results in the formation of construct 1 *in vivo* and/or *in vitro.* In another example, construct 3 can be used to generate construct 2 under the same principle wherein a linker is used to generate the active siNA construct 2 *in vivo* and/or *in vitro,* which can optionally utilize another biodegradable linker to generate the active siNA construct 1 *in vivo* and/or *in vitro.* As such, the stability and/or activity of the siNA constructs can be modulated based on the design of the siNA construct for use *in vivo* or *in vitro* and/or *in vitro.*

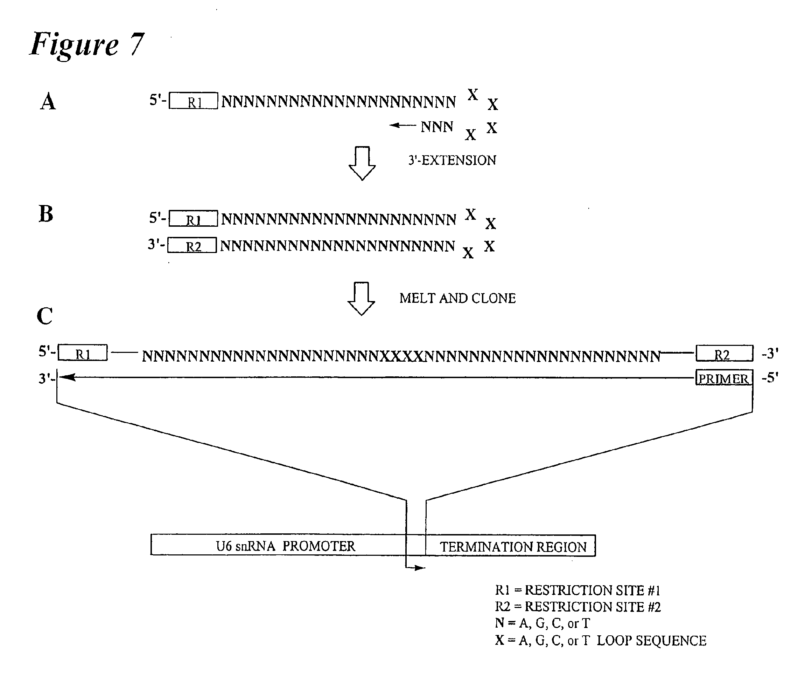

**Figure 7A-C** is a diagrammatic representation of a scheme utilized in generating an expression cassette to generate siNA hairpin constructs.
**Figure 7A:** A DNA oligomer is synthesized with a 5'-restriction site (R1) sequence followed by a region having sequence identical (sense region of siNA) to a predetermined BACE target sequence, wherein the sense region comprises, for example, about 19, 20, 21, or 22 nucleotides (N) in length, which is followed by a loop sequence of defined sequence (X), comprising, for example, about 3 to about 10 nucleotides.
**Figure 7B:** The synthetic construct is then extended by DNA polymerase to generate a hairpin structure having self-complementary sequence that will result in a siNA transcript having specificity for a BACE target sequence and having self-complementary sense and antisense regions.
**Figure 7C:** The construct is heated (for example to about 95°C) to linearize the sequence, thus allowing extension of a complementary second DNA strand using a primer to the 3'-restriction sequence of the first strand. The double-stranded DNA is then inserted into an appropriate vector for expression in cells. The construct can be designed such that a 3'-terminal nucleotide overhang results from the transcription, for example by engineering restriction sites and/or utilizing a poly-U termination region as described in Paul et al., 2002, Nature Biotechnology, 29, 505-508.
**Figure 8A-C** is a diagrammatic representation of a scheme utilized in generating an expression cassette to generate double-stranded siNA constructs.
**Figure 8A:** A DNA oligomer is synthesized with a 5'-restriction (R1) site sequence followed by a region having sequence identical (sense region of siNA) to a predetermined BACE target sequence, wherein the sense region comprises, for example, about 19, 20, 21, or 22 nucleotides (N) in length, and which is followed by a 3'-restriction site (R2) which is adjacent to a loop sequence of defined sequence (X).
**Figure 8B:** The synthetic construct is then extended by DNA polymerase to generate a hairpin structure having self-complementary sequence.
**Figure 8C:** The construct is processed by restriction enzymes specific to R1 and R2 to generate a double-stranded DNA which is then inserted into an appropriate vector for expression in cells. The transcription cassette is designed such that a U6 promoter region flancs each side of the dsDNA which generates the separate sense and antisense strands of the siNA. Poly T termination sequences can be added to the constructs to generate U overhangs in the resulting transcript.
**Figure 9A-E** is a diagrammatic representation of a method used to determine target sites for siNA mediated RNAi within a particular target nucleic acid sequence, such as messenger RNA.

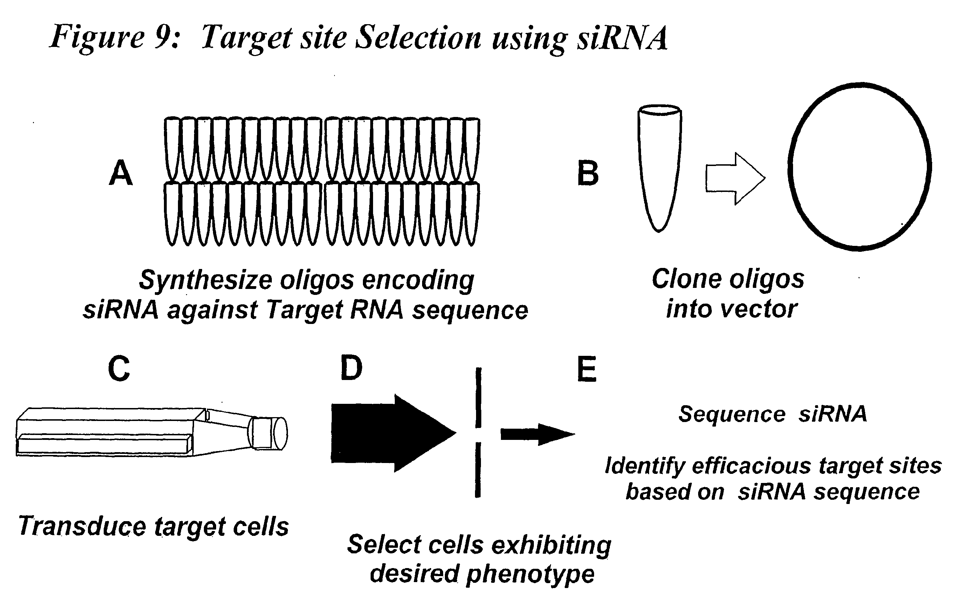

**Figure 9A:** A pool of siNA oligonucleotides are synthesized wherein the antisense region of the siNA constructs has complementarity to target sites across the target nucleic acid sequence, and wherein the sense region comprises sequence complementary to the antisense region of the siNA.
**Figure 9B&C: (Figure 9B)** The sequences are pooled and are inserted into vectors such that **(Figure 9C)** transfection of a vector into cells results in the expression of the siNA.
**Figure 9D:** Cells are sorted based on phenotypic change that is associated with modulation of the target nucleic acid sequence.
**Figure 9E:** The siNA is isolated from the sorted cells and is sequenced to identify efficacious target sites within the target nucleic acid sequence.
**Figure 10** shows non-limiting examples of different stabilization chemistries (1-10) that can be used, for example, to stabilize the 3'-end of siNA sequences of the invention, including **(1)** [3-3']-inverted deoxyribose; **(2)** deoxyribonucleotide; **(3)** [5'-3']-3'-deoxyribonucleotide; **(4)** [5'-3']-ribonucleotide; **(5)** [5'-3]-3'-O-methyl ribonucleotide; **(6)** 3'-glyceryl; **(7)** [3'-5']-3'-deoxyribonucleotide; **(8)** [3'-3']-deoxyribonucleotide; **(9)** [5'-2']-deoxyribonucleotide; and **(10)** [5-3']-dideoxyribonucleotide. In addition to modified and unmodified backbone chemistries indicated in the figure, these chemistries can be combined with different backbone modifications as described herein, for example, backbone modifications having Formula I. In addition, the 2'-deoxy nucleotide shown 5' to the terminal modifications shown can be another modified or unmodified nucleotide or non-nucleotide described herein, for example modifications having any of Formulae I-VII or any combination thereof.

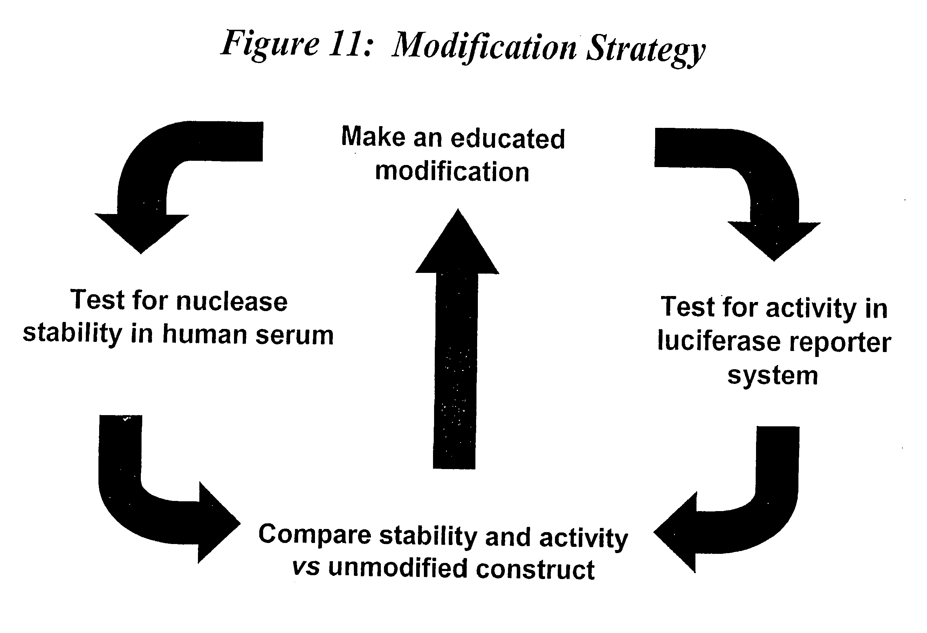

**Figure 11** shows a non-limiting example of a strategy used to identify chemically modified siNA constructs of the invention that are nuclease resistance while preserving the ability to mediate RNAi activity. Chemical modifications are introduced into the siNA construct based on educated design parameters (e.g. introducing 2'-mofications, base modifications, backbone modifications, terminal cap modifications etc). The modified construct in tested in an appropriate system (e.g. human serum for nuclease resistance, shown, or an animal model for PK/delivery parameters). In parallel, the siNA construct is tested for RNAi activity, for example in a cell culture system such as a luciferase reporter assay). Lead siNA constructs are then identified which possess a particular characteristic while maintaining RNAi activity, and can be further modified and assayed once again. This same approach can be used to identify siNA-conjugate molecules with improved pharmacokinetic profiles, delivery, and RNAi activity.

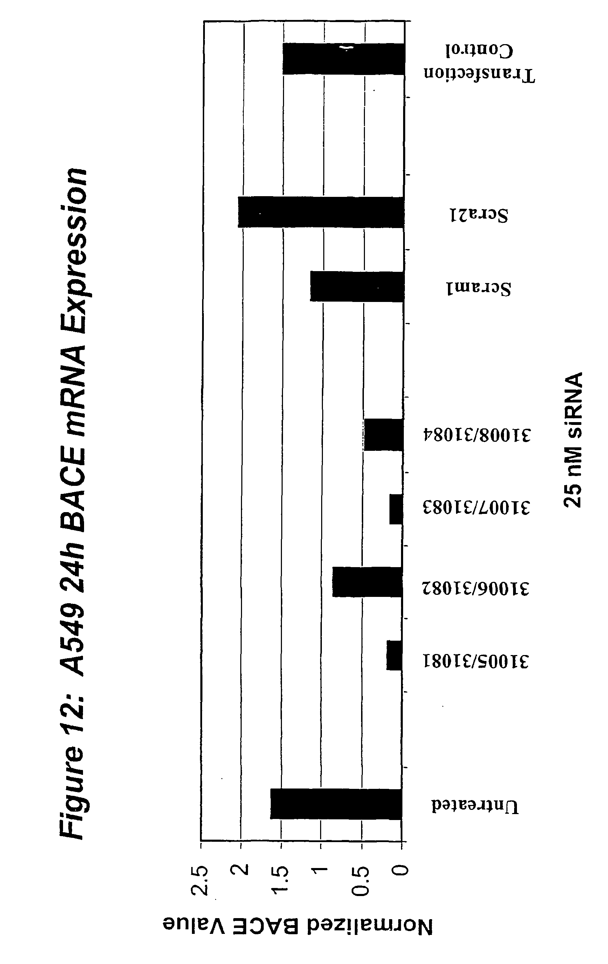

**Figure 12** shows a non-limiting example of reduction of BACE mRNA in A549 cells mediated by siNAs that target BACE mRNA. A549 cells were transfected with 0.25 ug/well of lipid complexed with 25 nM siNA. A screen of siNA constructs comprising ribonucleotides and 3'-terminal dithymidine caps was compared to untreated cells, scrambled siNA control constructs (Scram1 and Scram2), and cells transfected with lipid alone (transfection control). As shown in the figure, all of the siNA constructs show significant reduction of BACE RNA expression.

### DETAILED DESCRIPTION OF THE INVENTION

### Mechanism of action of Nucleic Acid Molecules of the Invention

The discussion that follows discusses the proposed mechanism of RNA interference mediated by short interfering RNA as is presently known, and is not meant to be limiting and is not an admission of prior art. Applicant demonstrates herein that chemically-modified short interfering nucleic acids possess similar or improved capacity to mediate RNAi as do siRNA molecules and are expected to possess improved stability and activity *in vivo;* therefore, this discussion is not meant to be limiting only to siRNA and can be applied to siNA as a whole. By "improved capacity to mediate RNAi" or "improved RNAi activity" is meant to include RNAi activity measured *in vitro* and/or *in vivo* where the RNAi activity is a reflection of both the ability of the siNA to mediate RNAi and the stability of the siNAs of the invention. In this invention, the product of these activities can be increased *in vitro* and/or *in vivo* compared to an all RNA siRNA or a siNA containing a plurality of ribonucleotides. In some cases, the activity or stability of the siNA molecule can be decreased (i.e., less than ten-fold), but the overall activity of the siNA molecule is enhanced *in vitro* and/or *in vivo*.

RNA interference refers to the process of sequence specific post-transcriptional gene silencing in animals mediated by short interfering RNAs (siRNAs) (Fire et al., 1998, Nature, 391, 806). The corresponding process in plants is commonly referred to as s post-transcriptional gene silencing or RNA silencing and is also referred to as quelling in fungi. The process of post-transcriptional gene silencing is thought to be an evolutionarily-conserved cellular defense mechanism used to prevent the expression of foreign genes which is commonly shared by diverse flora and phyla (Fire et al., 1999, Trends Genet., 15, 358). Such protection from foreign gene expression may have evolved in response to the production of double-stranded RNAs (dsRNAs) derived from viral infection or the random integration of transposon elements into a host genome via a cellular response that specifically destroys homologous single-stranded RNA or viral genomic RNA. The presence of dsRNA in cells triggers the RNAi response though a mechanism that has yet to be fully characterized. This mechanism appears to be different from the interferon response that results from dsRNA-mediated activation of protein kinase PKR and 2', 5'-oligoadenylate synthetase resulting in non-specific cleavage of mRNA by ribonuclease L.

The presence of long dsRNAs in cells stimulates the activity of a ribonuclease III enzyme referred to as Dicer. Dicer is involved in the processing of the dsRNA into short pieces of dsRNA known as short interfering RNAs (siRNAs) (Berstein et al., 2001, Nature, 409, 363). Short interfering RNAs derived from Dicer activity are typically about 21 to about 23 nucleotides in length and comprise about 19 base pair duplexes. Dicer has also been implicated in the excision of 21- and 22-nucleotide small temporal RNAs (stRNAs) from precursor RNA of conserved structure that are implicated in translational control (Hutvagner et al., 2001, Science, 293, 834). The RNAi response also features an endonuclease complex containing a siRNA, commonly referred to as an RNA-induced silencing complex (RISC), which mediates cleavage of single-stranded RNA having sequence homologous to the siRNA. Cleavage of the target RNA takes place in the middle of the region complementary to the guide sequence of the siRNA duplex (Elbashir et al., 2001, Genes Dev., 15, 188). In addition, RNA interference can also involve small RNA (e.g., micro-RNA or miRNA) mediated gene silencing, presumably though cellular mechanisms that regulate chromatin structure and thereby prevent transcription of target gene sequences (see for example Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237). As such, siNA molecules of the invention can be used to mediate gene silencing via interaction with RNA transcripts or alternately by interaction with particular gene sequences, wherein such interaction results in gene silencing either at the transcriptional level or post-transcriptional level.

RNAi has been studied in a variety of systems. Fire et al., 1998, Nature, 391, 806, were the first to observe RNAi in *C*. *elegans.* Wianny and Goetz, 1999, Nature Cell Biol., 2, 70, describe RNAi mediated by dsRNA in mouse embryos. Hammond et al., 2000, Nature, 404, 293, describe RNAi in *Drosophila* cells transfected with dsRNA. Elbashir et al., 2001, Nature, 411, 494, describe RNAi induced by introduction of duplexes of synthetic 21-nucleotide RNAs in cultured mammalian cells including human embryonic kidney and HeLa cells. Recent work in Drosophila embryonic lysates (Elbashir et al., 2001, EMBO J., 20, 6877) has revealed certain requirements for siRNA length, structure, chemical composition, and sequence that are essential to mediate efficient RNAi activity. These studies have shown that 21-nucleotide siRNA duplexes are most active when containing 3'-terminal dinucleotide overhangs. Furthermore, complete substitution of one or both siRNA strands with 2'-deoxy (2'-H) or 2'-O-methyl nucleotides abolishes RNAi activity, whereas substitution of the 3'-terminal siRNA overhang nucleotides with 2'-deoxy nucleotides (2'-H) was shown to be tolerated. Single mismatch sequences in the center of the siRNA duplex were also shown to abolish RNAi activity. In addition, these studies also indicate that the position of the cleavage site in the target RNA is defined by the 5'-end of the siRNA guide sequence rather than the 3'-end of the guide sequence (Elbashir et al., 2001, EMBO J., 20, 6877). Other studies have indicated that a 5'-phosphate on the target-complementary strand of a siRNA duplex is required for siRNA activity and that ATP is utilized to maintain the 5'-phosphate moiety on the siRNA (Nykanen et al., 2001, Cell, 107, 309).

### Synthesis of Nucleic acid Molecules

Synthesis of nucleic acids greater than 100 nucleotides in length is difficult using automated methods, and the therapeutic cost of such molecules is prohibitive. In this invention, small nucleic acid motifs ("small" refers to nucleic acid motifs no more than 100 nucleotides in length, preferably no more than 80 nucleotides in length, and most preferably no more than 50 nucleotides in length; *e.g.,* individual siNA oligonucleotide sequences or siNA sequences synthesized in tandem) are preferably used for exogenous delivery. The simple structure of these molecules increases the ability of the nucleic acid to invade targeted regions of protein and/or RNA structure. Exemplary molecules of the instant invention are chemically synthesized, and others can similarly be synthesized.

Oligonucleotides (*e.g*., certain modified oligonucleotides or portions of oligonucleotides lacking ribonucleotides) are synthesized using protocols known in the art, for example as described in Caruthers et al., 1992, Methods in. Enzymology 211, 3-19, Thompson et al., International PCT Publication No. WO 99/54459, Wincott et al., 1995, Nucleic Acids Res. 23, 2677-2684, Wincott et al., 1997, Methods Mol. Bio., 74, 59, Brennan et al., 1998, Biotechnol Bioeng., 61, 33-45, and Brennan, U.S. Pat. No. 6,001,311. All of these references are incorporated herein by reference. The synthesis of oligonucleotides makes use of common nucleic acid protecting and coupling groups, such as dimethoxytrityl at the 5'-end, and phosphoramidites at the 3'-end. In a non-limiting example, small scale syntheses are conducted on a 394 Applied Biosystems, Inc. synthesizer using a 0.2 µmol scale protocol with a 2.5 min coupling step for 2'-O-methylated nucleotides and a 45 sec coupling step for 2'-deoxy nucleotides or 2'-deoxy-2'-fluoro nucleotides. **Table V** outlines the amounts and the contact times of the reagents used in the synthesis cycle. Alternatively, syntheses at the 0.2 µmol scale can be performed on a 96-well plate synthesizer, such as the instrument produced by Protogene (Palo. Alto, CA) with minimal modification to the cycle. A 33-fold excess (60 µL of 0.11 M = 6.6 µmol) of 2'-O-methyl phosphoramidite and a 105-fold excess of S-ethyl tetrazole (60 µL of 0.25 M = 15 µmol) can be used in each coupling cycle of 2'-O-methyl residues relative to polymer-bound 5'-hydroxyl. A 22-fold excess (40 µL of 0.11 M = 4.4 µmol) of deoxy phosphoramidite and a 70-fold excess of S-ethyl tetrazole (40 µL of 0.25 M = 10 µmol) can be used in each coupling cycle of deoxy residues relative to polymer-bound 5'-hydroxyl. Average coupling yields on the 394 Applied Biosystems, Inc. synthesizer, determined by colorimetric quantitation of the trityl fractions, are typically 97.5-99%. Other oligonucleotide synthesis reagents for the 394 Applied Biosystems, Inc. synthesizer include the following: detritylation solution is 3% TCA in methylene chloride (ABI); capping is performed with 16% *N*-methyl imidazole in THF (ABI) and 10% acetic anhydride/10% 2,6-lutidine in THF (ABI); and oxidation solution is 16.9 mM I₂, 49 mM pyridine, 9% water in THF (PERSEPTIVE™). Burdick & Jackson Synthesis Grade acetonitrile is used directly from the reagent bottle. S-Ethyltetrazole solution (0.25 M in acetonitrile) is made up from the solid obtained from American International Chemical, Inc. Alternately, for the introduction of phosphorothioate linkages, Beaucage reagent (3H-1,2-Benzoditluol-3-one 1,1-dioxide, 0.05 M in acetonitrile) is used.

Deprotection of the DNA-based oligonucleotides is performed as follows: the polymer-bound trityl-on oligoribonucleotide is transferred to a 4 mL glass screw top vial and suspended in a solution of 40% aq. methylamine (1 mL) at 65 °C for 10 min. After cooling to -20°C, the supernatant is removed from the polymer support. The support is washed three times with 1.0 mL of EtOH:MeCN:H2O/3:1:1, vortexed and the supernatant is then added to the first supernatant. The combined supernatants, containing the oligoribonucleotide, are dried to a white powder.

The method of synthesis used for RNA including certain siNA molecules of the invention follows the procedure as described in Usman et al., 1987, J. Am. Chem. Soc., 109, 7845; Scaringe et al., 1990, Nucleic Acids Res., 18, 5433; and Wincott et al., 1995, Nucleic Acids Res. 23, 2677-2684 Wincott et al., 1997, Methods Mol. Bio., 74, 59, and makes use of common nucleic acid protecting and coupling groups, such as dimethoxytrityl at the 5'-end, and phosphoramidites at the 3'-end. In a non-limiting example, small scale syntheses are conducted on a 394 Applied Biosystems, Inc. synthesizer using a 0.2 µmol scale protocol with a 7.5 min coupling step for alkylsilyl protected nucleotides and a 2.5 min coupling step for 2'-O-methylated nucleotides. **Table V** outlines the amounts and the contact times of the reagents used in the synthesis cycle. Alternatively, syntheses at the 0.2 µmol scale can be done on a 96-well plate synthesizer, such as the instrument produced by Protogene (Palo Alto, CA) with minimal modification to the cycle. A 33-fold excess (60 µL of 0.11 M = 6.6 µmol) of 2'-O-methyl phosphoramidite and a 75-fold excess of S-ethyl tetrazole (60 µL of 0.25 M = 15 µmol) can be used in each coupling cycle of 2'-O-methyl residues relative to polymer-bound 5'-hydroxyl. A 66-fold excess (120 µL of 0.11 M = 13.2 µmol) of alkylsilyl (ribo) protected phosphoramidite and a 150-fold excess of S-ethyl tetrazole (120 µL of 0.25 M = 30 µmol) can be used in each coupling cycle of ribo residues relative to polymer-bound 5'-hydroxyl. Average coupling yields on the 394 Applied Biosystems, Inc. synthesizer, determined by colorimetric quantitation of the trityl fractions, are typically 97.5-99%. Other oligonucleotide synthesis reagents for the 394 Applied Biosystems, Inc. synthesizer include the following: detritylation solution is 3% TCA in methylene chloride (ABI); capping is performed with 16% *N*-methyl imidazole in THF (ABI) and 10% acetic anhydride/10% 2,6-lutidine in THF (ABI); oxidation solution is 16.9 mM I₂, 49 mM pyridine, 9% water in THF (PERSEPTIVE™). Burdick & Jackson Synthesis Grade acetonitrile is used directly from the reagent bottle. S-Ethyltetrazole solution (0.25 M in acetonitrile) is made up from the solid obtained from American International Chemical, Inc. Alternately, for the introduction of phosphorothioate linkages, Beaucage reagent (3H-1,2-Benzodithiol-3-one 1,1-dioxide0.05 M in acetonitrile) is used.

Deprotection of the RNA is performed using either a two-pot or one-pot protocol. For the two-pot protocol, the polymer-bound trityl-on oligoribonucleotide is transferred to a 4 mL glass screw top vial and suspended in a solution of 40% aq. methylamine (1 mL) at 65°C for 10 min. After cooling to -20°C, the supernatant is removed from the polymer support. The support is washed three times with 1.0 mL of EtOH:MeCN:H2O/3:1:1, vortexed and the supernatant is then added to the first supernatant. The combined supernatants, containing the oligoribonucleotide, are dried to a white powder. The base deprotected oligoribonucleotide is resuspended in anhydrous TEA/HF/NMP solution (300 µL of a solution of 1.5 mL N-methylpyrrolidinone, 750 µL TEA and 1 mL TEA•3HF to provide a 1.4 M HF concentration) and heated to 65 °C. After 1.5 h, the oligomer is quenched with 1.5 M NH₄HCO₃.

Alternatively, for the one-pot protocol, the polymer-bound trityl-on oligoribonucleotide is transferred to a 4 mL glass screw top vial and suspended in a solution of 33% ethanolic methylamine/DMSO: 1/1 (0.8 mL) at 65 °C for 15 min. The vial is brought to rt. TEA•3HF (0.1 mL) is added and the vial is heated at 65 °C for 15 min. The sample is cooled at -20°C and then quenched with 1.5 M NH₄HCO₃.

For purification of the trityl-on oligomers, the quenched NH₄HCO₃ solution is loaded onto a C-18 containing cartridge that had been pre-washed with acetonitrile followed by 50 mM TEAA. After washing the loaded cartridge with water, the RNA is detritylated with 0.5% TFA for 13 min. The cartridge is then washed again with water, salt exchanged with 1 M NaCl and washed with water again. The oligonucleotide is then eluted with 30% acetonitrile.

The average stepwise coupling yields are typically >98% (Wincott et al., 1995 Nucleic Acids Res. 23, 2677-2684). Those of ordinary skill in the art will recognize that the scale of synthesis can be adapted to be larger or smaller than the example described above including but not limited to 96-well format.

Alternatively, the nucleic acid molecules of the present invention can be synthesized separately and joined together post-synthetically, for example, by ligation (Moore et al., 1992, Science 256, 9923; Draper et al., International PCT publication No. WO 93/23569; Shabarova et al., 1991, Nucleic Acids Research 19, 4247; Bellon et al., 1997, Nucleosides & Nucleotides, 16, 951; Bellon et al., 1997, Bioconjugate Chem. 8, 204), or by hybridization following synthesis and/or deprotection.

The siNA molecules of the invention can also be synthesized via a tandem synthesis methodology as described in Example 1 herein, wherein both siNA strands are synthesized as a single contiguous oligonucleotide fragment or strand separated by a cleavable linker which is subsequently cleaved to provide separate siNA fragments or strands that hybridize and permit purification of the siNA duplex. The linker can be a polynucleotide linker or a non-nucleotide linker. The tandem synthesis of siNA as described herein can be readily adapted to both multiwell/multiplate synthesis platforms such as 96 well or similarly larger multi-well platforms. The tandem synthesis of siNA as described herein can also be readily adapted to large scale synthesis platforms employing batch reactors, synthesis columns and the like.

A siNA molecule can also be assembled from two distinct nucleic acid strands or fragments wherein one fragment comprises the sense region and the second fragment comprises the antisense region of the RNA molecule.

The nucleic acid molecules of the present invention can be modified extensively to enhance stability by modification with nuclease resistant groups, for example, 2'-amino, 2'-*C*-allyl, 2'-fluoro, 2'-*O*-methyl, 2'-H (for a review see Usman and Cedergren, 1992, *TIBS* 17, 34; Usman *et al.,* 1994, *Nucleic Acids Symp. Ser.* 31, 163). siNA constructs can be purified by gel electrophoresis using general methods or can be purified by high pressure liquid chromatography (HPLC; see Wincott *et al., supra,* the totality of which is hereby incorporated herein by reference) and re-suspended in water.

In another aspect of the invention, siNA molecules of the invention are expressed from transcription units inserted into DNA or RNA vectors. The recombinant vectors can be DNA plasmids or viral vectors. siNA expressing viral vectors can be constructed based on, but not limited to, adeno-associated virus, retrovirus, adenovirus, or alphavirus. The recombinant vectors capable of expressing the siNA molecules can be delivered as described herein, and persist in target cells. Alternatively, viral vectors can be used that provide for transient expression of siNA molecules.

### Optimizing Activity of the nucleic acid molecule of the invention.

Chemically synthesizing nucleic acid molecules with modifications (base, sugar and/or phosphate) can prevent their degradation by serum ribonucleases, which can increase their potency (see *e.g.,* Eckstein et al., International Publication No. WO 92/07065; Perrault et al., 1990 Nature 344, 565; Pieken et al., 1991, Science 253, 314; Usman and Cedergren, 1992, Trends in Biochem. Sci. 17, 334; Usman et al., International Publication No. WO 93/15187; and Rossi et al., International Publication No. WO 91/03162; Sproat, U.S. Pat. No. 5,334,711; Gold et al., U.S. Pat. No. 6,300,074; and Burgin *et al., supra;* all of which are incorporated by reference herein). All of the above references describe various chemical modifications that can be made to the base, phosphate and/or sugar moieties of the nucleic acid molecules described herein. Modifications that enhance their efficacy in cells, and removal of bases from nucleic acid molecules to shorten oligonucleotide synthesis times and reduce chemical requirements are desired.

There are several examples in the art describing sugar, base and phosphate modifications that can be introduced into nucleic acid molecules with significant enhancement in their nuclease stability and efficacy. For example, oligonucleotides are modified to enhance stability and/or enhance biological activity by modification with nuclease resistant groups, for example, 2'-amino, 2'-*C*-allyl, 2'-fluoro, 2'-*O*-methyl, 2'-O-allyl, 2'-H, nucleotide base modifications (for a review see Usman and Cedergren, 1992, TIBS. 17, 34; Usman et al., 1994, Nucleic Acids Symp. Ser. 31, 163; Burgin et al., 1996, Biochemistry, 35, 14090). Sugar modification of nucleic acid molecules have been extensively described in the art (see Eckstein et al., International Publication PCT No. WO 92/07065; Perrault et al. Nature, 1990, 344, 565-568; Pieken et al. Science, 1991, 253, 314-317; Usman and Cedergren, Trends in Biochem. Sci. , 1992, 17, 334-339; Usman *et al.* International Publication PCT No. WO 93/15187; Sproat, U.S. Pat. No. 5,334,711 and Beigelman et al., 1995, J. Biol. Chem., 270, 25702; Beigelman et al., International PCT publication No. WO 97/26270; Beigelman et al., U.S. Pat. No. 5,716,824; Usman *et al.,* U.S. Pat. No. 5,627,053; Woolf *et al.,* International PCT Publication No. WO 98/13526; Thompson et al., USSN 60/082,404 which was filed on April 20, 1998; Karpeishy et al., 1998, Tetrahedron Lett., 39, 1131; Earnshaw and Gait, 1998, Biopolymers (Nucleic Acid Sciences), 48, 39-55; Verma and Eckstein, 1998, Annu. Rev. Biochem., 67, 99-134; and Burlina et al., 1997, Bioorg. Med. Chem., 5, 1999-2010; all of the references are hereby incorporated in their totality by reference herein). Such publications describe general methods and strategies to determine the location of incorporation of sugar, base and/or phosphate modifications and the like into nucleic acid molecules without modulating catalysis, and are incorporated by reference herein. In view of such teachings, similar modifications can be used as described herein to modify the siNA nucleic acid molecules of the instant invention so long as the ability of siNA to promote RNAi is cells is not significantly inhibited.

While chemical modification of oligonucleotide internucleotide linkages with phosphorothioate, phosphorodithioate, and/or 5'-methylphosphonate linkages improves stability, excessive modifications can cause some toxicity or decreased activity. Therefore, when designing nucleic acid molecules, the amount of these internucleotide linkages should be minimized. The reduction in the concentration of these linkages should lower toxicity, resulting in increased efficacy and higher specificity of these molecules.

Short interfering nucleic acid (siNA) molecules having chemical modifications that maintain or enhance activity are provided. Such a nucleic acid is also generally more resistant to nucleases than an unmodified nucleic acid. Accordingly, the *in vitro* and/or *in vivo* activity should not be significantly lowered. In cases in which modulation is the goal, therapeutic nucleic acid molecules delivered exogenously should optimally be stable within cells until translation of the target RNA has been modulated long enough to reduce the levels of the undesirable protein. This period of time varies between hours to days depending upon the disease state. Improvements in the chemical synthesis of RNA and DNA (Wincott et al., 1995, Nucleic Acids Res. 23, 2677; Caruthers et al., 1992, Methods in Enzymology 211,3-19 (incorporated by reference herein)) have expanded the ability to modify nucleic acid molecules by introducing nucleotide modifications to enhance their nuclease stability, as described above.

In one embodiment, nucleic acid molecules of the invention include one or more (*e.g*., about 1, 2, 3, 4, 5, 6, 7, 8, 9, 10, or more) G-clamp nucleotides. A G-clamp nucleotide is a modified cytosine analog wherein the modifications confer the ability to hydrogen bond both Watson-Crick and Hoogsteen faces of a complementary guanine within a duplex, see for example Lin and Matteucci, 1998, J. Am. Chem. Soc., 120, 8531-8532. A single G-clamp analog substitution within an oligonucleotide can result in substantially enhanced helical thermal stability and mismatch discrimination when hybridized to complementary oligonucleotides. The inclusion of such nucleotides in nucleic acid molecules of the invention results in both enhanced affinity and specificity to nucleic acid targets, complementary sequences, or template strands. In another embodiment, nucleic acid molecules of the invention include one or more (e.g., about 1, 2, 3, 4, 5, 6, 7, 8, 9, 10, or more) LNA "locked nucleic acid" nucleotides such as a 2', 4'-C methylene bicyclo nucleotide (see for example Wengel et al., International PCT Publication No. WO 00/66604 and WO 99/14226).

In another embodiment, the invention features conjugates and/or complexes of siNA molecules of the invention. Such conjugates and/or complexes can be used to facilitate delivery of siNA molecules into a biological system, such as a cell. The conjugates and complexes provided by the instant invention can impart therapeutic activity by transferring therapeutic compounds across cellular membranes, altering the pharmacokinetics, and/or modulating the localization of nucleic acid molecules of the invention. The present invention encompasses the design and synthesis of novel conjugates and complexes for the delivery of molecules, including, but not limited to, small molecules, lipids, phospholipids, nucleosides, nucleotides, nucleic acids, antibodies, toxins, negatively charged polymers and other polymers, for example proteins, peptides, hormones, carbohydrates, polyethylene glycols, or polyamines, across cellular membranes. In general, the transporters described are designed to be used either individually or as part of a multi-component system, with or without degradable linkers. These compounds are expected to improve delivery and/or localization of nucleic acid molecules of the invention into a number of cell types originating from different tissues, in the presence or absence of serum (see Sullenger and Cech, U.S. Pat. No. 5,854,038). Conjugates of the molecules described herein can be attached to biologically active molecules via linkers that are biodegradable, such as biodegradable nucleic acid linker molecules.

The term "biodegradable linker" as used herein, refers to a nucleic acid or non-nucleic acid linker molecule that is designed as a biodegradable linker to connect one molecule to another molecule, for example, a biologically active molecule to a siNA molecule of the invention or the sense and antisense strands of a siNA molecule of the invention. The biodegradable linker is designed such that its stability can be modulated for a particular purpose, such as delivery to a particular tissue or cell type. The stability of a nucleic acid-based biodegradable linker molecule can be modulated by using various chemistries, for example combinations of ribonucleotides, deoxyribonucleotides, and chemically-modified nucleotides, such as 2'-O-methyl, 2'-fluoro, 2'-amino, 2'-O-amino, 2'-C-allyl, 2'-O-allyl, and other 2'-modified or base modified nucleotides. The biodegradable nucleic acid linker molecule can be a dimer, trimer, tetramer or longer nucleic acid molecule, for example, an oligonucleotide of about 2, 3, 4, 5, 6, 7, 8, 9, 10, 11, 12, 1.3, 14, 15, 16, 17, 18, 19, or 20 nucleotides in length, or can comprise a single nucleotide with a phosphorus-based linkage, for example, a phosphoramidate or phosphodiester linkage. The biodegradable nucleic acid linker molecule can also comprise nucleic acid backbone, nucleic acid sugar, or nucleic acid base modifications.

The term "biodegradable" as used herein, refers to degradation in a biological system, for example enzymatic degradation or chemical degradation.

The term "biologically active molecule" as used herein, refers to compounds or molecules that are capable of eliciting or modifying a biological response in a system. Non-limiting examples of biologically active siNA molecules either alone or in combination with other molecules contemplated by the instant invention include therapeutically active molecules such as antibodies, hormones, antivirals, peptides, proteins, chemotherapeutics, small molecules, vitamins, co-factors, nucleosides, nucleotides, oligonucleotides, enzymatic nucleic acids, antisense nucleic acids, triplex forming oligonucleotides, 2,5-A chimeras, siNA, dsRNA, allozymes, aptamers, decoys and analogs thereof. Biologically active molecules of the invention also include molecules capable of modulating the phamiacokinetics and/or pharmacodynamics of other biologically active molecules, for example, lipids and polymers such as polyamines, polyamides, polyethylene glycol and other polyethers.

The term "phospholipid" as used herein, refers to a hydrophobic molecule comprising at least one phosphorus group. For example, a phospholipid can comprise a phosphorus-containing group and saturated or unsaturated alkyl group, optionally substituted with OH, COOH, oxo, amine, or substituted or unsubstituted aryl groups.

Therapeutic nucleic acid molecules (*e.g*., siNA molecules) delivered exogenously optimally are stable within cells until reverse transcription of the RNA has been modulated long enough to reduce the levels of the RNA transcript. The nucleic acid molecules are resistant to nucleases in order to function as effective intracellular therapeutic agents. Improvements in the chemical synthesis of nucleic acid molecules described in the instant invention and in the art have expanded the ability to modify nucleic acid molecules by introducing nucleotide modifications to enhance their nuclease stability as described above.

In yet another embodiment, siNA molecules having chemical modifications that maintain or enhance enzymatic activity of proteins involved in RNAi are provided. Such nucleic acids are also generally more resistant to nucleases than unmodified nucleic acids. Thus, *in vitro* and/or *in vivo* the activity should not be significantly lowered.

Use of the nucleic acid-based molecules of the invention will lead to better treatment of the disease progression by affording the possibility of combination therapies (*e.g*., multiple siNA molecules targeted to different genes; nucleic acid molecules coupled with known small molecule modulators; or intermittent treatment with combinations of molecules, including different motifs and/or other chemical or biological molecules). The treatment of subjects with siNA molecules can also include combinations of different types of nucleic acid molecules, such as enzymatic nucleic acid molecules (ribozymes), allozymes, antisense, 2,5-A oligoadenylate, decoys, and aptamers.

In another aspect a siNA molecule of the invention comprises one or more 5' and/or a 3'- cap structure, for example on only the sense siNA strand, the antisense siNA strand, or both siNA strands.

By "cap structure" is meant chemical modifications, which have been incorporated at either terminus of the oligonucleotide (see, for example, Adamic et al., U.S. Pat. No. 5,998,203, incorporated by reference herein). These terminal modifications protect the nucleic acid molecule from exonuclease degradation, and may help in delivery and/or localization within a cell. The cap may be present at the 5'-terminus (5'-cap) or at the 3'-terminal (3'-cap) or may be present on both termini. In non-limiting examples, the 5'-cap is selected from the group consisting of glyceryl, inverted deoxy abasic residue (moiety); 4',5'-methylene nucleotide; 1-(beta-D-erythrofuranosyl) nucleotide, 4'-thio nucleotide; carbocyclic nucleotide; 1,5-anhydrohexitol nucleotide; L-nucleotides; alpha-nucleotides; modified base nucleotide; phosphorodithioate linkage; threo-pentofuranosyl nucleotide; acyclic 3',4'-seco nucleotide; acyclic 3,4-dihydroxybutyl nucleotide; acyclic 3,5-dihydroxypentyl nucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; an1Ínohexyl phosphate; 3'-phosphate; 3'-phosphorothioate; phosphorodithioate; or bridging or non-bridging methylphosphonate moiety.

In non-limiting examples, the 3'-cap is selected from the group consisting of glyceryl, inverted deoxy abasic residue (moiety), 4',5'-methylene nucleotide; 1-(beta-D-erythrofuranosyl) nucleotide; 4'-thio nucleotide, carbocyclic nucleotide; 5'-amino-alkyl phosphate; 1,3-diamino-2-propyl phosphate; 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate; *threo-*pentofuranosyl nucleotide; acyclic 3',4'-seco nucleotide; 3,4-dihydroxybutyl nucleotide; 3,5-dihydroxypentyl nucleotide, 5'-5'-inverted nucleotide moiety; 5'-5'-inverted abasic moiety; 5'-phosphoramidate; 5'-phosphorothioate; 1,4-butanediol phosphate; 5'-amino; bridging and/or non-bridging 5'-phosphoramidate, phosphorothioate and/or phosphorodithioate, bridging or non bridging methylphosphonate and 5'-mercapto moieties (for more details see Beaucage and Iyer, 1993, Tetrahedron 49, 1925; incorporated by reference herein).

By the term "non-nucleotide" is meant any group or compound which can be incorporated into a nucleic acid chain in the place of one or more nucleotide units, including either sugar and/or phosphate substitutions, and allows the remaining bases to exhibit their enzymatic activity. The group or compound is abasic in that it does not contain a commonly recognized nucleotide base, such as adenosine, guanine, cytosine, uracil or thymine and therefore lacks a base at the l'-position.

An "alkyl" group refers to a saturated aliphatic hydrocarbon, including straight-chain, branched-chain, and cyclic alkyl groups. Preferably, the alkyl group has 1 to 12 carbons. More preferably, it is a lower alkyl of from 1 to 7 carbons, more preferably 1 to 4 carbons. The alkyl group can be substituted or unsubstituted. When substituted the substituted group(s) is preferably, hydroxyl, cyano, alkoxy, =O, =S, NO₂ or N(CH₃)₂, amino, or SH. The term also includes alkenyl groups that are unsaturated hydrocarbon groups containing at least one carbon-carbon double bond, including straight-chain, branched-chain, and cyclic groups. Preferably, the alkenyl group has 1 to 12 carbons. More preferably, it is a lower alkenyl of from 1 to 7 carbons, more preferably 1 to 4 carbons. The alkenyl group may be substituted or unsubstituted. When substituted the substituted group(s) is preferably, hydroxyl, cyano, alkoxy, =O, =S, NO₂, halogen, N(CH₃)₂, amino, or SH. The term "alkyl" also includes alkynyl groups that have an unsaturated hydrocarbon group containing at least one carbon-carbon triple bond, including straight-chain, branched-chain, and cyclic groups. Preferably, the alkynyl group has 1 to 12 carbons. More preferably, it is a lower alkynyl of from 1 to 7 carbons, more preferably 1 to 4 carbons. The alkynyl group may be substituted or unsubstituted. When substituted the substituted group(s) is preferably, hydroxyl, cyano, alkoxy, =O, =S, NO₂ or N(CH₃)₂, amino or SH.

Such alkyl groups can also include aryl, alkylaryl, carbocyclic aryl, heterocyclic aryl, amide and ester groups. An "aryl" group refers to an aromatic group that has at least one ring having a conjugated pi electron system and includes carbocyclic aryl, heterocyclic aryl and biaryl groups, all of which may be optionally substituted. The preferred substituent(s) of aryl groups are halogen, trihalomethyl, hydroxyl, SH, OH, cyano, alkoxy, alkyl, alkenyl, alkynyl, and amino groups. An "alkylaryl" group refers to an alkyl group (as described above) covalently joined to an aryl group (as described above). Carbocyclic aryl groups are groups wherein the ring atoms on the aromatic ring are all carbon atoms. The carbon atoms are optionally substituted. Heterocyclic aryl groups are groups having from 1 to 3 heteroatoms as ring atoms in the aromatic ring and the remainder of the ring atoms are carbon atoms. Suitable heteroatoms include oxygen, sulfur, and nitrogen, and include furanyl, thienyl, pyridyl, pyrrolyl, N-lower alkyl pyrrolo, pyrimidyl, pyrazinyl, imidazolyl and the like, all optionally substituted. An "amide" refers to an -C(O)-NH-R, where R is either alkyl, aryl, alkylaryl or hydrogen. An "ester" refers to an -C(O)-OR', where R is either alkyl, aryl, alkylaryl or hydrogen.

By "nucleotide" as used herein is as recognized in the art to include natural bases (standard), and modified bases well known in the art. Such bases are generally located at the 1' position of a nucleotide sugar moiety. Nucleotides generally comprise a base, sugar and a phosphate group. The nucleotides can be unmodified or modified at the sugar, phosphate and/or base moiety, (also referred to interchangeably as nucleotide analogs, modified nucleotides, non-natural nucleotides, non-standard nucleotides and other; see, for example, Usman and McSwiggen, *supra;* Eckstein et al., International PCT Publication No. WO 92/07065; Usman et al., International PCT Publication No. WO 93/15187; Uhlman & Peyman, *supra,* all are hereby incorporated by reference herein). There are several examples of modified nucleic acid bases known in the art as summarized by Limbach et al., 1994, Nucleic Acids Res. 22, 2183. Some of the non-limiting examples of base modifications that can be introduced into nucleic acid molecules include, inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (*e.g*., 5-methylcytidine), 5-alkyluridines (*e.g.,* ribothymidine), 5-halouridine (*e.g*., 5-bromouridine) or 6-azapyrimidines or 6-alkylpyrimidines (e.g. 6-methyluridine), propyne, and others (Burgin et al., 1996, Biochemistry, 35, 14090; Uhlman & Peyman, *supra*)*.* By "modified bases" in this aspect is meant nucleotide bases other than adenine, guanine, cytosine and uracil at 1' position or their equivalents.

In one embodiment, the invention features modified siNA molecules, with phosphate backbone modifications comprising one or more phosphorothioate, phosphorodithioate, methylphosphonate, phosphotriester, morpholino, amidate carbamate, carboxymethyl, acetamidate, polyamide, sulfonate, sulfonamide, sulfamate, formacetal, thioformacetal, and/or alkylsilyl, substitutions. For a review of oligonucleotide backbone modifications, see Hunziker and Leumann, 1995, Nucleic Acid Analogues: Synthesis and Properties, in Modem Synthetic Methods, VCH, 331-417, and Mesmaeker et al., 1994, Novel Backbone Replacements for Oligonu.cleotides, in Carbohydrate Modifications in Antisense Research, ACS, 24-39.

By "abasic" is meant sugar moieties lacking a base or having other chemical groups in place of a base at the 1' position, see for example Adamic et al., U.S. Pat. No. 5,998,203.

By "unmodified nucleoside" is meant one of the bases adenine, cytosine, guanine, thymine, or uracil joined to the 1' carbon of β-D-ribo-furanose.

By "modified nucleoside" is meant any nucleotide base which contains a modification in the chemical structure of an unmodified nucleotide base, sugar and/or phosphate. Non-limiting examples of modified nucleotides are shown by Formulae I-VII and/or other modifications described herein.

In connection with 2'-modified nucleotides as described for the present invention, by "amino" is meant 2'-NH₂ or 2'-*O*- NH₂, which can be modified or unmodified. Such modified groups are described, for example, in Eckstein et al., U.S. Pat. No. 5,672,695 and Matulic-Adamic et al., U.S. Pat. No. 6,248,878, which are both incorporated by reference in their entireties.

Various modifications to nucleic acid siNA structure can be made to enhance the utility of these molecules. Such modifications will enhance shelf-life, half-life *in vitro,* stability, and ease of introduction of such oligonucleotides to the target site, *e.g*., to enhance penetration of cellular membranes, and confer the ability to recognize and bind to targeted cells.

### Administration of Nucleic Acid Molecules

A siNA molecule of the invention can be adapted for use to treat a variety of neurodegenerative diseases, including Alzheimer's disease, dementia, stroke (CVA), and any other diseases or conditions that are related to the levels of BACE in a cell or tissue, alone or in combination with other therapies. For example, a siNA molecule can comprise a delivery vehicle, including liposomes, for administration to a subject, carriers and diluents and their salts, and/or can be present in pharmaceutically acceptable formulations. Methods for the delivery of nucleic acid molecules are described in Akhtar et al., 1992, Trends Cell Bio., 2, 139; Delivery Strategies for Antisense Oligonucleotide Therapeutics, ed. Akhtar, 1995, Maurer et al., 1999, Mol. Membr. Biol., 16, 129-140; Hofland and Huang, 1999, Handb. Exp. Pharmacol., 137, 165-192; and Lee et al., 2000, ACS Symp. Ser., 752, 184-192, all of which are incorporated herein by reference. Beigelman et al., U.S. Pat. No. 6,395,713 and Sullivan et al., PCT WO 94/02595 further describe the general methods for delivery of nucleic acid molecules. These protocols can be utilized for the delivery of virtually any nucleic acid molecule. Nucleic acid molecules can be administered to cells by a variety of methods known to those of skill in the art, including, but not restricted to, encapsulation in liposomes, by iontophoresis, or by incorporation into other vehicles, such as hydrogels, cyclodextrins (see for example Gonzalez et al., 1999, Bioconjugate Chem., 10, 1068-1074), biodegradable nanocapsules, and bioadhesive microspheres, or by proteinaceous vectors (O'Hare and Normand, International PCT Publication No. WO 00/53722). Alternatively, the nucleic acid/vehicle combination is locally delivered by direct injection or by use of an infusion pump. Direct injection of the nucleic acid molecules of the invention, whether subcutaneous, intramuscular, or intradermal, can take place using standard needle and syringe methodologies, or by needle-free teclmologies such as those described in Conry et al., 1999, Clin. Cancer Res., 5, 2330-2337 and Barry et al., International PCT Publication No. WO 99/31262. The molecules of the instant invention can be used as pharmaceutical agents. Pharmaceutical agents prevent, modulate the occurrence, or treat (alleviate a symptom to some extent, preferably all of the symptoms) of a disease state in a subject.

Thus, the invention features a pharmaceutical composition comprising one or more nucleic acid(s) of the invention in an acceptable carrier, such as a stabilizer, buffer, and the like. The polynucleotides of the invention can be administered (*e.g.,* RNA, DNA or protein) and introduced into a subject by any standard means, with or without stabilizers, buffers, and the like, to form a pharmaceutical composition. When it is desired to use a liposome delivery mechanism, standard protocols for formation of liposomes can be followed. The compositions of the present invention can also be formulated and used as tablets, capsules or elixirs for oral administration, suppositories for rectal administration, sterile solutions, suspensions for injectable administration, and the other compositions known in the art.

The present invention also includes pharmaceutically acceptable formulations of the compounds described. These formulations include salts of the above compounds, *e.g*., acid addition salts, for example, salts of hydrochloric, hydrobromic, acetic acid, and benzene sulfonic acid.

A pharmacological composition or formulation refers to a composition or formulation in a form suitable for administration, *e.g*., systemic administration, into a cell or subject, including for example a human. Suitable forms, in part, depend upon the use or the route of entry, for example oral, transdermal, or by injection. Such forms should not prevent the composition or formulation from reaching a target cell (**i.e.,* a cell to which the negatively charged nucleic acid is desirable for delivery). For example, pharmacological compositions injected into the blood stream should be soluble. Other factors are known in the art, and include considerations such as toxicity and forms that prevent the composition or formulation from exerting its effect.

By "systemic administration" is meant *in vivo* systemic absorption or accumulation of drugs in the blood stream followed by distribution throughout the entire body. Administration routes that lead to systemic absorption include, without limitation: intravenous, subcutaneous, intraperitoneal, inhalation, oral, intrapulinonary and intramuscular. Each of these administration routes exposes the siNA molecules of the invention to an accessible diseased tissue. The rate of entry of a drug into the circulation has been shown to be a function of molecular weight or size. The use of a liposome or other drug carrier comprising the compounds of the instant invention can potentially localize the drug, for example, in certain tissue types, such as the tissues of the reticular endothelial system (RES). A liposome formulation that can facilitate the association of drug with the surface of cells, such as, lymphocytes and macrophages is also useful. This approach can provide enhanced delivery of the drug to target cells by taking advantage of the specificity of macrophage and lymphocyte immune recognition of abnormal cells, such as cells producing excess BACE.

By "pharmaceutically acceptable formulation" is meant, a composition or formulation that allows for the effective distribution of the nucleic acid molecules of the instant invention in the physical location most suitable for their desired activity. Non-limiting examples of agents suitable for formulation with the nucleic acid molecules of the instant invention include: P-glycoprotein inhibitors (such as Pluronic P85), which can enhance entry of drugs into the CNS (Jolliet-Riant and Tillement, 1999, Fundam. Clin. Pharmacol., 13, 16-26); biodegradable polymers, such as poly (DL-lactide-coglycolide) microspheres for sustained release delivery after intracerebral implantation (Emerich, DF et al, 1999, Cell Transplant, 8, 47-58) (Alkermes, Inc. Cambridge, MA); and loaded nanoparticles, such as those made of polybutylcyanoacrylate, which can deliver drugs across the blood brain barrier and can alter neuronal uptake mechanisms (Prog Neuropsychopharmacol Biol Psychiatry, 23, 941-949, 1999). Other non-limiting examples of delivery strategies for the nucleic acid molecules of the instant invention include material described in Boado et al., 1998, J. Pharm. Sci., 87, 1308-1315; Tyler et al., 1999, FEBS Lett., 421, 280-284; Pardridge et al., 1995, PNAS USA., 92, 5592-5596; Boado, 1995, Adv. Drug Delivery Rev., 15, 73-107; Aldrian-Herrada et al., 1998, Nucleic Acids Res., 26,4910-4916; and Tyler et al., 1999, PNAS USA., 96, 7053-7058.

The invention also features the use of the composition comprising surface-modified liposomes containing poly (ethylene glycol) lipids (PEG-modified, or long-circulating liposomes or stealth liposomes). These formulations offer a method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic et al. Chem. Rev. 1995, 95, 2601-2627; Ishiwata et al., Chem. Pharm. Bull. 1995, 43, 1005-1011). Such liposomes have been shown to accumulate selectively in tumors, presumably by extravasation and capture in the neovascularized target tissues (Lasic et al., Science 1995, 267, 1275-1276; Oku et al., 1995, Biochim. Biophys. Acta, 1238, 86-90). The long-circulating liposomes enhance the pharmacokinetics and pharmacodynamics of DNA and RNA, particularly compared to conventional cationic liposomes which are known to accumulate in tissues of the MPS (Liu et al., J. Biol. Chenx. 1995, 42, 24864-24870; Choi et al., International PCT Publication No. WO 96/10391; Ansell et al., International PCT Publication No. WO 96/10390; Holland et al., International PCT Publication No. WO 96/10392). Long-circulating liposomes are also likely to protect drugs from nuclease degradation to a greater extent compared to cationic liposomes, based on their ability to avoid accumulation in metabolically aggressive MPS tissues such as the liver and spleen.

The present invention also includes compositions prepared for storage or administration that include a pharmaceutically effective amount of the desired compounds in a pharmaceutically acceptable carrier or diluent. Acceptable carriers or diluents for therapeutic use are well known in the pharmaceutical art, and are described, for example, in *Remington's Pharmaceutical Sciences,* Mack Publishing Co. (A.R. Gennaro edit. 1985), hereby incorporated by reference herein. For example, preservatives, stabilizers, dyes and flavoring agents can be provided. These include sodium benzoate, sorbic acid and esters of *p*-hydroxybenzoic acid. In addition, antioxidants and suspending agents can be used.

A pharmaceutically effective dose is that dose required to prevent, inhibit the occurrence, or treat (alleviate a symptom to some extent, preferably all of the symptoms) of a disease state. The pharmaceutically effective dose depends on the type of disease, the composition used, the route of administration, the type of mammal being treated, the physical characteristics of the specific mammal under consideration, concurrent medication, and other factors that those skilled in the medical arts will recognize. Generally, an amount from about 0.1 mg/kg to about 100 mg/kg body weight/day of active ingredients is administered dependent upon potency of the negatively charged polymer.

The nucleic acid molecules of the invention and formulations thereof can be administered orally, topically, parenterally, by inhalation or spray, or rectally in dosage unit formulations containing conventional non-toxic phamiaceutically acceptable carriers, adjuvants and/or vehicles. The term parenteral as used herein includes percutaneous, subcutaneous, intravascular (*e.g*., intravenous), intramuscular, or intrathecal injection or infusion techniques and the like. In addition, there is provided a pharmaceutical formulation comprising a nucleic acid molecule of the invention and a pharmaceutically acceptable carrier. One or more nucleic acid molecules of the invention can be present in association with one or more non-toxic pharmaceutically acceptable carriers and/or diluents and/or adjuvants, and if desired other active ingredients. The pharmaceutical compositions containing nucleic acid molecules of the invention can be in a form suitable for oral use, for example, as tablets, troches, lozenges, aqueous or oily suspensions, dispersible powders or granules, emulsion, hard or soft capsules, or syrups or elixirs.

Compositions intended for oral use can be prepared according to any method known to the art for the manufacture of pharmaceutical compositions and such compositions can contain one or more such sweetening agents, flavoring agents, coloring agents or preservative agents in order to provide pharmaceutically elegant and palatable preparations. Tablets contain the active ingredient in admixture with non-toxic pharmaceutically acceptable excipients that are suitable for the manufacture of tablets. These excipients can be, for example, inert diluents; such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets can be uncoated or they can be coated by known techniques. In some cases such coatings can be prepared by known techniques to delay disintegration and absorption in the gastrointestinal tract and thereby provide a sustained action over a longer period. For example, a time delay material such as glyceryl monosterate or glyceryl distearate can be employed.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in a mixture with excipients suitable for the manufacture of aqueous suspensions. Such excipients are suspending agents, for example sodium carboxymethylcellulose, methylcellulose, hydropropylmethylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavoring agents, and one or more sweetening agents, such as sucrose or saccharin.

Oily suspensions can be formulated by suspending the active ingredients in a vegetable oil, for example arachis oil, olive oil, sesame oil or coconut oil, or in a mineral oil such as liquid paraffin. The oily suspensions can contain a thickening agent, for example beeswax, hard paraffin or cetyl alcohol. Sweetening agents and flavoring agents can be added to provide palatable oral preparations. These compositions can be preserved by the addition of an anti-oxidant such as ascorbic acid

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, can also be present.

Pharmaceutical compositions of the invention can also be in the form of oil-in-water emulsions. The oily phase can be a vegetable oil or a mineral oil or mixtures of these. Suitable emulsifying agents can be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions can also contain sweetening and flavoring agents.

Syrups and elixirs can be formulated with sweetening agents, for example glycerol, propylene glycol, sorbitol, glucose or sucrose. Such formulations can also contain a demulcent, a preservative and flavoring and coloring agents. The pharmaceutical compositions can be in the form of a sterile injectable aqueous or oleaginous suspension. This suspension can be formulated according to the known art using those suitable dispersing or wetting agents and suspending agents that have been mentioned above. The sterile injectable preparation can also be a sterile injectable solution or suspension in .. a non-toxic parentally acceptable diluent or solvent, for example as a solution in 1,3-butanediol. Among the acceptable vehicles and solvents that can be employed are water, Ringer's solution and isotonic sodium chloride solution. In addition, sterile, fixed oils are conventionally employed as a solvent or suspending medium. For this purpose, any bland fixed oil can be employed including synthetic mono-or diglycerides. In addition, fatty acids such as oleic acid find use in the preparation of injectables.

The nucleic acid molecules of the invention can also be administered in the form of suppositories, *e.g.,* for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

Nucleic acid molecules of the invention can be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

Dosage levels of the order of from about 0.1 mg to about 140 mg per kilogram of body weight per day are useful in the treatment of the above-indicated conditions (about 0.5 mg to about 7 g per subject per day). The amount of active ingredient that can be combined with the carrier materials to produce a single dosage form varies depending upon the host treated and the particular mode of administration. Dosage unit forms generally contain about 1 mg to about 500 mg of an active ingredient.

It is understood that the specific dose level for any particular subject depends upon a variety of factors including the activity of the specific compound employed, the age, body weight, general health, sex, diet, time of administration, route of administration, and rate of excretion, drug combination and the severity of the particular disease undergoing therapy.

For administration to non-human animals, the composition can also be added to the animal feed or drinking water. It can be convenient to formulate the animal feed and drinking water compositions so that the animal takes in a therapeutically appropriate quantity of the composition along with its diet. It can also be convenient to present the composition as a premix for addition to the feed or drinking water.

The nucleic acid molecules of the present invention can also be administered to a subject in combination with other therapeutic compounds to increase the overall therapeutic effect. The use of multiple compounds to treat an indication can increase the beneficial effects while reducing the presence of side effects.

In one embodiment, the invention provides compositions suitable for administering nucleic acid molecules of the invention to specific cell types. For example, the asialoglycoprotein receptor (ASGPr) (Wu and Wu, 1987, J. Biol. Chem. 262, 4429-4432) is unique to hepatocytes and binds branched galactose-terminal glycoproteins, such as asialoorosomucoid (ASOR). In another example, the folate receptor is overexpressed in many cancer cells. Binding of such glycoproteins, synthetic glycoconjugates, or folates to the receptor takes place with an affinity that strongly depends on the degree of branching of the oligosaccharide chain, for example, triatennary structures are bound with greater affinity than biatenarry or monoatennary chains (Baenziger and Fiete, 1980, Cell, 22, 611-620; Connolly et al., 1982, J. Biol. Chem., 257, 939-945). Lee and Lee, 1987, Glycoconjugate J., 4, 317-328, obtained this high specificity through the use of N-acetyl-D-galactosamine as the carbohydrate moiety, which has higher affinity for the receptor, compared to galactose. This "clustering effect" has also been described for the binding and uptake of mannosyl-terminating glycoproteins or glycoconjugates (Ponpipom et al., 1981, J. Med. Ghenz., 24, 1388-1395). The use of galactose, galactosamine, or folate based conjugates to transport exogenous compounds across cell membranes can provide a targeted delivery approach to, for example, the treatment of liver disease, cancers of the liver, or other cancers. The use of bioconjugates can also provide a reduction in the required dose of therapeutic compounds required for treatment. Furthermore, therapeutic bioavialability, pharmacodynamics, and pharmacokinetic parameters can be modulated through the use of nucleic acid bioconjugates of the invention. Non-limiting examples of such bioconjugates are described in Vargeese et al., USSN 10/201,394, filed August 13, 2001; and Matulic-Adamic et al., USSN 60/362,016, filed March 6, 2002.

Alternatively, certain siNA molecules of the instant invention can be expressed within cells from eukaryotic promoters (*e.g*., Izant and Weintraub, 1985, Science, 229, 345; McGarry and Lindquist, 1986, Proc. Natl. Acad. Sci., USA 83, 399; Scanlon et al., 1991, Proc. Natl. Acad. Sci. USA, 88, 10591-5; Kashani-Sabet et al., 1992, Antisense Res. Dev., 2, 3-15; Dropulic et al., 1992, J. Virol., 66, 1432-41; Weerasinghe et al., 1991, J. Virol., 65, 5531-4; Ojwang et al., 1992, Proc. Natl. Acad. Sci. USA, 89, 10802-6; Chen et al., 1992, Nucleic Acids Res., 20, 4581-9; Sarver et al., 1990 Science, 247, 1222-1225; Thompson et al., 1995, Nucleic Acids Res., 23, 2259; Good et al., 1997, Gene Therapy, 4, 45. Those skilled in the art realize that any nucleic acid can be expressed in eulcaryotic cells from the appropriate DNA/RNA vector. The activity of such nucleic acids can be augmented by their release from the primary transcript by a enzymatic nucleic acid (Draper et al., PCT WO 93/23569, and Sullivan et al., PCT WO 94/02595; Ohlcawa et al., 1992, Nucleic Acids Symp. Ser., 27, 15-6; Taira et al., 1991, Nucleic Acids Res., 19, 5125-30; Ventura et al., 1993, Nucleic Acids Res., 21, 3249-55; Chowrira et al., 1994, J. Biol. Chem., 269,25856.

In another aspect of the invention, RNA molecules of the present invention can be expressed from transcription units (see for example Couture et al., 1996, TIG., 12, 510) inserted into DNA or RNA vectors. The recombinant vectors can be DNA plasmids or viral vectors. siNA expressing viral vectors can be constructed based on, but not limited to, adeno-associated virus, retrovirus, adenovirus, or alphavirus. In another embodiment, pol III based constructs are used to express nucleic acid molecules of the invention (see for example Thompson, U.S. Pats. Nos. 5,902,880 and 6,146,886). The recombinant vectors capable of expressing the siNA molecules can be delivered as described above, and persist in target cells. Alternatively, viral vectors can be used that provide for transient expression of nucleic acid molecules. Such vectors can be repeatedly administered as necessary. Once expressed, the siNA molecule interacts with the target mRNA and generates an RNAi response. Delivery of siNA molecule expressing vectors can be systemic, such as by intravenous or intra-muscular administration, by administration to target cells ex-planted from a subject followed by reintroduction into the subject, or by any other means that would allow for introduction into the desired target cell (for a review see Couture et al., 1996, TIG., 12, 510).

In one aspect the invention features an expression vector comprising a nucleic acid sequence encoding at least one siNA molecule of the instant invention. The expression vector can encode one or both strands of a siNA duplex, or a single self-complementary strand that self hybridizes into a siNA duplex. The nucleic acid sequences encoding the siNA molecules of the instant invention can be operably linked in a manner that allows expression of the siNA molecule (see for example Paul et al., 2002, Nature Biotechnology, 19, 505; Miyagishi and Taira, 2002, Nature Biotechnology, 19, 497; Lee et al., 2002, Nature Biotechnology, 19, 500; and Novina et al., 2002, Nature Medicine, advance online publication doi:10.1038/nm725).

In another aspect, the invention features an expression vector comprising: a) a transcription initiation region (*e.g*., eukaryotic pol I, II or III initiation region); b) a transcription termination region (*e.g*., eukaryotic pol I, II or III termination region); and c) a nucleic acid sequence encoding at least one of the siNA molecules of the instant invention wherein said sequence is operably linked to said initiation region and said termination region in a manner that allows expression and/or delivery of the siNA molecule. The vector can optionally include an open reading frame (ORF) for a protein operably linked on the 5' side or the 3'-side of the sequence encoding the siNA of the invention, and/or an intron (intervening sequences).

Transcription of the siNA molecule sequences can be driven from a promoter for eukaryotic RNA polymerase I (pol I), RNA polymerase II (pol II), or RNA polymerase III (pol III). Transcripts from pol II or pol III promoters are expressed at high levels in all cells; the levels of a given pol II promoter in a given cell type depends on the nature of the gene regulatory sequences (enhancers, silencers, etc.) present nearby. Prokaryotic RNA polymerase promoters are also used, providing that the prokaryotic RNA polymerase enzyme is expressed in the appropriate cells (Elroy-Stein and Moss, 1990, Proc. Natl. Acad. Sci. U S A, 87, 6743-7; Gao and Huang 1993, Nucleic Acids Res., 21, 2867-72; Lieber et al., 1993, Methods Enzymol., 217,47-66; Zhou et al., 1990, Mol. Cell. Biol., 10, 4529-37). Several investigators have demonstrated that nucleic acid molecules expressed from such promoters can function in mammalian cells (e.g. Kashani-Sabet et al., 1992, Antisense Res. Dev., 2, 3-15; Ojwang et al., 1992, Proc. Natl. Acad. Sci. U S A, 89, 10802-6; Chen et al., 1992, Nucleic Acids Res., 20, 4581-9; Yu et al., 1993, Proc. Natl. Acad. Sci. USA, 90, 6340-4; L'Huillier et al., 1992, EMBO J., 11, 4411-8; Lisziewicz et al., 1993, Proc. Natl. Acad. Sci. U. S. A, 90, 8000-4; Thompson et al., 1995, Nucleic Acids Res., 23, 2259; Sullenger & Cech, 1993, Science, 262, 1566). More specifically, transcription units such as the ones derived from genes encoding U6 small nuclear (snRNA), transfer RNA (tRNA) and adenovirus VA RNA are useful in generating high concentrations of desired RNA molecules such as siNA in cells (Thompson *et al., supra;* Couture and Stinchcomb, *1996, supra;* Noonberg et al., 1994, Nucleic Acid Res., 22, 2830; Noonberg et al., U.S. Pat. No. 5,624,803; Good et al., 1997, Gene Ther., 4, 45; Beigelman et al., International PCT Publication No. WO 96/18736. The above siNA transcription units can be incorporated into a variety of vectors for introduction into mammalian cells, including but not restricted to, plasmid DNA vectors, viral DNA vectors (such as adenovirus or adeno-associated virus vectors), or viral RNA vectors (such as retroviral or alphavirus vectors) (for a review see Couture and Stinchcomb, *1996, supra*)*.*

In another aspect the invention features an expression vector comprising a nucleic acid sequence encoding at least one of the siNA molecules of the invention in a manner that allows expression of that siNA molecule. The expression vector comprises in one embodiment; a) a transcription initiation region; b) a transcription termination region; and c) a nucleic acid sequence encoding at least one strand of the siNA molecule, wherein the sequence is operably linked to the initiation region and the termination region in a manner that allows expression and/or delivery of the siNA molecule.

In another embodiment the expression vector comprises: a) a transcription initiation region; b) a transcription termination region; c) an open reading frame; and d) a nucleic acid sequence encoding at least one strand of a siNA molecule, wherein the sequence is operably linked to the 3'-end of the open reading frame and wherein the sequence is operably linked to the initiation region, the open reading frame and the termination region in a manner that allows expression and/or delivery of the siNA molecule. In yet another embodiment, the expression vector comprises: a) a transcription initiation region; b) a transcription termination region; c) an intron; and d) a nucleic acid sequence encoding at least one siNA molecule, wherein the sequence is operably linked to the initiation region, the intron and the termination region in a manner which allows expression and/or delivery of the nucleic acid molecule.

In another embodiment, the expression vector comprises: a) a transcription initiation region; b) a transcription termination region; c) an intron; d) an open reading frame; and e) a nucleic acid sequence encoding at least one strand of a siNA molecule, wherein the sequence is operably linked to the 3'-end of the open reading frame and wherein the sequence is operably linked to the initiation region, the intron, the open reading frame and the termination region in a manner which allows expression and/or delivery of the siNA molecule.

### BACE biology and biochemistry

Alzheimer's disease is characterized by the progressive formation of insoluble plaques and vascular deposits in the brain consisting of the 4 kD amyloid β peptide (Aβ). These plaques are characterized by dystrophic neurites that show profound synaptic loss, neurofibrillary tangle formation, and gliosis. Aβ arises from the proteolytic cleavage of the large type I transmembrane protein, β-amyloid precursor protein (APP) (Kang et al., 1987, Nature, 325, 733). Processing of APP to generate Aβ requires two sites of cleavage by a β-secretase and a γ-secretase. β-secretase cleavage of APP results in the cytoplasmic release of a 100 kD soluble ammo-terminal fragment, APPsβ, leaving behind a 12 kD transmembrane carboxy-terminal fragment, C99. Alternately, APP can be cleaved by a α-secretase to generate cytoplasmic APPsα and transmembrane C83 fragments. Both remaining transmembrane fragments, C99 and C83, can be further cleaved by a γ-secretase, leading to the release and secretion of Alzheimer's related Aβ and a non-pathogenic peptide, p3, respectively (Vassar et al., 1999, Science, 286, 735-741). Early onset familial Alzheimer's disease is characterized by mutant APP protein with a Met to Leu substitution at position P1, characterized as the "Swedish" familial mutation (Mullan et al., 1992, Nature Genet., 1, 345). This APP mutation is characterized by a dramatic enhancement in β-secretase cleavage (Citron et al., 1992, Nature, 360, 672).

The identification of β-secretase and γ-secretase constituents involved in the release of β-amyloid protein is of primary importance in the development of treatment strategies for Alzheimer's disease. Characterization of α-secretase is also important in this regard since α-secretase cleavage may compete with β-secretase cleavage resulting in changes in the relative amounts of non-pathogenic and pathogenic protein production. Involvement of the two metalloproteases, ADAM 10 and TACE, has been demonstrated in α-cleavage of AAP (Buxbaum et al., 1999, J. Biol. Chem., 273, 27765, and Lammich et al., 1999, Proc. Natl. Acad. Sci. U.S.A., 96, 3922). Studies of γ-secretase activity have demonstrated presenilin dependence (De Stooper et al., 1998, Nature, 391, 387, and De Stooper et al., 1999, Nature, 398, 518), and as such, presenilins have been proposed as γ-secretase even though presenilin does not present proteolytic activity (Wolfe et al., 1999, Nature, 398, 513).

Studies have shown β-secretase cleavage of AAP by the transmembrane aspartic protease beta site APP cleaving enzyme, BACE (Vassar *et al.,* supra). While other potential candidates for β-secretase have been proposed (for review see Evin et al., 1999, Proc. Natl. Acad. Sci. U.S.A., 96, 3922), none have demonstrated the full range of characteristics expected from this enzyme. Studies have shown that BACE expression and localization are as expected for β-secretase, that BACE overexpression in cells results in increased β-secretase cleavage of APP and Swedish APP, that isolated BACE demonstrates site specific proteolytic activity on APP derived peptide substrates, and that antisense mediated endogenous BACE inhibition results in dramatically reduced β-secretase activity (Vassar *et al.,* supra).

Current treatment strategies for Alzheimer's disease rely on either the prevention or the alleviation of symptoms and/or the slowing down of disease progression. Two drugs approved in the treatment of Alzheimer's, donepezil (Aricept®) and tacrine (Gognex®), both cholinomimetics, attempt to slow the loss of cognitive ability by increasing the amount of acetylcholine available to the brain. Antioxidant therapy through the use of antioxidant compounds such as alpha-tocopherol (vitamin E), melatonin, and selegeline (Eldepryl®) attempt to slow disease progression by minimizing free radical damage. Estrogen replacement therapy is thought to incur a possible preventative benefit in the development of Alzheimer's disease based on limited data. The use of anti-inflammatory drugs may be associated with a reduced risk of Alzheimer's as well. Calcium channel blockers such as Nimodipine® are considered to have a potential benefit in treating Alzheimer's disease due to protection of nerve cells from calcium overload, thereby prolonging nerve cell survival. Nootropic compounds, such as acetyl-L-camitine (Alcar®) and insulin, have been proposed to have some benefit in treating Alzheimer's due to enhancement of cognitive and memory function based on cellular metabolism.

Whereby the above treatment strategies can all improve quality of life in Alzheimer's patients, there exists an unmet need in the comprehensive treatment and prevention of this disease. As such, there exists the need for therapeutics effective in reversing the physiological changes associated with Alzheimer's disease, specifically, therapeutics that can eliminate and/or reverse the deposition of amyloid β peptide. The use of compounds,such as small nucleic acid molecules (e:g., short interfering nucleic acid (siNA), short interfering RNA (siRNA), double-stranded RNA (dsRNA), micro-RNA (miRNA), and short hairpin RNA (shRNA) molecules capable of mediating RNA interference (RNAi)), to modulate the expression of proteases that are instrumental in the release of amyloid β peptide, namely β-secretase (BACE), γ-secretase (presenilin), and the amyloid precursor protein (APP), is of therapeutic significance.

### Examples:

The following are non-limiting examples showing the selection, isolation, synthesis and activity of nucleic acids of the instant invention.

### Example 1: Tandem synthesis of siNA constructs

Exemplary siNA molecules of the invention are synthesized in tandem using a cleavable linker, for example, a succinyl-based linker. Tandem synthesis as described herein is followed by a one-step purification process that provides RNAi molecules in high yield. This approach is highly amenable to siNA synthesis in support of high throughput RNAi screening, and can be readily adapted to multi-column or multi-well synthesis platforms.

After completing a tandem synthesis of a siNA oligo and its complement in which the 5'-terminal dimethoxytrityl (5'-O-DMT) group remains intact (trityl on synthesis), the oligonucleotides are deprotected as described above. Following deprotection, the siNA sequence strands are allowed to spontaneously hybridize. This hybridization yields a duplex in which one strand has retained the 5'-O-DMT group while the complementary strand comprises a terminal 5'-hydroxyl. The newly formed duplex behaves as a single molecule during routine solid-phase extraction purification (Trityl-On purification) even though only one molecule has a dimethoxytrityl group. Because the strands form a stable duplex, this dimethoxytrityl group (or an equivalent group, such as other trityl groups or other hydrophobic moieties) is all that is required to purify the pair of oligos, for example, by using a C18 cartridge.

Standard phosphoramidite synthesis chemistry is used up to the point of introducing a tandem linker, such as an inverted deoxy abasic succinate or glyceryl succinate linker (see **Figure 1**) or an equivalent cleavable linker. A non-limiting example of linker coupling conditions that can be used includes a hindered base such as diisopropylethylamine (DIPA) and/or DMAP in the presence of an activator reagent such as Bromotripyrrolidinophosphoniumhexaflurorophosphate (PyBrOP). After the linker is coupled, standard synthesis chemistry is utilized to complete synthesis of the second sequence leaving the terminal the 5'-O-DMT intact. Following synthesis, the resulting oligonucleotide is deprotected according to the procedures described herein and quenched with a suitable buffer, for example with 50mM NaOAc or 1.5M NH₄H₂CO₃.

Purification of the siNA duplex can be readily accomplished using solid phase extraction, for example using a Waters C18 SepPak 1g cartridge conditioned with 1 column volume (CV) of acetonitrile, 2 CV H2O, and 2 CV 50mM NaOAc. The sample is loaded and then washed with 1 CV H2O or 50mM NaOAc. Failure sequences are eluted with 1 CV 14% ACN (Aqueous with 50mM NaOAc and 50mM NaCl). The column is then washed, for example with 1 CV H2O followed by on-column detritylation, for example by passing 1 CV of 1% aqueous trifluoroacetic acid (TFA) over the column, then adding a second CV of 1% aqueous TFA to the column and allowing to stand for approximately 10 minutes. The remaining TFA solution is removed and the column washed with H2O followed by 1 CV 1M NaCl and additional H2O. The siNA duplex product is then eluted, for example, using 1 CV 20% aqueous CAN.

**Figure 2** provides an example of MALDI-TOV mass spectrometry analysis of a purified siNA construct in which each peak corresponds to the calculated mass of an individual siNA strand of the siNA duplex. The same purified siNA provides three peaks when analyzed by capillary gel electrophoresis (CGE), one peak presumably corresponding to the duplex siNA, and two peaks presumably corresponding to the separate siNA sequence strands. Ion exchange HPLC analysis of the same siNA contract only shows a single peak. Testing of the purified siNA construct using a luciferase reporter assay described below demonstrated the same RNAi activity compared to siNA constructs generated from separately synthesized oligonucleotide sequence strands.

### Example 2: Identification of potential siNA target sites in any RNA sequence

The sequence of an RNA target of interest, such as a viral or human mRNA transcript, is screened for target sites, for example by using a computer folding algorithm. In a non-limiting example, the sequence of a gene or RNA gene transcript derived from a database, such as Genbank, is used to generate siNA targets having complementarity to the target. Such sequences can be obtained from a database, or can be determined experimentally as known in the art. Target sites that are known, for example, those target sites determined to be effective target sites based on studies with other nucleic acid molecules, for example ribozymes or antisense, or those targets known to be associated with a disease or condition such as those sites containing mutations or deletions, can be used to design siNA molecules targeting those sites. Various parameters can be used to determine which sites are the most suitable target sites within the target RNA sequence. These parameters include but are not limited to secondary or tertiary RNA structure, the nucleotide base composition of the target sequence, the degree of homology between various regions of the target sequence, or the relative position of the target sequence within the RNA transcript. Based on these determinations, any number of target sites within the RNA transcript can be chosen to screen siNA molecules for efficacy, for example by using in *vitro* RNA cleavage assays, cell culture, or animal models. In a non-limiting example, anywhere from 1 to 1000 target sites are chosen within the transcript based on the size of the siNA construct to be used. High throughput screening assays can be developed for screening siNA molecules using methods known in the art, such as with multi-well or multi-plate assays to determine efficient reduction in target gene expression.

### Example 3: Selection of siNA molecule target sites in a RNA

The following non-limiting steps can be used to carry out the selection of siNAs targeting a given gene sequence or transcript.
1. The target sequence is parsed *in silico* into a list of all fragments or subsequences of a particular length, for example 23 nucleotide fragments, contained within the target sequence. This step is typically carried out using a custom Perl script, but commercial sequence analysis programs such as Oligo, MacVector, or the GCG Wisconsin Package can be employed as well.
2. In some instances the siNAs correspond to more than one target sequence; such would be the case for example in targeting different transcripts of the same gene, targeting different transcripts of more than one gene, or for targeting both the human gene and an animal homolog. In this case, a subsequence list of a particular length is generated for each of the targets, and then the lists are compared to find matching sequences in each list. The subsequences are then ranked according to the number of target sequences that contain the given subsequence; the goal is to find subsequences that are present in most or all of the target sequences. Alternately, the ranking can identify subsequences that are unique to a target sequence, such as a mutant target sequence. Such an approach would enable the use of siNA to target specifically the mutant sequence and not effect the expression of the normal sequence.
3. In some instances the siNA subsequences are absent in one or more sequences while present in the desired target sequence; such would be the case if the siNA targets a gene with a paralogous family member that is to remain untargeted. As in case 2 above, a subsequence list of a particular length is generated for each of the targets, and then the lists are compared to find sequences that are present in the target gene but are absent in the untargeted paralog.
4. The ranked siNA subsequences can be further analyzed and ranked according to GC content. A preference can be given to sites containing 30-70% GC, with a further preference to sites containing 40-60% GC.
5. The ranked siNA subsequences can be further analyzed and ranked according to self-folding and internal hairpins. Weaker internal folds are preferred; strong hairpin structures are to be avoided.
6. The ranked siNA subsequences can be further analyzed and ranked according to whether they have runs of GGG or CCC in the sequence. GGG (or even more Gs) in either strand can make oligonucleotide synthesis problematic and can potentially interfere with RNAi activity, so it is avoided whenever better sequences are available. CCC is searched in the target strand because that will place GGG in the antisense strand.
7. The ranked siNA subsequences can be further analyzed and ranked according to whether they have the dinucleotide UU (uridine dinucleotide) on the 3'-end of the sequence, and/or AA on the 5'-end of the sequence (to yield 3' UU on the antisense sequence). These sequences allow one to design siNA molecules with terminal TT thymidine dinucleotides.
8. Four or five target sites are chosen from the ranked list of subsequences as described above. For example, in subsequences having 23 nucleotides, the right 21 nucleotides of each chosen 23-mer subsequence are then designed and synthesized for the upper (sense) strand of the siNA duplex, while the reverse complement of the left 21 nucleotides of each chosen 23-mer subsequence are then designed and synthesized for the lower (antisense) strand of the siNA duplex (see **Tables II and III**). If terminal TT residues are desired for the sequence (as described in paragraph 7), then the two 3' terminal nucleotides of both the sense and antisense strands are replaced by TT prior to synthesizing the oligos.
9. The siNA molecules are screened in an *in vitro,* cell culture or animal model system to identify the most active siNA molecule or the most preferred target site within the target RNA sequence.

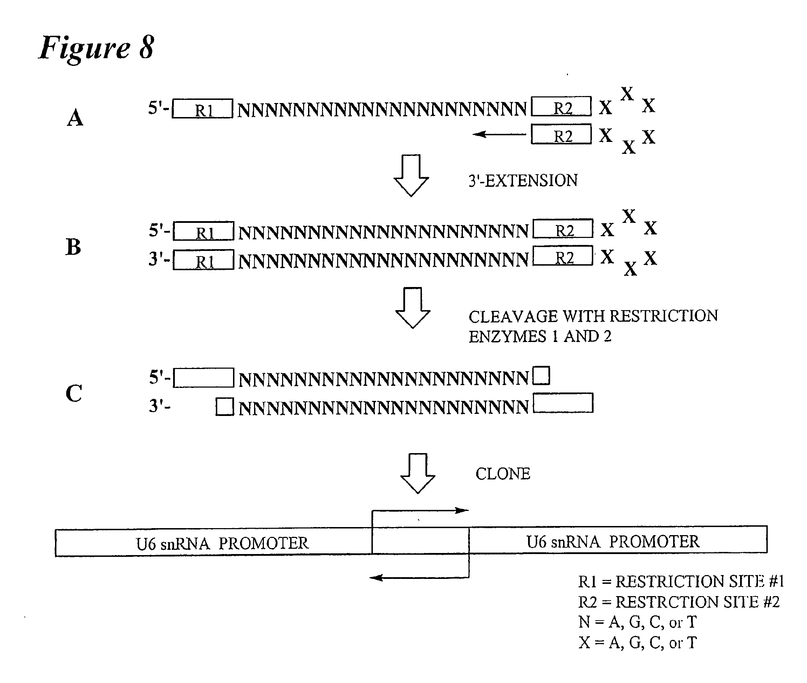

In an alternate approach, a pool of siNA constructs specific to a BACE target sequence is used to screen for target sites in cells expressing BACE RNA, such A549 cells, 7PA2 Chinese hamster ovary (CHO) cells or APPsw (Swedish type amyloid precursor protein expressing) cells. The general strategy used in this approach is shown in **Figure 9**. A non-limiting example of such as pool is a pool comprising sequences having sense sequences comprising SEQ ID NOs. 1-325, 651-658, 663-666, and 671-674, and antisense sequences comprising SEQ ID NOs. 326-650, 659-662, 667-670, and 675-675, respectively. Cells expressing BACE (e.g., A549 cells) are transfected with the pool of siNA constructs, and cells that demonstrate a phenotype associated with BACE inhibition are sorted. The pool of siNA constructs can be expressed from transcription cassettes inserted into appropriate vectors (see for example **Figure 7** and **Figure 8**). The siNA from cells demonstrating a positive phenotypic change (e.g., decreased proliferation, decreased BACE mRNA levels or decreased BACE protein expression), are sequenced to determine the most suitable target site(s) within the target BACE RNA sequence.

### Example 4: BACE targeted siNA design

siNA target sites were chosen by analyzing sequences of the BACE RNA target and optionally prioritizing the target sites on the basis of folding (structure of any given sequence analyzed to determine siNA accessibility to the target), by using a library of siNA molecules as described in Example 3, or alternately by using an *in vitro* siNA system as described in Example 6 herein. siNA molecules were designed that could bind each target and are optionally individually analyzed by computer folding to assess whether the siNA molecule can interact with the target sequence. Varying the length of the siNA molecules can be chosen to optimize activity. Generally, a sufficient number of complementary nucleotide bases are chosen to bind to, or otherwise interact with, the target RNA, but the degree of complementarity can be modulated to accommodate siNA duplexes or varying length or base composition. By using such methodologies, siNA molecules can be designed to target sites within any known RNA sequence, for example those RNA sequences corresponding to the any gene transcript.

Chemically modified siNA constructs are designed to provide nuclease stability for systemic administration in vivo and/or improved pharmacokinetic, localization, and delivery properties while preserving the ability to mediate RNAi activity. Chemical modifications as described herein are introduced synthetically using synthetic methods described herein and those generally known in the art. The synthetic siNA constructs are then assayed for nuclease stability in serum and/or cellular/tissue extracts (e.g. liver extracts). The synthetic siNA constructs are also tested in parallel for RNAi activity using an appropriate assay, such as a luciferase reporter assay as described herein or another suitable assay that can quantity RNAi activity. Synthetic siNA constructs that possess both nuclease stability and RNAi activity can be further modified and reevaluated in stability and activity assays. The chemical modifications of the stabilized active siNA constructs can then be applied to any siNA sequence targeting any chosen RNA and used, for example, in target screening assays to pick lead siNA compounds for therapeutic development (see for example **Figure 11**).

### Example 5: Chemical Synthesis and Purification of siNA

siNA molecules can be designed to interact with various sites in the RNA message, for example, target sequences within the RNA sequences described herein. The sequence of one strand of the siNA molecule(s) is complementary to the target site sequences described above. The siNA molecules can be chemically synthesized using methods described herein. Inactive siNA molecules that are used as control sequences can be synthesized by scrambling the sequence of the siNA molecules such that it is not complementary to the target sequence. Generally, siNA constructs can by synthesized using solid phase oligonucleotide synthesis methods as described herein (see for example Usman et al., US Patent Nos. 5,804,653; 5,831,071; 5,998,303; 6,117,657; 6,353,098; 6,362,323; 6,437,117; 6,469,158; Scaringe et al., US Patent Nos. 6,111,086; 6,008,400; 6,111,086 all incorporated by reference herein in their entirety).

In a non-limiting example, RNA oligonucleotides are synthesized in a stepwise fashion using the phosphoramidite chemistry as is known in the art. Standard phosphoramidite chemistry involves the use of nucleosides comprising any of 5'-O-dimethoxytrityl, 2'-O-tert-butyldimethylsilyl, 3'-O-2-Cyanoethyl N,N-diisopropylphosphoroamidite groups, and exocyclic amine protecting groups (e.g. N6-benzoyl adenosine, N4 acetyl cytidine, and N2-isobutyryl guanosine). Alternately, 2'-O-Silyl Ethers can be used in conjunction with acid-labile 2'-O-orthoester protecting groups in the synthesis of RNA as described by Scaringe *supra.* Differing 2' chemistries can require different protecting groups, for example 2'-deoxy-2'-amino nucleosides can utilize N-phthaloyl protection as described by Usman et al., US Patent 5,631,360, incorporated by reference herein in its entirety).

During solid phase synthesis, each nucleotide is added sequentially (3'- to 5'-direction) to the solid support-bound oligonucleotide. The first nucleoside at the 3'-end of the chain is covalently attached to a solid support (e.g., controlled pore glass or polystyrene) using various linkers. The nucleotide precursor, a ribonucleoside phosphoramidite, and activator are combined resulting in the coupling of the second nucleoside phosphoramidite onto the 5'-end of the first nucleoside. The support is then washed and any unreacted 5'-hydroxyl groups are capped with a capping reagent such as acetic anhydride to yield inactive 5'-acetyl moieties. The trivalent phosphorus linkage is then oxidized to a more stable phosphate linkage. At the end of the nucleotide addition cycle, the 5'-O-protecting group is cleaved under suitable conditions (e.g., acidic conditions for trityl-based groups and Fluoride for silyl-based groups). The cycle is repeated for each subsequent nucleotide.

Modification of synthesis conditions can be used to optimize coupling efficiency, for example, by using differing coupling times, differing reagent/phosphoramidite concentrations, differing contact times, differing solid supports and solid support linker chemistries depending on the particular chemical composition of the siNA to be synthesized. Deprotection and purification of the siNA can be performed as is generally described in Scaringe *supra,* Usman et al., US 5,831,071, US 6,353,098, US 6,437,117, and Bellon et al., US 6,054,576, US 6,162,909, US 6,303,773, all of which are incorporated by reference herein in their entireties.

Additionally, deprotection conditions can be modified to provide the best possible yield and purity of siNA constructs. For example, applicant has observed that oligonucleotides comprising 2'-deoxy-2'-fluoro nucleotides can degrade under inappropriate deprotection conditions. Such oligonucleotides are deprotected using aqueous methylamine at about 35°C for 30 minutes. If the 2'-deoxy-2'-fluoro containing oligonucleotide also comprises ribonucleotides, after deprotection with aqueous methylamine at about 35°C for 30 minutes, TEA-HF is added and the reaction maintained at about 65°C for an additional 15 minutes.

### Example 6: RNAi in vitro assay to assess siNA activity

An *in vitro* assay that recapitulates RNAi in a cell-free system is used to evaluate siNA constructs targeting BACE RNA targets. The assay comprises the system described by Tuschl et al., 1999, Genes and Development, 13, 3191-3197 and Zamore et al., 2000, Cell, 101, 25-33 adapted for use with BACE target RNA. A Drosophila extract derived from syncytial blastoderm is used to reconstitute RNAi activity *in vitro.* Target RNA is generated via in *vitro* transcription from an appropriate BACE expressing plasmid using T7 RNA polymerase or via chemical synthesis as described herein. Sense and antisense siNA strands (for example 20 uM each) are annealed by incubation in buffer (such as 100 mM potassium acetate, 30 mM HEPES-KOH, pH 7.4, 2 mM magnesium acetate) for 1 min. at 90°C followed by 1 hour at 37°C , then diluted in lysis buffer (for example 100 mM potassium acetate, 30 mM HEPES-KOH at pH 7.4, 2mM magnesium acetate). Annealing can be monitored by gel electrophoresis on an agarose gel in TBE buffer and stained with ethidium bromide. The Drosophila lysate is prepared using zero to two-hour-old embryos from Oregon R flies collected on yeasted molasses agar that are dechorionated and lysed. The lysate is centrifuged and the supernatant isolated. The assay comprises a reaction mixture containing 50% lysate [vol/vol], RNA (10-50 pM final concentration), and 10% [vol/vol] lysis buffer containing siNA (10 nM final concentration). The reaction mixture also contains 10 mM creatine phosphate, 10 ug.ml creatine phosphokinase, 100 um GTP, 100 uM UTP, 100 uM CTP, 500 uM ATP, 5 mM DTT, 0.1 U/uL RNasin (Promega), and 100 uM of each amino acid. The final concentration of potassium acetate is adjusted to 100 mM. The reactions are preassembled on ice and preincubated at 25° C for 10 minutes before adding RNA, then incubated at 25° C for an additional 60 minutes. Reactions are quenched with 4 volumes of 1.25 x Passive Lysis Buffer (Promega). Target RNA cleavage is assayed by RT-PCR analysis or other methods known in the art and are compared to control reactions in which siNA is omitted from the reaction.

Alternately, internally-labeled target RNA for the assay is prepared by *in vitro* transcription in the presence of [alpha-³²P] CTP, passed over a G 50 Sephadex column by spin chromatography and used as target RNA without further purification. Optionally, target RNA is 5'-³²P-end labeled using T4 polynucleotide kinase enzyme. Assays are performed as described above and target RNA and the specific RNA cleavage products generated by RNAi are visualized on an autoradiograph of a gel. The percentage of cleavage is determined by Phosphor Imager^{®} quantitation of bands representing intact control RNA or RNA from control reactions without siNA and the cleavage products generated by the assay.

In one embodiment, this assay is used to determine target sites the BACE RNA target for siNA mediated RNAi cleavage, wherein a plurality of siNA constructs are screened for RNAi mediated cleavage of the BACE RNA target, for example, by analyzing the assay reaction by electrophoresis of labeled target RNA, or by northern blotting, as well as by other methodology well known in the art.

### Example 7: Nucleic acid inhibition of BACE target RNA in vivo

siNA molecules targeted to the huma BACE RNA are designed and synthesized as described above. These nucleic acid molecules can be tested for cleavage activity *in vivo,* for example, using the following procedure. The target sequences and the nucleotide location within the BACE RNA are given in **Tables II and III.**

Two formats are used to test the efficacy of siNAs targeting BACE. First, the reagents are tested in cell culture using, for example, A549 cells, 7PA2 Chinese hamster ovary (CHO) cells or APPsw (Swedish type amyloid precursor protein expressing) cells to determine the extent of RNA and protein inhibition. siNA reagents (*e.g*., see **Tables II and III**) are selected against the BACE target as described herein. RNA inhibition is measured after delivery of these reagents by a suitable transfection agent to, for example, A549 cells, 7PA2 Chinese hamster ovary (CHO) cells or APPsw (Swedish type amyloid precursor protein expressing) cells. Relative amounts of target RNA are measured versus actin using real-time PCR monitoring of amplification (*eg*., ABI 7700 Taqman®). A comparison is made to a mixture of oligonucleotide sequences made to unrelated targets or to a randomized siNA control with the same overall length and chemistry, but randomly substituted at each position. Primary and secondary lead reagents are chosen for the target and optimization performed. After an optimal transfection agent concentration is chosen, a RNA time-course of inhibition is performed with the lead siNA molecule. In addition, a cell-plating format can be used to determine RNA inhibition.

### Delivery of siNA to Cells

Cells (e.g., A549 cells, 7PA2, CHO, or APPsw cells) are seeded, for example, at 1x10⁵ cells per well of a six-well dish in EGM-2 (BioWhittaker) the day before transfection. siNA (final concentration, for example 20nM) and cationic lipid (e.g., final concentration 2µg/ml) are complexed in EGM basal media (Biowhittaker) at 37°C for 30 mins in polystyrene tubes. Following vortexing, the complexed siNA is added to each well and incubated for the times indicated. For initial optimization experiments, cells are seeded, for example, at 1x10³ in 96 well plates and siNA complex added as described. Efficiency of delivery of siNA to cells is determined using a fluorescent siNA complexed with lipid. Cells in 6-well dishes are incubated with siNA for 24 hours, rinsed with PBS and fixed in 2% paraformaldehyde for 15 minutes at room temperature. Uptake of siNA is visualized using a fluorescent microscope.

### Taqman and Lightcycler quantification of mRNA

Total RNA is prepared from cells following siNA delivery, for example, using Qiagen RNA purification kits for 6-well or Rneasy extraction kits for 96-well assays. For Taqman analysis, dual-labeled probes are synthesized with the reporter dye, FAM or JOE, covalently linked at the 5'-end and the quencher dye TAMRA conjugated to the 3'-end. One-step RT-PCR amplifications are performed on, for example, an ABI PRISM 7700 Sequence Detector using 50 µl reactions consisting of 10 µl total RNA, 100 nM forward primer, 900 nM reverse primer, 100 nM probe, IX TaqMan PCR reaction buffer (PE-Applied Biosystems), 5.5 mM MgCl₂, 300 µM each dATP, dCTP, dGTP, and dTTP, 10U RNase Inhibitor (Promega), 1.25U AmpliTaq Gold (PE-Applied Biosystems) and 10U M-MLV Reverse Transcriptase (Promega). The thermal cycling conditions can consist of 30 min at 48°C, 10 min at 95°C, followed by 40 cycles of 15 sec at 95°C and 1 min at 60°C. Quantitation of mRNA levels is determined relative to standards generated from serially diluted total cellular RNA (300, 100, 33, 11 ng/rxn) and normalizing to β-actin or GAPDH mRNA in parallel TaqMan reactions. For each gene of interest an upper and lower primer and a fluorescently labeled probe are designed. Real time incorporation of SYBR Green I dye into a specific PCR product can be measured in glass capillary tubes using a lightcyler. A standard curve is generated for each primer pair using control cRNA. Values are represented as relative expression to GAPDH in each sample.

### Western blotting

Nuclear extracts can be prepared using a standard micro preparation technique (see for example Andrews and Faller, 1991, Nucleic Acids Research, 19, 2499). Protein extracts from supernatants are prepared, for example using TCA precipitation. An equal volume of 20% TCA is added to the cell supernatant, incubated on ice for 1 hour and pelleted by centrifugation for 5 minutes. Pellets are washed in acetone, dried and resuspended in water. Cellular protein extracts are run on a 10% Bis-Tris NuPage (nuclear extracts) or 4-12% Tris-Glycine (supernatant extracts) polyacrylamide gel and transferred onto nitro-cellulose membranes. Non-specific binding can be blocked by incubation, for example, with 5% non-fat milk for 1 hour followed by primary antibody for 16 hour at 4°C. Following washes, the secondary antibody is applied, for example (1:10,000 dilution) for 1 hour at room temperature and the signal detected with SuperSignal reagent (Pierce).

### Example 8: Models useful to evaluate the down-regulation of BACE gene expression

### Cell Culture

Vassar et al., 1999, Science, 286, 735-741, describe a cell culture model for studying BACE inhibition. Specific antisense nucleic acid molecules targeting BACE mRNA were used for inhibition studies of endogenous BACE expression in 101 cells and APPsw (Swedish type amyloid precursor protein expressing) cells via lipid mediated transfection. Antisense treatment resulted in dramatic reduction of both BACE mRNA by Northern blot analysis, and APPsβsw ("Swedish" type β-secretase cleavage product) by ELISA, with maximum inhibition of both parameters at 75-80%. This model was also used to study the effect of BACE inhibition on amyloid β-peptide production in APPsw cells. Similarly, such a model can be used to screen siRNA molecules of the instant invention for efficacy and potency.

In several cell culture systems, cationic lipids have been shown to enhance the bioavailability of oligonucleotides to cells in culture (Bennet, et al., 1992, Mol. Pharmacology, 41, 1023-1033). In one embodiment, siNA molecules of the invention are complexed with cationic lipids for cell culture experiments. siNA and cationic lipid mixtures are prepared in serum-free DMEM immediately prior to addition to the cells. DMEM plus additives are warmed to room temperature (about 20-25°C) and cationic lipid is added to the final desired concentration and the solution is vortexed briefly. siNA molecules are added to the final desired concentration and the solution is again vortexed briefly and incubated for 10 minutes at room temperature. In dose response experiments, the RNA/lipid complex is serially diluted into DMEM following the 10 minute incubation.

### Animal Models

Evaluating the efficacy of anti-BACE agents in animal models is an important prerequisite to human clinical trials. Games et al., 1995, Nature, 373, 523-527, describe a transgenic mouse model in which mutant human familial type APP (Phe 717 instead of Val) is overexpressed. This model results in mice that progressively develop many of the pathological halhnarks of Alzheimer's disease, and as such, provides a model for testing therapeutic drugs, including siNA constructs of the instant invention.

### Example 9: RNAi mediated inhibition of BACE RNA expression

siNA constructs (**Tables II and III**) are tested for efficacy in reducing BACE RNA expression in, for example in A549 cells. Cells are plated approximately 24h before transfection in 96-well plates at 5,000-7,500 cells/well, 100 µl/well, such that at the time of transfection cells are 70-90% confluent. For transfection, annealed siNAs are mixed with the transfection reagent (Lipofectamine 2000, Invitrogen) in a volume of 50 µl/well and incubated for 20 min. at room temperature. The siNA transfection mixtures are added to cells to give a final siNA concentration of 25 nM in a volume of 150 µl. Each siNA transfection mixture is added to 3 wells for triplicate siNA treatments. Cells are incubated at 37° for 24h in the continued presence of the siNA transfection mixture. At 24h, RNA is prepared from each well of treated cells. The supematants with the transfection mixtures are first removed and discarded, then the cells are lysed and RNA prepared from each well. Target gene expression following treatment is evaluated by RT-PCR for the target gene and for a control gene (36B4, an RNA polymerase subunit) for normalization. The triplicate data is averaged and the standard deviations determined for each treatment. Normalized data are graphed and the percent reduction of target mRNA by active siNAs in comparison to their respective inverted control siNAs was determined.

In a non-limiting example, siNA constructs were screened for activity (see Figure 12) and compared to untreated cells, scrambled siNA control constructs (Scram1 and Scram2), and cells transfected with lipid alone (transfection control). As shown in Figure 12, the siNA constructs show significant reduction of BACE RNA expression. Leads generated from such a screen are then further assayed. In a non-limiting example, siNA constructs comprising ribonucleotides and 3'-terminal dithymidine caps are assayed along with a chemically modified siNA construct comprising 2'-deoxy-2'-fluoro pyrimidine nucleotides and purine ribonucleotides, in which the sense strand of the siNA is further modified with 5' and 3'-terminal inverted deoxyabasic caps and the antisense strand comprises a 3'-terminal phosphorothioate internucleotide linkage. Additional stabilization chemistries as described in **Table IV** are similarly assayed for activity. These siNA constructs are compared to appropriate matched chemistry inverted controls. In addition, the siNA constructs are also compared to untreated cells, cells transfected with lipid and scrambled siNA constructs, and cells transfected with lipid alone (transfection control).

### Example 10: Indications

Particular degenerative and disease states that can be associated with BACE expression modulation include but are not limited to: Alzheimer's disease, dementia, stroke (CVA) and any other diseases or conditions that are related to the levels of BACE in a cell or tissue, alone or in combination with other therapies. The reduction of BACE expression (specifically BACE RNA levels) and thus reduction in the level of the respective protein relieves, to some extent, the symptoms of the disease or condition.

Those skilled in the art will recognize that other drug compounds and therapies may be readily combined with or used in conjuction with the nucleic acid molecules of the instant invention (e.g., siNA molecules) are hence within the scope of the instant invention.

### Example 11: Diagnostic uses

The siNA molecules of the invention can be used in a variety of diagnostic applications, such as in the identification of molecular targets (e.g., RNA) in a variety of applications, for example, in clinical, industrial, environmental, agricultural and/or research settings. Such diagnostic use of siNA molecules involves utilizing reconstituted RNAi systems, for example, using cellular lysates or partially purified cellular lysates. siNA molecules of this invention can be used as diagnostic tools to examine genetic drift and mutations within diseased cells or to detect the presence of endogenous or exogenous, for example viral, RNA in a cell. The close relationship between siNA activity and the structure of the target RNA allows the detection of mutations in any region of the molecule, which alters the base-pairing and three-dimensional structure of the target RNA. By using multiple siNA molecules described in this invention, one can map nucleotide changes, which are important to RNA structure and function *in vitro,* as well as in cells and tissues. Cleavage of target RNAs with siNA molecules can be used to inhibit gene expression and define the role of specified gene products in the progression of disease or infection. In this manner, other genetic targets can be defined as important mediators of the disease. These experiments will lead to better treatment of the disease progression by affording the possibility of combination therapies (e.g., multiple siNA molecules targeted to different genes, siNA molecules coupled with known small molecule inhibitors, or intermittent treatment with combinations siNA molecules and/or other chemical or biological molecules). Other *in vitro* uses of siNA molecules of this invention are well known in the art, and include detection of the presence of mRNAs associated with a disease, infection, or related condition. Such RNA is detected by determining the presence of a cleavage product after treatment with a siNA using standard methodologies, for example, fluorescence resonance emission transfer (FRET).

In a specific example, siNA molecules that cleave only wild-type or mutant forms of the target RNA are used for the assay. The first siNA molecules (*i.e*., those that cleave only wild-type forms of target RNA) are used to identify wild-type RNA present in the sample and the second siNA molecules (*i.e*., those that cleave only mutant forms of target RNA) are used to identify mutant RNA in the sample. As reaction controls, synthetic substrates of both wild-type and mutant RNA are cleaved by both siNA molecules to demonstrate the relative siNA efficiencies in the reactions and the absence of cleavage of the "non-targeted" RNA species. The cleavage products from the synthetic substrates also serve to generate size markers for the analysis of wild-type and mutant RNAs in the sample population. Thus, each analysis requires two siNA molecules, two substrates and one unknown sample, which is combined into six reactions. The presence of cleavage products is determined using an RNase protection assay so that full-length and cleavage fragments of each RNA can be analyzed in one lane of a polyacrylamide gel. It is not absolutely required to quantify the results to gain insight into the expression of mutant RNAs and putative risk of the desired phenotypic changes in target cells. The expression of mRNA whose protein product is implicated in the development of the phenotype (*i.e*., disease related or infection related) is adequate to establish risk. If probes of comparable specific activity are used for both transcripts, then a qualitative comparison of RNA levels is adequate and decreases the cost of the initial diagnosis. Higher mutant form to wild-type ratios are correlated with higher risk whether RNA levels are compared qualitatively or quantitatively.

All patents and publications mentioned in the specification are indicative of the levels of skill of those skilled in the art to which the invention pertains. All references cited in this disclosure are incorporated by reference to the same extent as if each reference had been incorporated by reference in its entirety individually.

One skilled in the art would readily appreciate that the present invention is well adapted to carry out the objects and obtain the ends and advantages mentioned, as well as those inherent therein. The methods and compositions described herein as presently representative of preferred embodiments are exemplary and are not intended as limitations on the scope of the invention. Changes therein and other uses will occur to those skilled in the art, which are encompassed within the spirit of the invention, are defined by the scope of the claims.

It will be readily apparent to one skilled in the art that varying substitutions and modifications can be made to the invention disclosed herein without departing from the scope and spirit of the invention. Thus, such additional embodiments are within the scope of the present invention and the following claims. The present invention teaches one skilled in the art to test various combinations and/or substitutions of chemical modifications described herein toward generating nucleic acid constructs with improved activity for mediating RNAi activity. Such improved activity can comprise improved stability, improved bioavailability, and/or improved activation of cellular responses mediating RNAi. Therefore, the specific embodiments described herein are not limiting and one skilled in the art can readily appreciate that specific combinations of the modifications described herein can be tested without undue experimentation toward identifying siNA molecules with improved RNAi activity.

The invention illustratively described herein suitably can be practiced in the absence of any element or elements, limitation or limitations that are not specifically disclosed herein. Thus, for example, in each instance herein any of the terms "comprising", "consisting essentially of', and "consisting of' may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments, optional features, modification and variation of the concepts herein disclosed may be resorted to by those skilled in the art, and that such modifications and variations are considered to be within the scope of this invention as defined by the description and the appended claims.

In addition, where features or aspects of the invention are described in terms of Markush groups or other grouping of alternatives, those skilled in the art will recognize that the invention is also thereby described in terms of any individual member or subgroup of members of the Marlcush group or other group.

**Table I: BACE Accession Numbers**

| |
|---|
| NM_012104 |
| Homo sapiens beta-site APP-cleaving enzyme (BACE), transcript variant a, mRNA |
| gi\|21040369\|ref\|NM_012104.2\|[21040369] |
| |
| NM_006222 |
| Homo sapiens protein (peptidyl-prolyl cis/trans isomerase) NIMA-interacting |
| 1-like (PIN1L), mRNA |
| gi\|5453899\|ref\|NM_006222.1\|[5453899] |
| |
| L76517 |
| Homo sapiens (clone cc44) senilin 1 (PS1; S182) mRNA, complete cds |
| gi\|1479973\|gb\|L76517.1\|HUMPS1MRNA[1479973] |
| |
| L43964 |
| Homo sapiens (clone F-T03796) STM-2 mRNA, complete cds |
| gi\|951202\|gb\|L43964.1\|HUMSTM2R[951202] |
| |
| NM_138973 |
| Homo sapiens beta-site APP-cleaving enzyme (BACE), transcript variant d, mRNA |
| gi\|21040367\|ref\|NM_138973.1\|[21040367] |
| |
| NM_138972 |
| Homo sapiens beta-site APP-cleaving enzyme (BACE), transcript variant b, mRNA |
| gi\|21040365\|ref\|NM_138972.1\|[21040365] |
| |
| NM_138971 |
| Homo sapiens beta-site APP-cleaving enzyme (BACE), transcript variant c, mRNA |
| gi\|21040363\|ref\|NM_138971.1\|[21040363] |
| |
| AK075049 |
| Homo sapiens cDNA FLJ90568 fis, clone OVARC1001570, highly similar to Homo |
| sapiens beta-site APP cleaving enzyme (BACE) mRNA |
| gi\|22760888\|dbj\|AK075049.1\|[22760888] |
| AF527782 |
| Homo sapiens beta-site APP-cleaving enzyme (BACE) mRNA, partial cds, |
| alternatively spliced |
| gi\|22094870\|gb\|AF527782.1\|[22094870] |
| |
| AF324837 |
| Homo sapiens beta-site APP cleaving enzyme mRNA, partial cds, alternatively spliced |
| gi\|21449275\|gb\|AF324837.1\|[21449275] |
| |
| AF338817 |
| Homo sapiens beta-site APP cleaving enzyme type C mRNA, complete cds |
| gi\|13699247\|gb\|AF338817.1\|[13699247] |
| |
| AF338816 |
| Homo sapiens beta-site APP cleaving enzyme type B mRNA, complete cds |
| gi\|13699245\|gb\|AF338816.1\|[13699245] |
| |
| AB050438 |
| Homo sapiens BACE mRNA for beta-site APP cleaving enzyme I-432, complete cds |
| gi\|13568410\|dbj\|AB050438.1\|[13568410] |
| |
| AB050437 |
| Homo sapiens BACE mRNA for beta-site APP cleaving enzyme I-457, complete cds |
| gi\|13568408\|dbj\|AB050437.1\|[13568408] |
| |
| AB050436 |
| Homo sapiens BACE mRNA for beta-site APP cleaving enzyme I-476, complete cds |
| gi\|13568406\|dbj\|AB050436.1\|[13568406] |
| |
| AF190725 |
| Homo sapiens beta-site APP cleaving enzyme (BACE) mRNA, complete cds |
| gi\|6118538\|gb\|AF190725.1\|AF190725[6118538] |
| NM 007319 |
| Homo sapiens presenilin 1 (Alzheimer disease 3) (PSEN1), transcript variant |
| I-374., mRNA |
| gi\|7549814\|ref\|NM_007319.1\|[7549814] |
| |
| NM_138992 |
| Homo sapiens beta-site APP-cleaving enzyme 2 (BACE2), transcript variant b, mRNA |
| gi\|21040361\|ref\|NM_138992.1\|[21040361] |
| |
| NM_138991 |
| Homo sapiens beta-site APP-cleaving enzyme 2 (BACE2), transcript variant c, mRNA |
| gi\|21040359\|ref\|NM_138991.1\|[21040359] |
| |
| NM_012105 |
| Homo sapiens beta-site APP-cleaving enzyme 2 (BACE2), transcript variant a, mRNA |
| gi\|21040358\|ref\|NM_012105.3\|[21040358] |
| |
| AB066441 |
| Homo sapiens APP mRNA for amyloid precursor protein, partial cds, D678N mutant |
| gi\|16904654\|dbj\|AB066441.1\|[16904654] |
| |
| AB050438 |
| Homo sapiens BACE mRNA for beta-site APP cleaving enzyme I-432, complete cds |
| gi\|13568410\|dbj\|AB050438.1\|[13568410] |
| |
| AB050437 |
| Homo sapiens BACE mRNA for beta-site APP cleaving enzyme I-457, complete cds |
| gi\|13568408\|dbj\|AB050437.1\|[13568408] |
| |
| AB050436 |
| Homo sapiens BACE mRNA for beta-site APP cleaving enzyme I-476, complete cds |
| gi\|13568406\|dbj\|AB050436.1\|[13568406] |
| |
| NM_012486 |
| Homo sapiens presenilin 2 (Alzheimer disease 4) (PSEN2), transcript variant 2, |
| mRNA |
| gi\|7108359\|ref\|NM_012486.1\|[7108359] |
| |
| NM_000447 |
| Homo sapiens presenilin 2 (Alzheimer disease 4) (PSEN2), transcript variant 1, |
| mRNA |
| gi\|4506164\|ref\|NM_000447.1\|[4506164] |
| |
| AF188277 |
| Homo sapiens aspartyl protease (BACE2) mRNA, complete cds, alternatively spliced |
| gi\|7025334\|gb\|AF188277.1\|AF188277[7025334] |
| |
| AF188276 |
| Homo sapiens aspartyl protease (BACE2) mRNA, complete cds, alternatively spliced |
| gi\|7025332\|gb\|AF188276.1\|AF188276[7025332] |
| |
| AF178532 |
| Homo sapiens aspartyl protease (BACE2) mRNA, complete cds |
| gi\|6851265\|gb\|AF17B532.1\|AF178532[6851265] |
| |
| D87675 |
| Homo sapiens DNA for amyloid precursor protein, complete cds |
| gi\|2429080\|dbj\|D87675.1\|[2429080] |
| |
| AF201468 |
| Homo sapiens APP beta-secretase mRNA, complete cds |
| gi\|6601444\|gb\|AF201468.1\|AF201468[6601444] |
| |
| AF190725 |
| Homo sapiens beta-site APP cleaving enzyme (BACE) mRNA, complete cds |
| gi\|6118538\|gb\|AF190725.1\|AF190725[6118538] |
| |
| E14707 |
| DNA encoding a mutated amyloid precursor protein |
| gi\|5709390\|dbj\|E14707.1\|\|pat\|JP\|1998001499\|1[5709390] |
| |
| AF168956 |
| Homo sapiens amyloid precursor protein homolog HSD-2. mRNA, complete cds |
| gi\|5702387\|gb\|AF168956.1\|AF168956[5702387] |
| |
| S60099 |
| APPH=amyloid precursor protein homolog [human, placenta, mRNA, 3727 nt] |
| gi\|300168\|bbm\|300685\|bbs\|131198\|gb\|S60099.1\|S60099[300 168] |
| |
| U50939 |
| Human amyloid precursor protein-binding protein 1 mRNA, complete cds |
| gi\|1314559\|gb\|U50939.1\|HSU50939[1314559] |

**Table II: BACE siNA and Target Sequences**

| **NM 012104\|BACE** | | | | | | | | |
|---|---|---|---|---|---|---|---|---|
| Pos | Target Sequence, | Seq ID | UPos | Upper seq | Seq ID | LPos | Lower seq | Seq ID |
| 1 | CGCACUCGUCCCCAGCCCG | 1 | 1 | CGCACUCGUCCCCAGCCCG | 1 | 23 | CGGGCUGGGGACGAGUGCG | 326 |
| 19 | GCCCGGGAGCUGCGAGCCG | 2 | 19 | GCCCGGGAGCUGCGAGCCG | 2 | 41 | CGGCUCGCAGCUCCCGGGC | 327 |
| 37 | GCGAGCUGGAUUAUGGUGG | 3 | 37 | GCGAGCUGGAUUAUGGUGG | 3 | 59 | CCACCAUAAUCCAGCUCGC | 328 |
| 55 | GCCUGAGCAGCCAACGCAG | 4 | 55 | GCCUGAGCAGCCAACGCAG | 4 | 77 | CUGCGUUGGCUGCUCAGGC | 329 |
| 73 | GCCGCAGGAGCCCGGAGCC | 5 | 73 | GCCGCAGGAGCCCGGAGCC | 5 | 95 | GGCUCCGGGCUCCUGCGGC | 330 |
| 91 | CCUUGCCCCUGCCCGCGCC | 6 | 91 | CCUUGCCCCUGCCCGCGCC | 6 | 113 | GGCGCGGGCAGGGGCAAGG | 331 |
| 109 | CGCCGCCCGCCGGGGGGAC | 7 | 109 | CGCCGCCCGCCGGGGGGAC | 7 | 131 | GUCCCCCCGGCGGGCGGCG | 332 |
| 127 | CCAGGGAAGCCGCCACCGG | 8 | 127 | CCAGGGAAGCCGCCACCGG | 8 | 149 | CCGGUGGCGGCUUCCCUGG | 333 |
| 145 | GCCCGCCAUGCCCGCCCCU | 9 | 145 | GCCCGCCAUGCCCGCCCCU | 9 | 167 | AGGGGCGGGCAUGGCGGGC | 334 |
| 163 | UCCCAGCCCCGCCGGGAGC | 10 | 163 | UCCCAGCCCCGCCGGGAGC | 10 | 185 | GCUCCCGGCGGGGCUGGGA | 335 |
| 181 | CCCGCGCCCGCUGCCCAGG | 11 | 181 | CCCGCGCCCGCUGCCCAGG | 11 | 203 | CCUGGGCAGCGGGCGCGGG | 336 |
| 199 | GCUGGCCGCCGCCGUGCCG | 12 | 199 | GCUGGCCGCCGCCGUGCCG | 12 | 221 | CGGCACGGCGGCGGCCAGC | 337 |
| 217 | GAUGUAGCGGGCUCCGGAU | 13 | 217 | GAUGUAGCGGGCUCCGGAU | 13 | 239 | AUCCGGAGCCCGCUACAUC | 338 |
| 235 | UCCCAGCCUCUCCCCUGCU | 14 | 235 | UCCCAGCCUCUCCCCUGCU | 14 | 257 | AGCAGGGGAGAGGCUGGGA | 339 |
| 253 | UCCCGUGCUCUGCGGAUCU | 15 | 253 | UCCCGUGCUCUGCGGAUCU | 15 | 275 | AGAUCCGCAGAGCACGGGA | 340 |
| 271 | UCCCCUGACCGCUCUCCAC | 16 | 271 | UCCCCUGACCGCUCUCCAC | 16 | 293 | GUGGAGAGCGGUCAGGGGA | 341 |
| 289 | CAGCCCGGACCCGGGGGCU | 17 | 289 | CAGCCCGGACCCGGGGGCU | 17 | 311 | AGCCCCCGGGUCCGGGCUG | 342 |
| 307 | UGGCCCAGGGCCCUGCAGG | 18 | 307 | UGGCCCAGGGCCCUGCAGG | 18 | 329 | CCUGCAGGGCCCUGGGCCA | 343 |
| 325 | GCCCUGGCGUCCUGAUGCC | 19 | 325 | GCCCUGGCGUCCUGAUGCC | 19 | 347 | GGCAUCAGGACGCCAGGGC | 344 |
| 343 | CCCCAAGCUCCCUCUCCUG | 20 | 343 | CCCCAAGCUCCCUCUCCUG | 20 | 365 | CAGGAGAGGGAGCUUGGGG | 345 |
| 361 | GAGAAGCCACCAGCACCAC | 21 | 361 | GAGAAGCCACCAGCACCAC | 21 | 383 | GUGGUGCUGGUGGCUUCUC | 346 |
| 379 | CCCAGACUUGGGGGCAGGC | 22 | 379 | CCCAGACUUGGGGGCAGGC | 22 | 401 | GCCUGCCCCCAAGUCUGGG | 347 |
| 397 | CGCCAGGGACGGACGUGGG | 23 | 397 | CGCCAGGGACGGACGUGGG | 23 | 419 | CCCACGUCCGUCCCUGGCG | 348 |
| 415 | GCCAGUGCGAGCCCAGAGG | 24 | 415 | GCCAGUGCGAGCCCAGAGG | 24 | 437 | CCUCUGGGCUCGCACUGGC | 349 |
| 433 | GGCCCGAAGGCCGGGGCCC | 25 | 433 | GGCCCGAAGGCCGGGGCCC | 25 | 455 | GGGCCCCGGCCUUCGGGCC | 350 |
| 451 | CACCAUGGCCCAAGCCCUG | 26 | 451 | CACCAUGGCCCAAGCCCUG | 26 | 473 | CAGGGCUUGGGCCAUGGUG | 351 |
| 469 | GCCCUGGCUCCUGCUGUGG | 27 | 469 | GCCCUGGCUCCUGCUGUGG | 27 | 491 | CCACAGCAGGAGCCAGGGC | 352 |
| 487 | GAUGGGCGCGGGAGUGCUG | 28 | 487 | GAUGGGCGCGGGAGUGCUG | 28 | 509 | CAGCACUCCCGCGCCCAUC | 353 |
| 505 | GCCUGCCCACGGCACCCAG | 29 | 505 | GCCUGCCCACGGCACCCAG | 29 | 527 | CUGGGUGCCGUGGGCAGGC | 354 |
| 523 | GCACGGCAUCCGGCUGCCC | 30 | 523 | GCACGGCAUCCGGCUGCCC | 30 | 545 | GGGCAGCCGGAUGCCGUGC | 355 |
| 541 | CCUGCGCAGCGGCCUGGGG | 31 | 541 | CCUGCGCAGCGGCCUGGGG | 31 | 563 | CCCCAGGCCGCUGCGCAGG | 356 |
| 559 | GGGCGCCCCCCUGGGGCUG | 32 | 559 | GGGCGCCCCCCUGGGGCUG | 32 | 581 | CAGCCCCAGGGGGGCGCCC | 357 |
| 577 | GCGGCUGCCCCGGGAGACC | 33 | 577 | GCGGCUGCCCCGGGAGACC | 33 | 599 | GGUCUCCCGGGGCAGCCGC | 358 |
| 595 | CGACGAAGAGCCCGAGGAG | 34 | 595 | CGACGAAGAGCCCGAGGAG | 34 | 617 | CUCCUCGGGCUCUUCGUCG | 359 |
| 613 | GCCCGGCCGGAGGGGCAGC | 35 | 613 | GCCCGGCCGGAGGGGCAGC | 35 | 635 | GCUGCCCCUCCGGCCGGGC | 360 |
| 631 | CUUUGUGGAGAUGGUGGAC | 36 | 631 | CUUUGUGGAGAUGGUGGAC | 36 | 653 | GUCCACCAUCUCCACAAAG | 361 |
| 649 | CAACCUGAGGGGCAAGUCG | 37 | 649 | CAACCUGAGGGGCAAGUCG | 37 | 671 | CGACUUGCCCCUCAGGUUG | 362 |
| 667 | GGGGCAGGGCUACUACGUG | 38 | 667 | GGGGCAGGGCUACUACGUG | 38 | 689 | CACGUAGUAGCCCUGCCCC | 363 |
| 685 | GGAGAUGACCGUGGGCAGC | 39 | 685 | GGAGAUGACCGUGGGCAGC | 39 | 707 | GCUGCCCACGGUCAUCUCC | 364 |
| 703 | CCCCCCGCAGACGCUCAAC | 40 | 703 | CCCCCCGCAGACGCUCAAC | 40 | 725 | GUUGAGCGUCUGCGGGGGG | 365 |
| 721 | CAUCCUGGUGGAUACAGGC | 41 | 721 | CAUCCUGGUGGAUACAGGC | 41 | 743 | GCCUGUAUCCACCAGGAUG | 366 |
| 739 | CAGCAGUAACUUUGCAGUG | 42 | 739 | CAGCAGUAACUUUGCAGUG | 42 | 761 | CACUGCAAAGUUACUGCUG | 367 |
| 757 | GGGUGCUGCCCCCCACCCC | 43 | 757 | GGGUGCUGCCCCCCACCCC | 43 | 779 | GGGGUGGGGGGCAGCACCC | 368 |
| 775 | CUUCCUGCAUCGCUACUAC | 44 | 775 | CUUCCUGCAUCGCUACUAC | 44 | 797 | GUAGUAGCGAUGCAGGAAG | 369 |
| 793 | CCAGAGGCAGCUGUCCAGC | 45 | 793 | CCAGAGGCAGCUGUCCAGC | 45 | 815 | GCUGGACAGCUGCCUCUGG | 370 |
| 811 | CACAUACCGGGACCUCCGG | 46 | 811 | CACAUACCGGGACCUCCGG | 46 | 833 | CCGGAGGUCCCGGUAUGUG | 371 |
| 829 | GAAGGGUGUGUAUGUGCCC | 47 | 829 | GAAGGGUGUGUAUGUGCCC | 47 | 851 | GGGCACAUACACACCCUUC | 372 |
| 847 | CUACACCCAGGGCAAGUGG | 48 | 847 | CUACACCCAGGGCAAGUGG | 48 | 869 | CCACUUGCCCUGGGUGUAG | 373 |
| 865 | GGAAGGGGAGCUGGGCACC | 49 | 865 | GGAAGGGGAGCUGGGCACC | 49 | 887 | GGUGCCCAGCUCCCCUUCC | 374 |
| 883 | CGACCUGGUAAGCAUCCCC | 50 | 883 | CGACCUGGUAAGCAUCCCC | 50 | 905 | GGGGAUGCUUACCAGGUCG | 375 |
| 901 | CCAUGGCCCCAACGUCACU | 51 | 901 | CCAUGGCCCCAACGUCACU | 51 | 923 | AGUGACGUUGGGGCCAUGG | 376 |
| 919 | UGUGCGUGCCAACAUUGCU | 52 | 919 | UGUGCGUGCCAACAUUGCU | 52 | 941 | AGCAAUGUUGGCACGCACA | 377 |
| 937 | UGCCAUCACUGAAUCAGAC | 53 | 937 | UGCCAUCACUGAAUCAGAC | 53 | 959 | GUCUGAUUCAGUGAUGGCA | 378 |
| 955 | CAAGUUCUUCAUCAACGGC | 54 | 955 | CAAGUUCUUCAUCAACGGC | 54 | 977 | GCCGUUGAUGAAGAACUUG | 379 |
| 973 | CUCCAACUGGGAAGGCAUC | 55 | 973 | CUCCAACUGGGAAGGCAUC | 55 | 995 | GAUGCCUUCCCAGUUGGAG | 380 |
| 991 | CCUGGGGCUGGCCUAUGCU | 56 | 991 | CCUGGGGCUGGCCUAUGCU | 56 | 1013 | AGCAUAGGCCAGCCCCAGG | 381 |
| 1009 | UGAGAUUGCCAGGCCUGAC | 57 | 1009 | UGAGAUUGCCAGGCCUGAC | 57 | 1031 | GUCAGGCCUGGCAAUCUCA | 382 |
| 1027 | CGACUCCCUGGAGCCUUUC | 58 | 1027 | CGACUCCCUGGAGCCUUUC | 58 | 1049 | GAAAGGCUCCAGGGAGUCG | 383 |
| 1045 | CUUUGACUCUCUGGUAAAG | 59 | 1045 | CUUUGACUCUCUGGUAAAG | 59 | 1067 | CUUUACCAGAGAGUCAAAG | 384 |
| 1063 | GCAGACCCACGUUCCCAAC | 60 | 1063 | GCAGACCCACGUUCCCAAC | 60 | 1085 | GUUGGGAACGUGGGUCUGC | 385 |
| 1081 | CCUCUUCUCCCUGCAGCUU | 61 | 1081 | CCUCUUCUCCCUGCAGCUU | 61 | 1103 | AAGCUGCAGGGAGAAGAGG | 386 |
| 1099 | UUGUGGUGCUGGCUUCCCC | 62 | 1099 | UUGUGGUGCUGGCUUCCCC | 62 | 1121 | GGGGAAGCCAGCACCACAA | 387 |
| 1117 | CCUCAACCAGUCUGAAGUG | 63 | 1117 | CCUCAACCAGUCUGAAGUG | 63 | 1139 | CACUUCAGACUGGUUGAGG | 388 |
| 1135 | GCUGGCCUCUGUCGGAGGG | 64 | 1135 | GCUGGCCUCUGUCGGAGGG | 64 | 1157 | CCCUCCGACAGAGGCCAGC | 389 |
| 1153 | GAGCAUGAUCAUUGGAGGU | 65 | 1153 | GAGCAUGAUCAUUGGAGGU | 65 | 1175 | ACCUCCAAUGAUCAUGCUC | 390 |
| 1171 | UAUCGACCACUCGCUGUAC | 66 | 1171 | UAUCGACCACUCGCUGUAC | 66 | 1193 | GUACAGCGAGUGGUCGAUA | 391 |
| 1189 | CACAGGCAGUCUCUGGUAU | 67 | 1189 | CACAGGCAGUCUCUGGUAU | 67 | 1211 | AUACCAGAGACUGCCUGUG | 392 |
| 1207 | UACACCCAUCCGGCGGGAG | 68 | 1207 | UACACCCAUCCGGCGGGAG | 68 | 1229 | CUCCCGCCGGAUGGGUGUA | 393 |
| 1225 | GUGGUAUUAUGAGGUCAUC | 69 | 1225 | GUGGUAUUAUGAGGUCAUC | 69 | 1247 | GAUGACCUCAUAAUACCAC | 394 |
| 1243 | CAUUGUGCGGGUGGAGAUC | 70 | 1243 | CAUUGUGCGGGUGGAGAUC | 70 | 1265 | GAUCUCCACCCGCACAAUG | 395 |
| 1261 | CAAUGGACAGGAUCUGAAA | 71 | 1261 | CAAUGGACAGGAUCUGAAA | 71 | 1283 | UUUCAGAUCCUGUCCAUUG | 396 |
| 1279 | AAUGGACUGCAAGGAGUAC | 72 | 1279 | AAUGGACUGCAAGGAGUAC | 72 | 1301 | GUACUCCUUGCAGUCCAUU | 397 |
| 1297 | CAACUAUGACAAGAGCAUU | 73 | 1297 | CAACUAUGACAAGAGCAUU | 73 | 1319 | AAUGCUCUUGUCAUAGUUG | 398 |
| 1315 | UGUGGACAGUGGCACCACC | 74 | 1315 | UGUGGACAGUGGCACCACC | 74 | 1337 | GGUGGUGCCACUGUCCACA | 399 |
| 1333 | CAACCUUCGUUUGCCCAAG | 75 | 1333 | CAACCUUCGUUUGCCCAAG | 75 | 1355 | CUUGGGCAAACGAAGGUUG | 400 |
| 1351 | GAAAGUGUUUGAAGCUGCA | 76 | 1351 | GAAAGUGUUUGAAGCUGCA | 76 | 1373 | UGCAGCUUCAAACACUUUC | 401 |
| 1369 | AGUCAAAUCCAUCAAGGCA | 77 | 1369 | AGUCAAAUCCAUCAAGGCA | 77 | 1391 | UGCCUUGAUGGAUUUGACU | 402 |
| 1387 | AGCCUCCUCCACGGAGAAG | 78 | 1387 | AGCCUCCUCCACGGAGAAG | 78 | 1409 | CUUCUCCGUGGAGGAGGCU | 403 |
| 1405 | GUUCCCUGAUGGUUUCUGG | 79 | 1405 | GUUCCCUGAUGGUUUCUGG | 79 | 1427 | CCAGAAACCAUCAGGGAAC | 404 |
| 1423 | GCUAGGAGAGCAGCUGGUG | 80 | 1423 | GCUAGGAGAGCAGCUGGUG | 80 | 1445 | CACCAGCUGCUCUCCUAGC | 405 |
| 1441 | GUGCUGGCAAGCAGGCACC | 81 | 1441 | GUGCUGGCAAGCAGGCACC | 81 | 1463 | GGUGCCUGCUUGCCAGCAC | 406 |
| 1459 | CACCCCUUGGAACAUUUUC | 82 | 1459 | CACCCCUUGGAACAUUUUC | 82 | 1481 | GAAAAUGUUCCAAGGGGUG | 407 |
| 1477 | CCCAGUCAUCUCACUCUAC | 83 | 1477 | CCCAGUCAUCUCACUCUAC | 83 | 1499 | GUAGAGUGAGAUGACUGGG | 408 |
| 1495 | CCUAAUGGGUGAGGUUACC | 84 | 1495 | CCUAAUGGGUGAGGUUACC | 84 | 1517 | GGUAACCUCACCCAUUAGG | 409 |
| 1513 | CAACCAGUCCUUCCGCAUC | 85 | 1513 | CAACCAGUCCUUCCGCAUC | 85 | 1535 | GAUGCGGAAGGACUGGUUG | 410 |
| 1531 | CACCAUCCUUCCGCAGCAA | 86 | 1531 | CACCAUCCUUCCGCAGCAA | 86 | 1553 | UUGCUGCGGAAGGAUGGUG | 411 |
| 1549 | AUACCUGCGGCCAGUGGAA | 87 | 1549 | AUACCUGCGGCCAGUGGAA | 87 | 1571 | UUCCACUGGCCGCAGGUAU | 412 |
| 1567 | AGAUGUGGCCACGUCCCAA | 88 | 1567 | AGAUGUGGCCACGUCCCAA | 88 | 1589 | UUGGGACGUGGCCACAUCU | 413 |
| 1585 | AGACGACUGUUACAAGUUU | 89 | 1585 | AGACGACUGUUACAAGUUU | 89 | 1607 | AAACUUGUAACAGUCGUCU | 414 |
| 1603 | UGCCAUCUCACAGUCAUCC | 90 | 1603 | UGCCAUCUCACAGUCAUCC | 90 | 1625 | GGAUGACUGUGAGAUGGCA | 415 |
| 1621 | CACGGGCACUGUUAUGGGA | 91 | 1621 | CACGGGCACUGUUAUGGGA | 91 | 1643 | UCCCAUAACAGUGCCCGUG | 416 |
| 1639 | AGCUGUUAUCAUGGAGGGC | 92 | 1639 | AGCUGUUAUCAUGGAGGGC | 92 | 1661 | GCCCUCCAUGAUAACAGCU | 417 |
| 1657 | CUUCUACGUUGUCUUUGAU | 93 | 1657 | CUUCUACGUUGUCUUUGAU | 93 | 1679 | AUCAAAGACAACGUAGAAG | 418 |
| 1675 | UCGGGCCCGAAAACGAAUU | 94 | 1675 | UCGGGCCCGAAAACGAAUU | 94 | 1697 | AAUUCGUUUUCGGGCCCGA | 419 |
| 1693 | UGGCUUUGCUGUCAGCGCU | 95 | 1693 | UGGCUUUGCUGUCAGCGCU | 95 | 1715 | AGCGCUGACAGCAAAGCCA | 420 |
| 1711 | UUGCCAUGUGCACGAUGAG | 96 | 1711 | UUGCCAUGUGCACGAUGAG | 96 | 1733 | CUCAUCGUGCACAUGGCAA | 421 |
| 1729 | GUUCAGGACGGCAGCGGUG | 97 | 1729 | GUUCAGGACGGCAGCGGUG | 97 | 1751 | CACCGCUGCCGUCCUGAAC | 422 |
| 1747 | GGAAGGCCCUUUUGUCACC | 98 | 1747 | GGAAGGCCCUUUUGUCACC | 98 | 1769 | GGUGACAAAAGGGCCUUCC | 423 |
| 1765 | CUUGGACAUGGAAGACUGU | 99 | 1765 | CUUGGACAUGGAAGACUGU | 99 | 1787 | ACAGUCUUCCAUGUCCAAG | 424 |
| 1783 | UGGCUACAACAUUCCACAG | 100 | 1783 | UGGCUACAACAUUCCACAG | 100 | 1805 | CUGUGGAAUGUUGUAGCCA | 425 |
| 1801 | GACAGAUGAGUCAACCCUC | 101 | 1801 | GACAGAUGAGUCAACCCUC | 101 | 1823 | GAGGGUUGACUCAUCUGUC | 426 |
| 1819 | CAUGACCAUAGCCUAUGUC | 102 | 1819 | CAUGACCAUAGCCUAUGUC | 102 | 1841 | GACAUAGGCUAUGGUCAUG | 427 |
| 1837 | CAUGGCUGCCAUCUGCGCC | 103 | 1837 | CAUGGCUGCCAUCUGCGCC | 103 | 1859 | GGCGCAGAUGGCAGCCAUG | 428 |
| 1855 | CCUCUUCAUGCUGCCACUC | 104 | 1855 | CCUCUUCAUGCUGCCACUC | 104 | 1877 | GAGUGGCAGCAUGAAGAGG | 429 |
| 1873 | CUGCCUCAUGGUGUGUCAG | 105 | 1873 | CUGCCUCAUGGUGUGUCAG | 105 | 1895 | CUGACACACCAUGAGGCAG | 430 |
| 1891 | GUGGCGCUGCCUCCGCUGC | 106 | 1891 | GUGGCGCUGCCUCCGCUGC | 106 | 1913 | GCAGCGGAGGCAGCGCCAC | 431 |
| 1909 | CCUGCGCCAGCAGCAUGAU | 107 | 1909 | CCUGCGCCAGCAGCAUGAU | 107 | 1931 | AUCAUGCUGCUGGCGCAGG | 432 |
| 1927 | UGACUUUGCUGAUGACAUC | 108 | 1927 | UGACUUUGCUGAUGACAUC | 108 | 1949 | GAUGUCAUCAGCAAAGUCA | 433 |
| 1945 | CUCCCUGCUGAAGUGAGGA | 109 | 1945 | CUCCCUGCUGAAGUGAGGA | 109 | 1967 | UCCUCACUUCAGCAGGGAG | 434 |
| 1963 | AGGCCCAUGGGCAGAAGAU | 110 | 1963 | AGGCCCAUGGGCAGAAGAU | 110 | 1985 | AUCUUCUGCCCAUGGGCCU | 435 |
| 1981 | UAGAGAUUCCCCUGGACCA | 111 | 1981 | UAGAGAUUCCCCUGGACCA | 111 | 2003 | UGGUCCAGGGGAAUCUCUA | 436 |
| 1999 | ACACCUCCGUGGUUCACUU | 112 | 1999 | ACACCUCCGUGGUUCACUU | 112 | 2021 | AAGUGAACCACGGAGGUGU | 437 |
| 2017 | UUGGUCACAAGUAGGAGAC | 113 | 2017 | UUGGUCACAAGUAGGAGAC | 113 | 2039 | GUCUCCUACUUGUGACCAA | 438 |
| 2035 | CACAGAUGGCACCUGUGGC | 114 | 2035 | CACAGAUGGCACCUGUGGC | 114 | 2057 | GCCACAGGUGCCAUCUGUG | 439 |
| 2053 | CCAGAGCACCUCAGGACCC | 115 | 2053 | CCAGAGCACCUCAGGACCC | 115 | 2075 | GGGUCCUGAGGUGCUCUGG | 440 |
| 2071 | CUCCCCACCCACCAAAUGC | 116 | 2071 | CUCCCCACCCACCAAAUGC | 116 | 2093 | GCAUUUGGUGGGUGGGGAG | 441 |
| 2089 | CCUCUGCCUUGAUGGAGAA | 117 | 2089 | CCUCUGCCUUGAUGGAGAA | 117 | 2111 | UUCUCCAUCAAGGCAGAGG | 442 |
| 2107 | AGGAAAAGGCUGGCAAGGU | 118 | 2107 | AGGAAAAGGCUGGCAAGGU | 118 | 2129 | ACCUUGCCAGCCUUUUCCU | 443 |
| 2125 | UGGGUUCCAGGGACUGUAC | 119 | 2125 | UGGGUUCCAGGGACUGUAC | 119 | 2147 | GUACAGUCCCUGGAACCCA | 444 |
| 2143 | CCUGUAGGAAACAGAAAAG | 120 | 2143 | CCUGUAGGAAACAGAAAAG | 120 | 2165 | CUUUUCUGUUUCCUACAGG | 445 |
| 2161 | GAGAAGAAAGAAGCACUCU | 121 | 2161 | GAGAAGAAAGAAGCACUCU | 121 | 2183 | AGAGUGCUUCUUUCUUCUC | 446 |
| 2179 | UGCUGGCGGGAAUACUCUU | 122 | 2179 | UGCUGGCGGGAAUACUCUU | 122 | 2201 | AAGAGUAUUCCCGCCAGCA | 447 |
| 2197 | UGGUCACCUCAAAUUUAAG | 123 | 2197 | UGGUCACCUCAAAUUUAAG | 123 | 2219 | CUUAAAUUUGAGGUGACCA | 448 |
| 2215 | GUCGGGAAAUUCUGCUGCU | 124 | 2215 | GUCGGGAAAUUCUGCUGCU | 124 | 2237 | AGCAGCAGAAUUUCCCGAC | 449 |
| 2233 | UUGAAACUUCAGCCCUGAA | 125 | 2233 | UUGAAACUUCAGCCCUGAA | 125 | 2255 | UUCAGGGCUGAAGUUUCAA | 450 |
| 2251 | ACCUUUGUCCACCAUUCCU | 126 | 2251 | ACCUUUGUCCACCAUUCCU | 126 | 2273 | AGGAAUGGUGGACAAAGGU | 451 |
| 2269 | UUUAAAUUCUCCAACCCAA | 127 | 2269 | UUUAAAUUCUCCAACCCAA | 127 | 2291 | UUGGGUUGGAGAAUUUAAA | 452 |
| 2287 | AAGUAUUCUUCUUUUCUUA | 128 | 2287 | AAGUAUUCUUCUUUUCUUA | 128 | 2309 | UAAGAAAAGAAGAAUACUU | 453 |
| 2305 | AGUUUCAGAAGUACUGGCA | 129 | 2305 | AGUUUCAGAAGUACUGGCA | 129 | 2327 | UGCCAGUACUUCUGAAACU | 454 |
| 2323 | AUCACACGCAGGUUACCUU | 130 | 2323 | AUCACACGCAGGUUACCUU | 130 | 2345 | AAGGUAACCUGCGUGUGAU | 455 |
| 2341 | UGGCGUGUGUCCCUGUGGU | 131 | 2341 | UGGCGUGUGUCCCUGUGGU | 131 | 2363 | ACCACAGGGACACACGCCA | 456 |
| 2359 | UACCCUGGCAGAGAAGAGA | 132 | 2359 | UACCCUGGCAGAGAAGAGA | 132 | 2381 | UCUCUUCUCUGCCAGGGUA | 457 |
| 2377 | ACCAAGCUUGUUUCCCUGC | 133 | 2377 | ACCAAGCUUGUUUCCCUGC | 133 | 2399 | GCAGGGAAACAAGCUUGGU | 458 |
| 2395 | CUGGCCAAAGUCAGUAGGA | 134 | 2395 | CUGGCCAAAGUCAGUAGGA | 134 | 2417 | UCCUACUGACUUUGGCCAG | 459 |
| 2413 | AGAGGAUGCACAGUUUGCU | 135 | 2413 | AGAGGAUGCACAGUUUGCU | 135 | 2435 | AGCAAACUGUGCAUCCUCU | 460 |
| 2431 | UAUUUGCUUUAGAGACAGG | 136 | 2431 | UAUUUGCUUUAGAGACAGG | 136 | 2453 | CCUGUCUCUAAAGCAAAUA | 461 |
| 2449 | GGACUGUAUAAACAAGCCU | 137 | 2449 | GGACUGUAUAAACAAGCCU | 137 | 2471 | AGGCUUGUUUAUACAGUCC | 462 |
| 2467 | UAACAUUGGUGCAAAGAUU | 138 | 2467 | UAACAUUGGUGCAAAGAUU | 138 | 2489 | AAUCUUUGCACCAAUGUUA | 463 |
| 2485 | UGCCUCUUGAAUUAAAAAA | 139 | 2485 | UGCCUCUUGAAUUAAAAAA | 139 | 2507 | UUUUUUAAUUCAAGAGGCA | 464 |
| 2503 | AAAAAACUAGAUUGACUAU | 140 | 2503 | AAAAAACUAGAUUGACUAU | 140 | 2525 | AUAGUCAAUCUAGUUUUUU | 465 |
| 2521 | UUUAUACAAAUGGGGGCGG | 141 | 2521 | UUUAUACAAAUGGGGGCGG | 141 | 2543 | CCGCCCCCAUUUGUAUAAA | 466 |
| 2539 | GCUGGAAAGAGGAGAAGGA | 142 | 2539 | GCUGGAAAGAGGAGAAGGA | 142 | 2561 | UCCUUCUCCUCUUUCCAGC | 467 |
| 2557 | AGAGGGAGUACAAAGACAG | 143 | 2557 | AGAGGGAGUACAAAGACAG | 143 | 2579 | CUGUCUUUGUACUCCCUCU | 468 |
| 2575 | GGGAAUAGUGGGAUCAAAG | 144 | 2575 | GGGAAUAGUGGGAUCAAAG | 144 | 2597 | CUUUGAUCCCACUAUUCCC | 469 |
| 2593 | GCUAGGAAAGGCAGAAACA | 145 | 2593 | GCUAGGAAAGGCAGAAACA | 145 | 2615 | UGUUUCUGCCUUUCCUAGC | 470 |
| 2611 | ACAACCACUCACCAGUCCU | 146 | 2611 | ACAACCACUCACCAGUCCU | 146 | 2633 | AGGACUGGUGAGUGGUUGU | 471 |
| 2629 | UAGUUUUAGACCUCAUCUC | 147 | 2629 | UAGUUUUAGACCUCAUCUC | 147 | 2651 | GAGAUGAGGUCUAAAACUA | 472 |
| 2647 | CCAAGAUAGCAUCCCAUCU | 148 | 2647 | CCAAGAUAGCAUCCCAUCU | 148 | 2669 | AGAUGGGAUGCUAUCUUGG | 473 |
| 2665 | UCAGAAGAUGGGUGUUGUU | 149 | 2665 | UCAGAAGAUGGGUGUUGUU | 149 | 2687 | AACAACACCCAUCUUCUGA | 474 |
| 2683 | UUUCAAUGUUUUCUUUUCU | 150 | 2683 | UUUCAAUGUUUUCUUUUCU | 150 | 2705 | AGAAAAGAAAACAUUGAAA | 475 |
| 2701 | UGUGGUUGCAGCCUGACCA | 151 | 2701 | UGUGGUUGCAGCCUGACCA | 151 | 2723 | UGGUCAGGCUGCAACCACA | 476 |
| 2719 | AAAAGUGAGAUGGGAAGGG | 152 | 2719 | AAAAGUGAGAUGGGAAGGG | 152 | 2741 | CCCUUCCCAUCUCACUUUU | 477 |
| 2737 | GCUUAUCUAGCCAAAGAGC | 153 | 2737 | GCUUAUCUAGCCAAAGAGC | 153 | 2759 | GCUCUUUGGCUAGAUAAGC | 478 |
| 2755 | CUCUUUUUUAGCUCUCUUA | 154 | 2755 | CUCUUUUUUAGCUCUCUUA | 154 | 2777 | UAAGAGAGCUAAAAAAGAG | 479 |
| 2773 | AAAUGAAGUGCCCACUAAG | 155 | 2773 | AAAUGAAGUGCCCACUAAG | 155 | 2795 | CUUAGUGGGCACUUCAUUU | 480 |
| 2791 | GAAGUUCCACUUAACACAU | 156 | 2791 | GAAGUUCCACUUAACACAU | 156 | 2813 | AUGUGUUAAGUGGAACUUC | 481 |
| 2809 | UGAAUUUCUGCCAUAUUAA | 157 | 2809 | UGAAUUUCUGCCAUAUUAA | 157 | 2831 | UUAAUAUGGCAGAAAUUCA | 482 |
| 2827 | AUUUCAUUGUCUCUAUCUG | 158 | 2827 | AUUUCAUUGUCUCUAUCUG | 158 | 2849 | CAGAUAGAGACAAUGAAAU | 483 |
| 2845 | GAACCACCCUUUAUUCUAC | 159 | 2845 | GAACCACCCUUUAUUCUAC | 159 | 2867 | GUAGAAUAAAGGGUGGUUC | 484 |
| 2863 | CAUAUGAUAGGCAGCACUG | 160 | 2863 | CAUAUGAUAGGCAGCACUG | 160 | 2885 | CAGUGCUGCCUAUCAUAUG | 485 |
| 2881 | GAAAUAUCCUAACCCCCUA | 161 | 2881 | GAAAUAUCCUAACCCCCUA | 161 | 2903 | UAGGGGGUUAGGAUAUUUC | 486 |
| 2899 | AAGCUCCAGGUGCCCUGUG | 162 | 2899 | AAGCUCCAGGUGCCCUGUG | 162 | 2921 | CACAGGGCACCUGGAGCUU | 487 |
| 2917 | GGGAGAGCAACUGGACUAU | 163 | 2917 | GGGAGAGCAACUGGACUAU | 163 | 2939 | AUAGUCCAGUUGCUCUCCC | 488 |
| 2935 | UAGCAGGGCUGGGCUCUGU | 164 | 2935 | UAGCAGGGCUGGGCUCUGU | 164 | 2957 | ACAGAGCCCAGCCCUGCUA | 489 |
| 2953 | UCUUCCUGGUCAUAGGCUC | 165 | 2953 | UCUUCCUGGUCAUAGGCUC | 165 | 2975 | GAGCCUAUGACCAGGAAGA | 490 |
| 2971 | CACUCUUUCCCCCAAAUCU | 166 | 2971 | CACUCUUUCCCCCAAAUCU | 166 | 2993 | AGAUUUGGGGGAAAGAGUG | 491 |
| 2989 | UUCCUCUGGAGCUUUGCAG | 167 | 2989 | UUCCUCUGGAGCUUUGCAG | 167 | 3011 | CUGCAAAGCUCCAGAGGAA | 492 |
| 3007 | GCCAAGGUGCUAAAAGGAA | 168 | 3007 | GCCAAGGUGCUAAAAGGAA | 168 | 3029 | UUCCUUUUAGCACCUUGGC | 493 |
| 3025 | AUAGGUAGGAGACCUCUUC | 169 | 3025 | AUAGGUAGGAGACCUCUUC | 169 | 3047 | GAAGAGGUCUCCUACCUAU | 494 |
| 3043 | CUAUCUAAUCCUUAAAAGC | 170 | 3043 | CUAUCUAAUCCUUAAAAGC | 170 | 3065 | GCUUUUAAGGAUUAGAUAG | 495 |
| 3061 | CAUAAUGUUGAACAUUCAU | 171 | 3061 | CAUAAUGUUGAACAUUCAU | 171 | 3083 | AUGAAUGUUCAACAUUAUG | 496 |
| 3079 | UUCAACAGCUGAUGCCCUA | 172 | 3079 | UUCAACAGCUGAUGCCCUA | 172 | 3101 | UAGGGCAUCAGCUGUUGAA | 497 |
| 3097 | AUAACCCCUGCCUGGAUUU | 173 | 3097 | AUAACCCCUGCCUGGAUUU | 173 | 3119 | AAAUCCAGGCAGGGGUUAU | 498 |
| 3115 | UCUUCCUAUUAGGCUAUAA | 174 | 3115 | UCUUCCUAUUAGGCUAUAA | 174 | 3137 | UUAUAGCCUAAUAGGAAGA | 499 |
| 3133 | AGAAGUAGCAAGAUCUUUA | 175 | 3133 | AGAAGUAGCAAGAUCUUUA | 175 | 3155 | UAAAGAUCUUGCUACUUCU | 500 |
| 3151 | ACAUAAUUCAGAGUGGUUU | 176 | 3151 | ACAUAAUUCAGAGUGGUUU | 176 | 3173 | AAACCACUCUGAAUUAUGU | 501 |
| 3169 | UCAUUGCCUUCCUACCCUC | 177 | 3169 | UCAUUGCCUUCCUACCCUC | 177 | 3191 | GAGGGUAGGAAGGCAAUGA | 502 |
| 3187 | CUCUAAUGGCCCCUCCAUU | 178 | 3187 | CUCUAAUGGCCCCUCCAUU | 178 | 3209 | AAUGGAGGGGCCAUUAGAG | 503 |
| 3205 | UUAUUUGACUAAAGCAUCA | 179 | 3205 | UUAUUUGACUAAAGCAUCA | 179 | 3227 | UGAUGCUUUAGUCAAAUAA | 504 |
| 3223 | ACACAGUGGCACUAGCAUU | 180 | 3223 | ACACAGUGGCACUAGCAUU | 180 | 3245 | AAUGCUAGUGCCACUGUGU | 505 |
| 3241 | UAUACCAAGAGUAUGAGAA | 181 | 3241 | UAUACCAAGAGUAUGAGAA | 181 | 3263 | UUCUCAUACUCUUGGUAUA | 506 |
| 3259 | AAUACAGUGCUUUAUGGCU | 182 | 3259 | AAUACAGUGCUUUAUGGCU | 182 | 3281 | AGCCAUAAAGCACUGUAUU | 507 |
| 3277 | UCUAACAUUACUGCCUUCA | 183 | 3277 | UCUAACAUUACUGCCUUCA | 183 | 3299 | UGAAGGCAGUAAUGUUAGA | 508 |
| 3295 | AGUAUCAAGGCUGCCUGGA | 184 | 3295 | AGUAUCAAGGCUGCCUGGA | 184 | 3317 | UCCAGGCAGCCUUGAUACU | 509 |
| 3313 | AGAAAGGAUGGCAGCCUCA | 185 | 3313 | AGAAAGGAUGGCAGCCUCA | 185 | 3335 | UGAGGCUGCCAUCCUUUCU | 510 |
| 3331 | AGGGCUUCCUUAUGUCCUC | 186 | 3331 | AGGGCUUCCUUAUGUCCUC | 186 | 3353 | GAGGACAUAAGGAAGCCCU | 511 |
| 3349 | CCACCACAAGAGCUCCUUG | 187 | 3349 | CCACCACAAGAGCUCCUUG | 187 | 3371 | CAAGGAGCUCUUGUGGUGG | 512 |
| 3367 | GAUGAAGGUCAUCUUUUUC | 188 | 3367 | GAUGAAGGUCAUCUUUUUC | 188 | 3389 | GAAAAAGAUGACCUUCAUC | 513 |
| 3385 | CCCCUAUCCUGUUCUUCCC | 189 | 3385 | CCCCUAUCCUGUUCUUCCC | 189 | 3407 | GGGAAGAACAGGAUAGGGG | 514 |
| 3403 | CCUCCCCGCUCCUAAUGGU | 190 | 3403 | CCUCCCCGCUCCUAAUGGU | 190 | 3425 | ACCAUUAGGAGCGGGGAGG | 515 |
| 3421 | UACGUGGGUACCCAGGCUG | 191 | 3421 | UACGUGGGUACCCAGGCUG | 191 | 3443 | CAGCCUGGGUACCCACGUA | 516 |
| 3439 | GGUUCUUGGGCUAGGUAGU | 192 | 3439 | GGUUCUUGGGCUAGGUAGU | 192 | 3461 | ACUACCUAGCCCAAGAACC | 517 |
| 3457 | UGGGGACCAAGUUCAUUAC | 193 | 3457 | UGGGGACCAAGUUCAUUAC | 193 | 3479 | GUAAUGAACUUGGUCCCCA | 518 |
| 3475 | CCUCCCUAUCAGUUCUAGC | 194 | 3475 | CCUCCCUAUCAGUUCUAGC | 194 | 3497 | GCUAGAACUGAUAGGGAGG | 519 |
| 3493 | CAUAGUAAACUACGGUACC | 195 | 3493 | CAUAGUAAACUACGGUACC | 195 | 3515 | GGUACCGUAGUUUACUAUG | 520 |
| 3511 | CAGUGUUAGUGGGAAGAGC | 196 | 3511 | CAGUGUUAGUGGGAAGAGC | 196 | 3533 | GCUCUUCCCACUAACACUG | 521 |
| 3529 | CUGGGUUUUCCUAGUAUAC | 197 | 3529 | CUGGGUUUUCCUAGUAUAC | 197 | 3551 | GUAUACUAGGAAAACCCAG | 522 |
| 3547 | CCCACUGCAUCCUACUCCU | 198 | 3547 | CCCACUGCAUCCUACUCCU | 198 | 3569 | AGGAGUAGGAUGCAGUGGG | 523 |
| 3565 | UACCUGGUCAACCCGCUGC | 199 | 3565 | UACCUGGUCAACCCGCUGC | 199 | 3587 | GCAGCGGGUUGACCAGGUA | 524 |
| 3583 | CUUCCAGGUAUGGGACCUG | 200 | 3583 | CUUCCAGGUAUGGGACCUG | 200 | 3605 | CAGGUCCCAUACCUGGAAG | 525 |
| 3601 | GCUAAGUGUGGAAUUACCU | 201 | 3601 | GCUAAGUGUGGAAUUACCU | 201 | 3623 | AGGUAAUUCCACACUUAGC | 526 |
| 3619 | UGAUAAGGGAGAGGGAAAU | 202 | 3619 | UGAUAAGGGAGAGGGAAAU | 202 | 3641 | AUUUCCCUCUCCCUUAUCA | 527 |
| 3637 | UACAAGGAGGGCCUCUGGU | 203 | 3637 | UACAAGGAGGGCCUCUGGU | 203 | 3659 | ACCAGAGGCCCUCCUUGUA | 528 |
| 3655 | UGUUCCUGGCCUCAGCCAG | 204 | 3655 | UGUUCCUGGCCUCAGCCAG | 204 | 3677 | CUGGCUGAGGCCAGGAACA | 529 |
| 3673 | GCUGCCCACAAGCCAUAAA | 205 | 3673 | GCUGCCCACAAGCCAUAAA | 205 | 3695 | UUUAUGGCUUGUGGGCAGC | 530 |
| 3691 | ACCAAUAAAACAAGAAUAC | 206 | 3691 | ACCAAUAAAACAAGAAUAC | 206 | 3713 | GUAUUCUUGUUUUAUUGGU | 531 |
| 3709 | CUGAGUCAGUUUUUUAUCU | 207 | 3709 | CUGAGUCAGUUUUUUAUCU | 207 | 3731 | AGAUAAAAAACUGACUCAG | 532 |
| 3727 | UGGGUUCUCUUCAUUCCCA | 208 | 3727 | UGGGUUCUCUUCAUUCCCA | 208 | 3749 | UGGGAAUGAAGAGAACCCA | 533 |
| 3745 | ACUGCACUUGGUGCUGCUU | 209 | 3745 | ACUGCACUUGGUGCUGCUU | 209 | 3767 | AAGCAGCACCAAGUGCAGU | 534 |
| 3763 | UUGGCUGACUGGGAACACC | 210 | 3763 | UUGGCUGACUGGGAACACC | 210 | 3785 | GGUGUUCCCAGUCAGCCAA | 535 |
| 3781 | CCCAUAACUACAGAGUCUG | 211 | 3781 | CCCAUAACUACAGAGUCUG | 211 | 3803 | CAGACUCUGUAGUUAUGGG | 536 |
| 3799 | GACAGGAAGACUGGAGACU | 212 | 3799 | GACAGGAAGACUGGAGACU | 212 | 3821 | AGUCUCCAGUCUUCCUGUC | 537 |
| 3817 | UGUCCACUUCUAGCUCGGA | 213 | 3817 | UGUCCACUUCUAGCUCGGA | 213 | 3839 | UCCGAGCUAGAAGUGGACA | 538 |
| 3835 | AACUUACUGUGUAAAUAAA | 214 | 3835 | AACUUACUGUGUAAAUAAA | 214 | 3857 | UUUAUUUACACAGUAAGUU | 539 |
| 3853 | ACUUUCAGAACUGCUACCA | 215 | 3853 | ACUUUCAGAACUGCUACCA | 215 | 3875 | UGGUAGCAGUUCUGAAAGU | 540 |
| 3871 | AUGAAGUGAAAAUGCCACA | 216 | 3871 | AUGAAGUGAAAAUGCCACA | 216 | 3893 | UGUGGCAUUUUCACUUCAU | 541 |
| 3889 | AUUUUGCUUUAUAAUUUCU | 217 | 3889 | AUUUUGCUUUAUAAUUUCU | 217 | 3911 | AGAAAUUAUAAAGCAAAAU | 542 |
| 3907 | UACCCAUGUUGGGAAAAAC | 218 | 3907 | UACCCAUGUUGGGAAAAAC | 218 | 3929 | GUUUUUCCCAACAUGGGUA | 543 |
| 3925 | CUGGCUUUUUCCCAGCCCU | 219 | 3925 | CUGGCUUUUUCCCAGCCCU | 219 | 3947 | AGGGCUGGGAAAAAGCCAG | 544 |
| 3943 | UUUCCAGGGCAUAAAACUC | 220 | 3943 | UUUCCAGGGCAUAAAACUC | 220 | 3965 | GAGUUUUAUGCCCUGGAAA | 545 |
| 3961 | CAACCCCUUCGAUAGCAAG | 221 | 3961 | CAACCCCUUCGAUAGCAAG | 221 | 3983 | CUUGCUAUCGAAGGGGUUG | 546 |
| 3979 | GUCCCAUCAGCCUAUUAUU | 222 | 3979 | GUCCCAUCAGCCUAUUAUU | 222 | 4001 | AAUAAUAGGCUGAUGGGAC | 547 |
| 3997 | UUUUUUAAAGAAAACUUGC | 223 | 3997 | UUUUUUAAAGAAAACUUGC | 223 | 4019 | GCAAGUUUUCUUUAAAAAA | 548 |
| 4015 | CACUUGUUUUUCUUUUUAC | 224 | 4015 | CACUUGUUUUUCUUUUUAC | 224 | 4037 | GUAAAAAGAAAAACAAGUG | 549 |
| 4033 | CAGUUACUUCCUUCCUGCC | 225 | 4033 | CAGUUACUUCCUUCCUGCC | 225 | 4055 | GGCAGGAAGGAAGUAACUG | 550 |
| 4051 | CCCAAAAUUAUAAACUCUA | 226 | 4051 | CCCAAAAUUAUAAACUCUA | 226 | 4073 | UAGAGUUUAUAAUUUUGGG | 551 |
| 4069 | AAGUGUAAAAAA,4AGUCUU | 227 | 4069 | AAGUGUAAAAAAAAGUCUU | 227 | 4091 | AAGACUUUUUUUUACACUU | 552 |
| 4087 | UAACAACAGCUUCUUGCUU | 228 | 4087 | UAACAACAGCUUCUUGCUU | 228 | 4109 | AAGCAAGAAGCUGUUGUUA | 553 |
| 4105 | UGUAAAAAUAUGUAUUAUA | 229 | 4105 | UGUAAAAAUAUGUAUUAUA | 229 | 4127 | UAUAAUACAUAUUUUUACA | 554 |
| 4123 | ACAUCUGUAUUUUUAAAUU | 230 | 4123 | ACAUCUGUAUUUUUAAAUU | 230 | 4145 | AAUUUAAAAAUACAGAUGU | 555 |
| 4141 | UCUGCUCCUGAAAAAUGAC | 231 | 4141 | UCUGCUCCUGAAAAAUGAC | 231 | 4163 | GUCAUUUUUCAGGAGCAGA | 556 |
| 4159 | CUGUCCCAUUCUCCACUCA | 232 | 4159 | CUGUCCCAUUCUCCACUCA | 232 | 4181 | UGAGUGGAGAAUGGGACAG | 557 |
| 4177 | ACUGCAUUUGGGGCCUUUC | 233 | 4177 | ACUGCAUUUGGGGCCUUUC | 233 | 4199 | GAAAGGCCCCAAAUGCAGU | 558 |
| 4195 | CCCAUUGGUCUGCAUGUCU | 234 | 4195 | CCCAUUGGUCUGCAUGUCU | 234 | 4217 | AGACAUGCAGACCAAUGGG | 559 |
| 4213 | UUUUAUCAUUGCAGGCCAG | 235 | 4213 | UUUUAUCAUUGCAGGCCAG | 235 | 4235 | CUGGCCUGCAAUGAUAAAA | 560 |
| 4231 | GUGGACAGAGGGAGAAGGG | 236 | 4231 | GUGGACAGAGGGAGAAGGG | 236 | 4253 | CCCUUCUCCCUCUGUCCAC | 561 |
| 4249 | GAGAACAGGGGUCGCCAAC | 237 | 4249 | GAGAACAGGGGUCGCCAAC | 237 | 4271 | GUUGGCGACCCCUGUUCUC | 562 |
| 4267 | CACUUGUGUUGCUUUCUGA | 238 | 4267 | CACUUGUGUUGCUUUCUGA | 238 | 4289 | UCAGAAAGCAACACAAGUG | 563 |
| 4285 | ACUGAUCCUGAACAAGAAA | 239 | 4285 | ACUGAUCCUGAACAAGAAA | 239 | 4307 | UUUCUUGUUCAGGAUCAGU | 564 |
| 4303 | AGAGUAACACUGAGGCGCU | 240 | 4303 | AGAGUAACACUGAGGCGCU | 240 | 4325 | AGCGCCUCAGUGUUACUCU | 565 |
| 4321 | UCGCUCCCAUGCACAACUC | 241 | 4321 | UCGCUCCCAUGCACAACUC | 241 | 4343 | GAGUUGUGCAUGGGAGCGA | 566 |
| 4339 | CUCCAAAACACUUAUCCUC | 242 | 4339 | CUCCAAAACACUUAUCCUC | 242 | 4361 | GAGGAUAAGUGUUUUGGAG | 567 |
| 4357 | CCUGCAAGAGUGGGCUUUC | 243 | 4357 | CCUGCAAGAGUGGGCUUUC | 243 | 4379 | GAAAGCCCACUCUUGCAGG | 568 |
| 4375 | CCAGGGUCUUUACUGGGAA | 244 | 4375 | CCAGGGUCUUUACUGGGAA | 244 | 4397 | UUCCCAGUAAAGACCCUGG | 569 |
| 4393 | AGCAGUUAAGCCCCCUCCU | 245 | 4393 | AGCAGUUAAGCCCCCUCCU | 245 | 4415 | AGGAGGGGGCUUAACUGCU | 570 |
| 4411 | UCACCCCUUCCUUUUUUCU | 246 | 4411 | UCACCCCUUCCUUUUUUCU | 246 | 4433 | AGAAAAAAGGAAGGGGUGA | 571 |
| 4429 | UUUCUUUACUCCUUUGGCU | 247 | 4429 | UUUCUUUACUCCUUUGGCU | 247 | 4451 | AGCCAAAGGAGUAAAGAAA | 572 |
| 4447 | UUCAAAGGAUUUUGGAAAA | 248 | 4447 | UUCAAAGGAUUUUGGAAAA | 248 | 4469 | UUUUCCAAAAUCCUUUGAA | 573 |
| 4465 | AGAAACAAUAUGCUUUACA | 249 | 4465 | AGAAACAAUAUGCUUUACA | 249 | 4487 | UGUAAAGCAUAUUGUUUCU | 574 |
| 4483 | ACUCAUUUUCAAUUUCUAA | 250 | 4483 | ACUCAUUUUCAAUUUCUAA | 250 | 4505 | UUAGAAAUUGAAAAUGAGU | 575 |
| 4501 | AAUUUGCAGGGGAUACUGA | 251 | 4501 | AAUUUGCAGGGGAUACUGA | 251 | 4523 | UCAGUAUCCCCUGCAAAUU | 576 |
| 4519 | AAAAAUACGGCAGGUGGCC | 252 | 4519 | AAAAAUACGGCAGGUGGCC | 252 | 4541 | GGCCACCUGCCGUAUUUUU | 577 |
| 4537 | CUAAGGCUGCUGUAAAGUU | 253 | 4537 | CUAAGGCUGCUGUAAAGUU | 253 | 4559 | AACUUUACAGCAGCCUUAG | 578 |
| 4555 | UGAGGGGAGAGGAAAUCUU | 254 | 4555 | UGAGGGGAGAGGAAAUCUU | 254 | 4577 | AAGAUUUCCUCUCCCCUCA | 579 |
| 4573 | UAAGAUUACAAGAUAAAAA | 255 | 4573 | UAAGAUUACAAGAUAAAAA | 255 | 4595 | UUUUUAUCUUGUAAUCUUA | 580 |
| 4591 | AACGAAUCCCCUAAACAAA | 256 | 4591 | AACGAAUCCCCUAAACAAA | 256 | 4613 | UUUGUUUAGGGGAUUCGUU | 581 |
| 4609 | AAAGAACAAUAGAACUGGU | 257 | 4609 | AAAGAACAAUAGAACUGGU | 257 | 4631 | ACCAGUUCUAUUGUUCUUU | 582 |
| 4627 | UCUUCCAUUUUGCCACCUU | 258 | 4627 | UCUUCCAUUUUGCCACCUU | 258 | 4649 | AAGGUGGCAAAAUGGAAGA | 583 |
| 4645 | UUCCUGUUCAUGACAGCUA | 259 | 4645 | UUCCUGUUCAUGACAGCUA | 259 | 4667 | UAGCUGUCAUGAACAGGAA | 584 |
| 4663 | ACUAACCUGGAGACAGUAA | 260 | 4663 | ACUAACCUGGAGACAGUAA | 260 | 4685 | UUACUGUCUCCAGGUUAGU | 585 |
| 4681 | ACAUUUCAUUAACCAAAGA | 261 | 4681 | ACAUUUCAUUAACCAAAGA | 261 | 4703 | UCUUUGGUUAAUGAAAUGU | 586 |
| 4699 | AAAGUGGGUCACCUGACCU | 262 | 4699 | AAAGUGGGUCACCUGACCU | 262 | 4721 | AGGUCAGGUGACCCACUUU | 587 |
| 4717 | UCUGAAGAGCUGAGUACUC | 263 | 4717 | UCUGAAGAGCUGAGUACUC | 263 | 4739 | GAGUACUCAGCUCUUCAGA | 588 |
| 4735 | CAGGCCACUCCAAUCACCC | 264 | 4735 | CAGGCCACUCCAAUCACCC | 264 | 4757 | GGGUGAUUGGAGUGGCCUG | 589 |
| 4753 | CUACAAGAUGCCAAGGAGG | 265 | 4753 | CUACAAGAUGCCAAGGAGG | 265 | 4775 | CCUCCUUGGCAUCUUGUAG | 590 |
| 4771 | GUCCCAGGAAGUCCAGCUC | 266 | 4771 | GUCCCAGGAAGUCCAGCUC | 266 | 4793 | GAGCUGGACUUCCUGGGAC | 591 |
| 4789 | CCUUAAACUGACGCUAGUC | 267 | 4789 | CCUUAAACUGACGCUAGUC | 267 | 4811 | GACUAGCGUCAGUUUAAGG | 592 |
| 4807 | CAAUAAACCUGGGCAAGUG | 268 | 4807 | CAAUAAACCUGGGCAAGUG | 268 | 4829 | CACUUGCCCAGGUUUAUUG | 593 |
| 4825 | GAGGCAAGAGAAAUGAGGA | 269 | 4825 | GAGGCAAGAGAAAUGAGGA | 269 | 4847 | UCCUCAUUUCUCUUGCCUC | 594 |
| 4843 | AAGAAUCCAUCUGUGAGGU | 270 | 4843 | AAGAAUCCAUCUGUGAGGU | 270 | 4865 | ACCUCACAGAUGGAUUCUU | 595 |
| 4861 | UGACAGGCAAGGAUGAAAG | 271 | 4861 | UGACAGGCAAGGAUGAAAG | 271 | 4883 | CUUUCAUCCUUGCCUGUCA | 596 |
| 4879 | GACAAAGAAGGAAAAGAGU | 272 | 4879 | GACAAAGAAGGAAAAGAGU | 272 | 4901 | ACUCUUUUCCUUCUUUGUC | 597 |
| 4897 | UAUCAAAGGCAGAAAGGAG | 273 | 4897 | UAUCAAAGGCAGAAAGGAG | 273 | 4919 | CUCCUUUCUGCCUUUGAUA | 598 |
| 4915 | GAUCAUUUAGUUGGGUCUG | 274 | 4915 | GAUCAUUUAGUUGGGUCUG | 274 | 4937 | CAGACCCAACUAAAUGAUC | 599 |
| 4933 | GAAAGGAAAAGUCUUUGCU | 275 | 4933 | GAAAGGAAAAGUCUUUGCU | 275 | 4955 | AGCAAAGACUUUUCCUUUC | 600 |
| 4951 | UAUCCGACAUGUACUGCUA | 276 | 4951 | UAUCCGACAUGUACUGCUA | 276 | 4973 | UAGCAGUACAUGUCGGAUA | 601 |
| 4969 | AGUACCUGUAAGCAUUUUA | 277 | 4969 | AGUACCUGUAAGCAUUUUA | 277 | 4991 | UAAAAUGCUUACAGGUACU | 602 |
| 4987 | AGGUCCCAGAAUGGAAAAA | 278 | 4987 | AGGUCCCAGAAUGGAAAAA | 278 | 5009 | UUUUUCCAUUCUGGGACCU | 603 |
| 5005 | AAAAAUCAGCUAUUGGUAA | 279 | 5005 | AAAAAUCAGCUAUUGGUAA | 279 | 5027 | UUACCAAUAGCUGAUUUUU | 604 |
| 5023 | AUAUAAUAAUGUCCUUUCC | 280 | 5023 | AUAUAAUAAUGUCCUUUCC | 280 | 5045 | GGAAAGGACAUUAUUAUAU | 605 |
| 5041 | CCUGGAGUCAGUUUUUUUA | 281 | 5041 | CCUGGAGUCAGUUUUUUUA | 281 | 5063 | UAAAAAAACUGACUCCAGG | 606 |
| 5059 | AAAAAGUUAACUCUUAGUU | 282 | 5059 | AAAAAGUUAACUCUUAGUU | 282 | 5081 | AACUAAGAGUUAACUUUUU | 607 |
| 5077 | UUUUACUUGUUUAAUUCUA | 283 | 5077 | UUUUACUUGUUUAAUUCUA | 283 | 5099 | UAGAAUUAAACAAGUAAAA | 608 |
| 5095 | AAAAGAGAAGGGAGCUGAG | 284 | 5095 | AAAAGAGAAGGGAGCUGAG | 284 | 5117 | CUCAGCUCCCUUCUCUUUU | 609 |
| 5113 | GGCCAUUCCCUGUAGGAGU | 285 | 5113 | GGCCAUUCCCUGUAGGAGU | 285 | 5135 | ACUCCUACAGGGAAUGGCC | 610 |
| 5131 | UAAAGAUAAAAGGAUAGGA | 286 | 5131 | UAAAGAUAAAAGGAUAGGA | 286 | 5153 | UCCUAUCCUUUUAUCUUUA | 611 |
| 5149 | AAAAGAUUCAAAGCUCUAA | 287 | 5149 | AAAAGAUUCAAAGCUCUAA | 287 | 5171 | UUAGAGCUUUGAAUCUUUU | 612 |
| 5167 | AUAGAGUCACAGCUUUCCC | 288 | 5167 | AUAGAGUCACAGCUUUCCC | 288 | 5189 | GGGAAAGCUGUGACUCUAU | 613 |
| 5185 | CAGGUAUAAAACCUAAAAU | 289 | 5185 | CAGGUAUAAAACCUAAAAU | 289 | 5207 | AUUUUAGGUUUUAUACCUG | 614 |
| 5203 | UUAAGAAGUACAAUAAGCA | 290 | 5203 | UUAAGAAGUACAAUAAGCA | 290 | 5225 | UGCUUAUUGUACUUCUUAA | 615 |
| 5221 | AGAGGUGGAAAAUGAUCUA | 291 | 5221 | AGAGGUGGAAAAUGAUCUA | 291 | 5243 | UAGAUCAUUUUCCACCUCU | 616 |
| 5239 | AGUUCCUGAUAGCUACCCA | 292 | 5239 | AGUUCCUGAUAGCUACCCA | 292 | 5261 | UGGGUAGCUAUCAGGAACU | 617 |
| 5257 | ACAGAGCAAGUGAUUUAUA | 293 | 5257 | ACAGAGCAAGUGAUUUAUA | 293 | 5279 | UAUAAAUCACUUGCUCUGU | 618 |
| 5275 | AAAUUUGAAAUCCAAACUA | 294 | 5275 | AAAUUUGAAAUCCAAACUA | 294 | 5297 | UAGUUUGGAUUUCAAAUUU | 619 |
| 5293 | ACUUUCUUAAUAUCACUUU | 295 | 5293 | ACUUUCUUAAUAUCACUUU | 295 | 5315 | AAAGUGAUAUUAAGAAAGU | 620 |
| 5311 | UGGUCUCCAUUUUUCCCAG | 296 | 5311 | UGGUCUCCAUUUUUCCCAG | 296 | 5333 | CUGGGAAAAAUGGAGACCA | 621 |
| 5329 | GGACAGGAAAUAUGUCCCC | 297 | 5329 | GGACAGGAAAUAUGUCCCC | 297 | 5351 | GGGGACAUAUUUCCUGUCC | 622 |
| 5347 | CCCCUAACUUUCUUGCUUC | 298 | 5347 | CCCCUAACUUUCUUGCUUC | 298 | 5369 | GAAGCAAGAAAGUUAGGGG | 623 |
| 5365 | CAAAAAUUAAAAUCCAGCA | 299 | 5365 | CAAAAAUUAAAAUCCAGCA | 299 | 5387 | UGCUGGAUUUUAAUUUUUG | 624 |
| 5383 | AUCCCAAGAUCAUUCUACA | 300 | 5383 | AUCCCAAGAUCAUUCUACA | 300 | 5405 | UGUAGAAUGAUCUUGGGAU | 625 |
| 5401 | AAGUAAUUUUGCACAGACA | 301 | 5401 | AAGUAAUUUUGCACAGACA | 301 | 5423 | UGUCUGUGCAAAAUUACUU | 626 |
| 5419 | AUCUCCUCACCCCAGUGCC | 302 | 5419 | AUCUCCUCACCCCAGUGCC | 302 | 5441 | GGCACUGGGGUGAGGAGAU | 627 |
| 5437 | CUGUCUGGAGCUCACCCAA | 303 | 5437 | CUGUCUGGAGCUCACCCAA | 303 | 5459 | UUGGGUGAGCUCCAGACAG | 628 |
| 5455 | AGGUCACCAAACAACUUGG | 304 | 5455 | AGGUCACCAAACAACUUGG | 304 | 5477 | CCAAGUUGUUUGGUGACCU | 629 |
| 5473 | GUUGUGAACCAACUGCCUU | 305 | 5473 | GUUGUGAACCAACUGCCUU | 305 | 5495 | AAGGCAGUUGGUUCACAAC | 630 |
| 5491 | UAACCUUCUGGGGGAGGGG | 306 | 5491 | UAACCUUCUGGGGGAGGGG | 306 | 5513 | CCCCUCCCCCAGAAGGUUA | 631 |
| 5509 | GGAUUAGCUAGACUAGGAG | 307 | 5509 | GGAUUAGCUAGACUAGGAG | 307 | 5531 | CUCCUAGUCUAGCUAAUCC | 632 |
| 5527 | GACCAGAAGUGAAUGGGAA | 308 | 5527 | GACCAGAAGUGAAUGGGAA | 308 | 5549 | UUCCCAUUCACUUCUGGUC | 633 |
| 5545 | AAGGGUGAGGACUUCACAA | 309 | 5545 | AAGGGUGAGGACUUCACAA | 309 | 5567 | UUGUGAAGUCCUCACCCUU | 634 . |
| 5563 | AUGUUGGCCUGUCAGAGCU | 310 | 5563 | AUGUUGGCCUGUCAGAGCU | 310 | 5585 | AGCUCUGACAGGCCAACAU | 635 |
| 5581 | UUGAUUAGAAGCCAAGACA | 311 | 5581 | UUGAUUAGAAGCCAAGACA | 311 | 5603 | UGUCUUGGCUUCUAAUCAA | 636 |
| 5599 | AGUGGCAGCAAAGGAAGAC | 312 | 5599 | AGUGGCAGCAAAGGAAGAC | 312 | 5621 | GUCUUCCUUUGCUGCCACU | 637 |
| 5617 | CUUGGCCCAGGAAAAACCU | 313 | 5617 | CUUGGCCCAGGAAAAACCU | 313 | 5639 | AGGUUUUUCCUGGGCCAAG | 638 |
| 5635 | UGUGGGUUGUGCUAAUUUC | 314 | 5635 | UGUGGGUUGUGCUAAUUUC | 314 | 5657 | GAAAUUAGCACAACCCACA | 639 |
| 5653 | CUGUCCAGAAAAUAGGGUG | 315 | 5653 | CUGUCCAGAAAAUAGGGUG | 315 | 5675 | CACCCUAUUUUCUGGACAG | 640 |
| 5671 | GGACAGAAGCUUGUGGGGU | 316 | 5671 | GGACAGAAGCUUGUGGGGU | 316 | 5693 | ACCCCACAAGCUUCUGUCC | 641 |
| 5689 | UGCAUGGAGGAAUUGGGAC | 317 | 5689 | UGCAUGGAGGAAUUGGGAC | 317 | 5711 | GUCCCAAUUCCUCCAUGCA | 642 |
| 5707 | CCUGGUUAUGUUGUUAUUC | 318 | 5707 | CCUGGUUAUGUUGUUAUUC | 318 | 5729 | GAAUAACAACAUAACCAGG | 643 |
| 5725 | CUCGGACUGUGAAUUUUGG | 319 | 5725 | CUCGGACUGUGAAUUUUGG | 319 | 5747 | CCAAAAUUCACAGUCCGAG | 644 |
| 5743 | GUGAUGUAAAACAGAAUAU | 320 | 5743 | GUGAUGUAAAACAGAAUAU | 320 | 5765 | AUAUUCUGUUUUACAUCAC | 645 |
| 5761 | UUCUGUAAACCUAAUGUCU | 321 | 5761 | UUCUGUAAACCUAAUGUCU | 321 | 5783 | AGACAUUAGGUUUACAGAA | 646 |
| 5779 | UGUAUAAAUAAUGAGCGUU | 322 | 5779 | UGUAUAAAUAAUGAGCGUU | 322 | 5801 | AACGCUCAUUAUUUAUACA | 647 |
| 5797 | UAACACAGUAAAAUAUUCA | 323 | 5797 | UAACACAGUAAAAUAUUCA | 323 | 5819 | UGAAUAUUUUACUGUGUUA | 648 |
| 5815 | AAUAAGAAGUCAAAAAAAA | 324 | 5815 | AAUAAGAAGUCAAAAAAAA | 324 | 5837 | UUUUUUUUGACUUCUUAUU | 649 |
| 5821 | AAGUCAAAAAAAAAAAAAA | 325 | 5821 | AAGUCAAAAAAAAAAAAAA | 325 | 5843 | UUUUUUUUUUUUUUGACUU | 650 |

The 3'-ends of the Upper sequence and the Lower sequence of the siNA construct can include an overhang sequence, for example about 1, 2, 3, or 4 nucleotides in length, preferably 2 nucleotides in length, wherein the overhanging sequence of the lower sequence is optionally complementary to a portion of the target sequence. The upper sequence is also referred to as the sense strand, whereas the lower sequence is also referred to as the antisense strand. The upper and lower sequences in the Table can further comprise a chemical modification having Formulae I-VII or any combination thereof

**Table III: BACE Synthetic Modified siNA constructs**

| Target Pos | Target | Seq ID | RPI# | Aliases | Sequence | Seq ID |
|---|---|---|---|---|---|---|
| 1490 | AAUGGGUGAGGUUACCAACCAGU | 651 | 31005 | BACE:1492U21 siRNA sense | UGGGUGAGGUUACCAACCATT | 655 |
| 1753 | UCACCUUGGACAUGGAAGACUGU | 652 | 31006 | BACE:1755U21 siRNA sense | ACCUUGGACAUGGAAGACUTT | 656 |
| 2457 | CCUAACAUUGGUGCAAAGAUUGC | 653 | 31007 | BACE:2459U21 siRNA sense | UAACAUUGGUGCAAAGAUUTT | 657 |
| 3563 | UAUGGGACCUGCUAAGUGUGGAA | 654 | 31008 | BACE:3585U21 siRNA sense | UGGGACCUGCUAAGUGUGGTT | 658 |
| 1490 | AAUGGGUGAGGUUACCAACCAGU | 651 | 31081 | BACE:1510L21 siRNA (1492C) antisense | UGGUUGGUAACCUCACCCATT | 659 |
| 1753 | UCACCUUGGACAUGGAAGACUGU | 652 | 31082 | BACE:1773L21 siRNA (1755C) antisense | AGUCUUCCAUGUCCAAGGUTT | 660 |
| 2457 | CCUAACAUUGGUGCAAAGAUUGC | 653 | 31083 | BACE:2477L21 siRNA (2459C) antlsense | AAUCUUUGCACCAAUGUUATT | 661 |
| 3583 | UAUGGGACCUGCUAAGUGUGGAA | 654 | 31084 | BACE:3603L21 siRNA (3585C) antisense | CCACACUUAGCAGGUCCCATT | 662 |
| 1490 | AAUGGGUGAGGUUACCAACCAGU | 651 | 30729 | BACE:1492U21 siRNA stab04 sense | B uGGGuGAGGuuAccAAccATT B | 663 |
| 1753 | UCACCUUGGACAUGGAAGACUGU | 652 | 30730 | BACE:1755U21 siRNA stab04 sense | B AccuuGGAcAuGGAAGAcuTT B | 664 |
| 2457 | CCUAACAUUGGUGCAAAGAUUGC | 653 | 31378 | BACE:2459U21 siRNA stab04 sense | B uAAcAuuGGuGcAAAGAuuTTB | 665 |
| 3583 | UAUGGGACCUGCUAAGUGUGGAA | 654 | 30732 | BACE:3585U21 siRNA stab04 sense | B uGGGAccuGcuAAGuGuGGTT B | 666 |
| 1490 | AAUGGGUGAGGUUACCAACCAGU | 651 | 30733 | BACE:151OL21 siRNA (1492C) stab05 antisense | uGGuuGGuAAccucAcccATsT | 667 |
| 1753 | UCACCUUGGACAUGGAAGACUGU | 652 | 30734 | BACE:1773L21 siRNA (1755C) stab05 antisense | AGucuuccAuGuccAAGGuTsT | 668 |
| 2457 | CCUAACAUUGGUGCAAAGAUUGC | 653 | 31381 | BACE:2477L21 siRNA (2459C) stab05 antisense | AAucuuuGcAccAAuGuuATsT | 669 |
| 3583 | UAUGGGACCUGCUAAGUGUGGAA | 654 | 30736 | BACE:3603L21 siRNA (3585C) stab05 antisense | ccAcAcuuAGcAGGucccATsT | 670 |
| 1490 | AAUGGGUGAGGUUACCAACCAGU | 651 | | BACE:149ZU21 siRNA stab07 sense | B u*GGG*u*GAGG*uu*A*cc*AA*cc*A*TT B | 671 |
| 1753 | UCACCUUGGACAUGGAAGACUGU | 652 | | BACE:1755U21 siRNA stab07 sense | B *A*ccuu*GGA*c*A*u*GGAAGA*cuTT B | 672 |
| 2457 | CCUAACAUUGGUGCAAAGAUUGC | 653 | 31384 | BACE:2459U21 siRNA stab07 sense | B u*AA*c*A*uu*GG*u*G*c*AAAGA*uuTr B | 673 |
| 3583 | UAUGGGACCUGCUAAGUGUGGAA | 654 | | BACE:3585U21 siRNA stabOT sense | B u*GGGA*ccu*G*cu*AAG*u*G*u*GGTT* B | 674 |
| 1490 | AAUGGGUGAGGUUACCAACCAGU | 651 | | BACE:1510L21 siRNA (1492C) slab11 antisense | u*GG*uu*GG*u*AA*ccur*A*ccc*A*TsT | 675 |
| 1753 | UCACCUUGGACAUGGAAGACUGU | 652 | | BACE:1773L21 siRNA (1755C) stab11 antisense | *AG*ucuucc*A*u*G*ucc*AAGG*uTsT | 676 |
| 2457 | CCUAACAUUGGUGCAAAGAUUGC | 653 | 31387 | BACE:2477L21 siRNA (2459C) stab11 antisense | *AA*ucuuu*G*c*A*cc*AA*u*G*uu*A*TsT | 677 |
| 3583 | UAUGGGACCUGCUAAGUGUGGAA | 654 | | BACE:3603L21 siRNA (3585C) slab11 antisense | cc*A*c*A*cuu*AG*c*AGG*uccc*A*TsT | 678 |
| 2457 | CCUAACAUUGGUGCAAAGAIJUGC | 653 | 31390 | BACE:2459U21 siRNA Inv stab04 | B uuAGAAAcGtiGGuuAcAALiTT B | 679 |
| 2457 | CCUAACAUUGGUGCAAAGAUUGC | 653 | 31393 | BACE:2477L21 siRNA (2459C) Inv stab05 | AuuGuAAccAcGuuucuAATsT | 680 |
| 2457 | CCUAACAUUGGUGCAAAGAUUGC | 653 | 31396 | BACE:2459U21 siRNA Inv stab07 | B uu*AGAAA*c*G*u*GG*uu*A*c*AA*uTT B | 681 |
| 2457 | CCUAACAUUGGUGCAAAGAUUGC | 653 | 31399 | BACE:247TL21 siRNA (2459C) Inv stab11 | *A*uu*G*u*AA*cc*A*c*G*uuucu*AA*TsT | 682 |

| | | | | | | |
|---|---|---|---|---|---|---|
| Uppercase = ribonucleotide u,c = 2'-deoxy-2'-fluoro U,C T = thymidine B = inverted deoxy abasic *s* = phosphorothioate linkage *A* = deoxy Adenosine *G* = deoxy Guanosine | | | | | | |

**Table IV Non-limiting examples of Stabilization Chemistries for chemically modified siNA constructs**

| **Chemistry** | **pyrimidine** | **Purine** | **Cap** | **p=S** | **Strand** |
|---|---|---|---|---|---|
| **"Stab 1"** | Ribo | Ribo | - | 5 at 5'-end | S/AS |
| | | | | 1 at 3'-end | |
| **"Stab 2"** | Ribo | Ribo | - | All linkages | Usually AS |
| **"Stab 3"** | 2'-fluoro | Ribo | - | 4 at 5'-end | Usually S |
| | | | | 4 at 3'-end | |
| **"Stab 4"** | 2'-fluoro | Ribo | 5' and 3'-ends | - | Usually S |
| **"Stab 5"** | 2'-fluoro | Ribo | - | 1 at 3'-end | Usually AS |
| **"Stab 6"** | 2'-O-Methyl | Ribo | 5'and 3'-ends | - | Usually S |
| **"Stab 7"** | 2'-fluoro | 2'-deoxy | 5'and 3'-ends | - | Usually S |
| **"Stab 8"** | 2'-fluoro | 2'-O-Methyl | - | 1 at 3'-end | Usually AS |
| **"Stab 9"** | Ribo | Ribo | 5' and 3'-ends | - | Usually S |
| **"Stab 10"** | Ribo | Ribo | - | 1 at 3'-end | Usually AS |
| **"Stab 11"** | 2'-fluoro | 2'-deoxy | - | 1 at 3'-end | Usually AS |

| | | | | | |
|---|---|---|---|---|---|
| Cap = any terminal cap, see for example **Figure 10.** All Stab 1-11 chemistries can comprise 3'-terminal thymidine (TT) residues All Stab 1-11 chemistries typically comprise 21 nucleotides, but can vary as described herein. S = sense strand AS = antisense strand | | | | | |

**Table V**

| A. 2.5 µmol Synthesis Cycle ABI 394 Instrument | | | | | | | |
|---|---|---|---|---|---|---|---|
| **Reagent** | **Equivalents** | **Amount** | **Wait Time* DNA** | | **Wait Time* 2'-O-methyl** | | **Wait Time*RNA** |
| | | | | | | | |
| Phosphoramidites | 6.5 | 163 µL | 45 sec | | 2.5 min | | 7.5 min |
| S-Ethyl Tetrazole | 23.8 | 238 µL | 45 sec | | 2.5 min | | 7.5 min |
| Acetic Anhydride | 100 | 233 µL | 5 sec | | 5 sec | | 5 sec |
| *N*-Methyl imidazole | 186 | 233 µL | 5 sec | | 5 sec | | 5 sec |
| TCA | 176 | 2.3 mL | 21 sec | | 21 sec | | 21 sec |
| Iodine | 11.2 | 1.7 mL | 45 sec | | 45 sec | | 45 sec |
| Beaucage | 12.9 | 645 µL | 100 sec | | 300 sec | | 300 sec |
| Acetonitrile | NA | 6.67 mL | NA | | NA | | NA |

| B. 0.2 µmol Synthesis Cycle ABI 394 Instrument | | | | | | | |
|---|---|---|---|---|---|---|---|
| **Reagent** | **Equivalents** | **Amount** | **Wait Time* DNA** | | **Wait Time* 2'-O-methyl** | | **Wait Time*RNA** |
| | | | | | | | |
| Phosphoramidites | 15 | 31 µL | 45 sec | | 233 sec | | 465 sec |
| *S*-Ethyl Tetrazole | 38.7 | 31 µL | 45 sec | | 233 min | | 465 sec |
| Acetic Anhydride | 655 | 124 µL | 5 sec | | 5 sec | | 5 sec |
| *N*-Methyl Imidazole | 1245 | 124 µL | 5 sec | | 5 sec | | 5 sec |
| TCA | 700 | 732 µL | 10 sec | | 10 sec | | 10 sec |
| lodine | 20.6 | 244 µL | 15 sec | | 15 sec | | 15 sec |
| Beaucage | 7.7 | 232 µL | 100 sec | | 300 sec | | 300 sec |
| Acetonitrile | NA | 2.64 mL | NA | | NA | | NA |

| C. 0.2 µmol Synthesis Cycle 96 well Instrument | | | | | | | |
|---|---|---|---|---|---|---|---|
| **Reagent** | **Equivalents:DNA/ 2'-O-methyl/Ribo** | **Amount: DNA/2'-O-methyl/Ribo** | | **Wait Time* DNA** | | **Wait Time* 2'-O-methyl** | **Wait Time* Ribo** |
| | | | | | | | |
| Phosphoramidites | 22/33/66 | 40/60/120 µL | | 60 sec | | 180 sec | 360sec |
| S-Ethyl Tetrazole | 70/105/210 | 40/601120 µL | | 60 sec | | 180 min | 360 sec |
| Acetic Anhydride | 265/265/265 | 50/50/50 µL | | 10 sec | | 10 sec | 10 sec |
| *N*-Methyl Imidazole | 502/502/502 | 50/50/50 µL | | 10 sec | | 10 sec | 10 sec |
| TCA | 238/475/475 | 250/500/500 µL | | 15 sec | | 15 sec | 15 sec |
| iodine | 6.816.a/6.8 | 80180/80 µL | | 30 sec | | 30 sec | 30 sec |
| Beaucage | 34/51151 | 80/120/120 | | 100 sec | | 200 sec | 200 sec |
| Acetonitrile | NA | 1150/1150/1150 µL | | NA | | NA | NA |

| | | | | | | | |
|---|---|---|---|---|---|---|---|
| • Wait time does not include contact time during delivery. • Tandem synthesis utilizes double coupling of linker molecule | | | | | | | |

## Claims

1. A chemically synthesized double stranded short interfering nucleic acid (siNA) molecule that directs cleavage of a APP RNA via RNA interference (RNAi), wherein:
a. each strand of said siNA molecule is 19 to 23 nucleotides in length;
b. one strand of said siNA molecule comprises nucleotide sequence having sufficient complementarity to said APP RNA for the siNA molecule to direct cleavage of the APP RNA via RNA interference; and
c. said siNA molecule comprises at least one chemically modified nucleotide or non-nucleotide at the 5' end and/or 3' end of the sense strand and the 3' end of the antisense strand.

2. The siNA molecule of claim 1, wherein said siNA molecule comprises no ribonucleotides.

3. The siNA molecule of claim 1, wherein said siNA molecule comprises ribonucleotides.

4. The siNA molecule of claim 1, wherein one strand of said double-stranded siNA molecule comprises a nucleotide sequence that is complementary to a nucleotide sequence of a APP gene or a portion thereof, and wherein a second strand of said double-stranded siNA molecule comprises a nucleotide sequence substantially similar to the nucleotide sequence or a portion thereof of said APP RNA.

5. The siNA molecule of claim 4, wherein each strand of the siNA molecule comprises 19 to 23 nucleotides, and wherein each strand comprises at least 19 nucleotides that are complementary to the nucleotides of the other strand.

6. The siNA molecule of claim 1, wherein said siNA molecule comprises an antisense region comprising a nucleotide sequence that is complementary to a nucleotide sequence of an APP gene or a portion thereof, and wherein said siNA further comprises a sense region, wherein said sense region comprises a nucleotide sequence substantially similar to the nucleotide sequence of said APP gene or a portion thereof.

7. The siNA molecule of claim 6, wherein said antisense region and said sense region comprises 19 to 23 nucleotides, and wherein said antisense region comprises at least 19 nucleotides that are complementary to nucleotides of the sense region.

8. The siNA molecule of claim 1, wherein said siNA molecule comprises a sense region and an antisense region, and wherein said antisense region comprises a nucleotide sequence that is complementary to a nucleotide sequence of RNA encoded by an APP gene, or a portion thereof, and said sense region comprises a nucleotide sequence that is complementary to said antisense region.

9. The siNA molecule of claim 6, wherein said siNA molecule is assembled from two separate oligonucleotide fragments wherein one fragment comprises the sense region and a second fragment comprises the antisense region of said siNA molecule.

10. The siNA molecule of claim claim 6, wherein said sense region is connected to the antisense region via a linker molecule.

11. The siNA molecule of claim 10, wherein said linker molecule is a polynucleotide linker.

12. The siNA molecule of claim 10, wherein said linker molecule is a non-nucleotide linker.

13. The siNA molecule of claim 6, wherein pyrimidine nucleotides in the sense region are 2'-O-methyl pyrimidine nucleotides.

14. The siNA molecule of claim 6, wherein purine nucleotides in the sense region are 2'-deoxy purine nucleotides.

15. The siNA molecule of claim 6, wherein pyrimidine nucleotides present in the sense region are 2'-deoxy-2'-fluoro pyrimidine nucleotides.

16. The siNA molecule of claim 9, wherein the fragment comprising said sense region includes a terminal cap moiety at the 5'-end, the 3'-end, or both of the 5' and 3' ends of the fragment comprising said sense region.

17. The siNA molecule of claim 16, wherein said terminal cap moiety is an inverted deoxy abasic moiety.

18. The siNA molecule of claim 6, wherein pyrimidine nucleotides of said antisense region are 2'-deoxy-2'-fluoro pyrimidine nucleotides

19. The siNA molecule of claim 6, wherein purine nucleotides of said antisense region are 2'-O-methyl purine nucleotides.

20. The siNA molecule of claim 6, wherein purine nucleotides present in said antisense region comprise 2'-deoxy- purine nucleotides.

21. The siNA molecule of claim 18, wherein said antisense region comprises a phosphorothioate internucleotide linkage at the 3' end of said antisense region.

22. The siNA molecule of claim 6, wherein said antisense region comprises a glyceryl modification at the 3' end of said antisense region.

23. The siNA molecule of claim 9, wherein each of the two fragments of said siNA molecule comprise 21 nucleotides.

24. The siNA molecule of claim 23, wherein about 19 nucleotides of each fragment of the siNA molecule are base-paired to the complementary nucleotides of the other fragment of the siNA molecule and wherein at least two 3' terminal nucleotides of each fragment of the siNA molecule are not base-paired to the nucleotides of the other fragment of the siNA molecule.

25. The siNA molecule of claim 24, wherein each of the two 3' terminal nucleotides of each fragment of the siNA molecule are 2'-deoxy-pyrimidines.

26. The siNA molecule of claim 25, wherein said 2'-deoxy-pyrimidine is 2'-deoxythymidine.

27. The siNA molecule of claim 23, wherein all 21 nucleotides of each fragment of the siNA molecule are base-paired to the complementary nucleotides of the other fragment of the siNA molecule.

28. The siNA molecule of claim 23, wherein 19 nucleotides of the antisense region are base-paired to the nucleotide sequence of the RNA encoded by an APP gene or a portion thereof.

29. The siNA molecule of claim 23, wherein 21 nucleotides of the antisense region are base-paired to the nucleotide sequence of the RNA encoded by an APP gene or a portion thereof.

30. The siNA molecule of claim 9, wherein the 5'-end of the fragment comprising said antisense region optionally includes a phosphate group.

31. A pharmaceutical composition comprising the siNA molecule of claim 1 in an acceptable carrier or diluent.


### Annex to the application documents - subsequently filed sequences listing

SEQUENCE LISTING

<110> Sirna Therapeutics, Inc

<120> RNA Interference Mediated Treatment of Alzheimer's Disease Using
Short Interfering Nucleic Acid (siNA)

<130> P019392EPA

<140> 06075998.2
<141> 2006-05-04

<150> US 10/205,309
<151> 2002-07-25

<150> US 60/358,580
<151> 2002-02-20

<150> US 60/363,124
<151> 2002-03-11

<150> US 60/386,782
<151> 2002-06-06

<150> US 60/406,784
<151> 2002-08-29

<150> US 60/408,378
<151> 2002-09-05

<150> US 60/409,293
<151> 2002-09-09

<150> US 60/440,129
<151> 2003-01-15

<160> 704

<170> PatentIn version 3.3

<210> 1
<211> 19
<212> RNA
<213> Artificial Sequence

<220>
<223> Description of Artificial Sequence: Target sequence/siNA sense region

<400> 1
cgcacucguc cccagcccg 19

<210> 2
<211> 19
<212> RNA
<213> Artificial Sequence

<220>
<223> Description of Artificial Sequence: Target sequence/siNA sense region

<400> 2
gcccgggagc ugcgagccg 19

<210> 3
<211> 19
<212> RNA
<213> Artificial Sequence

<220>
<223> Description of Artificial Sequence: Target sequence/siNA sense region

<400> 3
gcgagcugga uuauggugg 19


<210> 4
<211> 19
<212> RNA
<213> Artificial Sequence

<220>
<223> Description of Artificial Sequence: Target sequence/siNA sense region

<400> 4
gccugagcag ccaacgcag 19


<210> 5
<211> 19
<212> RNA
<213> Artificial Sequence

<220>
<223> Description of Artificial Sequence: Target sequence/siNA sense region

<400> 5
gccgcaggag cccggagcc 19


<210> 6
<211> 19
<212> RNA
<213> Artificial Sequence

<220>
<223> Description of Artificial Sequence: Target sequence/siNA sense region

<400> 6
ccuugccccu gcccgcgcc 19


<210> 7
<211> 19
<212> RNA
<213> Artificial Sequence

<220>
<223> Description of Artificial Sequence: Target sequence/siNA sense region

<400> 7
cgccgccccgc cgggggac 19


<210> 8
<211> 19
<212> RNA

<213>  Artificial Sequence

<220>
<223>  Description of Artificial Sequence:  Target sequence/siNA sense region

<400>  8
ccagggaagc cgccaccgg                                                    19


<210>  9
<211>  19
<212>  RNA
<213>  Artificial Sequence

<220>
<223>  Description of Artificial Sequence:  Target sequence/siNA sense region

<400>  9
gcccgccaug cccgccccu                                                    19


<210>  10
<211>  19
<212>  RNA
<213>  Artificial Sequence

<220>
<223>  Description of Artificial Sequence:  Target sequence/siNA sense region

<400>  10
ucccagcccc gccgggagc                                                    19


<210>  11
<211>  19
<212>  RNA
<213>  Artificial Sequence

<220>
<223>  Description of Artificial Sequence:  Target sequence/siNA sense region

<400>  11
cccgcgcccg cugcccagg                                                    19


<210>  12
<211>  19
<212>  RNA
<213>  Artificial Sequence

<220>
<223>  Description of Artificial Sequence:  Target sequence/siNA sense region

<400>  12
gcuggccgcc gccgugccg                                                    19


<210>  13
<211>  19
<212>  RNA
<213>  Artificial Sequence

<220>
<223>  Description of Artificial Sequence:  Target sequence/siNA sense region

<400>  13
gauguagcgg gcuccggau                                                    19


<210>  14
<211>  19
<212>  RNA
<213>  Artificial Sequence

<220>
<223>  Description of Artificial Sequence:  Target sequence/siNA sense region

<400>  14
ucccagccuc uccccugcu                                                    19


<210>  15
<211>  19
<212>  RNA
<213>  Artificial Sequence

<220>
<223>  Description of Artificial Sequence:  Target sequence/siNA sense region

<400>  15
ucccgugcuc ugcggaucu                                                    19


<210>  16
<211>  19
<212>  RNA
<213>  Artificial Sequence

<220>
<223>  Description of Artificial Sequence:  Target sequence/siNA sense region

<400>  16
uccccugacc gcucuccac                                                    19


<210>  17
<211>  19
<212>  RNA
<213>  Artificial Sequence

<220>
<223>  Description of Artificial Sequence:  Target sequence/siNA sense region

<400>  17
cagcccggac ccgggggcu                                                    19


<210>  18
<211>  19
<212>  RNA
<213>  Artificial Sequence

<220>
<223>  Description of Artificial Sequence:  Target sequence/siNA sense region

<400>  18
uggcccaggg cccugcagg                                                    19

<210> 19
<211> 19
<212> RNA
<213> Artificial Sequence

<220>
<223> Description of Artificial Sequence: Target sequence/siNA sense region

<400> 19
gcccuggcgu ccugaugcc 19


<210> 20
<211> 19
<212> RNA
<213> Artificial Sequence

<220>
<223> Description of Artificial Sequence: Target sequence/siNA sense region

<400> 20
ccccaagcuc ccucuccug 19


<210> 21
<211> 19
<212> RNA
<213> Artificial Sequence

<220>
<223> Description of Artificial Sequence: Target sequence/siNA sense region

<400> 21
gagaagccac cagcaccac 19


<210> 22
<211> 19
<212> RNA
<213> Artificial Sequence

<220>
<223> Description of Artificial Sequence: Target sequence/siNA sense region

<400> 22
cccagacuug gggcaggc 19


<210> 23
<211> 19
<212> RNA
<213> Artificial Sequence

<220>
<223> Description of Artificial Sequence: Target sequence/siNA sense region

<400> 23
cgccagggac ggacguggg 19


<210> 24
<211> 19
<212> RNA

<213>  Artificial Sequence

<220>
<223>  Description of Artificial Sequence:  Target sequence/siNA sense region

<400>  24
gccagugcga gcccagagg                                                    19


<210>  25
<211>  19
<212>  RNA
<213>  Artificial Sequence

<220>
<223>  Description of Artificial Sequence:  Target sequence/siNA sense region

<400>  25
ggcccgaagg ccggggccc                                                    19


<210>  26
<211>  19
<212>  RNA
<213>  Artificial Sequence

<220>
<223>  Description of Artificial Sequence:  Target sequence/siNA sense region

<400>  26
caccauggcc caagcccug                                                    19


<210>  27
<211>  19
<212>  RNA
<213>  Artificial Sequence

<220>
<223>  Description of Artificial Sequence:  Target sequence/siNA sense region

<400>  27
gcccuggcuc cugcugugg                                                    19


<210>  28
<211>  19
<212>  RNA
<213>  Artificial Sequence

<220>
<223>  Description of Artificial Sequence:  Target sequence/siNA sense region

<400>  28
gaugggcgcg ggagugcug                                                    19


<210>  29
<211>  19
<212>  RNA
<213>  Artificial Sequence

<220>
<223>  Description of Artificial Sequence:  Target sequence/siNA sense region

<400>  29
gccugcccac ggcacccag                                                    19


<210>  30
<211>  19
<212>  RNA
<213>  Artificial Sequence

<220>
<223>  Description of Artificial Sequence:  Target sequence/siNA sense region

<400>  30
gcacggcauc cggcugccc                                                    19


<210>  31
<211>  19
<212>  RNA
<213>  Artificial Sequence

<220>
<223>  Description of Artificial Sequence:  Target sequence/siNA sense region

<400>  31
ccugcgcagc ggccugggg                                                    19


<210>  32
<211>  19
<212>  RNA
<213>  Artificial Sequence

<220>
<223>  Description of Artificial Sequence:  Target sequence/siNA sense region

<400>  32
gggcgccccc cuggggcug                                                    19


<210>  33
<211>  19
<212>  RNA
<213>  Artificial Sequence

<220>
<223>  Description of Artificial Sequence:  Target sequence/siNA sense region

<400>  33
gcggcugccc cgggagacc                                                    19


<210>  34
<211>  19
<212>  RNA
<213>  Artificial Sequence

<220>
<223>  Description of Artificial Sequence:  Target sequence/siNA sense region

<400>  34
cgacgaagag cccgaggag                                                    19

<210> 35
<211> 19
<212> RNA
<213> Artificial Sequence

<220>
<223> Description of Artificial Sequence: Target sequence/siNA sense region

<400> 35
gcccggccgg aggggcagc 19


<210> 36
<211> 19
<212> RNA
<213> Artificial Sequence

<220>
<223> Description of Artificial Sequence: Target sequence/siNA sense region

<400> 36
cuuuguggag augguggac 19


<210> 37
<211> 19
<212> RNA
<213> Artificial Sequence

<220>
<223> Description of Artificial Sequence: Target sequence/siNA sense region

<400> 37
caaccugagg ggcaagucg 19


<210> 38
<211> 19
<212> RNA
<213> Artificial Sequence

<220>
<223> Description of Artificial Sequence: Target sequence/siNA sense region

<400> 38
ggggcagggc uacuacgug 19


<210> 39
<211> 19
<212> RNA
<213> Artificial Sequence

<220>
<223> Description of Artificial Sequence: Target sequence/siNA sense region

<400> 39
ggagaugacc gugggcagc 19


<210> 40
<211> 19
<212> RNA

<213>  Artificial Sequence

<220>
<223>  Description of Artificial Sequence:  Target sequence/siNA sense region

<400>  40
ccccccgcag acgcucaac                                                    19


<210>  41
<211>  19
<212>  RNA
<213>  Artificial Sequence

<220>
<223>  Description of Artificial Sequence:  Target sequence/siNA sense region

<400>  41
cauccuggug gauacaggc                                                    19


<210>  42
<211>  19
<212>  RNA
<213>  Artificial Sequence

<220>
<223>  Description of Artificial Sequence:  Target sequence/siNA sense region

<400>  42
cagcaguaac uuugcagug                                                    19


<210>  43
<211>  19
<212>  RNA
<213>  Artificial Sequence

<220>
<223>  Description of Artificial Sequence:  Target sequence/siNA sense region

<400>  43
gggugcugcc ccccacccc                                                    19


<210>  44
<211>  19
<212>  RNA
<213>  Artificial Sequence

<220>
<223>  Description of Artificial Sequence:  Target sequence/siNA sense region

<400>  44
cuuccugcau cgcuacuac                                                    19


<210>  45
<211>  19
<212>  RNA
<213>  Artificial Sequence

<220>
<223>  Description of Artificial Sequence:  Target sequence/siNA sense region

<400> 45
ccagaggcag cuguccagc                                                    19

<210> 46
<211> 19
<212> RNA
<213> Artificial Sequence

<220>
<223> Description of Artificial Sequence: Target sequence/siNA sense region

<400> 46
cacauaccgg gaccuccgg                                                    19

<210> 47
<211> 19
<212> RNA
<213> Artificial Sequence

<220>
<223> Description of Artificial Sequence: Target sequence/siNA sense region

<400> 47
gaagggugug uaugugccc                                                    19

<210> 48
<211> 19
<212> RNA
<213> Artificial Sequence

<220>
<223> Description of Artificial Sequence: Target sequence/siNA sense region

<400> 48
cuacacccag ggcaagugg                                                    19

<210> 49
<211> 19
<212> RNA
<213> Artificial Sequence

<220>
<223> Description of Artificial Sequence: Target sequence/siNA sense region

<400> 49
ggaaggggag cugggcacc                                                    19

<210> 50
<211> 19
<212> RNA
<213> Artificial Sequence

<220>
<223> Description of Artificial Sequence: Target sequence/siNA sense region

<400> 50
cgaccuggua agcaucccc                                                    19

<210>  51
<211>  19
<212>  RNA
<213>  Artificial Sequence

<220>
<223>  Description of Artificial Sequence:  Target sequence/siNA sense region

<400>  51
ccauggcccc aacgucacu                                                    19


<210>  52
<211>  19
<212>  RNA
<213>  Artificial Sequence

<220>
<223>  Description of Artificial Sequence:  Target sequence/siNA sense region

<400>  52
ugugcgugcc aacauugcu                                                    19


<210>  53
<211>  19
<212>  RNA
<213>  Artificial Sequence

<220>
<223>  Description of Artificial Sequence:  Target sequence/siNA sense region

<400>  53
ugccaucacu gaaucagac                                                    19


<210>  54
<211>  19
<212>  RNA
<213>  Artificial Sequence

<220>
<223>  Description of Artificial Sequence:  Target sequence/siNA sense region

<400>  54
caaguucuuc aucaacggc                                                    19


<210>  55
<211>  19
<212>  RNA
<213>  Artificial Sequence

<220>
<223>  Description of Artificial Sequence:  Target sequence/siNA sense region

<400>  55
cuccaacugg gaaggcauc                                                    19


<210>  56
<211>  19
<212>  RNA

<213>  Artificial Sequence

<220>
<223>  Description of Artificial Sequence:  Target sequence/siNA sense region

<400>  56
ccuggggcug gccuaugcu                                                    19


<210>  57
<211>  19
<212>  RNA
<213>  Artificial Sequence

<220>
<223>  Description of Artificial Sequence:  Target sequence/siNA sense region

<400>  57
ugagauugcc aggccugac                                                    19


<210>  58
<211>  19
<212>  RNA
<213>  Artificial Sequence

<220>
<223>  Description of Artificial Sequence:  Target sequence/siNA sense region

<400>  58
cgacucccug gagccuuuc                                                    19


<210>  59
<211>  19
<212>  RNA
<213>  Artificial Sequence

<220>
<223>  Description of Artificial Sequence:  Target sequence/siNA sense region

<400>  59
cuuugacucu cugguaaag                                                    19


<210>  60
<211>  19
<212>  RNA
<213>  Artificial Sequence

<220>
<223>  Description of Artificial Sequence:  Target sequence/siNA sense region

<400>  60
gcagacccac guucccaac                                                    19


<210>  61
<211>  19
<212>  RNA
<213>  Artificial Sequence

<220>
<223>  Description of Artificial Sequence:  Target sequence/siNA sense region

<400> 61
ccucuucucc cugcagcuu 19

<210> 62
<211> 19
<212> RNA
<213> Artificial Sequence

<220>
<223> Description of Artificial Sequence: Target sequence/siNA sense region

<400> 62
uuguggugcu ggcuucccc 19

<210> 63
<211> 19
<212> RNA
<213> Artificial Sequence

<220>
<223> Description of Artificial Sequence: Target sequence/siNA sense region

<400> 63
ccucaaccag ucugaagug 19

<210> 64
<211> 19
<212> RNA
<213> Artificial Sequence

<220>
<223> Description of Artificial Sequence: Target sequence/siNA sense region

<400> 64
gcuggccucu gucggaggg 19

<210> 65
<211> 19
<212> RNA
<213> Artificial Sequence

<220>
<223> Description of Artificial Sequence: Target sequence/siNA sense region

<400> 65
gagcaugauc auuggaggu 19

<210> 66
<211> 19
<212> RNA
<213> Artificial Sequence

<220>
<223> Description of Artificial Sequence: Target sequence/siNA sense region

<400> 66
uaucgaccac ucgcuguac 19

<210> 67
<211> 19
<212> RNA
<213> Artificial Sequence

<220>
<223> Description of Artificial Sequence: Target sequence/siNA sense region

<400> 67
cacaggcagu cucugguau 19


<210> 68
<211> 19
<212> RNA
<213> Artificial Sequence

<220>
<223> Description of Artificial Sequence: Target sequence/siNA sense region

<400> 68
uacacccauc cggcgggag 19


<210> 69
<211> 19
<212> RNA
<213> Artificial Sequence

<220>
<223> Description of Artificial Sequence: Target sequence/siNA sense region

<400> 69
gugguauuau gaggucauc 19


<210> 70
<211> 19
<212> RNA
<213> Artificial Sequence

<220>
<223> Description of Artificial Sequence: Target sequence/siNA sense region

<400> 70
cauugugcgg guggagauc 19


<210> 71
<211> 19
<212> RNA
<213> Artificial Sequence

<220>
<223> Description of Artificial Sequence: Target sequence/siNA sense region

<400> 71
caauggacag gaucugaaa 19


<210> 72
<211> 19
<212> RNA

<213>  Artificial Sequence

<220>
<223>  Description of Artificial Sequence:  Target sequence/siNA sense region

<400>  72
aauggacugc aaggaguac                                                    19


<210>  73
<211>  19
<212>  RNA
<213>  Artificial Sequence

<220>
<223>  Description of Artificial Sequence:  Target sequence/siNA sense region

<400>  73
caacuaugac aagagcauu                                                    19


<210>  74
<211>  19
<212>  RNA
<213>  Artificial Sequence

<220>
<223>  Description of Artificial Sequence:  Target sequence/siNA sense region

<400>  74
uguggacagu ggcaccacc                                                    19


<210>  75
<211>  19
<212>  RNA
<213>  Artificial Sequence

<220>
<223>  Description of Artificial Sequence:  Target sequence/siNA sense region

<400>  75
caaccuucgu uugcccaag                                                    19


<210>  76
<211>  19
<212>  RNA
<213>  Artificial Sequence

<220>
<223>  Description of Artificial Sequence:  Target sequence/siNA sense region

<400>  76
gaaaguguuu gaagcugca                                                    19


<210>  77
<211>  19
<212>  RNA
<213>  Artificial Sequence

<220>
<223>  Description of Artificial Sequence:  Target sequence/siNA sense region

<400>  77
agucaaaucc aucaaggca                                                    19


<210>  78
<211>  19
<212>  RNA
<213>  Artificial Sequence

<220>
<223>  Description of Artificial Sequence:  Target sequence/siNA sense region

<400>  78
agccuccucc acggagaag                                                    19


<210>  79
<211>  19
<212>  RNA
<213>  Artificial Sequence

<220>
<223>  Description of Artificial Sequence:  Target sequence/siNA sense region

<400>  79
guucccugau gguuucugg                                                    19


<210>  80
<211>  19
<212>  RNA
<213>  Artificial Sequence

<220>
<223>  Description of Artificial Sequence:  Target sequence/siNA sense region

<400>  80
gcuaggagag cagcuggug                                                    19


<210>  81
<211>  19
<212>  RNA
<213>  Artificial Sequence

<220>
<223>  Description of Artificial Sequence:  Target sequence/siNA sense region

<400>  81
gugcuggcaa gcaggcacc                                                    19


<210>  82
<211>  19
<212>  RNA
<213>  Artificial Sequence

<220>
<223>  Description of Artificial Sequence:  Target sequence/siNA sense region

<400>  82
caccccuugg aacauuuuc                                                    19

<210>  83
<211>  19
<212>  RNA
<213>  Artificial Sequence

<220>
<223>  Description of Artificial Sequence:  Target sequence/siNA sense region

<400>  83
cccagucauc ucacucuac                                                    19


<210>  84
<211>  19
<212>  RNA
<213>  Artificial Sequence

<220>
<223>  Description of Artificial Sequence:  Target sequence/siNA sense region

<400>  84
ccuaaugggu gagguuacc                                                    19


<210>  85
<211>  19
<212>  RNA
<213>  Artificial Sequence

<220>
<223>  Description of Artificial Sequence:  Target sequence/siNA sense region

<400>  85
caaccagucc uuccgcauc                                                    19


<210>  86
<211>  19
<212>  RNA
<213>  Artificial Sequence

<220>
<223>  Description of Artificial Sequence:  Target sequence/siNA sense region

<400>  86
caccauccuu ccgcagcaa                                                    19


<210>  87
<211>  19
<212>  RNA
<213>  Artificial Sequence

<220>
<223>  Description of Artificial Sequence:  Target sequence/siNA sense region

<400>  87
auaccugcgg ccaguggaa                                                    19


<210>  88
<211>  19
<212>  RNA

<213>  Artificial Sequence

<220>
<223>  Description of Artificial Sequence:  Target sequence/siNA sense region

<400>  88
agauguggcc acgucccaa                                                    19


<210>  89
<211>  19
<212>  RNA
<213>  Artificial Sequence

<220>
<223>  Description of Artificial Sequence:  Target sequence/siNA sense region

<400>  89
agacgacugu uacaaguuu                                                    19


<210>  90
<211>  19
<212>  RNA
<213>  Artificial Sequence

<220>
<223>  Description of Artificial Sequence:  Target sequence/siNA sense region

<400>  90
ugccaucuca cagucaucc                                                    19


<210>  91
<211>  19
<212>  RNA
<213>  Artificial Sequence

<220>
<223>  Description of Artificial Sequence:  Target sequence/siNA sense region

<400>  91
cacgggcacu guuauggga                                                    19


<210>  92
<211>  19
<212>  RNA
<213>  Artificial Sequence

<220>
<223>  Description of Artificial Sequence:  Target sequence/siNA sense region

<400>  92
agcuguuauc auggagggc                                                    19


<210>  93
<211>  19
<212>  RNA
<213>  Artificial Sequence

<220>
<223>  Description of Artificial Sequence:  Target sequence/siNA sense region

<400> 93
cuucuacguu gucuuugau                                                    19

<210> 94
<211> 19
<212> RNA
<213> Artificial Sequence

<220>
<223> Description of Artificial Sequence: Target sequence/siNA sense region

<400> 94
ucgggcccga aaacgaauu                                                    19

<210> 95
<211> 19
<212> RNA
<213> Artificial Sequence

<220>
<223> Description of Artificial Sequence: Target sequence/siNA sense region

<400> 95
uggcuuugcu gucagcgcu                                                    19

<210> 96
<211> 19
<212> RNA
<213> Artificial Sequence

<220>
<223> Description of Artificial Sequence: Target sequence/siNA sense region

<400> 96
uugccaugug cacgaugag                                                    19

<210> 97
<211> 19
<212> RNA
<213> Artificial Sequence

<220>
<223> Description of Artificial Sequence: Target sequence/siNA sense region

<400> 97
guucaggacg gcagcggug                                                    19

<210> 98
<211> 19
<212> RNA
<213> Artificial Sequence

<220>
<223> Description of Artificial Sequence: Target sequence/siNA sense region

<400> 98
ggaaggcccu uuugucacc                                                    19

<210>  99
<211>  19
<212>  RNA
<213>  Artificial Sequence

<220>
<223>  Description of Artificial Sequence:  Target sequence/siNA sense region

<400>  99
cuuggacaug gaagacugu                                                    19


<210>  100
<211>  19
<212>  RNA
<213>  Artificial Sequence

<220>
<223>  Description of Artificial Sequence:  Target sequence/siNA sense region

<400>  100
uggcuacaac auuccacag                                                    19


<210>  101
<211>  19
<212>  RNA
<213>  Artificial Sequence

<220>
<223>  Description of Artificial Sequence:  Target sequence/siNA sense region

<400>  101
gacagaugag ucaacccuc                                                    19


<210>  102
<211>  19
<212>  RNA
<213>  Artificial Sequence

<220>
<223>  Description of Artificial Sequence:  Target sequence/siNA sense region

<400>  102
caugaccaua gccuauguc                                                    19


<210>  103
<211>  19
<212>  RNA
<213>  Artificial Sequence

<220>
<223>  Description of Artificial Sequence:  Target sequence/siNA sense region

<400>  103
cauggcugcc aucugcgcc                                                    19


<210>  104
<211>  19
<212>  RNA

<213>  Artificial Sequence

<220>
<223>  Description of Artificial Sequence:  Target sequence/siNA sense region

<400>  104
ccucuucaug cugccacuc                                                    19


<210>  105
<211>  19
<212>  RNA
<213>  Artificial Sequence

<220>
<223>  Description of Artificial Sequence:  Target sequence/siNA sense region

<400>  105
cugccucaug gugugucag                                                    19


<210>  106
<211>  19
<212>  RNA
<213>  Artificial Sequence

<220>
<223>  Description of Artificial Sequence:  Target sequence/siNA sense region

<400>  106
guggcgcugc cuccgcugc                                                    19


<210>  107
<211>  19
<212>  RNA
<213>  Artificial Sequence

<220>
<223>  Description of Artificial Sequence:  Target sequence/siNA sense region

<400>  107
ccugcgccag cagcaugau                                                    19


<210>  108
<211>  19
<212>  RNA
<213>  Artificial Sequence

<220>
<223>  Description of Artificial Sequence:  Target sequence/siNA sense region

<400>  108
ugacuuugcu gaugacauc                                                    19


<210>  109
<211>  19
<212>  RNA
<213>  Artificial Sequence

<220>
<223>  Description of Artificial Sequence:  Target sequence/siNA sense region

<400>  109
cucccugcug aagugagga                                                    19


<210>  110
<211>  19
<212>  RNA
<213>  Artificial Sequence

<220>
<223>  Description of Artificial Sequence:  Target sequence/siNA sense region

<400>  110
aggcccaugg gcagaagau                                                    19


<210>  111
<211>  19
<212>  RNA
<213>  Artificial Sequence

<220>
<223>  Description of Artificial Sequence:  Target sequence/siNA sense region

<400>  111
uagagauucc ccuggacca                                                    19


<210>  112
<211>  19
<212>  RNA
<213>  Artificial Sequence

<220>
<223>  Description of Artificial Sequence:  Target sequence/siNA sense region

<400>  112
acaccuccgu gguucacuu                                                    19


<210>  113
<211>  19
<212>  RNA
<213>  Artificial Sequence

<220>
<223>  Description of Artificial Sequence:  Target sequence/siNA sense region

<400>  113
uuggucacaa guaggagac                                                    19


<210>  114
<211>  19
<212>  RNA
<213>  Artificial Sequence

<220>
<223>  Description of Artificial Sequence:  Target sequence/siNA sense region

<400>  114
cacagauggc accuguggc                                                    19

<210>  115
<211>  19
<212>  RNA
<213>  Artificial Sequence

<220>
<223>  Description of Artificial Sequence:  Target sequence/siNA sense region

<400>  115
ccagagcacc ucaggaccc                                                    19


<210>  116
<211>  19
<212>  RNA
<213>  Artificial Sequence

<220>
<223>  Description of Artificial Sequence:  Target sequence/siNA sense region

<400>  116
cuccccaccc accaaaugc                                                    19


<210>  117
<211>  19
<212>  RNA
<213>  Artificial Sequence

<220>
<223>  Description of Artificial Sequence:  Target sequence/siNA sense region

<400>  117
ccucugccuu gauggagaa                                                    19


<210>  118
<211>  19
<212>  RNA
<213>  Artificial Sequence

<220>
<223>  Description of Artificial Sequence:  Target sequence/siNA sense region

<400>  118
aggaaaaggc uggcaaggu                                                    19


<210>  119
<211>  19
<212>  RNA
<213>  Artificial Sequence

<220>
<223>  Description of Artificial Sequence:  Target sequence/siNA sense region

<400>  119
uggguuccag ggacuguac                                                    19


<210>  120
<211>  19
<212>  RNA

<213>  Artificial Sequence

<220>
<223>  Description of Artificial Sequence:  Target sequence/siNA sense region

<400>  120
ccuguaggaa acagaaaag                                                    19


<210>  121
<211>  19
<212>  RNA
<213>  Artificial Sequence

<220>
<223>  Description of Artificial Sequence:  Target sequence/siNA sense region

<400>  121
gagaagaaag aagcacucu                                                    19


<210>  122
<211>  19
<212>  RNA
<213>  Artificial Sequence

<220>
<223>  Description of Artificial Sequence:  Target sequence/siNA sense region

<400>  122
ugcuggcggg aauacucuu                                                    19


<210>  123
<211>  19
<212>  RNA
<213>  Artificial Sequence

<220>
<223>  Description of Artificial Sequence:  Target sequence/siNA sense region

<400>  123
uggucaccuc aaauuuaag                                                    19


<210>  124
<211>  19
<212>  RNA
<213>  Artificial Sequence

<220>
<223>  Description of Artificial Sequence:  Target sequence/siNA sense region

<400>  124
gucgggaaau ucugcugcu                                                    19


<210>  125
<211>  19
<212>  RNA
<213>  Artificial Sequence

<220>
<223>  Description of Artificial Sequence:  Target sequence/siNA sense region

<400> 125
uugaaacuuc agcccugaa 19

<210> 126
<211> 19
<212> RNA
<213> Artificial Sequence

<220>
<223> Description of Artificial Sequence: Target sequence/siNA sense region

<400> 126
accuuugucc accauuccu 19

<210> 127
<211> 19
<212> RNA
<213> Artificial Sequence

<220>
<223> Description of Artificial Sequence: Target sequence/siNA sense region

<400> 127
uuuaaauucu ccaacccaa 19

<210> 128
<211> 19
<212> RNA
<213> Artificial Sequence

<220>
<223> Description of Artificial Sequence: Target sequence/siNA sense region

<400> 128
aaguauucuu cuuuucuua 19

<210> 129
<211> 19
<212> RNA
<213> Artificial Sequence

<220>
<223> Description of Artificial Sequence: Target sequence/siNA sense region

<400> 129
aguuucagaa guacuggca 19

<210> 130
<211> 19
<212> RNA
<213> Artificial Sequence

<220>
<223> Description of Artificial Sequence: Target sequence/siNA sense region

<400> 130
aucacacgca gguuaccuu 19

<210>  131
<211>  19
<212>  RNA
<213>  Artificial Sequence

<220>
<223>  Description of Artificial Sequence:  Target sequence/siNA sense region

<400>  131
uggcgugugu cccuguggu                                                    19


<210>  132
<211>  19
<212>  RNA
<213>  Artificial Sequence

<220>
<223>  Description of Artificial Sequence:  Target sequence/siNA sense region

<400>  132
uacccuggca gagaagaga                                                    19


<210>  133
<211>  19
<212>  RNA
<213>  Artificial Sequence

<220>
<223>  Description of Artificial Sequence:  Target sequence/siNA sense region

<400>  133
accaagcuug uuucccugc                                                    19


<210>  134
<211>  19
<212>  RNA
<213>  Artificial Sequence

<220>
<223>  Description of Artificial Sequence:  Target sequence/siNA sense region

<400>  134
cuggccaaag ucaguagga                                                    19


<210>  135
<211>  19
<212>  RNA
<213>  Artificial Sequence

<220>
<223>  Description of Artificial Sequence:  Target sequence/siNA sense region

<400>  135
agaggaugca caguuugcu                                                    19


<210>  136
<211>  19
<212>  RNA

<213>  Artificial Sequence

<220>
<223>  Description of Artificial Sequence:  Target sequence/siNA sense region

<400>  136
uauuugcuuu agagacagg                                                    19


<210>  137
<211>  19
<212>  RNA
<213>  Artificial Sequence

<220>
<223>  Description of Artificial Sequence:  Target sequence/siNA sense region

<400>  137
ggacuguaua aacaagccu                                                    19


<210>  138
<211>  19
<212>  RNA
<213>  Artificial Sequence

<220>
<223>  Description of Artificial Sequence:  Target sequence/siNA sense region

<400>  138
uaacauuggu gcaaagauu                                                    19


<210>  139
<211>  19
<212>  RNA
<213>  Artificial Sequence

<220>
<223>  Description of Artificial Sequence:  Target sequence/siNA sense region

<400>  139
ugccucuuga auuaaaaaa                                                    19


<210>  140
<211>  19
<212>  RNA
<213>  Artificial Sequence

<220>
<223>  Description of Artificial Sequence:  Target sequence/siNA sense region

<400>  140
aaaaaacuag auugacuau                                                    19


<210>  141
<211>  19
<212>  RNA
<213>  Artificial Sequence

<220>
<223>  Description of Artificial Sequence:  Target sequence/siNA sense region

<400> 141
uuuauacaaa ugggggcgg 19

<210> 142
<211> 19
<212> RNA
<213> Artificial Sequence

<220>
<223> Description of Artificial Sequence: Target sequence/siNA sense region

<400> 142
gcuggaaaga ggagaagga 19

<210> 143
<211> 19
<212> RNA
<213> Artificial Sequence

<220>
<223> Description of Artificial Sequence: Target sequence/siNA sense region

<400> 143
agagggagua caaagacag 19

<210> 144
<211> 19
<212> RNA
<213> Artificial Sequence

<220>
<223> Description of Artificial Sequence: Target sequence/siNA sense region

<400> 144
gggaauagug ggaucaaag 19

<210> 145
<211> 19
<212> RNA
<213> Artificial Sequence

<220>
<223> Description of Artificial Sequence: Target sequence/siNA sense region

<400> 145
gcuaggaaag gcagaaaca 19

<210> 146
<211> 19
<212> RNA
<213> Artificial Sequence

<220>
<223> Description of Artificial Sequence: Target sequence/siNA sense region

<400> 146
acaaccacuc accaguccu 19

<210> 147
<211> 19
<212> RNA
<213> Artificial Sequence

<220>
<223> Description of Artificial Sequence: Target sequence/siNA sense region

<400> 147
uaguuuuaga ccucaucuc 19


<210> 148
<211> 19
<212> RNA
<213> Artificial Sequence

<220>
<223> Description of Artificial Sequence: Target sequence/siNA sense region

<400> 148
ccaagauagc aucccaucu 19


<210> 149
<211> 19
<212> RNA
<213> Artificial Sequence

<220>
<223> Description of Artificial Sequence: Target sequence/siNA sense region

<400> 149
ucagaagaug gguguuguu 19


<210> 150
<211> 19
<212> RNA
<213> Artificial Sequence

<220>
<223> Description of Artificial Sequence: Target sequence/siNA sense region

<400> 150
uuucaauguu uucuuuucu 19


<210> 151
<211> 19
<212> RNA
<213> Artificial Sequence

<220>
<223> Description of Artificial Sequence: Target sequence/siNA sense region

<400> 151
ugugguugca gccugacca 19


<210> 152
<211> 19
<212> RNA

<213>  Artificial Sequence

<220>
<223>  Description of Artificial Sequence:  Target sequence/siNA sense region

<400>  152
aaaagugaga ugggaaggg                                                    19


<210>  153
<211>  19
<212>  RNA
<213>  Artificial Sequence

<220>
<223>  Description of Artificial Sequence:  Target sequence/siNA sense region

<400>  153
gcuuaucuag ccaaagagc                                                    19


<210>  154
<211>  19
<212>  RNA
<213>  Artificial Sequence

<220>
<223>  Description of Artificial Sequence:  Target sequence/siNA sense region

<400>  154
cucuuuuuua gcucucuua                                                    19


<210>  155
<211>  19
<212>  RNA
<213>  Artificial Sequence

<220>
<223>  Description of Artificial Sequence:  Target sequence/siNA sense region

<400>  155
aaaugaagug cccacuaag                                                    19


<210>  156
<211>  19
<212>  RNA
<213>  Artificial Sequence

<220>
<223>  Description of Artificial Sequence:  Target sequence/siNA sense region

<400>  156
gaaguuccac uuaacacau                                                    19


<210>  157
<211>  19
<212>  RNA
<213>  Artificial Sequence

<220>
<223>  Description of Artificial Sequence:  Target sequence/siNA sense region

<400>  157
ugaauuucug ccauauuaa                                                    19

<210>  158
<211>  19
<212>  RNA
<213>  Artificial Sequence

<220>
<223>  Description of Artificial Sequence:  Target sequence/siNA sense region

<400>  158
auuucauugu cucuaucug                                                    19

<210>  159
<211>  19
<212>  RNA
<213>  Artificial Sequence

<220>
<223>  Description of Artificial Sequence:  Target sequence/siNA sense region

<400>  159
gaaccacccu uuauucuac                                                    19

<210>  160
<211>  19
<212>  RNA
<213>  Artificial Sequence

<220>
<223>  Description of Artificial Sequence:  Target sequence/siNA sense region

<400>  160
cauaugauag gcagcacug                                                    19

<210>  161
<211>  19
<212>  RNA
<213>  Artificial Sequence

<220>
<223>  Description of Artificial Sequence:  Target sequence/siNA sense region

<400>  161
gaaauauccu aacccccua                                                    19

<210>  162
<211>  19
<212>  RNA
<213>  Artificial Sequence

<220>
<223>  Description of Artificial Sequence:  Target sequence/siNA sense region

<400>  162
aagcuccagg ugcccugug                                                    19

<210> 163
<211> 19
<212> RNA
<213> Artificial Sequence

<220>
<223> Description of Artificial Sequence: Target sequence/siNA sense region

<400> 163
gggagagcaa cuggacuau 19


<210> 164
<211> 19
<212> RNA
<213> Artificial Sequence

<220>
<223> Description of Artificial Sequence: Target sequence/siNA sense region

<400> 164
uagcagggcu gggcucugu 19


<210> 165
<211> 19
<212> RNA
<213> Artificial Sequence

<220>
<223> Description of Artificial Sequence: Target sequence/siNA sense region

<400> 165
ucuuccuggu cauaggcuc 19


<210> 166
<211> 19
<212> RNA
<213> Artificial Sequence

<220>
<223> Description of Artificial Sequence: Target sequence/siNA sense region

<400> 166
cacucuuucc cccaaaucu 19


<210> 167
<211> 19
<212> RNA
<213> Artificial Sequence

<220>
<223> Description of Artificial Sequence: Target sequence/siNA sense region

<400> 167
uuccucugga gcuuugcag 19


<210> 168
<211> 19
<212> RNA

<213>  Artificial Sequence

<220>
<223>  Description of Artificial Sequence:  Target sequence/siNA sense region

<400>  168
gccaaggugc uaaaaggaa                                                    19


<210>  169
<211>  19
<212>  RNA
<213>  Artificial Sequence

<220>
<223>  Description of Artificial Sequence:  Target sequence/siNA sense region

<400>  169
auagguagga gaccucuuc                                                    19


<210>  170
<211>  19
<212>  RNA
<213>  Artificial Sequence

<220>
<223>  Description of Artificial Sequence:  Target sequence/siNA sense region

<400>  170
cuaucuaauc cuuaaaagc                                                    19


<210>  171
<211>  19
<212>  RNA
<213>  Artificial Sequence

<220>
<223>  Description of Artificial Sequence:  Target sequence/siNA sense region

<400>  171
cauaauguug aacauucau                                                    19


<210>  172
<211>  19
<212>  RNA
<213>  Artificial Sequence

<220>
<223>  Description of Artificial Sequence:  Target sequence/siNA sense region

<400>  172
uucaacagcu gaugcccua                                                    19


<210>  173
<211>  19
<212>  RNA
<213>  Artificial Sequence

<220>
<223>  Description of Artificial Sequence:  Target sequence/siNA sense region

<400>  173
auaaccccug ccuggauuu                                                    19

<210>  174
<211>  19
<212>  RNA
<213>  Artificial Sequence

<220>
<223>  Description of Artificial Sequence:  Target sequence/siNA sense region

<400>  174
ucuuccuauu aggcuauaa                                                    19

<210>  175
<211>  19
<212>  RNA
<213>  Artificial Sequence

<220>
<223>  Description of Artificial Sequence:  Target sequence/siNA sense region

<400>  175
agaaguagca agaucuuua                                                    19

<210>  176
<211>  19
<212>  RNA
<213>  Artificial Sequence

<220>
<223>  Description of Artificial Sequence:  Target sequence/siNA sense region

<400>  176
acauaauuca gagugguuu                                                    19

<210>  177
<211>  19
<212>  RNA
<213>  Artificial Sequence

<220>
<223>  Description of Artificial Sequence:  Target sequence/siNA sense region

<400>  177
ucauugccuu ccuacccuc                                                    19

<210>  178
<211>  19
<212>  RNA
<213>  Artificial Sequence

<220>
<223>  Description of Artificial Sequence:  Target sequence/siNA sense region

<400>  178
cucuaauggc ccucccauu                                                    19

<210>  179
<211>  19
<212>  RNA
<213>  Artificial Sequence

<220>
<223>  Description of Artificial Sequence:  Target sequence/siNA sense region

<400>  179
uuauuugacu aaagcauca                                                    19


<210>  180
<211>  19
<212>  RNA
<213>  Artificial Sequence

<220>
<223>  Description of Artificial Sequence:  Target sequence/siNA sense region

<400>  180
acacaguggc acuagcauu                                                    19


<210>  181
<211>  19
<212>  RNA
<213>  Artificial Sequence

<220>
<223>  Description of Artificial Sequence:  Target sequence/siNA sense region

<400>  181
uauaccaaga guaugagaa                                                    19


<210>  182
<211>  19
<212>  RNA
<213>  Artificial Sequence

<220>
<223>  Description of Artificial Sequence:  Target sequence/siNA sense region

<400>  182
aauacagugc uuuauggcu                                                    19


<210>  183
<211>  19
<212>  RNA
<213>  Artificial Sequence

<220>
<223>  Description of Artificial Sequence:  Target sequence/siNA sense region

<400>  183
ucuaacauua cugccuuca                                                    19


<210>  184
<211>  19
<212>  RNA

<213>  Artificial Sequence

<220>
<223>  Description of Artificial Sequence:  Target sequence/siNA sense region

<400>  184
aguaucaagg cugccugga                                                    19


<210>  185
<211>  19
<212>  RNA
<213>  Artificial Sequence

<220>
<223>  Description of Artificial Sequence:  Target sequence/siNA sense region

<400>  185
agaaaggaug gcagccuca                                                    19


<210>  186
<211>  19
<212>  RNA
<213>  Artificial Sequence

<220>
<223>  Description of Artificial Sequence:  Target sequence/siNA sense region

<400>  186
agggcuuccu uauguccuc                                                    19


<210>  187
<211>  19
<212>  RNA
<213>  Artificial Sequence

<220>
<223>  Description of Artificial Sequence:  Target sequence/siNA sense region

<400>  187
ccaccacaag agcuccuug                                                    19


<210>  188
<211>  19
<212>  RNA
<213>  Artificial Sequence

<220>
<223>  Description of Artificial Sequence:  Target sequence/siNA sense region

<400>  188
gaugaagguc aucuuuuuc                                                    19


<210>  189
<211>  19
<212>  RNA
<213>  Artificial Sequence

<220>
<223>  Description of Artificial Sequence:  Target sequence/siNA sense region

<400>  189
ccccuauccu guucuuccc                                                    19

<210>  190
<211>  19
<212>  RNA
<213>  Artificial Sequence

<220>
<223>  Description of Artificial Sequence:  Target sequence/siNA sense region

<400>  190
   ccuccccgcu ccuaauggu                                                   19

<210>  191
<211>  19
<212>  RNA
<213>  Artificial Sequence

<220>
<223>  Description of Artificial Sequence:  Target sequence/siNA sense region

<400>  191
uacgugggua cccaggcug                                                    19

<210>  192
<211>  19
<212>  RNA
<213>  Artificial Sequence

<220>
<223>  Description of Artificial Sequence:  Target sequence/siNA sense region

<400>  192
gguucuuggg cuagguagu                                                    19

<210>  193
<211>  19
<212>  RNA
<213>  Artificial Sequence

<220>
<223>  Description of Artificial Sequence:  Target sequence/siNA sense region

<400>  193
uggggaccaa guucauuac                                                    19

<210>  194
<211>  19
<212>  RNA
<213>  Artificial Sequence

<220>
<223>  Description of Artificial Sequence:  Target sequence/siNA sense region

<400>  194
ccucccuauc aguucuagc                                                    19

<210>  195
<211>  19
<212>  RNA
<213>  Artificial Sequence

<220>
<223>  Description of Artificial Sequence:  Target sequence/siNA sense region

<400>  195
cauaguaaac uacgguacc                                                    19


<210>  196
<211>  19
<212>  RNA
<213>  Artificial Sequence

<220>
<223>  Description of Artificial Sequence:  Target sequence/siNA sense region

<400>  196
caguguuagu gggaagagc                                                    19


<210>  197
<211>  19
<212>  RNA
<213>  Artificial Sequence

<220>
<223>  Description of Artificial Sequence:  Target sequence/siNA sense region

<400>  197
cuggguuuuc cuaguauac                                                    19


<210>  198
<211>  19
<212>  RNA
<213>  Artificial Sequence

<220>
<223>  Description of Artificial Sequence:  Target sequence/siNA sense region

<400>  198
cccacugcau ccuacuccu                                                    19


<210>  199
<211>  19
<212>  RNA
<213>  Artificial Sequence

<220>
<223>  Description of Artificial Sequence:  Target sequence/siNA sense region

<400>  199
uaccugguca acccgcugc                                                    19


<210>  200
<211>  19
<212>  RNA

<213>  Artificial Sequence

<220>
<223>  Description of Artificial Sequence:  Target sequence/siNA sense region

<400>  200
cuuccaggua ugggaccug                                                    19


<210>  201
<211>  19
<212>  RNA
<213>  Artificial Sequence

<220>
<223>  Description of Artificial Sequence:  Target sequence/siNA sense region

<400>  201
gcuaagugug gaauuaccu                                                    19


<210>  202
<211>  19
<212>  RNA
<213>  Artificial Sequence

<220>
<223>  Description of Artificial Sequence:  Target sequence/siNA sense region

<400>  202
ugauaaggga gagggaaau                                                    19


<210>  203
<211>  19
<212>  RNA
<213>  Artificial Sequence

<220>
<223>  Description of Artificial Sequence:  Target sequence/siNA sense region

<400>  203
uacaaggagg gccucuggu                                                    19


<210>  204
<211>  19
<212>  RNA
<213>  Artificial Sequence

<220>
<223>  Description of Artificial Sequence:  Target sequence/siNA sense region

<400>  204
uguuccuggc cucagccag                                                    19


<210>  205
<211>  19
<212>  RNA
<213>  Artificial Sequence

<220>
<223>  Description of Artificial Sequence:  Target sequence/siNA sense region

<400>  205
gcugcccaca agccauaaa                                                    19


<210>  206
<211>  19
<212>  RNA
<213>  Artificial Sequence

<220>
<223>  Description of Artificial Sequence:  Target sequence/siNA sense region

<400>  206
accaauaaaa caagaauac                                                    19


<210>  207
<211>  19
<212>  RNA
<213>  Artificial Sequence

<220>
<223>  Description of Artificial Sequence:  Target sequence/siNA sense region

<400>  207
cugagucagu uuuuuaucu                                                    19


<210>  208
<211>  19
<212>  RNA
<213>  Artificial Sequence

<220>
<223>  Description of Artificial Sequence:  Target sequence/siNA sense region

<400>  208
uggguucucu ucauuccca                                                    19


<210>  209
<211>  19
<212>  RNA
<213>  Artificial Sequence

<220>
<223>  Description of Artificial Sequence:  Target sequence/siNA sense region

<400>  209
acugcacuug gugcugcuu                                                    19


<210>  210
<211>  19
<212>  RNA
<213>  Artificial Sequence

<220>
<223>  Description of Artificial Sequence:  Target sequence/siNA sense region

<400>  210
uuggcugacu gggaacacc                                                    19

<210> 211
<211> 19
<212> RNA
<213> Artificial Sequence

<220>
<223> Description of Artificial Sequence: Target sequence/siNA sense region

<400> 211
cccauaacua cagagucug 19


<210> 212
<211> 19
<212> RNA
<213> Artificial Sequence

<220>
<223> Description of Artificial Sequence: Target sequence/siNA sense region

<400> 212
gacaggaaga cuggagacu 19


<210> 213
<211> 19
<212> RNA
<213> Artificial Sequence

<220>
<223> Description of Artificial Sequence: Target sequence/siNA sense region

<400> 213
uguccacuuc uagcucgga 19


<210> 214
<211> 19
<212> RNA
<213> Artificial Sequence

<220>
<223> Description of Artificial Sequence: Target sequence/siNA sense region

<400> 214
aacuuacugu guaaauaaa 19


<210> 215
<211> 19
<212> RNA
<213> Artificial Sequence

<220>
<223> Description of Artificial Sequence: Target sequence/siNA sense region

<400> 215
acuuucagaa cugcuacca 19


<210> 216
<211> 19
<212> RNA

<213>  Artificial Sequence

<220>
<223>  Description of Artificial Sequence:  Target sequence/siNA sense region

<400>  216
augaagugaa aaugccaca                                                    19


<210>  217
<211>  19
<212>  RNA
<213>  Artificial Sequence

<220>
<223>  Description of Artificial Sequence:  Target sequence/siNA sense region

<400>  217
auuuugcuuu auaauuucu                                                    19


<210>  218
<211>  19
<212>  RNA
<213>  Artificial Sequence

<220>
<223>  Description of Artificial Sequence:  Target sequence/siNA sense region

<400>  218
uacccauguu gggaaaaac                                                    19


<210>  219
<211>  19
<212>  RNA
<213>  Artificial Sequence

<220>
<223>  Description of Artificial Sequence:  Target sequence/siNA sense region

<400>  219
cuggcuuuuu cccagcccu                                                    19


<210>  220
<211>  19
<212>  RNA
<213>  Artificial Sequence

<220>
<223>  Description of Artificial Sequence:  Target sequence/siNA sense region

<400>  220
uuuccagggc auaaaacuc                                                    19


<210>  221
<211>  19
<212>  RNA
<213>  Artificial Sequence

<220>
<223>  Description of Artificial Sequence:  Target sequence/siNA sense region

<400> 221
caaccccuuc gauagcaag 19

<210> 222
<211> 19
<212> RNA
<213> Artificial Sequence

<220>
<223> Description of Artificial Sequence: Target sequence/siNA sense region

<400> 222
gucccaucag ccuauuauu 19

<210> 223
<211> 19
<212> RNA
<213> Artificial Sequence

<220>
<223> Description of Artificial Sequence: Target sequence/siNA sense region

<400> 223
uuuuuuaaag aaaacuugc 19

<210> 224
<211> 19
<212> RNA
<213> Artificial Sequence

<220>
<223> Description of Artificial Sequence: Target sequence/siNA sense region

<400> 224
cacuuguuuu ucuuuuuac 19

<210> 225
<211> 19
<212> RNA
<213> Artificial Sequence

<220>
<223> Description of Artificial Sequence: Target sequence/siNA sense region

<400> 225
caguuacuuc cuuccugcc 19

<210> 226
<211> 19
<212> RNA
<213> Artificial Sequence

<220>
<223> Description of Artificial Sequence: Target sequence/siNA sense region

<400> 226
cccaaaauua uaaacucua 19

<210> 227
<211> 19
<212> RNA
<213> Artificial Sequence

<220>
<223> Description of Artificial Sequence: Target sequence/siNA sense region

<400> 227
aaguguaaaa aaaagucuu 19

<210> 228
<211> 19
<212> RNA
<213> Artificial Sequence

<220>
<223> Description of Artificial Sequence: Target sequence/siNA sense region

<400> 228
uaacaacagc uucuugcuu 19

<210> 229
<211> 19
<212> RNA
<213> Artificial Sequence

<220>
<223> Description of Artificial Sequence: Target sequence/siNA sense region

<400> 229
uguaaaaaua uguauuaua 19

<210> 230
<211> 19
<212> RNA
<213> Artificial Sequence

<220>
<223> Description of Artificial Sequence: Target sequence/siNA sense region

<400> 230
acaucuguau uuuuaaauu 19

<210> 231
<211> 19
<212> RNA
<213> Artificial Sequence

<220>
<223> Description of Artificial Sequence: Target sequence/siNA sense region

<400> 231
ucugcuccug aaaaaugac 19

<210> 232
<211> 19
<212> RNA

<213>  Artificial Sequence

<220>
<223>  Description of Artificial Sequence:  Target sequence/siNA sense region

<400>  232
cugucccauu cuccacuca                                                    19


<210>  233
<211>  19
<212>  RNA
<213>  Artificial Sequence

<220>
<223>  Description of Artificial Sequence:  Target sequence/siNA sense region

<400>  233
acugcauuug gggccuuuc                                                    19


<210>  234
<211>  19
<212>  RNA
<213>  Artificial Sequence

<220>
<223>  Description of Artificial Sequence:  Target sequence/siNA sense region

<400>  234
cccauugguc ugcaugucu                                                    19


<210>  235
<211>  19
<212>  RNA
<213>  Artificial Sequence

<220>
<223>  Description of Artificial Sequence:  Target sequence/siNA sense region

<400>  235
uuuuaucauu gcaggccag                                                    19


<210>  236
<211>  19
<212>  RNA
<213>  Artificial Sequence

<220>
<223>  Description of Artificial Sequence:  Target sequence/siNA sense region

<400>  236
guggacagag ggagaaggg                                                    19


<210>  237
<211>  19
<212>  RNA
<213>  Artificial Sequence

<220>
<223>  Description of Artificial Sequence:  Target sequence/siNA sense region

<400>  237
gagaacaggg gucgccaac                                                    19


<210>  238
<211>  19
<212>  RNA
<213>  Artificial Sequence

<220>
<223>  Description of Artificial Sequence:  Target sequence/siNA sense region

<400>  238
cacuuguguu gcuuucuga                                                    19


<210>  239
<211>  19
<212>  RNA
<213>  Artificial Sequence

<220>
<223>  Description of Artificial Sequence:  Target sequence/siNA sense region

<400>  239
acugauccug aacaagaaa                                                    19


<210>  240
<211>  19
<212>  RNA
<213>  Artificial Sequence

<220>
<223>  Description of Artificial Sequence:  Target sequence/siNA sense region

<400>  240
agaguaacac ugaggcgcu                                                    19


<210>  241
<211>  19
<212>  RNA
<213>  Artificial Sequence

<220>
<223>  Description of Artificial Sequence:  Target sequence/siNA sense region

<400>  241
ucgcucccau gcacaacuc                                                    19


<210>  242
<211>  19
<212>  RNA
<213>  Artificial Sequence

<220>
<223>  Description of Artificial Sequence:  Target sequence/siNA sense region

<400>  242
cuccaaaaca cuuauccuc                                                    19

<210>  243
<211>  19
<212>  RNA
<213>  Artificial Sequence

<220>
<223>  Description of Artificial Sequence:  Target sequence/siNA sense region

<400>  243
ccugcaagag ugggcuuuc                                                    19


<210>  244
<211>  19
<212>  RNA
<213>  Artificial Sequence

<220>
<223>  Description of Artificial Sequence:  Target sequence/siNA sense region

<400>  244
ccagggucuu uacugggaa                                                    19


<210>  245
<211>  19
<212>  RNA
<213>  Artificial Sequence

<220>
<223>  Description of Artificial Sequence:  Target sequence/siNA sense region

<400>  245
agcaguuaag cccccuccu                                                    19


<210>  246
<211>  19
<212>  RNA
<213>  Artificial Sequence

<220>
<223>  Description of Artificial Sequence:  Target sequence/siNA sense region

<400>  246
ucaccccuuc cuuuuuucu                                                    19


<210>  247
<211>  19
<212>  RNA
<213>  Artificial Sequence

<220>
<223>  Description of Artificial Sequence:  Target sequence/siNA sense region

<400>  247
uuucuuuacu ccuuuggcu                                                    19


<210>  248
<211>  19
<212>  RNA

<213>  Artificial Sequence

<220>
<223>  Description of Artificial Sequence:  Target sequence/siNA sense region

<400>  248
uucaaaggau uuuggaaaa                                                    19


<210>  249
<211>  19
<212>  RNA
<213>  Artificial Sequence

<220>
<223>  Description of Artificial Sequence:  Target sequence/siNA sense region

<400>  249
agaaacaaua ugcuuuaca                                                    19


<210>  250
<211>  19
<212>  RNA
<213>  Artificial Sequence

<220>
<223>  Description of Artificial Sequence:  Target sequence/siNA sense region

<400>  250
acucauuuuc aauuucuaa                                                    19


<210>  251
<211>  19
<212>  RNA
<213>  Artificial Sequence

<220>
<223>  Description of Artificial Sequence:  Target sequence/siNA sense region

<400>  251
aauuugcagg ggauacuga                                                    19


<210>  252
<211>  19
<212>  RNA
<213>  Artificial Sequence

<220>
<223>  Description of Artificial Sequence:  Target sequence/siNA sense region

<400>  252
aaaaauacgg cagguggcc                                                    19


<210>  253
<211>  19
<212>  RNA
<213>  Artificial Sequence

<220>
<223>  Description of Artificial Sequence:  Target sequence/siNA sense region

<400>  253
cuaaggcugc uguaaaguu                                                    19


<210>  254
<211>  19
<212>  RNA
<213>  Artificial Sequence

<220>
<223>  Description of Artificial Sequence:  Target sequence/siNA sense region

<400>  254
ugaggggaga ggaaaucuu                                                    19


<210>  255
<211>  19
<212>  RNA
<213>  Artificial Sequence

<220>
<223>  Description of Artificial Sequence:  Target sequence/siNA sense region

<400>  255
uaagauuaca agauaaaaa                                                    19


<210>  256
<211>  19
<212>  RNA
<213>  Artificial Sequence

<220>
<223>  Description of Artificial Sequence:  Target sequence/siNA sense region

<400>  256
aacgaauccc cuaaacaaa                                                    19


<210>  257
<211>  19
<212>  RNA
<213>  Artificial Sequence

<220>
<223>  Description of Artificial Sequence:  Target sequence/siNA sense region

<400>  257
aaagaacaau agaacuggu                                                    19


<210>  258
<211>  19
<212>  RNA
<213>  Artificial Sequence

<220>
<223>  Description of Artificial Sequence:  Target sequence/siNA sense region

<400>  258
ucuuccauuu ugccaccuu                                                    19

<210>  259
<211>  19
<212>  RNA
<213>  Artificial Sequence

<220>
<223>  Description of Artificial Sequence:  Target sequence/siNA sense region

<400>  259
uuccuguuca ugacagcua                                                    19


<210>  260
<211>  19
<212>  RNA
<213>  Artificial Sequence

<220>
<223>  Description of Artificial Sequence:  Target sequence/siNA sense region

<400>  260
acuaaccugg agacaguaa                                                    19


<210>  261
<211>  19
<212>  RNA
<213>  Artificial Sequence

<220>
<223>  Description of Artificial Sequence:  Target sequence/siNA sense region

<400>  261
acauuucauu aaccaaaga                                                    19


<210>  262
<211>  19
<212>  RNA
<213>  Artificial Sequence

<220>
<223>  Description of Artificial Sequence:  Target sequence/siNA sense region

<400>  262
aaaguggguc accugaccu                                                    19


<210>  263
<211>  19
<212>  RNA
<213>  Artificial Sequence

<220>
<223>  Description of Artificial Sequence:  Target sequence/siNA sense region

<400>  263
ucugaagagc ugaguacuc                                                    19


<210>  264
<211>  19
<212>  RNA

<213>  Artificial Sequence

<220>
<223>  Description of Artificial Sequence:  Target sequence/siNA sense region

<400>  264
caggccacuc caaucaccc                                                    19


<210>  265
<211>  19
<212>  RNA
<213>  Artificial Sequence

<220>
<223>  Description of Artificial Sequence:  Target sequence/siNA sense region

<400>  265
cuacaagaug ccaaggagg                                                    19


<210>  266
<211>  19
<212>  RNA
<213>  Artificial Sequence

<220>
<223>  Description of Artificial Sequence:  Target sequence/siNA sense region

<400>  266
gucccaggaa guccagcuc                                                    19


<210>  267
<211>  19
<212>  RNA
<213>  Artificial Sequence

<220>
<223>  Description of Artificial Sequence:  Target sequence/siNA sense region

<400>  267
ccuuaaacug acgcuaguc                                                    19


<210>  268
<211>  19
<212>  RNA
<213>  Artificial Sequence

<220>
<223>  Description of Artificial Sequence:  Target sequence/siNA sense region

<400>  268
caauaaaccu gggcaagug                                                    19


<210>  269
<211>  19
<212>  RNA
<213>  Artificial Sequence

<220>
<223>  Description of Artificial Sequence:  Target sequence/siNA sense region

<400> 269
gaggcaagag aaaugagga                                                    19


<210> 270
<211> 19
<212> RNA
<213> Artificial Sequence

<220>
<223> Description of Artificial Sequence: Target sequence/siNA sense region

<400> 270
aagaauccau cugugaggu                                                    19


<210> 271
<211> 19
<212> RNA
<213> Artificial Sequence

<220>
<223> Description of Artificial Sequence: Target sequence/siNA sense region

<400> 271
ugacaggcaa ggaugaaag                                                    19


<210> 272
<211> 19
<212> RNA
<213> Artificial Sequence

<220>
<223> Description of Artificial Sequence: Target sequence/siNA sense region

<400> 272
gacaaagaag gaaaagagu                                                    19


<210> 273
<211> 19
<212> RNA
<213> Artificial Sequence

<220>
<223> Description of Artificial Sequence: Target sequence/siNA sense region

<400> 273
uaucaaaggc agaaaggag                                                    19


<210> 274
<211> 19
<212> RNA
<213> Artificial Sequence

<220>
<223> Description of Artificial Sequence: Target sequence/siNA sense region

<400> 274
gaucauuuag uugggucug                                                    19

<210>  275
<211>  19
<212>  RNA
<213>  Artificial Sequence

<220>
<223>  Description of Artificial Sequence:  Target sequence/siNA sense region

<400>  275
gaaaggaaaa gucuuugcu                                                    19


<210>  276
<211>  19
<212>  RNA
<213>  Artificial Sequence

<220>
<223>  Description of Artificial Sequence:  Target sequence/siNA sense region

<400>  276
uauccgacau guacugcua                                                    19


<210>  277
<211>  19
<212>  RNA
<213>  Artificial Sequence

<220>
<223>  Description of Artificial Sequence:  Target sequence/siNA sense region

<400>  277
aguaccugua agcauuuua                                                    19


<210>  278
<211>  19
<212>  RNA
<213>  Artificial Sequence

<220>
<223>  Description of Artificial Sequence:  Target sequence/siNA sense region

<400>  278
   aggucccaga auggaaaaa                                                    19


<210>  279
<211>  19
<212>  RNA
<213>  Artificial Sequence

<220>
<223>  Description of Artificial Sequence:  Target sequence/siNA sense region

<400>  279
aaaaaucagc uauugguaa                                                    19


<210>  280
<211>  19
<212>  RNA

<213>  Artificial Sequence

<220>
<223>  Description of Artificial Sequence:  Target sequence/siNA sense region

<400>  280
auauaauaau guccuuucc                                                    19


<210>  281
<211>  19
<212>  RNA
<213>  Artificial Sequence

<220>
<223>  Description of Artificial Sequence:  Target sequence/siNA sense region

<400>  281
ccuggaguca guuuuuuua                                                    19


<210>  282
<211>  19
<212>  RNA
<213>  Artificial Sequence

<220>
<223>  Description of Artificial Sequence:  Target sequence/siNA sense region

<400>  282
aaaaaguuaa cucuuaguu                                                    19


<210>  283
<211>  19
<212>  RNA
<213>  Artificial Sequence

<220>
<223>  Description of Artificial Sequence:  Target sequence/siNA sense region

<400>  283
uuuuacuugu uuaauucua                                                    19


<210>  284
<211>  19
<212>  RNA
<213>  Artificial Sequence

<220>
<223>  Description of Artificial Sequence:  Target sequence/siNA sense region

<400>  284
aaaagagaag ggagcugag                                                    19


<210>  285
<211>  19
<212>  RNA
<213>  Artificial Sequence

<220>
<223>  Description of Artificial Sequence:  Target sequence/siNA sense region

<400> 285
ggccauuccc uguaggagu 19

<210> 286
<211> 19
<212> RNA
<213> Artificial Sequence

<220>
<223> Description of Artificial Sequence: Target sequence/siNA sense region

<400> 286
uaaagauaaa aggauagga 19

<210> 287
<211> 19
<212> RNA
<213> Artificial Sequence

<220>
<223> Description of Artificial Sequence: Target sequence/siNA sense region

<400> 287
aaaagauuca aagcucuaa 19

<210> 288
<211> 19
<212> RNA
<213> Artificial Sequence

<220>
<223> Description of Artificial Sequence: Target sequence/siNA sense region

<400> 288
auagagucac agcuuuccc 19

<210> 289
<211> 19
<212> RNA
<213> Artificial Sequence

<220>
<223> Description of Artificial Sequence: Target sequence/siNA sense region

<400> 289
cagguauaaa accuaaaau 19

<210> 290
<211> 19
<212> RNA
<213> Artificial Sequence

<220>
<223> Description of Artificial Sequence: Target sequence/siNA sense region

<400> 290
uuaagaagua caauaagca 19

<210>  291
<211>  19
<212>  RNA
<213>  Artificial Sequence

<220>
<223>  Description of Artificial Sequence:  Target sequence/siNA sense region

<400>  291
agagguggaa aaugaucua                                                    19


<210>  292
<211>  19
<212>  RNA
<213>  Artificial Sequence

<220>
<223>  Description of Artificial Sequence:  Target sequence/siNA sense region

<400>  292
aguuccugau agcuaccca                                                    19


<210>  293
<211>  19
<212>  RNA
<213>  Artificial Sequence

<220>
<223>  Description of Artificial Sequence:  Target sequence/siNA sense region

<400>  293
acagagcaag ugauuuaua                                                    19


<210>  294
<211>  19
<212>  RNA
<213>  Artificial Sequence

<220>
<223>  Description of Artificial Sequence:  Target sequence/siNA sense region

<400>  294
aaauuugaaa uccaaacua                                                    19


<210>  295
<211>  19
<212>  RNA
<213>  Artificial Sequence

<220>
<223>  Description of Artificial Sequence:  Target sequence/siNA sense region

<400>  295
acuuucuuaa uaucacuuu                                                    19


<210>  296
<211>  19
<212>  RNA

<213>  Artificial Sequence

<220>
<223>  Description of Artificial Sequence:  Target sequence/siNA sense region

<400>  296
uggucuccau uuuucccag                                                    19


<210>  297
<211>  19
<212>  RNA
<213>  Artificial Sequence

<220>
<223>  Description of Artificial Sequence:  Target sequence/siNA sense region

<400>  297
ggacaggaaa uaugucccc                                                    19


<210>  298
<211>  19
<212>  RNA
<213>  Artificial Sequence

<220>
<223>  Description of Artificial Sequence:  Target sequence/siNA sense region

<400>  298
ccccuaacuu ucuugcuuc                                                    19


<210>  299
<211>  19
<212>  RNA
<213>  Artificial Sequence

<220>
<223>  Description of Artificial Sequence:  Target sequence/siNA sense region

<400>  299
caaaaauuaa aauccagca                                                    19


<210>  300
<211>  19
<212>  RNA
<213>  Artificial Sequence

<220>
<223>  Description of Artificial Sequence:  Target sequence/siNA sense region

<400>  300
aucccaagau cauucuaca                                                    19


<210>  301
<211>  19
<212>  RNA
<213>  Artificial Sequence

<220>
<223>  Description of Artificial Sequence:  Target sequence/siNA sense region

<400> 301
aaguaauuuu gcacagaca 19

<210> 302
<211> 19
<212> RNA
<213> Artificial Sequence

<220>
<223> Description of Artificial Sequence: Target sequence/siNA sense region

<400> 302
aucuccucac cccagugcc 19

<210> 303
<211> 19
<212> RNA
<213> Artificial Sequence

<220>
<223> Description of Artificial Sequence: Target sequence/siNA sense region

<400> 303
cugucuggag cucacccaa 19

<210> 304
<211> 19
<212> RNA
<213> Artificial Sequence

<220>
<223> Description of Artificial Sequence: Target sequence/siNA sense region

<400> 304
aggucaccaa acaacuugg 19

<210> 305
<211> 19
<212> RNA
<213> Artificial Sequence

<220>
<223> Description of Artificial Sequence: Target sequence/siNA sense region

<400> 305
guugugaacc aacugccuu 19

<210> 306
<211> 19
<212> RNA
<213> Artificial Sequence

<220>
<223> Description of Artificial Sequence: Target sequence/siNA sense region

<400> 306
uaaccuucug ggggagggg 19

<210> 307
<211> 19
<212> RNA
<213> Artificial Sequence

<220>
<223> Description of Artificial Sequence: Target sequence/siNA sense region

<400> 307
ggauuagcua gacuaggag 19


<210> 308
<211> 19
<212> RNA
<213> Artificial Sequence

<220>
<223> Description of Artificial Sequence: Target sequence/siNA sense region

<400> 308
gaccagaagu gaaugggaa 19


<210> 309
<211> 19
<212> RNA
<213> Artificial Sequence

<220>
<223> Description of Artificial Sequence: Target sequence/siNA sense region

<400> 309
aagggugagg acuucacaa 19


<210> 310
<211> 19
<212> RNA
<213> Artificial Sequence

<220>
<223> Description of Artificial Sequence: Target sequence/siNA sense region

<400> 310
auguuggccu gucagagcu 19


<210> 311
<211> 19
<212> RNA
<213> Artificial Sequence

<220>
<223> Description of Artificial Sequence: Target sequence/siNA sense region

<400> 311
uugauuagaa gccaagaca 19


<210> 312
<211> 19
<212> RNA

<213>  Artificial Sequence

<220>
<223>  Description of Artificial Sequence:  Target sequence/siNA sense region

<400>  312
aguggcagca aaggaagac                                                    19


<210>  313
<211>  19
<212>  RNA
<213>  Artificial Sequence

<220>
<223>  Description of Artificial Sequence:  Target sequence/siNA sense region

<400>  313
cuuggcccag gaaaaaccu                                                    19


<210>  314
<211>  19
<212>  RNA
<213>  Artificial Sequence

<220>
<223>  Description of Artificial Sequence:  Target sequence/siNA sense region

<400>  314
ugugguugu gcuaauuuc                                                     19


<210>  315
<211>  19
<212>  RNA
<213>  Artificial Sequence

<220>
<223>  Description of Artificial Sequence:  Target sequence/siNA sense region

<400>  315
cuguccagaa aauagggug                                                    19


<210>  316
<211>  19
<212>  RNA
<213>  Artificial Sequence

<220>
<223>  Description of Artificial Sequence:  Target sequence/siNA sense region

<400>  316
ggacagaagc uuguggggu                                                    19


<210>  317
<211>  19
<212>  RNA
<213>  Artificial Sequence

<220>
<223>  Description of Artificial Sequence:  Target sequence/siNA sense region

<400>  317
ugcauggagg aauugggac                                                    19


<210>  318
<211>  19
<212>  RNA
<213>  Artificial Sequence

<220>
<223>  Description of Artificial Sequence:  Target sequence/siNA sense region

<400>  318
ccugguuaug uuguuauuc                                                    19


<210>  319
<211>  19
<212>  RNA
<213>  Artificial Sequence

<220>
<223>  Description of Artificial Sequence:  Target sequence/siNA sense region

<400>  319
cucggacugu gaauuuugg                                                    19


<210>  320
<211>  19
<212>  RNA
<213>  Artificial Sequence

<220>
<223>  Description of Artificial Sequence:  Target sequence/siNA sense region

<400>  320
gugauguaaa acagaauau                                                    19


<210>  321
<211>  19
<212>  RNA
<213>  Artificial Sequence

<220>
<223>  Description of Artificial Sequence:  Target sequence/siNA sense region

<400>  321
uucuguaaac cuaaugucu                                                    19


<210>  322
<211>  19
<212>  RNA
<213>  Artificial Sequence

<220>
<223>  Description of Artificial Sequence:  Target sequence/siNA sense region

<400>  322
uguauaaaua augagcguu                                                    19

<210>  323
<211>  19
<212>  RNA
<213>  Artificial Sequence

<220>
<223>  Description of Artificial Sequence:  Target sequence/siNA sense region

<400>  323
uaacacagua aaauauuca                                                    19


<210>  324
<211>  19
<212>  RNA
<213>  Artificial Sequence

<220>
<223>  Description of Artificial Sequence:  Target sequence/siNA sense region

<400>  324
aauaagaagu caaaaaaaa                                                    19


<210>  325
<211>  19
<212>  RNA
<213>  Artificial Sequence

<220>
<223>  Description of Artificial Sequence:  Target sequence/siNA sense region

<400>  325
aagucaaaaa aaaaaaaaa                                                    19


<210>  326
<211>  19
<212>  RNA
<213>  Artificial Sequence

<220>
<223>  Description of Artificial Sequence:  siNA antisense region

<400>  326
cgggcugggg acgagugcg                                                    19


<210>  327
<211>  19
<212>  RNA
<213>  Artificial Sequence

<220>
<223>  Description of Artificial Sequence:  siNA antisense region

<400>  327
cggcucgcag cucccgggc                                                    19


<210>  328
<211>  19
<212>  RNA

<213>  Artificial Sequence

<220>
<223>  Description of Artificial Sequence:  siNA antisense region

<400>  328
ccaccauaau ccagcucgc                                                    19


<210>  329
<211>  19
<212>  RNA
<213>  Artificial Sequence

<220>
<223>  Description of Artificial Sequence:  siNA antisense region

<400>  329
cugcguuggc ugcucaggc                                                    19


<210>  330
<211>  19
<212>  RNA
<213>  Artificial Sequence

<220>
<223>  Description of Artificial Sequence:  siNA antisense region

<400>  330
ggcuccgggc uccugcggc                                                    19


<210>  331
<211>  19
<212>  RNA
<213>  Artificial Sequence

<220>
<223>  Description of Artificial Sequence:  siNA antisense region

<400>  331
ggcgcgggca ggggcaagg                                                    19


<210>  332
<211>  19
<212>  RNA
<213>  Artificial Sequence

<220>
<223>  Description of Artificial Sequence:  siNA antisense region

<400>  332
   guccccccgg cgggcggcg                                                     19


<210>  333
<211>  19
<212>  RNA
<213>  Artificial Sequence

<220>
<223>  Description of Artificial Sequence:  siNA antisense region

<400>  333
ccgguggcgg cuucccugg                                                    19

<210>  334
<211>  19
<212>  RNA
<213>  Artificial Sequence

<220>
<223>  Description of Artificial Sequence:  siNA antisense region

<400>  334
aggggcgggc auggcgggc                                                    19

<210>  335
<211>  19
<212>  RNA
<213>  Artificial Sequence

<220>
<223>  Description of Artificial Sequence:  siNA antisense region

<400>  335
gcucccggcg gggcuggga                                                    19

<210>  336
<211>  19
<212>  RNA
<213>  Artificial Sequence

<220>
<223>  Description of Artificial Sequence:  siNA antisense region

<400>  336
ccugggcagc gggcgcggg                                                    19

<210>  337
<211>  19
<212>  RNA
<213>  Artificial Sequence

<220>
<223>  Description of Artificial Sequence:  siNA antisense region

<400>  337
cggcacggcg gcggccagc                                                    19

<210>  338
<211>  19
<212>  RNA
<213>  Artificial Sequence

<220>
<223>  Description of Artificial Sequence:  siNA antisense region

<400>  338
auccggagcc cgcuacauc                                                    19

<210>  339
<211>  19
<212>  RNA
<213>  Artificial Sequence

<220>
<223>  Description of Artificial Sequence:  siNA antisense region

<400>  339
agcaggggag aggcuggga                                                    19


<210>  340
<211>  19
<212>  RNA
<213>  Artificial Sequence

<220>
<223>  Description of Artificial Sequence:  siNA antisense region

<400>  340
agauccgcag agcacggga                                                    19


<210>  341
<211>  19
<212>  RNA
<213>  Artificial Sequence

<220>
<223>  Description of Artificial Sequence:  siNA antisense region

<400>  341
guggagagcg gucagggga                                                    19


<210>  342
<211>  19
<212>  RNA
<213>  Artificial Sequence

<220>
<223>  Description of Artificial Sequence:  siNA antisense region

<400>  342
agcccccggg uccgggcug                                                    19


<210>  343
<211>  19
<212>  RNA
<213>  Artificial Sequence

<220>
<223>  Description of Artificial Sequence:  siNA antisense region

<400>  343
ccugcagggc ccugggcca                                                    19


<210>  344
<211>  19
<212>  RNA

<213>  Artificial Sequence

<220>
<223>  Description of Artificial Sequence:  siNA antisense region

<400>  344
ggcaucagga cgccagggc                                                    19


<210>  345
<211>  19
<212>  RNA
<213>  Artificial Sequence

<220>
<223>  Description of Artificial Sequence:  siNA antisense region

<400>  345
caggagaggg agcuugggg                                                    19


<210>  346
<211>  19
<212>  RNA
<213>  Artificial Sequence

<220>
<223>  Description of Artificial Sequence:  siNA antisense region

<400>  346
guggugcugg uggcuucuc                                                    19


<210>  347
<211>  19
<212>  RNA
<213>  Artificial Sequence

<220>
<223>  Description of Artificial Sequence:  siNA antisense region

<400>  347
gccugccccc aagucuggg                                                    19


<210>  348
<211>  19
<212>  RNA
<213>  Artificial Sequence

<220>
<223>  Description of Artificial Sequence:  siNA antisense region

<400>  348
cccacguccg ucccuggcg                                                    19


<210>  349
<211>  19
<212>  RNA
<213>  Artificial Sequence

<220>
<223>  Description of Artificial Sequence:  siNA antisense region

<400>  349
ccucugggcu cgcacuggc                                                    19


<210>  350
<211>  19
<212>  RNA
<213>  Artificial Sequence

<220>
<223>  Description of Artificial Sequence:  siNA antisense region

<400>  350
gggccccggc cuucgggcc                                                    19


<210>  351
<211>  19
<212>  RNA
<213>  Artificial Sequence

<220>
<223>  Description of Artificial Sequence:  siNA antisense region

<400>  351
cagggcuugg gccauggug                                                    19


<210>  352
<211>  19
<212>  RNA
<213>  Artificial Sequence

<220>
<223>  Description of Artificial Sequence:  siNA antisense region

<400>  352
ccacagcagg agccagggc                                                    19


<210>  353
<211>  19
<212>  RNA
<213>  Artificial Sequence

<220>
<223>  Description of Artificial Sequence:  siNA antisense region

<400>  353
cagcacuccc gcgcccauc                                                    19


<210>  354
<211>  19
<212>  RNA
<213>  Artificial Sequence

<220>
<223>  Description of Artificial Sequence:  siNA antisense region

<400>  354
cugggugccg ugggcaggc                                                    19

<210>  355
<211>  19
<212>  RNA
<213>  Artificial Sequence

<220>
<223>  Description of Artificial Sequence:  siNA antisense region

<400>  355
gggcagccgg augccgugc                                                    19


<210>  356
<211>  19
<212>  RNA
<213>  Artificial Sequence

<220>
<223>  Description of Artificial Sequence:  siNA antisense region

<400>  356
ccccaggccg cugcgcagg                                                    19


<210>  357
<211>  19
<212>  RNA
<213>  Artificial Sequence

<220>
<223>  Description of Artificial Sequence:  siNA antisense region

<400>  357
cagccccagg ggggcgccc                                                    19


<210>  358
<211>  19
<212>  RNA
<213>  Artificial Sequence

<220>
<223>  Description of Artificial Sequence:  siNA antisense region

<400>  358
ggucucccgg ggcagccgc                                                    19


<210>  359
<211>  19
<212>  RNA
<213>  Artificial Sequence

<220>
<223>  Description of Artificial Sequence:  siNA antisense region

<400>  359
cuccucgggc ucuucgucg                                                    19


<210>  360
<211>  19
<212>  RNA

<213>  Artificial Sequence

<220>
<223>  Description of Artificial Sequence:  siNA antisense region

<400>  360
gcugccccuc cggccgggc                                                    19


<210>  361
<211>  19
<212>  RNA
<213>  Artificial Sequence

<220>
<223>  Description of Artificial Sequence:  siNA antisense region

<400>  361
guccaccauc uccacaaag                                                    19


<210>  362
<211>  19
<212>  RNA
<213>  Artificial Sequence

<220>
<223>  Description of Artificial Sequence:  siNA antisense region

<400>  362
cgacuugccc cucagguug                                                    19


<210>  363
<211>  19
<212>  RNA
<213>  Artificial Sequence

<220>
<223>  Description of Artificial Sequence:  siNA antisense region

<400>  363
cacguaguag cccugcccc                                                    19


<210>  364
<211>  19
<212>  RNA
<213>  Artificial Sequence

<220>
<223>  Description of Artificial Sequence:  siNA antisense region

<400>  364
gcugcccacg gucaucucc                                                    19


<210>  365
<211>  19
<212>  RNA
<213>  Artificial Sequence

<220>
<223>  Description of Artificial Sequence:  siNA antisense region

<400>  365
guugagcguc ugcgggggg                                                    19


<210>  366
<211>  19
<212>  RNA
<213>  Artificial Sequence

<220>
<223>  Description of Artificial Sequence:  siNA antisense region

<400>  366
gccuguaucc accaggaug                                                    19


<210>  367
<211>  19
<212>  RNA
<213>  Artificial Sequence

<220>
<223>  Description of Artificial Sequence:  siNA antisense region

<400>  367
cacugcaaag uuacugcug                                                    19


<210>  368
<211>  19
<212>  RNA
<213>  Artificial Sequence

<220>
<223>  Description of Artificial Sequence:  siNA antisense region

<400>  368
gggguggggg gcagcaccc                                                    19


<210>  369
<211>  19
<212>  RNA
<213>  Artificial Sequence

<220>
<223>  Description of Artificial Sequence:  siNA antisense region

<400>  369
guaguagcga ugcaggaag                                                    19


<210>  370
<211>  19
<212>  RNA
<213>  Artificial Sequence

<220>
<223>  Description of Artificial Sequence:  siNA antisense region

<400>  370
gcuggacagc ugccucugg                                                    19

<210>  371
<211>  19
<212>  RNA
<213>  Artificial Sequence

<220>
<223>  Description of Artificial Sequence:  siNA antisense region

<400>  371
ccggaggucc cgguaugug                                                    19


<210>  372
<211>  19
<212>  RNA
<213>  Artificial Sequence

<220>
<223>  Description of Artificial Sequence:  siNA antisense region

<400>  372
gggcacauac acacccuuc                                                    19


<210>  373
<211>  19
<212>  RNA
<213>  Artificial Sequence

<220>
<223>  Description of Artificial Sequence:  siNA antisense region

<400>  373
ccacuugccc uggguguag                                                    19


<210>  374
<211>  19
<212>  RNA
<213>  Artificial Sequence

<220>
<223>  Description of Artificial Sequence:  siNA antisense region

<400>  374
ggugcccagc uccccuucc                                                    19


<210>  375
<211>  19
<212>  RNA
<213>  Artificial Sequence

<220>
<223>  Description of Artificial Sequence:  siNA antisense region

<400>  375
ggggaugcuu accaggucg                                                    19


<210>  376
<211>  19
<212>  RNA

<213>  Artificial Sequence

<220>
<223>  Description of Artificial Sequence:  siNA antisense region

<400>  376
agugacguug gggccaugg                                                    19


<210>  377
<211>  19
<212>  RNA
<213>  Artificial Sequence

<220>
<223>  Description of Artificial Sequence:  siNA antisense region

<400>  377
agcaauguug gcacgcaca                                                    19


<210>  378
<211>  19
<212>  RNA
<213>  Artificial Sequence

<220>
<223>  Description of Artificial Sequence:  siNA antisense region

<400>  378
gucugauuca gugauggca                                                    19


<210>  379
<211>  19
<212>  RNA
<213>  Artificial Sequence

<220>
<223>  Description of Artificial Sequence:  siNA antisense region

<400>  379
gccguugaug aagaacuug                                                    19


<210>  380
<211>  19
<212>  RNA
<213>  Artificial Sequence

<220>
<223>  Description of Artificial Sequence:  siNA antisense region

<400>  380
gaugccuucc caguuggag                                                    19


<210>  381
<211>  19
<212>  RNA
<213>  Artificial Sequence

<220>
<223>  Description of Artificial Sequence:  siNA antisense region

<400> 381
agcauaggcc agccccagg 19

<210> 382
<211> 19
<212> RNA
<213> Artificial Sequence

<220>
<223> Description of Artificial Sequence: siNA antisense region

<400> 382
gucaggccug gcaaucuca 19

<210> 383
<211> 19
<212> RNA
<213> Artificial Sequence

<220>
<223> Description of Artificial Sequence: siNA antisense region

<400> 383
gaaaggcucc agggagucg 19

<210> 384
<211> 19
<212> RNA
<213> Artificial Sequence

<220>
<223> Description of Artificial Sequence: siNA antisense region

<400> 384
cuuuaccaga gagucaaag 19

<210> 385
<211> 19
<212> RNA
<213> Artificial Sequence

<220>
<223> Description of Artificial Sequence: siNA antisense region

<400> 385
guugggaacg ugggucugc 19

<210> 386
<211> 19
<212> RNA
<213> Artificial Sequence

<220>
<223> Description of Artificial Sequence: siNA antisense region

<400> 386
aagcugcagg gagaagagg 19

<210> 387
<211> 19
<212> RNA
<213> Artificial Sequence

<220>
<223> Description of Artificial Sequence: siNA antisense region

<400> 387
ggggaagcca gcaccacaa 19


<210> 388
<211> 19
<212> RNA
<213> Artificial Sequence

<220>
<223> Description of Artificial Sequence: siNA antisense region

<400> 388
cacuucagac ugguugagg 19


<210> 389
<211> 19
<212> RNA
<213> Artificial Sequence

<220>
<223> Description of Artificial Sequence: siNA antisense region

<400> 389
cccuccgaca gaggccagc 19


<210> 390
<211> 19
<212> RNA
<213> Artificial Sequence

<220>
<223> Description of Artificial Sequence: siNA antisense region

<400> 390
accuccaaug aucaugcuc 19


<210> 391
<211> 19
<212> RNA
<213> Artificial Sequence

<220>
<223> Description of Artificial Sequence: siNA antisense region

<400> 391
guacagcgag uggucgaua 19


<210> 392
<211> 19
<212> RNA

<213>  Artificial Sequence

<220>
<223>  Description of Artificial Sequence:  siNA antisense region

<400>  392
auaccagaga cugccugug                                                    19


<210>  393
<211>  19
<212>  RNA
<213>  Artificial Sequence

<220>
<223>  Description of Artificial Sequence:  siNA antisense region

<400>  393
cucccgccgg augggugua                                                    19


<210>  394
<211>  19
<212>  RNA
<213>  Artificial Sequence

<220>
<223>  Description of Artificial Sequence:  siNA antisense region

<400>  394
gaugaccuca uaauaccac                                                    19


<210>  395
<211>  19
<212>  RNA
<213>  Artificial Sequence

<220>
<223>  Description of Artificial Sequence:  siNA antisense region

<400>  395
gaucuccacc cgcacaaug                                                    19


<210>  396
<211>  19
<212>  RNA
<213>  Artificial Sequence

<220>
<223>  Description of Artificial Sequence:  siNA antisense region

<400>  396
uuucagaucc uguccauug                                                    19


<210>  397
<211>  19
<212>  RNA
<213>  Artificial Sequence

<220>
<223>  Description of Artificial Sequence:  siNA antisense region

<400>  397
guacuccuug caguccauu                                                    19


<210>  398
<211>  19
<212>  RNA
<213>  Artificial Sequence

<220>
<223>  Description of Artificial Sequence:  siNA antisense region

<400>  398
aaugcucuug ucauaguug                                                    19


<210>  399
<211>  19
<212>  RNA
<213>  Artificial Sequence

<220>
<223>  Description of Artificial Sequence:  siNA antisense region

<400>  399
gguggugcca cuguccaca                                                    19


<210>  400
<211>  19
<212>  RNA
<213>  Artificial Sequence

<220>
<223>  Description of Artificial Sequence:  siNA antisense region

<400>  400
cuugggcaaa cgaagguug                                                    19


<210>  401
<211>  19
<212>  RNA
<213>  Artificial Sequence

<220>
<223>  Description of Artificial Sequence:  siNA antisense region

<400>  401
ugcagcuuca aacacuuuc                                                    19


<210>  402
<211>  19
<212>  RNA
<213>  Artificial Sequence

<220>
<223>  Description of Artificial Sequence:  siNA antisense region

<400>  402
ugccuugaug gauuugacu                                                    19

<210> 403
<211> 19
<212> RNA
<213> Artificial Sequence

<220>
<223> Description of Artificial Sequence: siNA antisense region

<400> 403
cuucuccgug gaggaggcu 19


<210> 404
<211> 19
<212> RNA
<213> Artificial Sequence

<220>
<223> Description of Artificial Sequence: siNA antisense region

<400> 404
ccagaaacca ucagggaac 19


<210> 405
<211> 19
<212> RNA
<213> Artificial Sequence

<220>
<223> Description of Artificial Sequence: siNA antisense region

<400> 405
caccagcugc ucuccuagc 19


<210> 406
<211> 19
<212> RNA
<213> Artificial Sequence

<220>
<223> Description of Artificial Sequence: siNA antisense region

<400> 406
ggugccugcu ugccagcac 19


<210> 407
<211> 19
<212> RNA
<213> Artificial Sequence

<220>
<223> Description of Artificial Sequence: siNA antisense region

<400> 407
gaaaauguuc caaggggug 19


<210> 408
<211> 19
<212> RNA

<213>  Artificial Sequence

<220>
<223>  Description of Artificial Sequence:  siNA antisense region

<400>  408
guagagugag augacuggg                                                    19


<210>  409
<211>  19
<212>  RNA
<213>  Artificial Sequence

<220>
<223>  Description of Artificial Sequence:  siNA antisense region

<400>  409
gguaaccuca cccauuagg                                                    19


<210>  410
<211>  19
<212>  RNA
<213>  Artificial Sequence

<220>
<223>  Description of Artificial Sequence:  siNA antisense region

<400>  410
gaugcggaag gacugguug                                                    19


<210>  411
<211>  19
<212>  RNA
<213>  Artificial Sequence

<220>
<223>  Description of Artificial Sequence:  siNA antisense region

<400>  411
uugcugcgga aggauggug                                                    19


<210>  412
<211>  19
<212>  RNA
<213>  Artificial Sequence

<220>
<223>  Description of Artificial Sequence:  siNA antisense region

<400>  412
uuccacuggc cgcagguau                                                    19


<210>  413
<211>  19
<212>  RNA
<213>  Artificial Sequence

<220>
<223>  Description of Artificial Sequence:  siNA antisense region

<400>  413
uugggacgug gccacaucu                                                    19


<210>  414
<211>  19
<212>  RNA
<213>  Artificial Sequence

<220>
<223>  Description of Artificial Sequence:  siNA antisense region

<400>  414
aaacuuguaa cagucgucu                                                    19


<210>  415
<211>  19
<212>  RNA
<213>  Artificial Sequence

<220>
<223>  Description of Artificial Sequence:  siNA antisense region

<400>  415
ggaugacugu gagauggca                                                    19


<210>  416
<211>  19
<212>  RNA
<213>  Artificial Sequence

<220>
<223>  Description of Artificial Sequence:  siNA antisense region

<400>  416
ucccauaaca gugcccgug                                                    19


<210>  417
<211>  19
<212>  RNA
<213>  Artificial Sequence

<220>
<223>  Description of Artificial Sequence:  siNA antisense region

<400>  417
gcccuccaug auaacagcu                                                    19


<210>  418
<211>  19
<212>  RNA
<213>  Artificial Sequence

<220>
<223>  Description of Artificial Sequence:  siNA antisense region

<400>  418
aucaaagaca acguagaag                                                    19

<210>  419
<211>  19
<212>  RNA
<213>  Artificial Sequence

<220>
<223>  Description of Artificial Sequence:  siNA antisense region

<400>  419
aauucguuuu cgggcccga                                                    19


<210>  420
<211>  19
<212>  RNA
<213>  Artificial Sequence

<220>
<223>  Description of Artificial Sequence:  siNA antisense region

<400>  420
agcgcugaca gcaaagcca                                                    19


<210>  421
<211>  19
<212>  RNA
<213>  Artificial Sequence

<220>
<223>  Description of Artificial Sequence:  siNA antisense region

<400>  421
cucaucgugc acauggcaa                                                    19


<210>  422
<211>  19
<212>  RNA
<213>  Artificial Sequence

<220>
<223>  Description of Artificial Sequence:  siNA antisense region

<400>  422
caccgcugcc guccugaac                                                    19


<210>  423
<211>  19
<212>  RNA
<213>  Artificial Sequence

<220>
<223>  Description of Artificial Sequence:  siNA antisense region

<400>  423
ggugacaaaa gggccuucc                                                    19


<210>  424
<211>  19
<212>  RNA

<213>  Artificial Sequence

<220>
<223>  Description of Artificial Sequence:  siNA antisense region

<400>  424
acagucuucc auguccaag                                                    19


<210>  425
<211>  19
<212>  RNA
<213>  Artificial Sequence

<220>
<223>  Description of Artificial Sequence:  siNA antisense region

<400>  425
cuguggaaug uuguagcca                                                    19


<210>  426
<211>  19
<212>  RNA
<213>  Artificial Sequence

<220>
<223>  Description of Artificial Sequence:  siNA antisense region

<400>  426
gaggguugac ucaucuguc                                                    19


<210>  427
<211>  19
<212>  RNA
<213>  Artificial Sequence

<220>
<223>  Description of Artificial Sequence:  siNA antisense region

<400>  427
gacauaggcu auggucaug                                                    19


<210>  428
<211>  19
<212>  RNA
<213>  Artificial Sequence

<220>
<223>  Description of Artificial Sequence:  siNA antisense region

<400>  428
ggcgcagaug gcagccaug                                                    19


<210>  429
<211>  19
<212>  RNA
<213>  Artificial Sequence

<220>
<223>  Description of Artificial Sequence:  siNA antisense region

<400> 429
gaguggcagc augaagagg 19

<210> 430
<211> 19
<212> RNA
<213> Artificial Sequence

<220>
<223> Description of Artificial Sequence: siNA antisense region

<400> 430
cugacacacc augaggcag 19

<210> 431
<211> 19
<212> RNA
<213> Artificial Sequence

<220>
<223> Description of Artificial Sequence: siNA antisense region

<400> 431
gcagcggagg cagcgccac 19

<210> 432
<211> 19
<212> RNA
<213> Artificial Sequence

<220>
<223> Description of Artificial Sequence: siNA antisense region

<400> 432
aucaugcugc uggcgcagg 19

<210> 433
<211> 19
<212> RNA
<213> Artificial Sequence

<220>
<223> Description of Artificial Sequence: siNA antisense region

<400> 433
gaugucauca gcaaaguca 19

<210> 434
<211> 19
<212> RNA
<213> Artificial Sequence

<220>
<223> Description of Artificial Sequence: siNA antisense region

<400> 434
uccucacuuc agcagggag 19

<210>  435
<211>  19
<212>  RNA
<213>  Artificial Sequence

<220>
<223>  Description of Artificial Sequence:  siNA antisense region

<400>  435
aucuucugcc caugggccu                                                    19


<210>  436
<211>  19
<212>  RNA
<213>  Artificial Sequence

<220>
<223>  Description of Artificial Sequence:  siNA antisense region

<400>  436
ugguccaggg gaaucucua                                                    19


<210>  437
<211>  19
<212>  RNA
<213>  Artificial Sequence

<220>
<223>  Description of Artificial Sequence:  siNA antisense region

<400>  437
aagugaacca cggaggugu                                                    19


<210>  438
<211>  19
<212>  RNA
<213>  Artificial Sequence

<220>
<223>  Description of Artificial Sequence:  siNA antisense region

<400>  438
gucuccuacu ugugaccaa                                                    19


<210>  439
<211>  19
<212>  RNA
<213>  Artificial Sequence

<220>
<223>  Description of Artificial Sequence:  siNA antisense region

<400>  439
gccacaggug ccaucugug                                                    19


<210>  440
<211>  19
<212>  RNA

<213>  Artificial Sequence

<220>
<223>  Description of Artificial Sequence:  siNA antisense region

<400>  440
ggguccugag gugcucugg                                                    19


<210>  441
<211>  19
<212>  RNA
<213>  Artificial Sequence

<220>
<223>  Description of Artificial Sequence:  siNA antisense region

<400>  441
   gcauuuggug gguggggag                                                     19


<210>  442
<211>  19
<212>  RNA
<213>  Artificial Sequence

<220>
<223>  Description of Artificial Sequence:  siNA antisense region

<400>  442
uucuccauca aggcagagg                                                    19


<210>  443
<211>  19
<212>  RNA
<213>  Artificial Sequence

<220>
<223>  Description of Artificial Sequence:  siNA antisense region

<400>  443
accuugccag ccuuuuccu                                                    19


<210>  444
<211>  19
<212>  RNA
<213>  Artificial Sequence

<220>
<223>  Description of Artificial Sequence:  siNA antisense region

<400>  444
guacaguccc uggaaccca                                                    19


<210>  445
<211>  19
<212>  RNA
<213>  Artificial Sequence

<220>
<223>  Description of Artificial Sequence:  siNA antisense region

<400>  445
cuuuucuguu uccuacagg                                                    19

<210>  446
<211>  19
<212>  RNA
<213>  Artificial Sequence

<220>
<223>  Description of Artificial Sequence:  siNA antisense region

<400>  446
agagugcuuc uuucuucuc                                                    19

<210>  447
<211>  19
<212>  RNA
<213>  Artificial Sequence

<220>
<223>  Description of Artificial Sequence:  siNA antisense region

<400>  447
aagaguauuc ccgccagca                                                    19

<210>  448
<211>  19
<212>  RNA
<213>  Artificial Sequence

<220>
<223>  Description of Artificial Sequence:  siNA antisense region

<400>  448
cuuaaauuug aggugacca                                                    19

<210>  449
<211>  19
<212>  RNA
<213>  Artificial Sequence

<220>
<223>  Description of Artificial Sequence:  siNA antisense region

<400>  449
agcagcagaa uuucccgac                                                    19

<210>  450
<211>  19
<212>  RNA
<213>  Artificial Sequence

<220>
<223>  Description of Artificial Sequence:  siNA antisense region

<400>  450
uucagggcug aaguuucaa                                                    19

<210>  451
<211>  19
<212>  RNA
<213>  Artificial Sequence

<220>
<223>  Description of Artificial Sequence:  siNA antisense region

<400>  451
aggaauggug gacaaaggu                                                    19


<210>  452
<211>  19
<212>  RNA
<213>  Artificial Sequence

<220>
<223>  Description of Artificial Sequence:  siNA antisense region

<400>  452
uuggguugga gaauuuaaa                                                    19


<210>  453
<211>  19
<212>  RNA
<213>  Artificial Sequence

<220>
<223>  Description of Artificial Sequence:  siNA antisense region

<400>  453
uaagaaaaga agaauacuu                                                    19


<210>  454
<211>  19
<212>  RNA
<213>  Artificial Sequence

<220>
<223>  Description of Artificial Sequence:  siNA antisense region

<400>  454
ugccaguacu ucugaaacu                                                    19


<210>  455
<211>  19
<212>  RNA
<213>  Artificial Sequence

<220>
<223>  Description of Artificial Sequence:  siNA antisense region

<400>  455
aagguaaccu gcgugugau                                                    19


<210>  456
<211>  19
<212>  RNA

<213>  Artificial Sequence

<220>
<223>  Description of Artificial Sequence:  siNA antisense region

<400>  456
accacaggga cacacgcca                                                    19


<210>  457
<211>  19
<212>  RNA
<213>  Artificial Sequence

<220>
<223>  Description of Artificial Sequence:  siNA antisense region

<400>  457
ucucuucucu gccagggua                                                    19


<210>  458
<211>  19
<212>  RNA
<213>  Artificial Sequence

<220>
<223>  Description of Artificial Sequence:  siNA antisense region

<400>  458
gcagggaaac aagcuuggu                                                    19


<210>  459
<211>  19
<212>  RNA
<213>  Artificial Sequence

<220>
<223>  Description of Artificial Sequence:  siNA antisense region

<400>  459
uccuacugac uuuggccag                                                    19


<210>  460
<211>  19
<212>  RNA
<213>  Artificial Sequence

<220>
<223>  Description of Artificial Sequence:  siNA antisense region

<400>  460
agcaaacugu gcauccucu                                                    19


<210>  461
<211>  19
<212>  RNA
<213>  Artificial Sequence

<220>
<223>  Description of Artificial Sequence:  siNA antisense region

<400> 461
ccugucucua aagcaaaua 19

<210> 462
<211> 19
<212> RNA
<213> Artificial Sequence

<220>
<223> Description of Artificial Sequence: siNA antisense region

<400> 462
aggcuuguuu auacagucc 19

<210> 463
<211> 19
<212> RNA
<213> Artificial Sequence

<220>
<223> Description of Artificial Sequence: siNA antisense region

<400> 463
aaucuuugca ccaauguua 19

<210> 464
<211> 19
<212> RNA
<213> Artificial Sequence

<220>
<223> Description of Artificial Sequence: siNA antisense region

<400> 464
uuuuuuaauu caagaggca 19

<210> 465
<211> 19
<212> RNA
<213> Artificial Sequence

<220>
<223> Description of Artificial Sequence: siNA antisense region

<400> 465
auagucaauc uaguuuuuu 19

<210> 466
<211> 19
<212> RNA
<213> Artificial Sequence

<220>
<223> Description of Artificial Sequence: siNA antisense region

<400> 466
ccgcccccau uuguauaaa 19

<210> 467
<211> 19
<212> RNA
<213> Artificial Sequence

<220>
<223> Description of Artificial Sequence: siNA antisense region

<400> 467
uccuucuccu cuuuccagc 19


<210> 468
<211> 19
<212> RNA
<213> Artificial Sequence

<220>
<223> Description of Artificial Sequence: siNA antisense region

<400> 468
cugucuuugu acucccucu 19


<210> 469
<211> 19
<212> RNA
<213> Artificial Sequence

<220>
<223> Description of Artificial Sequence: siNA antisense region

<400> 469
cuuugauccc acuauuccc 19


<210> 470
<211> 19
<212> RNA
<213> Artificial Sequence

<220>
<223> Description of Artificial Sequence: siNA antisense region

<400> 470
uguuucugcc uuuccuagc 19


<210> 471
<211> 19
<212> RNA
<213> Artificial Sequence

<220>
<223> Description of Artificial Sequence: siNA antisense region

<400> 471
aggacuggug agugguugu 19


<210> 472
<211> 19
<212> RNA

<213>  Artificial Sequence

<220>
<223>  Description of Artificial Sequence:  siNA antisense region

<400>  472
gagaugaggu cuaaaacua                                                    19


<210>  473
<211>  19
<212>  RNA
<213>  Artificial Sequence

<220>
<223>  Description of Artificial Sequence:  siNA antisense region

<400>  473
agaugggaug cuaucuugg                                                    19


<210>  474
<211>  19
<212>  RNA
<213>  Artificial Sequence

<220>
<223>  Description of Artificial Sequence:  siNA antisense region

<400>  474
aacaacaccc aucuucuga                                                    19


<210>  475
<211>  19
<212>  RNA
<213>  Artificial Sequence

<220>
<223>  Description of Artificial Sequence:  siNA antisense region

<400>  475
agaaaagaaa acauugaaa                                                    19


<210>  476
<211>  19
<212>  RNA
<213>  Artificial Sequence

<220>
<223>  Description of Artificial Sequence:  siNA antisense region

<400>  476
uggucaggcu gcaaccaca                                                    19


<210>  477
<211>  19
<212>  RNA
<213>  Artificial Sequence

<220>
<223>  Description of Artificial Sequence:  siNA antisense region

<400>  477
cccuucccau cucacuuuu                                                    19


<210>  478
<211>  19
<212>  RNA
<213>  Artificial Sequence

<220>
<223>  Description of Artificial Sequence:  siNA antisense region

<400>  478
gcucuuuggc uagauaagc                                                    19


<210>  479
<211>  19
<212>  RNA
<213>  Artificial Sequence

<220>
<223>  Description of Artificial Sequence:  siNA antisense region

<400>  479
uaagagagcu aaaaaagag                                                    19


<210>  480
<211>  19
<212>  RNA
<213>  Artificial Sequence

<220>
<223>  Description of Artificial Sequence:  siNA antisense region

<400>  480
cuuagugggc acuucauuu                                                    19


<210>  481
<211>  19
<212>  RNA
<213>  Artificial Sequence

<220>
<223>  Description of Artificial Sequence:  siNA antisense region

<400>  481
auguguuaag uggaacuuc                                                    19


<210>  482
<211>  19
<212>  RNA
<213>  Artificial Sequence

<220>
<223>  Description of Artificial Sequence:  siNA antisense region

<400>  482
uuaauauggc agaaauuca                                                    19

<210> 483
<211> 19
<212> RNA
<213> Artificial Sequence

<220>
<223> Description of Artificial Sequence: siNA antisense region

<400> 483
cagauagaga caaugaaau 19


<210> 484
<211> 19
<212> RNA
<213> Artificial Sequence

<220>
<223> Description of Artificial Sequence: siNA antisense region

<400> 484
   guagaauaaa gggugguuc 19


<210> 485
<211> 19
<212> RNA
<213> Artificial Sequence

<220>
<223> Description of Artificial Sequence: siNA antisense region

<400> 485
cagugcugcc uaucauaug 19


<210> 486
<211> 19
<212> RNA
<213> Artificial Sequence

<220>
<223> Description of Artificial Sequence: siNA antisense region

<400> 486
uaggggguua ggauauuuc 19


<210> 487
<211> 19
<212> RNA
<213> Artificial Sequence

<220>
<223> Description of Artificial Sequence: siNA antisense region

<400> 487
cacagggcac cuggagcuu 19


<210> 488
<211> 19
<212> RNA

<213>  Artificial Sequence

<220>
<223>  Description of Artificial Sequence:  siNA antisense region

<400>  488
auaguccagu ugcucuccc                                                    19


<210>  489
<211>  19
<212>  RNA
<213>  Artificial Sequence

<220>
<223>  Description of Artificial Sequence:  siNA antisense region

<400>  489
acagagccca gcccugcua                                                    19


<210>  490
<211>  19
<212>  RNA
<213>  Artificial Sequence

<220>
<223>  Description of Artificial Sequence:  siNA antisense region

<400>  490
gagccuauga ccaggaaga                                                    19


<210>  491
<211>  19
<212>  RNA
<213>  Artificial Sequence

<220>
<223>  Description of Artificial Sequence:  siNA antisense region

<400>  491
agauuugggg gaaagagug                                                    19


<210>  492
<211>  19
<212>  RNA
<213>  Artificial Sequence

<220>
<223>  Description of Artificial Sequence:  siNA antisense region

<400>  492
cugcaaagcu ccagaggaa                                                    19


<210>  493
<211>  19
<212>  RNA
<213>  Artificial Sequence

<220>
<223>  Description of Artificial Sequence:  siNA antisense region

<400>  493
uuccuuuuag caccuuggc                                                    19


<210>  494
<211>  19
<212>  RNA
<213>  Artificial Sequence

<220>
<223>  Description of Artificial Sequence:  siNA antisense region

<400>  494
gaagaggucu ccuaccuau                                                    19


<210>  495
<211>  19
<212>  RNA
<213>  Artificial Sequence

<220>
<223>  Description of Artificial Sequence:  siNA antisense region

<400>  495
gcuuuuaagg auuagauag                                                    19


<210>  496
<211>  19
<212>  RNA
<213>  Artificial Sequence

<220>
<223>  Description of Artificial Sequence:  siNA antisense region

<400>  496
augaauguuc aacauuaug                                                    19


<210>  497
<211>  19
<212>  RNA
<213>  Artificial Sequence

<220>
<223>  Description of Artificial Sequence:  siNA antisense region

<400>  497
uagggcauca gcuguugaa                                                    19


<210>  498
<211>  19
<212>  RNA
<213>  Artificial Sequence

<220>
<223>  Description of Artificial Sequence:  siNA antisense region

<400>  498
aaauccaggc agggguuau                                                    19

<210> 499
<211> 19
<212> RNA
<213> Artificial Sequence

<220>
<223> Description of Artificial Sequence: siNA antisense region

<400> 499
uuauagccua auaggaaga 19


<210> 500
<211> 19
<212> RNA
<213> Artificial Sequence

<220>
<223> Description of Artificial Sequence: siNA antisense region

<400> 500
uaaagaucuu gcuacuucu 19


<210> 501
<211> 19
<212> RNA
<213> Artificial Sequence

<220>
<223> Description of Artificial Sequence: siNA antisense region

<400> 501
aaaccacucu gaauuaugu 19


<210> 502
<211> 19
<212> RNA
<213> Artificial Sequence

<220>
<223> Description of Artificial Sequence: siNA antisense region

<400> 502
gaggguagga aggcaauga 19


<210> 503
<211> 19
<212> RNA
<213> Artificial Sequence

<220>
<223> Description of Artificial Sequence: siNA antisense region

<400> 503
aauggagggg ccauuagag 19


<210> 504
<211> 19
<212> RNA

<213>  Artificial Sequence

<220>
<223>  Description of Artificial Sequence:  siNA antisense region

<400>  504
ugaugcuuua gucaaauaa                                                    19


<210>  505
<211>  19
<212>  RNA
<213>  Artificial Sequence

<220>
<223>  Description of Artificial Sequence:  siNA antisense region

<400>  505
aaugcuagug ccacugugu                                                    19


<210>  506
<211>  19
<212>  RNA
<213>  Artificial Sequence

<220>
<223>  Description of Artificial Sequence:  siNA antisense region

<400>  506
uucucauacu cuugguaua                                                    19


<210>  507
<211>  19
<212>  RNA
<213>  Artificial Sequence

<220>
<223>  Description of Artificial Sequence:  siNA antisense region

<400>  507
agccauaaag cacuguauu                                                    19


<210>  508
<211>  19
<212>  RNA
<213>  Artificial Sequence

<220>
<223>  Description of Artificial Sequence:  siNA antisense region

<400>  508
ugaaggcagu aauguuaga                                                    19


<210>  509
<211>  19
<212>  RNA
<213>  Artificial Sequence

<220>
<223>  Description of Artificial Sequence:  siNA antisense region

<400>  509
uccaggcagc cuugauacu                                                    19


<210>  510
<211>  19
<212>  RNA
<213>  Artificial Sequence

<220>
<223>  Description of Artificial Sequence:  siNA antisense region

<400>  510
ugaggcugcc auccuuucu                                                    19


<210>  511
<211>  19
<212>  RNA
<213>  Artificial Sequence

<220>
<223>  Description of Artificial Sequence:  siNA antisense region

<400>  511
gaggacauaa ggaagcccu                                                    19


<210>  512
<211>  19
<212>  RNA
<213>  Artificial Sequence

<220>
<223>  Description of Artificial Sequence:  siNA antisense region

<400>  512
caaggagcuc uuguggugg                                                    19


<210>  513
<211>  19
<212>  RNA
<213>  Artificial Sequence

<220>
<223>  Description of Artificial Sequence:  siNA antisense region

<400>  513
gaaaaagaug accuucauc                                                    19


<210>  514
<211>  19
<212>  RNA
<213>  Artificial Sequence

<220>
<223>  Description of Artificial Sequence:  siNA antisense region

<400>  514
gggaagaaca ggauagggg                                                    19

<210> 515
<211> 19
<212> RNA
<213> Artificial Sequence

<220>
<223> Description of Artificial Sequence: siNA antisense region

<400> 515
accauuagga gcggggagg 19


<210> 516
<211> 19
<212> RNA
<213> Artificial Sequence

<220>
<223> Description of Artificial Sequence: siNA antisense region

<400> 516
cagccugggu acccacgua 19


<210> 517
<211> 19
<212> RNA
<213> Artificial Sequence

<220>
<223> Description of Artificial Sequence: siNA antisense region

<400> 517
acuaccuagc ccaagaacc 19


<210> 518
<211> 19
<212> RNA
<213> Artificial Sequence

<220>
<223> Description of Artificial Sequence: siNA antisense region

<400> 518
   guaaugaacu uggucccca 19


<210> 519
<211> 19
<212> RNA
<213> Artificial Sequence

<220>
<223> Description of Artificial Sequence: siNA antisense region

<400> 519
gcuagaacug auagggagg 19


<210> 520
<211> 19
<212> RNA

<213>  Artificial Sequence

<220>
<223>  Description of Artificial Sequence:  siNA antisense region

<400>  520
gguaccguag uuuacuaug                                                    19


<210>  521
<211>  19
<212>  RNA
<213>  Artificial Sequence

<220>
<223>  Description of Artificial Sequence:  siNA antisense region

<400>  521
gcucuuccca cuaacacug                                                    19


<210>  522
<211>  19
<212>  RNA
<213>  Artificial Sequence

<220>
<223>  Description of Artificial Sequence:  siNA antisense region

<400>  522
guauacuagg aaaacccag                                                    19


<210>  523
<211>  19
<212>  RNA
<213>  Artificial Sequence

<220>
<223>  Description of Artificial Sequence:  siNA antisense region

<400>  523
aggaguagga ugcaguggg                                                    19


<210>  524
<211>  19
<212>  RNA
<213>  Artificial Sequence

<220>
<223>  Description of Artificial Sequence:  siNA antisense region

<400>  524
gcagcggguu gaccaggua                                                    19


<210>  525
<211>  19
<212>  RNA
<213>  Artificial Sequence

<220>
<223>  Description of Artificial Sequence:  siNA antisense region

<400>  525
caggucccau accuggaag                                                    19


<210>  526
<211>  19
<212>  RNA
<213>  Artificial Sequence

<220>
<223>  Description of Artificial Sequence:  siNA antisense region

<400>  526
agguaauucc acacuuagc                                                    19


<210>  527
<211>  19
<212>  RNA
<213>  Artificial Sequence

<220>
<223>  Description of Artificial Sequence:  siNA antisense region

<400>  527
auuucccucu cccuuauca                                                    19


<210>  528
<211>  19
<212>  RNA
<213>  Artificial Sequence

<220>
<223>  Description of Artificial Sequence:  siNA antisense region

<400>  528
accagaggcc cuccuugua                                                    19


<210>  529
<211>  19
<212>  RNA
<213>  Artificial Sequence

<220>
<223>  Description of Artificial Sequence:  siNA antisense region

<400>  529
cuggcugagg ccaggaaca                                                    19


<210>  530
<211>  19
<212>  RNA
<213>  Artificial Sequence

<220>
<223>  Description of Artificial Sequence:  siNA antisense region

<400>  530
uuuauggcuu gugggcagc                                                    19

<210>  531
<211>  19
<212>  RNA
<213>  Artificial Sequence

<220>
<223>  Description of Artificial Sequence:  siNA antisense region

<400>  531
guauucuugu uuuauuggu                                                    19


<210>  532
<211>  19
<212>  RNA
<213>  Artificial Sequence

<220>
<223>  Description of Artificial Sequence:  siNA antisense region

<400>  532
agauaaaaaa cugacucag                                                    19


<210>  533
<211>  19
<212>  RNA
<213>  Artificial Sequence

<220>
<223>  Description of Artificial Sequence:  siNA antisense region

<400>  533
ugggaaugaa gagaaccca                                                    19


<210>  534
<211>  19
<212>  RNA
<213>  Artificial Sequence

<220>
<223>  Description of Artificial Sequence:  siNA antisense region

<400>  534
aagcagcacc aagugcagu                                                    19


<210>  535
<211>  19
<212>  RNA
<213>  Artificial Sequence

<220>
<223>  Description of Artificial Sequence:  siNA antisense region

<400>  535
gguguuccca gucagccaa                                                    19


<210>  536
<211>  19
<212>  RNA

<213>  Artificial Sequence

<220>
<223>  Description of Artificial Sequence:  siNA antisense region

<400>  536
cagacucugu aguuauggg                                                    19


<210>  537
<211>  19
<212>  RNA
<213>  Artificial Sequence

<220>
<223>  Description of Artificial Sequence:  siNA antisense region

<400>  537
agucuccagu cuuccuguc                                                    19


<210>  538
<211>  19
<212>  RNA
<213>  Artificial Sequence

<220>
<223>  Description of Artificial Sequence:  siNA antisense region

<400>  538
uccgagcuag aaguggaca                                                    19


<210>  539
<211>  19
<212>  RNA
<213>  Artificial Sequence

<220>
<223>  Description of Artificial Sequence:  siNA antisense region

<400>  539
uuuauuuaca caguaaguu                                                    19


<210>  540
<211>  19
<212>  RNA
<213>  Artificial Sequence

<220>
<223>  Description of Artificial Sequence:  siNA antisense region

<400>  540
ugguagcagu ucugaaagu                                                    19


<210>  541
<211>  19
<212>  RNA
<213>  Artificial Sequence

<220>
<223>  Description of Artificial Sequence:  siNA antisense region

<400>  541
uguggcauuu ucacuucau                                                    19


<210>  542
<211>  19
<212>  RNA
<213>  Artificial Sequence

<220>
<223>  Description of Artificial Sequence:  siNA antisense region

<400>  542
agaaauuaua aagcaaaau                                                    19


<210>  543
<211>  19
<212>  RNA
<213>  Artificial Sequence

<220>
<223>  Description of Artificial Sequence:  siNA antisense region

<400>  543
guuuuuccca acaugggua                                                    19


<210>  544
<211>  19
<212>  RNA
<213>  Artificial Sequence

<220>
<223>  Description of Artificial Sequence:  siNA antisense region

<400>  544
agggcuggga aaaagccag                                                    19


<210>  545
<211>  19
<212>  RNA
<213>  Artificial Sequence

<220>
<223>  Description of Artificial Sequence:  siNA antisense region

<400>  545
gaguuuuaug cccuggaaa                                                    19


<210>  546
<211>  19
<212>  RNA
<213>  Artificial Sequence

<220>
<223>  Description of Artificial Sequence:  siNA antisense region

<400>  546
   cuugcuaucg aagggguug                                                    19

<210>  547
<211>  19
<212>  RNA
<213>  Artificial Sequence

<220>
<223>  Description of Artificial Sequence:  siNA antisense region

<400>  547
aauaauaggc ugaugggac                                                    19


<210>  548
<211>  19
<212>  RNA
<213>  Artificial Sequence

<220>
<223>  Description of Artificial Sequence:  siNA antisense region

<400>  548
gcaaguuuuc uuuaaaaaa                                                    19


<210>  549
<211>  19
<212>  RNA
<213>  Artificial Sequence

<220>
<223>  Description of Artificial Sequence:  siNA antisense region

<400>  549
guaaaaagaa aaacaagug                                                    19


<210>  550
<211>  19
<212>  RNA
<213>  Artificial Sequence

<220>
<223>  Description of Artificial Sequence:  siNA antisense region

<400>  550
ggcaggaagg aaguaacug                                                    19


<210>  551
<211>  19
<212>  RNA
<213>  Artificial Sequence

<220>
<223>  Description of Artificial Sequence:  siNA antisense region

<400>  551
uagaguuuau aauuuuggg                                                    19


<210>  552
<211>  19
<212>  RNA

<213>  Artificial Sequence

<220>
<223>  Description of Artificial Sequence:  siNA antisense region

<400>  552
aagacuuuuu uuuacacuu                                                    19


<210>  553
<211>  19
<212>  RNA
<213>  Artificial Sequence

<220>
<223>  Description of Artificial Sequence:  siNA antisense region

<400>  553
aagcaagaag cuguuguua                                                    19


<210>  554
<211>  19
<212>  RNA
<213>  Artificial Sequence

<220>
<223>  Description of Artificial Sequence:  siNA antisense region

<400>  554
uauaauacau auuuuuaca                                                    19


<210>  555
<211>  19
<212>  RNA
<213>  Artificial Sequence

<220>
<223>  Description of Artificial Sequence:  siNA antisense region

<400>  555
aauuuaaaaa uacagaugu                                                    19


<210>  556
<211>  19
<212>  RNA
<213>  Artificial Sequence

<220>
<223>  Description of Artificial Sequence:  siNA antisense region

<400>  556
gucauuuuuc aggagcaga                                                    19


<210>  557
<211>  19
<212>  RNA
<213>  Artificial Sequence

<220>
<223>  Description of Artificial Sequence:  siNA antisense region

<400> 557
ugaguggaga augggacag 19

<210> 558
<211> 19
<212> RNA
<213> Artificial Sequence

<220>
<223> Description of Artificial Sequence: siNA antisense region

<400> 558
gaaaggcccc aaaugcagu 19

<210> 559
<211> 19
<212> RNA
<213> Artificial Sequence

<220>
<223> Description of Artificial Sequence: siNA antisense region

<400> 559
agacaugcag accaauggg 19

<210> 560
<211> 19
<212> RNA
<213> Artificial Sequence

<220>
<223> Description of Artificial Sequence: siNA antisense region

<400> 560
cuggccugca augauaaaa 19

<210> 561
<211> 19
<212> RNA
<213> Artificial Sequence

<220>
<223> Description of Artificial Sequence: siNA antisense region

<400> 561
cccuucuccc ucuguccac 19

<210> 562
<211> 19
<212> RNA
<213> Artificial Sequence

<220>
<223> Description of Artificial Sequence: siNA antisense region

<400> 562
guuggcgacc ccuguucuc 19

<210> 563
<211> 19
<212> RNA
<213> Artificial Sequence

<220>
<223> Description of Artificial Sequence: siNA antisense region

<400> 563
ucagaaagca acacaagug 19


<210> 564
<211> 19
<212> RNA
<213> Artificial Sequence

<220>
<223> Description of Artificial Sequence: siNA antisense region

<400> 564
uuucuuguuc aggaucagu 19


<210> 565
<211> 19
<212> RNA
<213> Artificial Sequence

<220>
<223> Description of Artificial Sequence: siNA antisense region

<400> 565
agcgccucag uguuacucu 19


<210> 566
<211> 19
<212> RNA
<213> Artificial Sequence

<220>
<223> Description of Artificial Sequence: siNA antisense region

<400> 566
gaguugugca ugggagcga 19


<210> 567
<211> 19
<212> RNA
<213> Artificial Sequence

<220>
<223> Description of Artificial Sequence: siNA antisense region

<400> 567
gaggauaagu guuuuggag 19


<210> 568
<211> 19
<212> RNA

<213>  Artificial Sequence

<220>
<223>  Description of Artificial Sequence:  siNA antisense region

<400>  568
gaaagcccac ucuugcagg                                                    19


<210>  569
<211>  19
<212>  RNA
<213>  Artificial Sequence

<220>
<223>  Description of Artificial Sequence:  siNA antisense region

<400>  569
uucccaguaa agacccugg                                                    19


<210>  570
<211>  19
<212>  RNA
<213>  Artificial Sequence

<220>
<223>  Description of Artificial Sequence:  siNA antisense region

<400>  570
aggaggggc uuaacugcu                                                     19


<210>  571
<211>  19
<212>  RNA
<213>  Artificial Sequence

<220>
<223>  Description of Artificial Sequence:  siNA antisense region

<400>  571
agaaaaaagg aagggguga                                                    19


<210>  572
<211>  19
<212>  RNA
<213>  Artificial Sequence

<220>
<223>  Description of Artificial Sequence:  siNA antisense region

<400>  572
agccaaagga guaaagaaa                                                    19


<210>  573
<211>  19
<212>  RNA
<213>  Artificial Sequence

<220>
<223>  Description of Artificial Sequence:  siNA antisense region

<400>  573
uuuuccaaaa uccuuugaa                                                    19

<210>  574
<211>  19
<212>  RNA
<213>  Artificial Sequence

<220>
<223>  Description of Artificial Sequence:  siNA antisense region

<400>  574
uguaaagcau auuguuucu                                                    19

<210>  575
<211>  19
<212>  RNA
<213>  Artificial Sequence

<220>
<223>  Description of Artificial Sequence:  siNA antisense region

<400>  575
uuagaaauug aaaaugagu                                                    19

<210>  576
<211>  19
<212>  RNA
<213>  Artificial Sequence

<220>
<223>  Description of Artificial Sequence:  siNA antisense region

<400>  576
ucaguauccc cugcaaauu                                                    19

<210>  577
<211>  19
<212>  RNA
<213>  Artificial Sequence

<220>
<223>  Description of Artificial Sequence:  siNA antisense region

<400>  577
ggccaccugc cguauuuuu                                                    19

<210>  578
<211>  19
<212>  RNA
<213>  Artificial Sequence

<220>
<223>  Description of Artificial Sequence:  siNA antisense region

<400>  578
aacuuuacag cagccuuag                                                    19

<210> 579
<211> 19
<212> RNA
<213> Artificial Sequence

<220>
<223> Description of Artificial Sequence: siNA antisense region

<400> 579
aagauuuccu cuccccuca 19

<210> 580
<211> 19
<212> RNA
<213> Artificial Sequence

<220>
<223> Description of Artificial Sequence: siNA antisense region

<400> 580
uuuuuaucuu guaaucuua 19

<210> 581
<211> 19
<212> RNA
<213> Artificial Sequence

<220>
<223> Description of Artificial Sequence: siNA antisense region

<400> 581
uuuguuuagg ggauucguu 19

<210> 582
<211> 19
<212> RNA
<213> Artificial Sequence

<220>
<223> Description of Artificial Sequence: siNA antisense region

<400> 582
accaguucua uuguucuuu 19

<210> 583
<211> 19
<212> RNA
<213> Artificial Sequence

<220>
<223> Description of Artificial Sequence: siNA antisense region

<400> 583
aagguggcaa aauggaaga 19

<210> 584
<211> 19
<212> RNA

<213>  Artificial Sequence

<220>
<223>  Description of Artificial Sequence:  siNA antisense region

<400>  584
uagcugucau gaacaggaa                                                    19


<210>  585
<211>  19
<212>  RNA
<213>  Artificial Sequence

<220>
<223>  Description of Artificial Sequence:  siNA antisense region

<400>  585
uuacugucuc cagguuagu                                                    19


<210>  586
<211>  19
<212>  RNA
<213>  Artificial Sequence

<220>
<223>  Description of Artificial Sequence:  siNA antisense region

<400>  586
ucuuugguua augaaaugu                                                    19


<210>  587
<211>  19
<212>  RNA
<213>  Artificial Sequence

<220>
<223>  Description of Artificial Sequence:  siNA antisense region

<400>  587
aggucaggug acccacuuu                                                    19


<210>  588
<211>  19
<212>  RNA
<213>  Artificial Sequence

<220>
<223>  Description of Artificial Sequence:  siNA antisense region

<400>  588
gaguacucag cucuucaga                                                    19


<210>  589
<211>  19
<212>  RNA
<213>  Artificial Sequence

<220>
<223>  Description of Artificial Sequence:  siNA antisense region

<400>  589
gggugauugg aguggccug                                                    19


<210>  590
<211>  19
<212>  RNA
<213>  Artificial Sequence

<220>
<223>  Description of Artificial Sequence:  siNA antisense region

<400>  590
ccuccuuggc aucuuguag                                                    19


<210>  591
<211>  19
<212>  RNA
<213>  Artificial Sequence

<220>
<223>  Description of Artificial Sequence:  siNA antisense region

<400>  591
gagcuggacu uccugggac                                                    19


<210>  592
<211>  19
<212>  RNA
<213>  Artificial Sequence

<220>
<223>  Description of Artificial Sequence:  siNA antisense region

<400>  592
gacuagcguc aguuuaagg                                                    19


<210>  593
<211>  19
<212>  RNA
<213>  Artificial Sequence

<220>
<223>  Description of Artificial Sequence:  siNA antisense region

<400>  593
cacuugccca gguuuauug                                                    19


<210>  594
<211>  19
<212>  RNA
<213>  Artificial Sequence

<220>
<223>  Description of Artificial Sequence:  siNA antisense region

<400>  594
uccucauuuc ucuugccuc                                                    19

<210>  595
<211>  19
<212>  RNA
<213>  Artificial Sequence

<220>
<223>  Description of Artificial Sequence:  siNA antisense region

<400>  595
accucacaga uggauucuu                                                    19


<210>  596
<211>  19
<212>  RNA
<213>  Artificial Sequence

<220>
<223>  Description of Artificial Sequence:  siNA antisense region

<400>  596
cuuucauccu ugccuguca                                                    19


<210>  597
<211>  19
<212>  RNA
<213>  Artificial Sequence

<220>
<223>  Description of Artificial Sequence:  siNA antisense region

<400>  597
acucuuuucc uucuuuguc                                                    19


<210>  598
<211>  19
<212>  RNA
<213>  Artificial Sequence

<220>
<223>  Description of Artificial Sequence:  siNA antisense region

<400>  598
cuccuuucug ccuuugaua                                                    19


<210>  599
<211>  19
<212>  RNA
<213>  Artificial Sequence

<220>
<223>  Description of Artificial Sequence:  siNA antisense region

<400>  599
cagacccaac uaaaugauc                                                    19


<210>  600
<211>  19
<212>  RNA

<213>  Artificial Sequence

<220>
<223>  Description of Artificial Sequence:  siNA antisense region

<400>  600
agcaaagacu uuuccuuuc                                                    19


<210>  601
<211>  19
<212>  RNA
<213>  Artificial Sequence

<220>
<223>  Description of Artificial Sequence:  siNA antisense region

<400>  601
uagcaguaca ugucggaua                                                    19


<210>  602
<211>  19
<212>  RNA
<213>  Artificial Sequence

<220>
<223>  Description of Artificial Sequence:  siNA antisense region

<400>  602
uaaaaugcuu acagguacu                                                    19


<210>  603
<211>  19
<212>  RNA
<213>  Artificial Sequence

<220>
<223>  Description of Artificial Sequence:  siNA antisense region

<400>  603
uuuuuccauu cugggaccu                                                    19


<210>  604
<211>  19
<212>  RNA
<213>  Artificial Sequence

<220>
<223>  Description of Artificial Sequence:  siNA antisense region

<400>  604
uuaccaauag cugauuuuu                                                    19


<210>  605
<211>  19
<212>  RNA
<213>  Artificial Sequence

<220>
<223>  Description of Artificial Sequence:  siNA antisense region

<400> 605
ggaaaggaca uuauuauau 19

<210> 606
<211> 19
<212> RNA
<213> Artificial Sequence

<220>
<223> Description of Artificial Sequence: siNA antisense region

<400> 606
uaaaaaaacu gacuccagg 19

<210> 607
<211> 19
<212> RNA
<213> Artificial Sequence

<220>
<223> Description of Artificial Sequence: siNA antisense region

<400> 607
aacuaagagu uaacuuuuu 19

<210> 608
<211> 19
<212> RNA
<213> Artificial Sequence

<220>
<223> Description of Artificial Sequence: siNA antisense region

<400> 608
uagaauuaaa caaguaaaa 19

<210> 609
<211> 19
<212> RNA
<213> Artificial Sequence

<220>
<223> Description of Artificial Sequence: siNA antisense region

<400> 609
cucagcuccc uucucuuuu 19

<210> 610
<211> 19
<212> RNA
<213> Artificial Sequence

<220>
<223> Description of Artificial Sequence: siNA antisense region

<400> 610
acuccuacag ggaauggcc 19

<210> 611
<211> 19
<212> RNA
<213> Artificial Sequence

<220>
<223> Description of Artificial Sequence: siNA antisense region

<400> 611
uccuauccuu uuaucuuua 19


<210> 612
<211> 19
<212> RNA
<213> Artificial Sequence

<220>
<223> Description of Artificial Sequence: siNA antisense region

<400> 612
uuagagcuuu gaaucuuuu 19


<210> 613
<211> 19
<212> RNA
<213> Artificial Sequence

<220>
<223> Description of Artificial Sequence: siNA antisense region

<400> 613
gggaaagcug ugacucuau 19


<210> 614
<211> 19
<212> RNA
<213> Artificial Sequence

<220>
<223> Description of Artificial Sequence: siNA antisense region

<400> 614
auuuuagguu uuauaccug 19


<210> 615
<211> 19
<212> RNA
<213> Artificial Sequence

<220>
<223> Description of Artificial Sequence: siNA antisense region

<400> 615
ugcuuauugu acuucuuaa 19


<210> 616
<211> 19
<212> RNA

<213>  Artificial Sequence

<220>
<223>  Description of Artificial Sequence:  siNA antisense region

<400>  616
uagaucauuu uccaccucu                                                    19


<210>  617
<211>  19
<212>  RNA
<213>  Artificial Sequence

<220>
<223>  Description of Artificial Sequence:  siNA antisense region

<400>  617
uggguagcua ucaggaacu                                                    19


<210>  618
<211>  19
<212>  RNA
<213>  Artificial Sequence

<220>
<223>  Description of Artificial Sequence:  siNA antisense region

<400>  618
uauaaaucac uugcucugu                                                    19


<210>  619
<211>  19
<212>  RNA
<213>  Artificial Sequence

<220>
<223>  Description of Artificial Sequence:  siNA antisense region

<400>  619
uaguuuggau uucaaauuu                                                    19


<210>  620
<211>  19
<212>  RNA
<213>  Artificial Sequence

<220>
<223>  Description of Artificial Sequence:  siNA antisense region

<400>  620
aaagugauau uaagaaagu                                                    19


<210>  621
<211>  19
<212>  RNA
<213>  Artificial Sequence

<220>
<223>  Description of Artificial Sequence:  siNA antisense region

<400>  621
cugggaaaaa uggagacca                                                    19


<210>  622
<211>  19
<212>  RNA
<213>  Artificial Sequence

<220>
<223>  Description of Artificial Sequence:  siNA antisense region

<400>  622
ggggacauau uuccugucc                                                    19


<210>  623
<211>  19
<212>  RNA
<213>  Artificial Sequence

<220>
<223>  Description of Artificial Sequence:  siNA antisense region

<400>  623
gaagcaagaa aguuagggg                                                    19


<210>  624
<211>  19
<212>  RNA
<213>  Artificial Sequence

<220>
<223>  Description of Artificial Sequence:  siNA antisense region

<400>  624
ugcuggauuu uaauuuuug                                                    19


<210>  625
<211>  19
<212>  RNA
<213>  Artificial Sequence

<220>
<223>  Description of Artificial Sequence:  siNA antisense region

<400>  625
uguagaauga ucuugggau                                                    19


<210>  626
<211>  19
<212>  RNA
<213>  Artificial Sequence

<220>
<223>  Description of Artificial Sequence:  siNA antisense region

<400>  626
ugucugugca aaauuacuu                                                    19

<210>  627
<211>  19
<212>  RNA
<213>  Artificial Sequence

<220>
<223>  Description of Artificial Sequence:  siNA antisense region

<400>  627
ggcacugggg ugaggagau                                                    19


<210>  628
<211>  19
<212>  RNA
<213>  Artificial Sequence

<220>
<223>  Description of Artificial Sequence:  siNA antisense region

<400>  628
uugggugagc uccagacag                                                    19


<210>  629
<211>  19
<212>  RNA
<213>  Artificial Sequence

<220>
<223>  Description of Artificial Sequence:  siNA antisense region

<400>  629
ccaaguuguu uggugaccu                                                    19


<210>  630
<211>  19
<212>  RNA
<213>  Artificial Sequence

<220>
<223>  Description of Artificial Sequence:  siNA antisense region

<400>  630
aaggcaguug guucacaac                                                    19


<210>  631
<211>  19
<212>  RNA
<213>  Artificial Sequence

<220>
<223>  Description of Artificial Sequence:  siNA antisense region

<400>  631
ccccuccccc agaagguua                                                    19


<210>  632
<211>  19
<212>  RNA

<213>  Artificial Sequence

<220>
<223>  Description of Artificial Sequence:  siNA antisense region

<400>  632
cuccuagucu agcuaaucc                                                    19


<210>  633
<211>  19
<212>  RNA
<213>  Artificial Sequence

<220>
<223>  Description of Artificial Sequence:  siNA antisense region

<400>  633
uucccauuca cuucugguc                                                    19


<210>  634
<211>  19
<212>  RNA
<213>  Artificial Sequence

<220>
<223>  Description of Artificial Sequence:  siNA antisense region

<400>  634
uugugaaguc cucacccuu                                                    19


<210>  635
<211>  19
<212>  RNA
<213>  Artificial Sequence

<220>
<223>  Description of Artificial Sequence:  siNA antisense region

<400>  635
agcucugaca ggccaacau                                                    19


<210>  636
<211>  19
<212>  RNA
<213>  Artificial Sequence

<220>
<223>  Description of Artificial Sequence:  siNA antisense region

<400>  636
ugucuuggcu ucuaaucaa                                                    19


<210>  637
<211>  19
<212>  RNA
<213>  Artificial Sequence

<220>
<223>  Description of Artificial Sequence:  siNA antisense region

<400>  637
gucuuccuuu gcugccacu                                                    19

<210>  638
<211>  19
<212>  RNA
<213>  Artificial Sequence

<220>
<223>  Description of Artificial Sequence:  siNA antisense region

<400>  638
agguuuuucc ugggccaag                                                    19

<210>  639
<211>  19
<212>  RNA
<213>  Artificial Sequence

<220>
<223>  Description of Artificial Sequence:  siNA antisense region

<400>  639
gaaauuagca caacccaca                                                    19

<210>  640
<211>  19
<212>  RNA
<213>  Artificial Sequence

<220>
<223>  Description of Artificial Sequence:  siNA antisense region

<400>  640
cacccuauuu ucuggacag                                                    19

<210>  641
<211>  19
<212>  RNA
<213>  Artificial Sequence

<220>
<223>  Description of Artificial Sequence:  siNA antisense region

<400>  641
accccacaag cuucugucc                                                    19

<210>  642
<211>  19
<212>  RNA
<213>  Artificial Sequence

<220>
<223>  Description of Artificial Sequence:  siNA antisense region

<400>  642
gucccaauuc cuccaugca                                                    19

<210> 643
<211> 19
<212> RNA
<213> Artificial Sequence

<220>
<223> Description of Artificial Sequence: siNA antisense region

<400> 643
gaauaacaac auaaccagg 19

<210> 644
<211> 19
<212> RNA
<213> Artificial Sequence

<220>
<223> Description of Artificial Sequence: siNA antisense region

<400> 644
ccaaaauuca caguccgag 19

<210> 645
<211> 19
<212> RNA
<213> Artificial Sequence

<220>
<223> Description of Artificial Sequence: siNA antisense region

<400> 645
auauucuguu uuacaucac 19

<210> 646
<211> 19
<212> RNA
<213> Artificial Sequence

<220>
<223> Description of Artificial Sequence: siNA antisense region

<400> 646
agacauuagg uuuacagaa 19

<210> 647
<211> 19
<212> RNA
<213> Artificial Sequence

<220>
<223> Description of Artificial Sequence: siNA antisense region

<400> 647
aacgcucauu auuuauaca 19

<210> 648
<211> 19
<212> RNA

<213>  Artificial Sequence

<220>
<223>  Description of Artificial Sequence:  siNA antisense region

<400>  648
ugaauauuuu acuguguua                                                    19


<210>  649
<211>  19
<212>  RNA
<213>  Artificial Sequence

<220>
<223>  Description of Artificial Sequence:  siNA antisense region

<400>  649
uuuuuuuuga cuucuuauu                                                    19


<210>  650
<211>  19
<212>  RNA
<213>  Artificial Sequence

<220>
<223>  Description of Artificial Sequence:  siNA antisense region

<400>  650
uuuuuuuuuu uuuugacuu                                                    19


<210>  651
<211>  23
<212>  RNA
<213>  Artificial Sequence

<220>
<223>  Description of Artificial Sequence:  Target Sequence/siNA sense
       region

<400>  651
aaugggugag guuaccaacc agu                                               23


<210>  652
<211>  23
<212>  RNA
<213>  Artificial Sequence

<220>
<223>  Description of Artificial Sequence:  Target Sequence/siNA sense
       region

<400>  652
ucaccuugga cauggaagac ugu                                               23


<210>  653
<211>  23
<212>  RNA
<213>  Artificial Sequence

<220>
<223>  Description of Artificial Sequence:  Target Sequence/siNA sense
      region

<400>  653
ccuaacauug gugcaaagau ugc                                              23


<210>  654
<211>  23
<212>  RNA
<213>  Artificial Sequence

<220>
<223>  Description of Artificial Sequence:  Target Sequence/siNA sense
      region

<400>  654
uaugggaccu gcuaagugug gaa                                              23


<210>  655
<211>  21
<212>  DNA
<213>  Artificial

<220>
<223>  Description of Artificial Sequence:  siNA sense region

<400>  655
ugggugaggu uaccaaccat t                                                21


<210>  656
<211>  21
<212>  DNA
<213>  Artificial

<220>
<223>  Description of Artificial Sequence:  siNA sense region

<400>  656
accuuggaca uggaagacut t                                                21


<210>  657
<211>  21
<212>  DNA
<213>  Artificial

<220>
<223>  Description of Artificial Sequence:  siNA sense region

<400>  657
uaacauuggu gcaaagauut t                                                21


<210>  658
<211>  21
<212>  DNA
<213>  Artificial

<220>
<223>  Description of Artificial Sequence:  siNA sense region

<400>  658
ugggaccugc uaaguguggt t                                                21

<210>  659
<211>  21
<212>  DNA
<213>  Artificial

<220>
<223>  Description of Artificial Sequence:  siNA antisense region

<400>  659
ugguugguaa ccucacccat t                                                21

<210>  660
<211>  21
<212>  DNA
<213>  Artificial

<220>
<223>  Description of Artificial Sequence:  siNA antisense region

<400>  660
agucuuccau guccaaggut t                                                21

<210>  661
<211>  21
<212>  DNA
<213>  Artificial

<220>
<223>  Description of Artificial Sequence:  siNA antisense region

<400>  661
aaucuuugca ccaauguuat t                                                21

<210>  662
<211>  21
<212>  DNA
<213>  Artificial

<220>
<223>  Description of Artificial Sequence:  siNA antisense region

<400>  662
ccacacuuag caggucccat t                                                21

<210>  663
<211>  21
<212>  DNA
<213>  Artificial

<220>
<223>  Description of Artificial Sequence:  siNA sense region

<220>
<221>  misc_feature

<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (5)..(5)
<223>  2'-deoxy-2'-fluoro

<220>
<221>  misc_feature
<222>  (10)..(11)
<223>  2'-deoxy-2'-fluoro

<220>
<221>  misc_feature
<222>  (13)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (21)..(21)
<223>  3'-3 attached terminal deoxyabasic moeity

<400>  663
ugggugaggu uaccaaccat t                                                21


<210>  664
<211>  21
<212>  DNA
<213>  Artificial

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(5)
<223>  2'-deoxy-2'-fluoro

<220>
<221>  misc_feature
<222>  (9)..(9)
<223>  2'-deoxy-2'-fluoro

<220>
<221>  misc_feature
<222>  (11)..(11)

<223>  2'-deoxy-2'-fluoro

<220>
<221>  misc_feature
<222>  (18)..(19)
<223>  2'-deoxy-2'-fluoro

<220>
<221>  misc_feature
<222>  (21)..(21)
<223>  3'-3 attached terminal deoxyabasic moeity

<400>  664
accuuggaca uggaagacut t                                                21


<210>  665
<211>  21
<212>  DNA
<213>  Artificial

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (6)..(7)
<223>  2'-deoxy-2'-fluoro

<220>
<221>  misc_feature
<222>  (10)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (18)..(19)
<223>  2'-deoxy-2'-fluoro

<220>
<221>  misc_feature
<222>  (21)..(21)
<223>  3'-3 attached terminal deoxyabasic moeity

<400>  665
uaacauuggu gcaaagauut t                                                21

<210>  666
<211>  21
<212>  DNA
<213>  Artificial

<220>
<223>  Description of Artificial Sequence:  siNA sense region

<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (6)..(8)
<223>  2'-deoxy-2'-fluoro

<220>
<221>  misc_feature
<222>  (10)..(11)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (21)..(21)
<223>  3'-3 attached terminal deoxyabasic moeity

<400>  666
ugggaccugc uaaguguggt t                                                21

<210>  667
<211>  21
<212>  DNA
<213>  Artificial

<220>
<223>  Description of Artificial Sequence:  siNA antisense region

<220>
<221>  misc_feature

<222>  (1)..(1)
<223>  2'-deoxy-2'-fluoro

<220>
<221>  misc_feature
<222>  (4)..(5)
<223>  2'-deoxy-2'-fluoro

<220>
<221>  misc_feature
<222>  (8)..(8)
<223>  2'-deoxy-2'-fluoro

<220>
<221>  misc_feature
<222>  (11)..(14)
<223>  2'-deoxy-2'-fluoro

<220>
<221>  misc_feature
<222>  (16)..(18)
<223>  2'-deoxy-2'-fluoro

<220>
<221>  misc_feature
<222>  (20)..(20)
<223>  Phosphorothioate 3'-Internucleotide Linkage

<400>  667
ugguugguaa ccucacccat t                                                21


<210>  668
<211>  21
<212>  DNA
<213>  Artificial

<220>
<223>  Description of Artificial Sequence:  siNA antisense region


<220>
<221>  misc_feature
<222>  (3)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (12)..(14)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(20)

<223>  Phosphorothioate 3'-Internucleotide Linkage

<400>  668
agucuuccau guccaaggut t                                                21


<210>  669
<211>  21
<212>  DNA
<213>  Artificial

<220>
<223>  Description of Artificial Sequence:  siNA antisense region


<220>
<221>  misc_feature
<222>  (3)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (11)..(12)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(20)
<223>  Phosphorothioate 3'-Internucleotide Linkage

<400>  669
aaucuuugca ccaauguuat t                                                21


<210>  670
<211>  21
<212>  DNA
<213>  Artificial

<220>
<223>  Description of Artificial Sequence:  siNA antisense region


<220>
<221>  misc_feature
<222>  (1)..(2)
<223>  2'-deoxy-2'-fluoro

<220>

<221>  misc_feature
<222>  (4)..(4)
<223>  2'-deoxy-2'-fluoro

<220>
<221>  misc_feature
<222>  (6)..(8)
<223>  2'-deoxy-2'-fluoro

<220>
<221>  misc_feature
<222>  (11)..(11)
<223>  2'-deoxy-2'-fluoro

<220>
<221>  misc_feature
<222>  (15)..(18)
<223>  2'-deoxy-2'-fluoro

<220>
<221>  misc_feature
<222>  (20)..(20)
<223>  Phosphorothioate 3'-Internucleotide Linkage

<400>  670
ccacacuuag caggucccat t                                                21


<210>  671
<211>  21
<212>  DNA
<213>  Artificial

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (2)..(4)
<223>  2'-deoxy

<220>
<221>  misc_feature
<222>  (5)..(5)
<223>  2'-deoxy-2'-fluoro

<220>
<221>  misc_feature
<222>  (6)..(9)
<223>  2'-deoxy

<220>
<221>  misc_feature

<222>  (10)..(11)
<223>  2'-deoxy-2'-fluoro

<220>
<221>  misc_feature
<222>  (12)..(12)
<223>  2'-deoxy

<220>
<221>  misc_feature
<222>  (13)..(14)
<223>  2'-deoxy-2'-fluoro

<220>
<221>  misc_feature
<222>  (15)..(16)
<223>  2'-deoxy

<220>
<221>  misc_feature
<222>  (17)..(18)
<223>  2'-deoxy-2'-fluoro

<220>
<221>  misc_feature
<222>  (19)..(19)
<223>  2'-deoxy

<220>
<221>  misc_feature
<222>  (21)..(21)
<223>  3'-3 attached terminal deoxyabasic moeity

<400>  671
ugggugaggu uaccaaccat t                                                21


<210>  672
<211>  21
<212>  DNA
<213>  Artificial

<220>
<223>  Description of Artificial Sequence:  siNA sense region


<220>
<221>  misc_feature
<222>  (1)..(1)
<223>  2'-deoxy

<220>
<221>  misc_feature
<222>  (1)..(1)
<223>  5'-3 attached terminal deoxyabasic moeity

<220>
<221>  misc_feature
<222>  (2)..(5)
<223>  2'-deoxy-2'-fluoro

<220>
<221>  misc_feature
<222>  (6)..(8)

<223>  2'-deoxy

<220>
<221>  misc_feature
<222>  (9)..(9)
<223>  2'-deoxy-2'-fluoro

<220>
<221>  misc_feature
<222>  (10)..(10)
<223>  2'-deoxy

<220>
<221>  misc_feature
<222>  (11)..(11)
<223>  2'-deoxy-2'-fluoro

<220>
<221>  misc_feature
<222>  (12)..(17)
<223>  2'-deoxy

<220>
<221>  misc_feature
<222>  (18)..(19)
<223>  2'-deoxy-2'-fluoro

<220>
<221>  misc_feature
<222>  (21)..(21)
<223>  3'-3 attached terminal deoxyabasic moeity

<400>  672
accuuggaca uggaagacut t                                                21


<210>  673
<211>  21
<212>  DNA
<213>  Artificial

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (2)..(3)
<223>  2'-deoxy

<220>
<221>  misc_feature
<222>  (4)..(4)
<223>  2'-deoxy-2'-fluoro

<220>
<221> misc_feature
<222> (5)..(5)
<223> 2'-deoxy

<220>
<221> misc_feature
<222> (6)..(7)
<223> 2'-deoxy-2'-fluoro

<220>
<221> misc_feature
<222> (8)..(9)
<223> 2'-deoxy

<220>
<221> misc_feature
<222> (10)..(10)
<223> 2'-deoxy-2'-fluoro

<220>
<221> misc_feature
<222> (11)..(11)
<223> 2'-deoxy

<220>
<221> misc_feature
<222> (12)..(12)
<223> 2'-deoxy-2'-fluoro

<220>
<221> misc_feature
<222> (13)..(17)
<223> 2'-deoxy

<220>
<221> misc_feature
<222> (18)..(19)
<223> 2'-deoxy-2'-fluoro

<220>
<221> misc_feature
<222> (21)..(21)
<223> 3'-3 attached terminal deoxyabasic moeity

<400> 673
uaacauuggu gcaaagauut t                                                21


<210> 674
<211> 21
<212> DNA
<213> Artificial

<220>
<223> Description of Artificial Sequence: siNA sense region


<220>
<221> misc_feature
<222> (1)..(1)
<223> 2'-deoxy-2'-fluoro

<220>
<221>  misc_feature
<222>  (1)..(1)
<223>  5'-3 attached terminal deoxyabasic moeity

<220>
<221>  misc_feature
<222>  (2)..(5)
<223>  2'-deoxy

<220>
<221>  misc_feature
<222>  (6)..(8)
<223>  2'-deoxy-2'-fluoro

<220>
<221>  misc_feature
<222>  (9)..(9)
<223>  2'-deoxy

<220>
<221>  misc_feature
<222>  (10)..(11)
<223>  2'-deoxy-2'-fluoro

<220>
<221>  misc_feature
<222>  (12)..(14)
<223>  2'-deoxy

<220>
<221>  misc_feature
<222>  (15)..(15)
<223>  2'-deoxy-2'-fluoro

<220>
<221>  misc_feature
<222>  (16)..(16)
<223>  2'-deoxy

<220>
<221>  misc_feature
<222>  (17)..(17)
<223>  2'-deoxy-2'-fluoro

<220>
<221>  misc_feature
<222>  (18)..(19)
<223>  2'-deoxy

<220>
<221>  misc_feature
<222>  (21)..(21)
<223>  3'-3 attached terminal deoxyabasic moeity

<400>  674
ugggaccugc uaaguguggt t                                                21


<210>  675
<211>  21
<212>  DNA
<213>  Artificial

<220>
<223>  Description of Artificial Sequence:  siNA antisense region

<220>
<221>  misc_feature
<222>  (1)..(1)
<223>  2'-deoxy-2'-fluoro

<220>
<221>  misc_feature
<222>  (2)..(3)
<223>  2'-deoxy

<220>
<221>  misc_feature
<222>  (4)..(5)
<223>  2'-deoxy-2'-fluoro

<220>
<221>  misc_feature
<222>  (6)..(7)
<223>  2'-deoxy

<220>
<221>  misc_feature
<222>  (8)..(8)
<223>  2'-deoxy-2'-fluoro

<220>
<221>  misc_feature
<222>  (9)..(10)
<223>  2'-deoxy

<220>
<221>  misc_feature
<222>  (11)..(14)
<223>  2'-deoxy-2'-fluoro

<220>
<221>  misc_feature
<222>  (15)..(15)
<223>  2'-deoxy

<220>
<221>  misc_feature
<222>  (16)..(18)
<223>  2'-deoxy-2'-fluoro

<220>
<221>  misc_feature
<222>  (19)..(19)
<223>  2'-deoxy

<220>
<221>  misc_feature
<222>  (20)..(20)
<223>  Phosphorothioate 3'-Internucleotide Linkage

<400>  675
ugguugguaa ccucacccat t                                                21


<210>  676
<211>  21

<212>  DNA
<213>  Artificial

<220>
<223>  Description of Artificial Sequence:  siNA antisense region


<220>
<221>  misc_feature
<222>  (1)..(2)
<223>  2'-deoxy

<220>
<221>  misc_feature
<222>  (3)..(8)
<223>  2'-deoxy-2'-fluoro

<220>
<221>  misc_feature
<222>  (9)..(9)
<223>  2'-deoxy

<220>
<221>  misc_feature
<222>  (10)..(10)
<223>  2'-deoxy-2'-fluoro

<220>
<221>  misc_feature
<222>  (11)..(11)
<223>  2'-deoxy

<220>
<221>  misc_feature
<222>  (12)..(14)
<223>  2'-deoxy-2'-fluoro

<220>
<221>  misc_feature
<222>  (15)..(18)
<223>  2'-deoxy

<220>
<221>  misc_feature
<222>  (19)..(19)
<223>  2'-deoxy-2'-fluoro

<220>
<221>  misc_feature
<222>  (20)..(20)
<223>  Phosphorothioate 3'-Internucleotide Linkage

<400>  676
agucuuccau guccaaggut t                                                21


<210>  677
<211>  21
<212>  DNA
<213>  Artificial

<220>
<223>  Description of Artificial Sequence:  siNA antisense region

<220>
<221> misc_feature
<222> (1)..(2)
<223> 2'-deoxy

<220>
<221> misc_feature
<222> (3)..(7)
<223> 2'-deoxy-2'-fluoro

<220>
<221> misc_feature
<222> (8)..(8)
<223> 2'-deoxy

<220>
<221> misc_feature
<222> (9)..(9)
<223> 2'-deoxy-2'-fluoro

<220>
<221> misc_feature
<222> (10)..(10)
<223> 2'-deoxy

<220>
<221> misc_feature
<222> (11)..(12)
<223> 2'-deoxy-2'-fluoro

<220>
<221> misc_feature
<222> (13)..(14)
<223> 2'-deoxy

<220>
<221> misc_feature
<222> (15)..(15)
<223> 2'-deoxy-2'-fluoro

<220>
<221> misc_feature
<222> (16)..(16)
<223> 2'-deoxy

<220>
<221> misc_feature
<222> (17)..(18)
<223> 2'-deoxy-2'-fluoro

<220>
<221> misc_feature
<222> (19)..(19)
<223> 2'-deoxy

<220>
<221> misc_feature
<222> (20)..(20)
<223> Phosphorothioate 3'-Internucleotide Linkage

<400> 677
aaucuuugca ccaauguuat t 21

<210>  678
<211>  21
<212>  DNA
<213>  Artificial

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (3)..(3)
<223>  2'-deoxy

<220>
<221>  misc_feature
<222>  (4)..(4)
<223>  2'-deoxy-2'-fluoro

<220>
<221>  misc_feature
<222>  (5)..(5)
<223>  2'-deoxy

<220>
<221>  misc_feature
<222>  (6)..(8)
<223>  2'-deoxy-2'-fluoro

<220>
<221>  misc_feature
<222>  (9)..(10)
<223>  2'-deoxy

<220>
<221>  misc_feature
<222>  (11)..(11)
<223>  2'-deoxy-2'-fluoro

<220>
<221>  misc_feature
<222>  (12)..(14)
<223>  2'-deoxy

<220>
<221>  misc_feature
<222>  (15)..(18)
<223>  2'-deoxy-2'-fluoro

<220>
<221>  misc_feature
<222>  (19)..(19)
<223>  2'-deoxy

<220>
<221>  misc_feature
<222>  (20)..(20)
<223>  Phosphorothioate 3'-Internucleotide Linkage

<400>  678
ccacacuuag caggucccat t                                                21


<210>  679
<211>  21
<212>  DNA
<213>  Artificial

<220>
<223>  Description of Artificial Sequence:  siNA sense region


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (8)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (13)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (19)..(19)
<223>  2'-deoxy-2'-fluoro

<220>
<221>  misc_feature
<222>  (21)..(21)
<223>  3'-3 attached terminal deoxyabasic moeity

<400>  679
uuagaaacgu gguuacaaut t                                                21


<210>  680
<211>  21
<212>  DNA
<213>  Artificial

<220>

<223>  Description of Artificial Sequence:  siNA antisense region

<220>
<221>  misc_feature
<222>  (2)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (8)..(9)
<223>  2'-deoxy-2'-fluoro

<220>
<221>  misc_feature
<222>  (11)..(11)
<223>  2'-deoxy-2'-fluoro

<220>
<221>  misc_feature
<222>  (13)..(17)
<223>  2'-deoxy-2'-fluoro

<220>
<221>  misc_feature
<222>  (20)..(20)
<223>  Phosphorothioate 3'-Internucleotide Linkage

<400>  680
auuguaacca cguuucuaat t                                                21

<210>  681
<211>  21
<212>  DNA
<213>  Artificial

<220>
<223>  Description of Artificial Sequence:  siNA sense region

<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(7)
<223>  2'-deoxy

<220>
<221>  misc_feature
<222>  (8)..(8)

<223> 2'-deoxy-2'-fluoro

<220>
<221> misc_feature
<222> (9)..(9)
<223> 2'-deoxy

<220>
<221> misc_feature
<222> (10)..(10)
<223> 2'-deoxy-2'-fluoro

<220>
<221> misc_feature
<222> (11)..(12)
<223> 2'-deoxy

<220>
<221> misc_feature
<222> (13)..(14)
<223> 2'-deoxy-2'-fluoro

<220>
<221> misc_feature
<222> (15)..(15)
<223> 2'-deoxy

<220>
<221> misc_feature
<222> (16)..(16)
<223> 2'-deoxy-2'-fluoro

<220>
<221> misc_feature
<222> (17)..(18)
<223> 2'-deoxy

<220>
<221> misc_feature
<222> (19)..(19)
<223> 2'-deoxy-2'-fluoro

<220>
<221> misc_feature
<222> (21)..(21)
<223> 3'-3 attached terminal deoxyabasic moeity

<400> 681
uuagaaacgu gguuacaaut t                                                21


<210> 682
<211> 21
<212> DNA
<213> Artificial

<220>
<223> Description of Artificial Sequence: siNA antisense region


<220>
<221> misc_feature
<222> (1)..(1)
<223> 2'-deoxy

<220>
<221> misc_feature
<222> (2)..(3)
<223> 2'-deoxy-2'-fluoro

<220>
<221> misc_feature
<222> (4)..(4)
<223> 2'-deoxy

<220>
<221> misc_feature
<222> (5)..(5)
<223> 2'-deoxy-2'-fluoro

<220>
<221> misc_feature
<222> (6)..(7)
<223> 2'-deoxy

<220>
<221> misc_feature
<222> (8)..(9)
<223> 2'-deoxy-2'-fluoro

<220>
<221> misc_feature
<222> (10)..(10)
<223> 2'-deoxy

<220>
<221> misc_feature
<222> (11)..(11)
<223> 2'-deoxy-2'-fluoro

<220>
<221> misc_feature
<222> (12)..(12)
<223> 2'-deoxy

<220>
<221> misc_feature
<222> (13)..(17)
<223> 2'-deoxy-2'-fluoro

<220>
<221> misc_feature
<222> (18)..(19)
<223> 2'-deoxy

<220>
<221> misc_feature
<222> (20)..(20)
<223> Phosphorothioate 3'-Internucleotide Linkage

<400> 682
auuguaacca cguuucuaat t                                                21


<210> 683
<211> 21
<212> DNA
<213> Artificial

<220>
<223>  Description of Artificial Sequence:  siNA sense region


<220>
<221>  misc_feature
<222>  (1)..(19)
<223>  may be any nucleotide wherein any perimidine nucleotide present
      is 2'-O-methyl or 2'-Fluoro

<220>
<221>  misc_feature
<222>  (1)..(4)
<223>  Phosphorothioate 3'-Internucleotide Linkage

<220>
<221>  misc_feature
<222>  (17)..(20)
<223>  Phosphorothioate 3'-Internucleotide Linkage

<220>
<221>  misc_feature
<222>  (20)..(21)
<223>  may be any nucleotide

<400>  683
aaaaaaaaaa aaaaaaaaaa a                                                21


<210>  684
<211>  21
<212>  DNA
<213>  Artificial

<220>
<223>  Description of Artificial Sequence:  siNA antisense region


<220>
<221>  misc_feature
<222>  (1)..(4)
<223>  Phosphorothioate 3'-Internucleotide Linkage

<220>
<221>  misc_feature
<222>  (1)..(19)
<223>  may be any nucleotide wherein any perimidine nucleotide present
      is 2' fluoro

<220>
<221>  misc_feature
<222>  (17)..(20)
<223>  Phosphorothioate 3'-Internucleotide Linkage

<220>
<221>  misc_feature
<222>  (20)..(21)
<223>  may be any nucleotide

<400>  684
aaaaaaaaaa aaaaaaaaaa a                                                21

<210>  685
<211>  21
<212>  DNA
<213>  Artificial

<220>
<223>  Description of Artificial Sequence:  siNA sense region


<220>
<221>  misc_feature
<222>  (1)..(19)
<223>  may be any nucleotide wherein any perimidine nucleotide present
       is 2'-O-methyl or 2'-Fluoro

<220>
<221>  misc_feature
<222>  (20)..(21)
<223>  may be any nucleotide

<400>  685
aaaaaaaaaa aaaaaaaaaa a                                                21


<210>  686
<211>  21
<212>  DNA
<213>  Artificial

<220>
<223>  Description of Artificial Sequence:  siNA antisense region


<220>
<221>  misc_feature
<222>  (1)..(19)
<223>  may be any nucleotide wherein any perimidine nucleotide present
       is 2' fluoro

<220>
<221>  misc_feature
<222>  (20)..(21)
<223>  may be any nucleotide

<220>
<221>  misc_feature
<222>  (21)..(21)
<223>  3'-3 attached terminal glyceryl moeity

<400>  686
aaaaaaaaaa aaaaaaaaaa a                                                21


<210>  687
<211>  21
<212>  DNA
<213>  Artificial

<220>
<223>  Description of Artificial Sequence:  siNA sense region


<220>
<221>  misc_feature

<222>  (1)..(19)
<223>  may be any nucleotide wherein any perimidine nucleotide present
       is 2'-O-methyl or 2'-Fluoro

<220>
<221>  misc_feature
<222>  (1)..(1)
<223>  5'-3 attached terminal deoxyabasic moeity

<220>
<221>  misc_feature
<222>  (20)..(21)
<223>  may be any nucleotide

<220>
<221>  misc_feature
<222>  (21)..(21)
<223>  3'-3 attached terminal deoxyabasic moeity

<400>  687
aaaaaaaaaa aaaaaaaaaa a                                                21


<210>  688
<211>  21
<212>  DNA
<213>  Artificial

<220>
<223>  Description of Artificial Sequence:  siNA antisense region


<220>
<221>  misc_feature
<222>  (1)..(19)
<223>  may be any nucleotide wherein any perimidine nucleotide present
       is 2' fluoro

<220>
<221>  misc_feature
<222>  (20)..(21)
<223>  may be any nucleotide

<220>
<221>  misc_feature
<222>  (20)..(20)
<223>  Phosphorothioate 3'-Internucleotide Linkage

<220>
<221>  misc_feature
<222>  (21)..(21)
<223>  3'-3 attached terminal glyceryl moeity

<400>  688
aaaaaaaaaa aaaaaaaaaa a                                                21


<210>  689
<211>  21
<212>  DNA
<213>  Artificial

<220>
<223>  Description of Artificial Sequence:  siNA sense region

<220>
<221> misc_feature
<222> (1)..(19)
<223> may be any nucleotide wherein any pyrimidine nucleotide present
      is 2'-Fluoro and any purine nucleotide present is 2'-Deoxy

<220>
<221> misc_feature
<222> (20)..(21)
<223> may be any nucleotide

<220>
<221> misc_feature
<222> (1)..(1)
<223> 5'-3 attached terminal deoxyabasic moeity

<220>
<221> misc_feature
<222> (21)..(21)
<223> 3'-3 attached terminal deoxyabasic moeity

<400> 689
aaaaaaaaaa aaaaaaaaaa a                                                21


<210> 690
<211> 21
<212> DNA
<213> Artificial

<220>
<223> Description of Artificial Sequence: siNA antisense region


<220>
<221> misc_feature
<222> (1)..(19)
<223> may be any nucleotide wherein any pyrimidine nucleotide present
      is 2'-Fluoro and any purine nucleotide present is 2'-O-methyl

<220>
<221> misc_feature
<222> (20)..(20)
<223> Phosphorothioate 3'-Internucleotide Linkage

<220>
<221> misc_feature
<222> (20)..(21)
<223> may be any nucleotide

<220>
<221> misc_feature
<222> (21)..(21)
<223> 3'-3 attached terminal glyceryl moeity

<400> 690
aaaaaaaaaa aaaaaaaaaa a                                                21


<210> 691
<211> 21
<212> DNA
<213> Artificial

<220>
<223>  Description of Artificial Sequence:  siNA sense region

<220>
<221>  misc_feature
<222>  (1)..(19)
<223>  may be any nucleotide wherein any pyrimidine nucleotide present
      is 2'-Fluoro

<220>
<221>  misc_feature
<222>  (1)..(1)
<223>  5'-3 attached terminal deoxyabasic moeity

<220>
<221>  misc_feature
<222>  (20)..(21)
<223>  may be any nucleotide

<220>
<221>  misc_feature
<222>  (21)..(21)
<223>  3'-3 attached terminal deoxyabasic moeity

<400>  691
aaaaaaaaaa aaaaaaaaaa a                                                21

<210>  692
<211>  21
<212>  DNA
<213>  Artificial

<220>
<223>  Description of Artificial Sequence:  siNA antisense region

<220>
<221>  misc_feature
<222>  (1)..(19)
<223>  may be any nucleotide wherein any pyrimidine nucleotide present
      is 2'-Fluoro and any purine nucleotide present is 2'-O-methyl

<220>
<221>  misc_feature
<222>  (20)..(21)
<223>  may be any nucleotide

<220>
<221>  misc_feature
<222>  (21)..(21)
<223>  3'-3 attached terminal glyceryl moeity

<400>  692
aaaaaaaaaa aaaaaaaaaa a                                                21

<210>  693
<211>  21
<212>  DNA
<213>  Artificial

<220>
<223>  Description of Artificial Sequence:  siNA antisense region


<220>
<221>  misc_feature
<222>  (1)..(19)
<223>  may be any nucleotide wherein any pyrimidine nucleotide present
      is 2'-Fluoro and any purine nucleotide present is 2'-Deoxy

<220>
<221>  misc_feature
<222>  (20)..(20)
<223>  Phosphorothioate 3'-Internucleotide Linkage

<220>
<221>  misc_feature
<222>  (20)..(21)
<223>  may be any nucleotide

<220>
<221>  misc_feature
<222>  (21)..(21)
<223>  3'-3 attached terminal glyceryl moeity

<400>  693
aaaaaaaaaa aaaaaaaaaa a                                                21


<210>  694
<211>  21
<212>  DNA
<213>  Artificial

<220>
<223>  Description of Artificial Sequence:  siNA sense region


<220>
<221>  misc_feature
<222>  (1)..(4)
<223>  Phosphorothioate 3'-Internucleotide Linkage

<220>
<221>  misc_feature
<222>  (1)..(1)
<223>  2'-O-Methyl or 2'-deoxy-2'-fluoro

<220>
<221>  misc_feature
<222>  (3)..(3)
<223>  2'-O-Methyl or 2'-deoxy-2'-fluoro

<220>
<221>  misc_feature
<222>  (6)..(7)
<223>  2'-O-Methyl or 2'-deoxy-2'-fluoro

<220>
<221>  misc_feature
<222>  (9)..(10)
<223>  2'-O-Methyl or 2'-deoxy-2'-fluoro

<220>

<221>  misc_feature
<222>  (12)..(14)
<223>  2'-O-Methyl or 2'-deoxy-2'-fluoro

<220>
<221>  misc_feature
<222>  (16)..(16)
<223>  2'-O-Methyl or 2'-deoxy-2'-fluoro

<220>
<221>  misc_feature
<222>  (17)..(20)
<223>  Phosphorothioate 3'-Internucleotide Linkage

<220>
<221>  misc_feature
<222>  (18)..(19)
<223>  2'-O-Methyl or 2'-deoxy-2'-fluoro

<220>
<221>  misc_feature
<222>  (20)..(21)
<223>  2'-deoxy

<400>  694
cauggcugcc aucugcgcct t                                                21


<210>  695
<211>  21
<212>  DNA
<213>  Artificial

<220>
<223>  Description of Artificial Sequence:  siNA antisense region


<220>
<221>  misc_feature
<222>  (1)..(4)
<223>  Phosphorothioate 3'-Internucleotide Linkage

<220>
<221>  misc_feature
<222>  (3)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (9)..(9)
<223>  2'-deoxy-2'-fluoro

<220>
<221>  misc_feature
<222>  (12)..(12)
<223>  2'-deoxy-2'-fluoro

<220>
<221>  misc_feature

<222>  (15)..(16)
<223>  2'-deoxy-2'-fluoro

<220>
<221>  misc_feature
<222>  (18)..(18)
<223>  2'-O-Methyl

<220>
<221>  misc_feature
<222>  (20)..(20)
<223>  Phosphorothioate 3'-Internucleotide Linkage

<220>
<221>  misc_feature
<222>  (20)..(21)
<223>  2' -deoxy

<220>
<221>  misc_feature
<222>  (21)..(21)
<223>  3'-3 attached terminal glyceryl moeity

<400>  695
ggcgcagaug gcagccaugt t                                                21


<210>  696
<211>  21
<212>  DNA
<213>  Artificial

<220>
<223>  Description of Artificial Sequence:  siNA sense region


<220>
<221>  misc_feature
<222>  (1)..(1)
<223>  2'-O-Methyl or 2'-deoxy-2'-fluoro

<220>
<221>  misc_feature
<222>  (3)..(3)
<223>  2'-O-Methyl or 2'-deoxy-2'-fluoro

<220>
<221>  misc_feature
<222>  (6)..(7)
<223>  2'-O-Methyl or 2'-deoxy-2'-fluoro

<220>
<221>  misc_feature
<222>  (9)..(10)
<223>  2'-O-Methyl or 2'-deoxy-2'-fluoro

<220>
<221>  misc_feature
<222>  (12)..(14)
<223>  2'-O-Methyl or 2'-deoxy-2'-fluoro

<220>
<221>  misc_feature
<222>  (16)..(16)

<223>  2'-O-Methyl or 2'-deoxy-2'-fluoro

<220>
<221>  misc_feature
<222>  (18)..(19)
<223>  2'-O-Methyl or 2'-deoxy-2'-fluoro

<220>
<221>  misc_feature
<222>  (20)..(21)
<223>  2'-deoxy

<400>  696
cauggcugcc aucugcgcct t                                                21


<210>  697
<211>  21
<212>  DNA
<213>  Artificial

<220>
<223>  Description of Artificial Sequence:  siNA antisense region


<220>
<221>  misc_feature
<222>  (3)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (9)..(9)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(16)
<223>  2'-deoxy-2'-fluoro

<220>
<221>  misc_feature
<222>  (18)..(18)
<223>  2'-O-Methyl or 2'-deoxy-2'-fluoro

<220>
<221>  misc_feature
<222>  (20)..(21)
<223>  2'-deoxy

<220>
<221>  misc_feature
<222>  (21)..(21)
<223>  3'-3 attached terminal glyceryl moeity

<400>  697
ggcgcagaug gcagccaugt t                                                21


<210>  698
<211>  21
<212>  DNA
<213>  Artificial

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (1)..(1)
<223>  2'-O-Methyl or 2'-deoxy-2'-fluoro

<220>
<221>  misc_feature
<222>  (3)..(3)
<223>  2'-O-Methyl or 2'-deoxy-2'-fluoro

<220>
<221>  misc_feature
<222>  (6)..(7)
<223>  2'-O-Methyl or 2'-deoxy-2'-fluoro

<220>
<221>  misc_feature
<222>  (9)..(10)
<223>  2'-O-Methyl or 2'-deoxy-2'-fluoro

<220>
<221>  misc_feature
<222>  (12)..(14)
<223>  2'-O-Methyl or 2'-deoxy-2'-fluoro

<220>
<221>  misc_feature
<222>  (16)..(16)
<223>  2'-O-Methyl or 2'-deoxy-2'-fluoro

<220>
<221>  misc_feature
<222>  (18)..(19)
<223>  2'-O-Methyl or 2'-deoxy-2'-fluoro

<220>
<221>  misc_feature
<222>  (20)..(21)
<223>  2' -deoxy

<220>
<221>  misc_feature
<222>  (21)..(21)
<223>  3'-3 attached terminal deoxyabasic moeity

<400>  698
cauggcugcc aucugcgcct t                                                21


<210>  699
<211>  21
<212>  DNA
<213>  Artificial

<220>
<223>  Description of Artificial Sequence:  siNA antisense region


<220>
<221>  misc_feature
<222>  (3)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (9)..(9)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(16)
<223>  2'-deoxy-2'-fluoro

<220>
<221>  misc_feature
<222>  (18)..(18)
<223>  2'-O-Methyl

<220>
<221>  misc_feature
<222>  (20)..(21)
<223>  2' -deoxy

<220>
<221>  misc_feature
<222>  (20)..(20)
<223>  Phosphorothioate 3'-Internucleotide Linkage

<220>
<221>  misc_feature
<222>  (21)..(21)
<223>  3'-3 attached terminal glyceryl moeity

<400>  699
ggcgcagaug gcagccaugt t                                                21


<210>  700
<211>  21

<212>  DNA
<213>  Artificial

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (1)..(1)
<223>  2'-deoxy-2'-fluoro

<220>
<221>  misc_feature
<222>  (2)..(2)
<223>  2'-deoxy

<220>
<221>  misc_feature
<222>  (3)..(3)
<223>  2'-deoxy-2'-fluoro

<220>
<221>  misc_feature
<222>  (4)..(5)
<223>  2'-deoxy

<220>
<221>  misc_feature
<222>  (6)..(7)
<223>  2'-deoxy-2'-fluoro

<220>
<221>  misc_feature
<222>  (8)..(8)
<223>  2'-deoxy

<220>
<221>  misc_feature
<222>  (9)..(10)
<223>  2'-deoxy-2'-fluoro

<220>
<221>  misc_feature
<222>  (11)..(11)
<223>  2'-deoxy

<220>
<221>  misc_feature
<222>  (12)..(14)
<223>  2'-deoxy-2'-fluoro

<220>
<221>  misc_feature
<222>  (15)..(15)
<223>  2'-deoxy

<220>
<221>  misc_feature

<222>  (16)..(16)
<223>  2'-deoxy-2'-fluoro

<220>
<221>  misc_feature
<222>  (17)..(17)
<223>  2'-deoxy

<220>
<221>  misc_feature
<222>  (18)..(19)
<223>  2'-deoxy-2'-fluoro

<220>
<221>  misc_feature
<222>  (20)..(21)
<223>  2'-deoxy

<220>
<221>  misc_feature
<222>  (21)..(21)
<223>  3'-3 attached terminal deoxyabasic moeity

<400>  700
cauggcugcc aucugcgcct t                                                21


<210>  701
<211>  21
<212>  DNA
<213>  Artificial

<220>
<223>  Description of Artificial Sequence:  siNA antisense region


<220>
<221>  misc_feature
<222>  (1)..(2)
<223>  2'-O-Methyl or 2'-deoxy-2'-fluoro

<220>
<221>  misc_feature
<222>  (3)..(3)
<223>  2'-deoxy-2'Fluoro

<220>
<221>  misc_feature
<222>  (4)..(4)
<223>  2'-O-Methyl or 2'-deoxy-2'-fluoro

<220>
<221>  misc_feature
<222>  (5)..(5)
<223>  2'-deoxy-2'Fluoro

<220>
<221>  misc_feature
<222>  (6)..(6)
<223>  2'-O-Methyl

<220>
<221>  misc_feature
<222>  (7)..(7)

<223>  2'-O-Methyl or 2'-deoxy-2'-fluoro

<220>
<221>  misc_feature
<222>  (8)..(8)
<223>  2'-O-Methyl

<220>
<221>  misc_feature
<222>  (9)..(9)
<223>  2'-deoxy-2'Fluoro

<220>
<221>  misc_feature
<222>  (10)..(11)
<223>  2'-O-Methyl or 2'-deoxy-2'-fluoro

<220>
<221>  misc_feature
<222>  (12)..(12)
<223>  2'-deoxy-2'Fluoro

<220>
<221>  misc_feature
<222>  (13)..(13)
<223>  2'-O-Methyl

<220>
<221>  misc_feature
<222>  (14)..(14)
<223>  2'-O-Methyl or 2'-deoxy-2'-fluoro

<220>
<221>  misc_feature
<222>  (15)..(16)
<223>  2'-deoxy-2'Fluoro

<220>
<221>  misc_feature
<222>  (17)..(17)
<223>  2'-O-Methyl

<220>
<221>  misc_feature
<222>  (18)..(19)
<223>  2'-O-Methyl or 2'-deoxy-2'-fluoro

<220>
<221>  misc_feature
<222>  (20)..(20)
<223>  Phosphorothioate or Phosphorodithioate 3'-Internucleotide Linkage

<220>
<221>  misc_feature
<222>  (20)..(21)
<223>  2'-deoxy

<220>
<221>  misc_feature
<222>  (21)..(21)
<223>  3'-3 attached terminal glyceryl moeity

<400>  701
ggcgcagaug gcagccaugt t                                                21

<210>  702
<211>  21
<212>  DNA
<213>  Artificial

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (1)..(1)
<223>  2'-deoxy-2'-fluoro

<220>
<221>  misc_feature
<222>  (3)..(3)
<223>  2'-deoxy-2'-fluoro

<220>
<221>  misc_feature
<222>  (6)..(7)
<223>  2'-deoxy-2'-fluoro

<220>
<221>  misc_feature
<222>  (9)..(10)
<223>  2'-deoxy-2'-fluoro

<220>
<221>  misc_feature
<222>  (12)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  2'-deoxy

<220>
<221>  misc_feature
<222>  (21)..(21)
<223>  3'-3 attached terminal deoxyabasic moeity

<400>  702
cauggcugcc aucugcgcct t                                                21

<210>  703
<211>  21
<212>  DNA
<213>  Artificial

<220>
<223>  Description of Artificial Sequence:  siNA antisense region


<220>
<221>  misc_feature
<222>  (1)..(2)
<223>  2'-O-Methyl or 2'-deoxy-2'-fluoro

<220>
<221>  misc_feature
<222>  (3)..(3)
<223>  2'-deoxy-2'Fluoro

<220>
<221>  misc_feature
<222>  (4)..(4)
<223>  2'-O-Methyl or 2'-deoxy-2'-fluoro

<220>
<221>  misc_feature
<222>  (5)..(5)
<223>  2'-deoxy-2'Fluoro

<220>
<221>  misc_feature
<222>  (6)..(6)
<223>  2'-O-Methyl

<220>
<221>  misc_feature
<222>  (7)..(7)
<223>  2'-O-Methyl or 2'-deoxy-2'-fluoro

<220>
<221>  misc_feature
<222>  (8)..(8)
<223>  2'-O-Methyl

<220>
<221>  misc_feature
<222>  (9)..(9)
<223>  2'-deoxy-2'Fluoro

<220>
<221>  misc_feature
<222>  (10)..(11)
<223>  2'-O-Methyl or 2'-deoxy-2'-fluoro

<220>
<221>  misc_feature
<222>  (12)..(12)
<223>  2'-deoxy-2'Fluoro

<220>
<221>  misc_feature
<222>  (13)..(13)
<223>  2'-O-Methyl

<220>
<221> misc_feature
<222> (14)..(14)
<223> 2'-O-Methyl or 2'-deoxy-2'-fluoro

<220>
<221> misc_feature
<222> (15)..(16)
<223> 2'-deoxy-2'Fluoro

<220>
<221> misc_feature
<222> (17)..(17)
<223> 2'-O-Methyl

<220>
<221> misc_feature
<222> (18)..(19)
<223> 2'-O-Methyl or 2'-deoxy-2'-fluoro

<220>
<221> misc_feature
<222> (20)..(21)
<223> 2' -deoxy

<220>
<221> misc_feature
<222> (21)..(21)
<223> 3'-3 attached terminal glyceryl moeity

<400> 703
ggcgcagaug gcagccaugt t                                                21


<210> 704
<211> 21
<212> DNA
<213> Artificial

<220>
<223> Description of Artificial Sequence: siNA antisense region


<220>
<221> misc_feature
<222> (1)..(2)
<223> 2'-deoxy

<220>
<221> misc_feature
<222> (3)..(4)
<223> 2'-deoxy-2'Fluoro

<220>
<221> misc_feature
<222> (5)..(7)
<223> 2'-deoxy

<220>
<221> misc_feature
<222> (8)..(8)
<223> 2'-deoxy-2'Fluoro

<220>
<221>  misc_feature
<222>  (9)..(9)
<223>  2'-deoxy

<220>
<221>  misc_feature
<222>  (10)..(10)
<223>  2'-deoxy-2'Fluoro

<220>
<221>  misc_feature
<222>  (11)..(11)
<223>  2'-deoxy

<220>
<221>  misc_feature
<222>  (12)..(14)
<223>  2'-deoxy-2'Fluoro

<220>
<221>  misc_feature
<222>  (15)..(21)
<223>  2'-deoxy

<220>
<221>  misc_feature
<222>  (20)..(20)
<223>  Phosphorothioate 3'-Internucleotide Linkage

<220>
<221>  misc_feature
<222>  (21)..(21)
<223>  3'-3 attached terminal glyceryl moeity

<400>  704
ggcgcagaug gcagccaugt t                                                21